(19) Europäisches Patentamt / European Patent Office / Office européen des brevets

(11) **EP 3 209 774 B1**

(12) **EUROPEAN PATENT SPECIFICATION**

(45) Date of publication and mention
of the grant of the patent:
**26.02.2020  Bulletin 2020/09**

(21) Application number: **15794483.6**

(22) Date of filing: **23.10.2015**

(51) Int Cl.:
*C12N 9/48* *(2006.01)*     *C12P 21/06* *(2006.01)*
*A23J 3/34* *(2006.01)*     *A23K 10/14* *(2016.01)*

(86) International application number:
**PCT/EP2015/074602**

(87) International publication number:
**WO 2016/062857 (28.04.2016 Gazette 2016/17)**

(54) **USE OF PROLINE TOLERANT TRIPEPTIDYL PEPTIDASES IN FEED ADDITIVE COMPOSITIONS**

VERWENDUNG VON PROLINTOLERANTEN TRIPEPTIDYLPEPTIDASEN IN
FUTTERZUSATZZUSAMMENSETZUNGEN

UTILISATION DE TRIPEPTIDYL PEPTIDASES TOLÉRANTES À LA PROLINE DANS DES
COMPOSITIONS D'ADDITIFS ALIMENTAIRES

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **24.10.2014   US 201462068282 P
24.10.2014   US 201462068264 P
24.10.2014   US 201462068243 P
17.12.2014   US 201462093301 P**

(43) Date of publication of application:
**30.08.2017   Bulletin 2017/35**

(73) Proprietor: **DuPont Nutrition Biosciences ApS
1411 Copenhagen K (DK)**

(72) Inventors:
• **HAANING, Svend
8464 Galten (DK)**
• **KRAGH, Karsten Matthias
8270 Hoejbjerg (DK)**
• **SHIPOVSKOV, Stepan
8250 Egå (DK)**
• **MIASNIKOV, Andrei
Union City
California 94587 (US)**
• **MA, Maria
Fremont
California 94539 (US)**
• **MILLAN, Luis Fernando Romero
Swindon SN251 TP (GB)**
• **BARNARD, Luke
Wiltshire SN1 7BJ (GB)**

• **YU, Shukun
212 40 Malmö (SE)**
• **MEINJOHANNS, Ernst
1801 Frederiksberg C (DK)**
• **BAK, Steffen Yde
8200 Aarhus N (DK)**
• **NURMINEN, Paivi
02570 Siuntio (FI)**
• **PUTAALA, Heli
02480 Kirkkonummi (FI)**

(74) Representative: **D Young & Co LLP
120 Holborn
London EC1N 2DY (GB)**

(56) References cited:
**WO-A2-02/068623          WO-A2-2011/077359
WO-A2-2013/102674      US-A- 5 821 104
US-B2- 7 972 808**

• **U. REICHARD ET AL: "Sedolisins, a New Class of
Secreted Proteases from Aspergillus fumigatus
with Endoprotease or Tripeptidyl-Peptidase
Activity at Acidic pHs", APPLIED AND
ENVIRONMENTAL MICROBIOLOGY, vol. 72, no.
3, 1 March 2006 (2006-03-01), pages 1739-1748,
XP055000173, ISSN: 0099-2240, DOI:
10.1128/AEM.72.3.1739-1748.2006 cited in the
application**

Remarks:

The file contains technical information submitted after the application was filed and not included in this specification

**Description**

**FIELD OF THE INVENTION**

The present invention relates to proline tolerant tripeptidyl peptidases for use in feed additive compositions and/or feed and/or feedstuffs and methods and/or uses encompassing the use of said proline tolerant tripeptidyl peptidases.

**BACKGROUND**

Proteases (synonymous with peptidases) are enzymes that are capable of cleaving peptide bonds between amino acids in substrate peptides, oligopeptides and/or proteins.

Proteases are grouped into 7 families based on their catalytic reaction mechanism and the amino acid residue involved in the active site for catalysis. The serine proteases, aspartic acid proteases, cysteine proteases and metalloprotease are the 4 major families, whilst the threonine proteases, glutamic acid proteases and ungrouped proteases make up the remaining 3 families.

The substrate specificity of a protease is usually defined in terms of preferential cleavage of bonds between particular amino acid residues in a substrate. Typically, amino acid positions in a substrate peptide are defined relative to the location of the scissile bond (i.e. the position at which a protease cleaves):

$$NH_2\text{-......P3-P2-P1*P1'-P2'-P3'......-COOH}$$

Illustrated using the hypothetical peptide above, the scissile bond is indicated by the asterisk (*) whilst amino acid residues are represented by the letter 'P', with the residues N-terminal to the scissile bond beginning at P1 and increasing in number when moving away from the scissile bond towards the N-terminus. Amino acid residues C-terminal to the scissile bond begin at P1' and increase in number moving towards the C-terminal residue.

Proteases can be also generally subdivided into two broad groups based on their substrate-specificity. The first group is that of the endoproteases, which are proteolytic peptidases capable of cleaving internal peptide bonds of a peptide or protein substrate and tending to act away from the N-terminus or C-terminus. Examples of endoproteases include trypsin, chymotrypsin and pepsin. In contrast, the second group of proteases is the exopeptidases which cleave peptide bonds between amino acid residues located towards the C or N-terminus of a protein or peptide substrate.

Certain enzymes of the exopeptidase group may have tripeptidyl peptidase activity. Such enzymes are therefore capable of cleaving 3 amino acid fragments (tripeptides) from the unsubstituted N-terminus of substrate peptides, oligopeptides and/or proteins. Tripeptidyl peptidases are known to cleave tripeptide sequences from the N-terminus of a substrate except bonds with proline at the P1 and/or P1' position. Alternatively tripeptidyl peptidases may be proline-specific and only capable of cleaving substrates having a proline residue N-terminal to the scissile bond (i.e. in the P1 position).

Both exopeptidases and endoproteases have many applications.

Increasing protein digestibility, and therefore reducing the cost of the diet and increasing the efficiency of nutrient utilization in poultry and swine diets, as well as aqua and ruminant diets, is a big commercial area. Current commercially available proteases that are used for feed are alkaline proteases that are active at a high pH (8), which means they are active lower down in the gastrointestinal tract (the pH in the early digestive tract is more acidic, and becomes closer to neutral in the later part of the small intestine and the large intestine and caecum). By being active later (i.e. lower down) in the gastrointestinal tract, they produce oligopeptides later in the gastrointestinal tract where they appear to increase populations of microbes that excel at utilising easily digestible protein, which could lead to enteric disease challenges and reduced nutrients for the animal.

Additionally, at later parts of the gastrointestinal tract, the mucosa is less well-protected than in the tough gizzard, proventriculus or stomach and so is more easily damaged causing inflammation, a phenomenon that has been associated with protease use in young birds.

A tripeptidyl aminopeptidase, a DNA construct encoding the tripeptidyl aminopeptidase, a method of producing tripeptidyl aminopeptidase and methods of reducing the tripeptidyl aminopeptidase production in cells in which tripeptidyl aminopeptidase production is undesirable are described in US5821104.

**SUMMARY OF THE INVENTION**

According to a first aspect of the invention there is provided a method of preparing a feed additive composition comprising:

(a) admixing at least one proline tolerant tripeptidyl peptidase predominantly having exopeptidase activity wherein said proline tolerant tripeptidyl peptidase is capable of cleaving tri-peptides from the N-terminus of peptides having

(i)

(A) Proline at P1; and
(B) An amino acid selected from alanine, arginine, asparagine, aspartic acid, cysteine, glutamine, glutamic acid, glycine, histidine, isoleucine, leucine, lysine, methionine, phenylalanine, serine, threonine, tryptophan, tyrosine, valine or synthetic amino acids at P1; and/or

(ii)

(a') Proline at P1'; and
(b') An amino acid selected from alanine, arginine, asparagine, aspartic acid, cysteine, glutamine, glutamic acid, glycine, histidine, isoleucine, leucine, lysine, methionine, phenylalanine, serine, threonine, tryptophan, tyrosine, valine or synthetic amino acids or amines at P1'; and

one or more ingredients selected from the group consisting of: a salt, polyol including sorbitol and glycerol, wheat or a wheat component, sodium acetate, sodium acetate trihydrate, potassium sorbate Talc, polyvinyl alcohol (PVA), benzoate, sorbiate, 1,3-propane diol, glucose, parabens, sodium chloride, citrate, metabisulfite, formate or a combination thereof; and

optionally packaging,
wherein the at least one proline tolerant tripeptidyl peptidase:

(a) comprises the amino acid sequence SEQ ID No. 29, SEQ ID No. 1, SEQ ID No. 2 or a functional fragment thereof that retains said peptidase activity;
(b) comprises an amino acid having at least 80% or at least 90% identity to SEQ ID No. 29, SEQ ID No. 1, SEQ ID No. 2 or a functional fragment thereof that retains said peptidase activity;
(c) is encoded by a nucleotide sequence comprising the sequence SEQ ID No. 56;
(d) is encoded by a nucleotide sequence comprising at least 80% or at least 90% sequence identity to SEQ ID No. 56;
(e) is encoded by a nucleotide sequence which hybridises to SEQ ID No. 56 under high stringency conditions; or
(f) is encoded by a nucleotide sequence which differs from SEQ ID No. 56 due to degeneracy of the genetic code.

In a second aspect there is provided a feed additive composition or a feed ingredient comprising at least one proline tolerant tripeptidyl peptidase predominantly having exopeptidase activity wherein said proline tolerant tripeptidyl peptidase is capable of cleaving tri-peptides from the N-terminus of peptides having:

(i)

(A) Proline at P1; and
(B) An amino acid selected from alanine, arginine, asparagine, aspartic acid, cysteine, glutamine, glutamic acid, glycine, histidine, isoleucine, leucine, lysine, methionine, phenylalanine, serine, threonine, tryptophan, tyrosine, valine or synthetic amino acids at P1; and/or

(ii)

(a') Proline at P1'; and
(b') An amino acid selected from alanine, arginine, asparagine, aspartic acid, cysteine, glutamine, glutamic acid, glycine, histidine, isoleucine, leucine, lysine, methionine, phenylalanine, serine, threonine, tryptophan, tyrosine, valine or synthetic amino acids at or amines P1'; and

one or more ingredients selected from the group consisting of: a salt, polyol including sorbitol and glycerol, wheat or a wheat component, sodium acetate, sodium acetate trihydrate, potassium sorbate Talc, polyvinyl alcohol (PVA), benzoate, sorbiate, 1,3-propane diol, glucose, parabens, sodium chloride, citrate, metabisulfite, formate or a combination thereof, wherein the at least one proline tolerant tripeptidyl peptidase:

(a) comprises the amino acid sequence SEQ ID No. 29, SEQ ID No. 1, SEQ ID No. 2 or a functional fragment thereof that retains said peptidase activity;
(b) comprises an amino acid having at least 80% or at least 90% identity to SEQ ID No. 29, SEQ ID No. 1, SEQ ID No. 2 or a functional fragment thereof that retains said peptidase activity;
(c) is encoded by a nucleotide sequence comprising the sequence SEQ ID No. 56;
(d) is encoded by a nucleotide sequence comprising at least 80% or at least 90% sequence identity to SEQ ID No. 56;
(e) is encoded by a nucleotide sequence which hybridises to SEQ ID No. 56 under high stringency conditions; or
(f) is encoded by a nucleotide sequence which differs from SEQ ID No. 56 due to degeneracy of the genetic code.

According to a third aspect of the invention there is provided a kit comprising at least one proline tolerant tripeptidyl peptidase predominantly having exopeptidase activity wherein said proline tolerant tripeptidyl peptidase is capable of cleaving tri-peptides from the N-terminus of peptides having:

(i)

(A) Proline at P1; and
(B) An amino acid selected from alanine, arginine, asparagine, aspartic acid, cysteine, glutamine, glutamic acid, glycine, histidine, isoleucine, leucine, lysine, methionine, phenylalanine, serine, threonine, tryptophan, tyrosine, valine or synthetic amino acids at P1; and/or

(ii)

(a') Proline at P1'; and
(b') An amino acid selected from alanine, arginine, asparagine, aspartic acid, cysteine, glutamine, glutamic acid, glycine, histidine, isoleucine, leucine, lysine, methionine, phenylalanine, serine, threonine, tryptophan, tyrosine, valine or synthetic amino acids or amines at P1'; and instructions for administering same to an animal,

wherein the at least one proline tolerant tripeptidyl peptidase:

(a) comprises the amino acid sequence SEQ ID No. 29, SEQ ID No. 1, SEQ ID No. 2 or a functional fragment thereof that retains said peptidase activity;
(b) comprises an amino acid having at least 80% or at least 90% identity to SEQ ID No. 29, SEQ ID No. 1, SEQ ID No. 2 or a functional fragment thereof that retains said peptidase activity;
(c) is encoded by a nucleotide sequence comprising the sequence SEQ ID No. 56;
(d) is encoded by a nucleotide sequence comprising at least 80% or at least 90% sequence identity to SEQ ID No. 56;
(e) is encoded by a nucleotide sequence which hybridises to SEQ ID No. 56 under high stringency conditions; or
(f) is encoded by a nucleotide sequence which differs from SEQ ID No. 56 due to degeneracy of the genetic code.

According to a fourth aspect of the invention there is provided a premix comprising a feed additive composition or feed ingredient of the invention and at least one mineral and/or at least one vitamin.
In a fifth aspect there is provided a method of preparing a feedstuff comprising contacting a feed component with a feed additive composition or feed ingredient of the invention or a premix of the invention or at least one proline tolerant tripeptidyl peptidase predominantly having exopeptidase activity wherein said proline tolerant tripeptidyl peptidase is capable of cleaving tri-peptides from the N-terminus of peptides having:

(i)

(A) Proline at P1; and
(B) An amino acid selected from alanine, arginine, asparagine, aspartic acid, cysteine, glutamine, glutamic acid, glycine, histidine, isoleucine, leucine, lysine, methionine, phenylalanine, serine, threonine, tryptophan, tyrosine, valine or synthetic amino acids at P1; and/or

(ii)

(a') Proline at P1'; and
(b') An amino acid selected from alanine, arginine, asparagine, aspartic acid, cysteine, glutamine, glutamic acid, glycine, histidine, isoleucine, leucine, lysine, methionine, phenylalanine, serine, threonine, tryptophan, tyrosine, valine or synthetic amino acids or amines at P1'

optionally in combination with at least one endoprotease, wherein the at least one proline tolerant tripeptidyl peptidase:

(a) comprises the amino acid sequence SEQ ID No. 29, SEQ ID No. 1, SEQ ID No. 2 or a functional fragment thereof that retains said peptidase activity;
(b) comprises an amino acid having at least 80% or at least 90% identity to SEQ ID No. 29, SEQ ID No. 1, SEQ ID No. 2 or a functional fragment thereof that retains said peptidase activity;
(c) is encoded by a nucleotide sequence comprising the sequence SEQ ID No. 56;
(d) is encoded by a nucleotide sequence comprising at least 80% or at least 90% sequence identity to SEQ ID No. 56;
(e) is encoded by a nucleotide sequence which hybridises to SEQ ID No. 56 under high stringency conditions; or
(f) is encoded by a nucleotide sequence which differs from SEQ ID No. 56 due to degeneracy of the genetic code.

In a sixth aspect there is provided a feedstuff comprising a feed additive composition or feed ingredient of the invention or a premix of the invention or comprising at least one proline tolerant tripeptidyl peptidase predominantly having exopeptidase activity wherein said proline tolerant tripeptidyl peptidase is capable of cleaving tri-peptides from the N-terminus of peptides having

(i)

(A) Proline at P1; and
(B) An amino acid selected from alanine, arginine, asparagine, aspartic acid, cysteine, glutamine, glutamic acid, glycine, histidine, isoleucine, leucine, lysine, methionine, phenylalanine, serine, threonine, tryptophan, tyrosine, valine or synthetic amino acids at P1; and/or

(ii)

(a') Proline at P1'; and
(b') An amino acid selected from alanine, arginine, asparagine, aspartic acid, cysteine, glutamine, glutamic acid, glycine, histidine, isoleucine, leucine, lysine, methionine, phenylalanine, serine, threonine, tryptophan, tyrosine, valine or synthetic amino acids or amines at P1' preferably wherein the endoprotease and/or proline tolerant tripeptidyl peptidase are inactive (or substantially inactive) in the in the feedstuff prior to feeding the feedstuff to an animal, wherein the at least one proline tolerant tripeptidyl peptidase:

(a) comprises the amino acid sequence SEQ ID No. 29, SEQ ID No. 1, SEQ ID No. 2 or a functional fragment thereof that retains said peptidase activity;
(b) comprises an amino acid having at least 80% or at least 90% identity to SEQ ID No. 29, SEQ ID No. 1, SEQ ID No. 2 or a functional fragment thereof that retains said peptidase activity;
(c) is encoded by a nucleotide sequence comprising the sequence SEQ ID No. 56;
(d) is encoded by a nucleotide sequence comprising at least 80% or at least 90% sequence identity to SEQ ID No. 56;
(e) is encoded by a nucleotide sequence which hybridises to SEQ ID No. 56 under high stringency conditions; or
(f) is encoded by a nucleotide sequence which differs from SEQ ID No. 56 due to degeneracy of the genetic code.

According to a seventh aspect there is provided a non-therapeutic method for improving a biophysical characteristic of an animal or for improving protein digestibility of an animal which method comprises administering to an animal a feed additive composition obtainable (e.g. obtained) by a method or use of the invention or a feed additive composition, feedstuff or premix of the invention or at least one proline tolerant tripeptidyl peptidase predominantly having exopeptidase activity wherein said proline tolerant tripeptidyl peptidase is capable of cleaving tri-peptides from the N-terminus of peptides having:

(i)

(A) Proline at P1; and

(B) An amino acid selected from alanine, arginine, asparagine, aspartic acid, cysteine, glutamine, glutamic acid, glycine, histidine, isoleucine, leucine, lysine, methionine, phenylalanine, serine, threonine, tryptophan, tyrosine, valine or synthetic amino acids at P1; and/or

(ii)

(a') Proline at P1'; and
(b') An amino acid selected from alanine, arginine, asparagine, aspartic acid, cysteine, glutamine, glutamic acid, glycine, histidine, isoleucine, leucine, lysine, methionine, phenylalanine, serine, threonine, tryptophan, tyrosine, valine or synthetic amino acids or amines at P1'; and

optionally at least one feed component and/or at least one mineral and/or at least one vitamin, preferably wherein the method comprises administering to an animal at least one endoprotease, wherein the at least one proline tolerant tripeptidyl peptidase:

(a) comprises the amino acid sequence SEQ ID No. 29, SEQ ID No. 1, SEQ ID No. 2 or a functional fragment thereof that retains said peptidase activity;
(b) comprises an amino acid having at least 80% or at least 90% identity to SEQ ID No. 29, SEQ ID No. 1, SEQ ID No. 2 or a functional fragment thereof that retains said peptidase activity;
(c) is encoded by a nucleotide sequence comprising the sequence SEQ ID No. 56;
(d) is encoded by a nucleotide sequence comprising at least 80% or at least 90% sequence identity to SEQ ID No. 56;
(e) is encoded by a nucleotide sequence which hybridises to SEQ ID No. 56 under high stringency conditions; or
(f) is encoded by a nucleotide sequence which differs from SEQ ID No. 56 due to degeneracy of the genetic code.

In an eighth aspect there is provided the use of a feed additive composition or feed ingredient of the invention or a feedstuff or premix of the invention or at least one proline tolerant tripeptidyl peptidase predominantly having exopeptidase activity wherein said proline tolerant tripeptidyl peptidase is capable of cleaving tri-peptides from the N-terminus of peptides having:

(i)

(A) Proline at P1; and
(B) An amino acid selected from alanine, arginine, asparagine, aspartic acid, cysteine, glutamine, glutamic acid, glycine, histidine, isoleucine, leucine, lysine, methionine, phenylalanine, serine, threonine, tryptophan, tyrosine, valine or synthetic amino acids at P1; and/or

(ii)

(a') Proline at P1'; and
(b') An amino acid selected from alanine, arginine, asparagine, aspartic acid, cysteine, glutamine, glutamic acid, glycine, histidine, isoleucine, leucine, lysine, methionine, phenylalanine, serine, threonine, tryptophan, tyrosine, valine or synthetic amino acids or amines at P1'

for improving protein digestibility in an animal or for non-therapeutically improving a biophysical characteristic of an animal, preferably wherein at least one endoprotease is used in combination, wherein the at least one proline tolerant tripeptidyl peptidase:

(a) comprises the amino acid sequence SEQ ID No. 29, SEQ ID No. 1, SEQ ID No. 2 or a functional fragment thereof that retains said peptidase activity;
(b) comprises an amino acid having at least 80% or at least 90% identity to SEQ ID No. 29, SEQ ID No. 1, SEQ ID No. 2 or a functional fragment thereof that retains said peptidase activity;
(c) is encoded by a nucleotide sequence comprising the sequence SEQ ID No. 56;
(d) is encoded by a nucleotide sequence comprising at least 80% or at least 90% sequence identity to SEQ ID No. 56;
(e) is encoded by a nucleotide sequence which hybridises to SEQ ID No. 56 under high stringency conditions; or
(f) is encoded by a nucleotide sequence which differs from SEQ ID No. 56 due to degeneracy of the genetic code.

## BRIEF DESCRIPTION OF THE DRAWINGS

Embodiments of the invention will now be described, by way of example only, with reference to accompanying drawings, in which:

**Figure 1** shows a plasmid map of the expression vector pTTT-pyrG-TRI083.

**Figure 2** shows a pH profile for a proline tolerant tripeptidyl peptidase.

**Figure 3** shows a graph displaying the activity of a proline tolerant tripeptidyl peptidase at various temperatures.

**Figure 4** shows enzyme activity when Alphalase® AFP (herein referred to as AFP) (an acid protease) is used in combination with a proline tolerant tripeptidyl peptidase at different dosages of each enzyme.

**Figure 5** shows the ability of a proline tolerant tripeptidyl peptidase to cleave the substrate AAPPA over time.

**Figure 6** shows the production of the cleavage product AAP from an AAPPA substrate over time.

**Figure 7** shows a plasmid map of the expression vector pTTT-pyrG13-TRI071. The endogenous signal sequence was replaced by the secretion signal sequence from the Trichoderma reesei acidic fungal protease (AFP) and an intron from a Trichoderma reesei glycoamylase gene (TrGA1) (see lower portion of Figure 7).

**Figure 8** shows dose response of Alphalase® AFP on protein hydrolysis of corn soy feed. Dashed line represents the control where only pepsin and pancreatin were used.

**Figure 9** shows the dependence of DH on enzyme composition in the feed sample.

**Figure 10** shows the effect of feed treatment by Alphalase® AFP and proline tolerant tripeptidyl peptidase at different conditions. Solid bars represent the treatment at 40°C for 100 min. Empty bars represent the treatment at 40°C for 200 min.

**Figure 11** shows the effect of commercial proteases compared to a proline tolerant tripeptidyl peptidase on ileal N digestibility %.

**Figure 12** shows the effect of commercial proteases compared to a proline tolerant tripeptidyl peptidase on ileal digestiblity of energy (MJ/kg).

**Figure 13** shows the ATP content of the intestinal epithelial cells treated with proteases mixed directly with cell cultivation medium for one hour. Full bars - treatment by Commercial Protease 1; Empty bars - treatment by Commercial Protease 2; Bars with diagonal stripes - treatment by TRI083 (TRI083 as used herein mean an enzyme having the mature peptide sequence shown herein as SEQ ID No. 29); Bars with horizontal stripes - treatment by combination of TRI083 and Alphalase® AFP (dosages indicated for each enzyme).

**Figure 14** shows the ATP content of the intestinal epithelial cells treated for one hour with *in vitro* digested feed with proteases. Full bars - treatment by Commercial Protease 1 (plus control of digestion); Empty bars - treatment by Commercial Protease 2; Bars with diagonal stripes - treatment by TRI083; Bars with horizontal stripes - treatment by combination of TRI083 and Alphalase® AFP (dosages indicated for each enzyme).

**Figure 15** shows FITC-Dextran permeability after four hours treatment. Full bars - treatment by Commercial Protease 1; Empty bars - treatment by Commercial Protease 2; Bars with diagonal stripes - treatment by TRI083; Bars with horizontal stripes - treatment by combination of TRI083 and Alphalase® AFP (dosages indicated for each enzyme).

**Figure 16** shows relative changes in TEER for different treatments calculated from the value before the application of the test substances and after 4 hours of application. Full bars - treatment by Commercial Protease 1; Empty bars - treatment by Commercial Protease 2; Bars with diagonal stripes - treatment by TRI083; Bars with horizontal stripes - treatment by combination of TRI083 and Alphalase® AFP (dosages indicated for each enzyme)

**Figure 17** shows alignments between a number of proline tolerant tripeptidyl peptidase amino acid sequences. The xEANLD, y'Tzx'G and QNFSV motifs are shown (boxed).

**Figure 18** shows improvements in ileal N digestibility (%) versus the NC with supplementation of commercial proteases A + B and tripeptidyl peptidase (TRI083).

**Figure 19** shows improvements in ileal digestible energy (MJ/kg) versus the NC with supplementation of commercial proteases A + B and tripeptidyl peptidase (TRI083).

**Figure 20** shows the effect of supplementation of commercial proteases A + B and tripeptidyl peptidase (TRI083) on BWG of broilers.

**Figure 21** shows the effect of supplementation of commercial proteases A + B and tripeptidyl peptidase (TRI083) on FCR of broilers.

**Figure 22** shows the effect of pH on TRI045 (a tripeptidyl peptidase having pre-pro sequence SEQ ID No. 98 and mature protein SEQ ID No. 99) activity using AAF-pNA as substrate (values are the average of one test with 0.8 μl TRI045 (n=2).

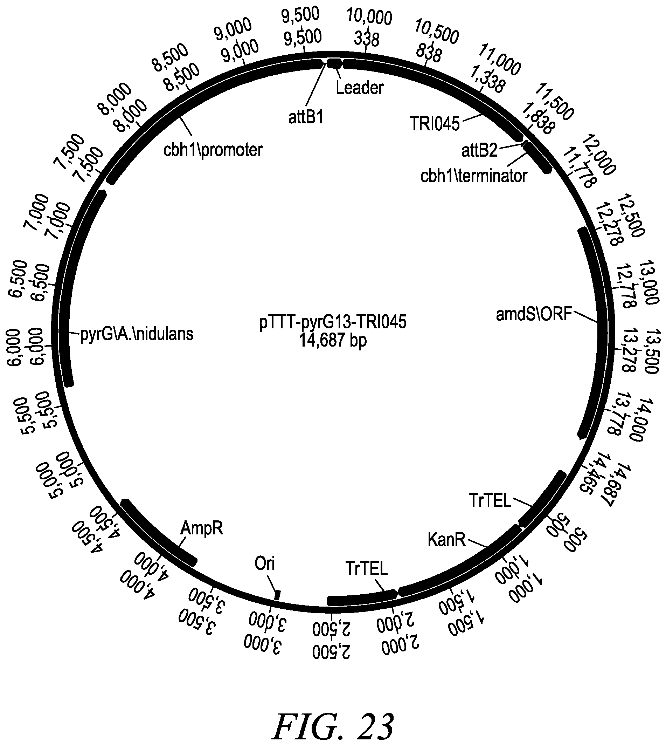

**Figure 23** shows a plasmid map of the expression vector pTTT-pyrG13-TRI045.

**Figure 24** shows a pH profile for the tripeptidyl peptidase TRI045.

## DETAILED DESCRIPTION

A seminal finding of the present invention is that a tripeptidyl peptidase can have exopeptidase activity on a substrate having proline at P1 and/or P1' as well as any other amino acid at P1 and/or P1'. This is highly surprising as tripeptidyl peptidases that have been documented in the art typically are inhibited when proline is at P1 or are active when proline is at P1 but inactive when an amino acid other than proline is present at position P1 in the substrate, this is sometimes referred to herein as a proline-specific tripeptidyl peptidase. The inventors have shown for the first time that a proline tolerant tripeptidyl peptidase according to the present invention is highly advantageous for use in feed and feedstuffs and confers advantages to an animal fed the proline tolerant tripeptidyl peptidase or a feed additive composition comprising the same.

Advantageously, a proline tolerant tripeptidyl peptidase taught for use in the present invention is capable of acting on a wide range of peptide and/or protein substrates and due to having such a broad substrate-specificity is not readily inhibited from cleaving substrates enriched in certain amino acids (e.g. proline). The use of such a proline tolerant tripeptidyl peptidase therefore may efficiently and/or rapidly breakdown protein substrates (e.g. present in feed and/or feedstuffs). This confers the further advantage of efficiently and/or rapidly digesting a protein substrate *in situ* in an animal fed with such a protein substrate (e.g. as present in a feed or feedstuff) allowing rapid and/or efficient uptake of digested peptides by the animal.

Based on these findings, there is provided a method of preparing a feed additive composition comprising: admixing at least one proline tolerant tripeptidyl peptidase predominantly having exopeptidase activity wherein said proline tolerant tripeptidyl peptidase is capable of cleaving tri-peptides from the N-terminus of peptides having (i) (A) Proline at P1; and (B) An amino acid selected from alanine, arginine, asparagine, aspartic acid, cysteine, glutamine, glutamic acid, glycine, histidine, isoleucine, leucine, lysine, methionine, phenylalanine, serine, threonine, tryptophan, tyrosine, valine or synthetic amino acids at P1; or (ii) (a') Proline at P1'; and (b') An amino acid selected from alanine, arginine, asparagine, aspartic acid, cysteine, glutamine, glutamic acid, glycine, histidine, isoleucine, leucine, lysine, methionine, phenylalanine, serine, threonine, tryptophan, tyrosine, valine or synthetic amino acids at P1'; and one or more ingredients selected from the group consisting of: a salt, polyol including sorbitol and glycerol, wheat or a wheat component, sodium acetate, sodium acetate trihydrate, potassium sorbate Talc, polyvinyl alcohol (PVA), benzoate, sorbiate, 1,3-propane diol, glucose, parabens, sodium chloride, citrate, metabisulfite, formate or a combination thereof; and optionally packaging, wherein the at least one proline tolerant tripeptidyl peptidase:

(a) comprises the amino acid sequence SEQ ID No. 29, SEQ ID No. 1, SEQ ID No. 2 or a functional fragment thereof that retains said peptidase activity;
(b) comprises an amino acid having at least 80% or at least 90% identity to SEQ ID No. 29, SEQ ID No. 1, SEQ ID No. 2 or a functional fragment thereof that retains said peptidase activity;
(c) is encoded by a nucleotide sequence comprising the sequence SEQ ID No. 56;
(d) is encoded by a nucleotide sequence comprising at least 80% or at least 90% sequence identity to SEQ ID No. 56;
(e) is encoded by a nucleotide sequence which hybridises to SEQ ID No. 56 under high stringency conditions; or
(f) is encoded by a nucleotide sequence which differs from SEQ ID No. 56 due to degeneracy of the genetic code.

Described herein is a feed additive composition obtainable (preferably obtained) by the method of the foregoing embodiment.

The term "admixing" as used herein refers to the mixing of one or more ingredients and/or enzymes where the one or more ingredients or enzymes are added in any order and in any combination. Suitably, admixing may relate to mixing one or more ingredients and/or enzymes simultaneously or sequentially.

In one embodiment the one or more ingredients and/or enzymes may be mixed sequentially. Preferably, the one or more ingredients and/or enzymes may be mixed simultaneously.

The proline tolerant tripeptidyl peptidase for use in the methods and/or uses or comprised in any of the products of the present invention is capable of cleaving tri-peptides from the N-terminus of peptides having proline at P1; and an amino acid selected from alanine, arginine, asparagine, aspartic acid, cysteine, glutamine, glutamic acid, glycine, histidine, isoleucine, leucine, lysine, methionine, phenylalanine, serine, threonine, tryptophan, tyrosine, valine or synthetic amino acids at P1.

Alternatively or additionally, the proline tolerant tripeptidyl peptidase for use in the methods and/or uses or comprised in any of the products of the present invention is capable of cleaving tri-peptides from the N-terminus of peptides having proline at P1'; and an amino acid selected from alanine, arginine, asparagine, aspartic acid, cysteine, glutamine, glutamic acid, glycine, histidine, isoleucine, leucine, lysine, methionine, phenylalanine, serine, threonine, tryptophan, tyrosine, valine or synthetic amino acids at P1'.

The term "proline tolerant tripeptidyl peptidase" as used herein relates to an exopeptidase which can cleave tripeptides from the N-terminus of a peptide, oligopeptide and/or protein substrate. A "proline tolerant tripeptidyl peptidase"

is capable of cleaving peptide bonds where proline is at position P1 as well as cleaving peptide bonds where an amino acid other than proline is at P1 and/or capable of cleaving peptide bonds where proline is at position P1' as well as cleaving peptide bonds where an amino acid other than proline is at P1'.

In one embodiment the proline tolerant tripeptidyl peptidase is not an endoprotease.

In another embodiment the proline tolerant tripeptidyl peptidase is not an enzyme which cleaves tetrapeptides from the N-terminus of a substrate.

In a further embodiment the proline tolerant tripeptidyl peptidase is not an enzyme which cleaves dipeptides from the N-terminus of a substrate.

In a yet further embodiment the proline tolerant tripeptidyl peptidase is not an enzyme which cleaves single amino acids from the N-terminus of a substrate.

In one embodiment the proline tolerant tripeptidyl peptidase may be capable of cleaving peptide bonds where proline is at position P1 as well as cleaving peptide bonds where an amino acid other than proline is at P1.

In another embodiment the proline tolerant tripeptidyl peptidase may be capable of cleaving peptide bonds where proline is at position P1' as well as cleaving peptide bonds where an amino acid other than proline is at P1'.

Suitably, the proline tolerant tripeptidyl peptidase may also be able to cleave peptide bonds where the proline present at position P1 and/or P1' is present in its *cis* or *trans* configuration. Suitably an "amino acid other than proline" may be an amino acid selected from alanine, arginine, asparagine, aspartic acid, cysteine, glutamine, glutamic acid, glycine, histidine, isoleucine, leucine, lysine, methionine, phenylalanine, serine, threonine, tryptophan, tyrosine, valine or synthetic amino acids.

In another embodiment the "amino acid other than proline" may be an amino acid selected from alanine, arginine, asparagine, aspartic acid, cysteine, glutamine, glutamic acid, glycine, histidine, isoleucine, leucine, lysine, methionine, phenylalanine, serine, threonine, tryptophan, tyrosine or valine.

Suitably, in such an embodiment synthetic amino acids may be excluded.

Preferably, the proline tolerant tripeptidyl peptidase may be able to cleave peptide bonds where proline is present at position P1 and P1'.

It is surprising that a tripeptidyl peptidase can act on a substrate having proline at position P1 and/or P1'. It is even more surprising that in addition to this activity a tripeptidyl peptidase may also have activity when an amino acid other than proline is present at position P1 and/or P1'.

In addition to having activity on any of the various substrates as described above the proline tolerant tripeptidyl peptidase for use in the present invention may additionally be tolerant of proline at one or more positions selected from the group consisting of: P2, P2', P3 and P3'. Suitably the proline tolerant tripeptidyl peptidase in addition to having the activities described above may be tolerant of proline at position P2, P2', P3 and P3'.

This is advantageous as it allows the efficient cleavage of peptide and/or protein substrates having stretches of proline and allows cleavage of a wide range of peptide and/or protein substrates.

The proline tolerant tripeptidyl peptidase may have a high activity on peptides and/or proteins having one or more of lysine, arginine or glycine in the P1 position.

The tripeptidyl peptide, e.g. proline tolerant tripeptidyl peptidase for use in the methods and/or uses of the present invention may be formulated in any appropriate manner known in the art.

In some embodiments further ingredients may be admixed with the tripeptidyl peptidase such as salts such as $Na_2SO_4$, maltodextrin, limestone (calcium carbonate), cyclodextrin, wheat or a wheat component, starch, Talc, polyvinyl alcohol (PVA), polyols such as sorbitol and glycerol, benzoate, sorbiate, sugars such as sucrose and glucose, propylene glycol, 1,3-propane diol, parabens, sodium chloride, citrate, acetate, sodium acetate, phosphate, calcium, metabisulfite, formate or mixtures thereof.

In a preferred embodiment the food additive composition or feed additive composition according to the present invention comprises the tripeptidyl peptidase according to the present invention or fermentate according to the present invention and further comprises one or more ingredients selected from the group consisting of: a salt, polyol including sorbitol and glycerol, wheat or a wheat component, sodium acetate, sodium acetate trihydrate, potassium sorbate Talc, polyvinyl alcohol (PVA), benzoate, sorbiate, 1,3-propane diol, glucose, parabens, sodium chloride, citrate, metabisulfite, formate or a combination thereof.

In one embodiment the salt may be selected from the group consisting of: $Na_2SO_4$, $NaH_2PO_4$, $Na_2HPO_4$, $Na_3PO_4$, $(NH_4)H_2PO_4$, $K_2HPO_4$, $KH_2PO_4$, $K_2SO_4$, $KHSO_4$, $ZnSO_4$, $MgSO_4$, $CuSO_4$, $Mg(NO_3)_2$, $(NH_4)_2SO_4$, sodium borate, magnesium acetate, sodium citrate or combinations thereof.

In some embodiment polyols may be admixed with the proline tolerant tripeptidyl peptidase. Polyols such as glycerol and/or sorbitol may be admixed in amounts from 5% (w/w) - 70% (w/w), preferably 10-20% (w/w), preferably 20-50% (w/w) and more preferably 10-50% (w/w) and more preferably 10- 30% (w/w) with % (w/w) meaning % (weight polyol / weight solution), without wishing to be bound by theory a lower concentration of 10% polyol might help increasing the solubility and storage stability of the enzyme. However many commercial enzymes require 30% glycerol to keep the enzyme stable over time in the concentration of interest. Higher polyol at 50% might still improve stability further, but at this polyol level also the benefit of lower water activity is an advantage for microbial preservation. In particular for food enzymes this can be very important at neutral pH, where the choice of good preservatives are limited.

Sugars (in particular glucose) may be admixed with the proline tolerant tripeptidyl peptidase. Sugars like glucose, fructose, sucrose, maltose, lactose, trehalose are all examples of substances that for many enzymes can be an alternative to using polyols. Suitably they (particularly glucose) may be used in the range 5% (w/w) - 50% (w/w) either alone or in combination with polyols.

Sodium acetate may be admixed in amounts from 5% (w/w) - 50% (w/w), preferably 8 - 40% preferably 8 - 12 % (w/w), preferably 10 - 50% and more preferably 10 - 30% (w/w) with % (w/w) meaning % (weight sodium acetate / weight solution).

In one embodiment the proline tolerant tripeptidyl peptidase may be admixed with a preservative.

Suitably the preservative may be benzoate, such as sodium benzoate, and/or potassium sorbate. These preservatives can be typically used in a combined concentration of about 0.1 - 1%, suitably about 0.2 - 0.5%. Sodium benzoate is most efficient at pH < 5.5 and sodium sorbate at pH < 6.

In one embodiment the one or more ingredients (e.g. used for the formulation of the enzyme when used in the methods and/or uses of the present invention) may be selected from the group consisting of: a wheat carrier, a polyol, a sugar, a salt and a preservative.

Suitably the sugar is sorbitol.

Suitably the salt is sodium sulphate.

In one embodiment the one or more ingredients (e.g. used for the formulation of the enzyme when used in the methods and/or uses of the present invention) may be selected from the group consisting of: a wheat carrier, a polyol, a sorbitol, sodium sulphate and a preservative. Suitably the one or more ingredients (e.g. used for the formulation of the feed additive composition and/or feed and/or feedstuff and/or premix) may be selected from the group consisting of: a wheat carrier, sorbitol and sodium sulphate.

Suitably, the proline tolerant tripeptidyl peptidase may be admixed with a wheat carrier. Suitably, the proline tolerant tripeptidyl peptidase may be admixed with sorbitol.

Suitably the proline tolerant tripeptidyl peptidase may be admixed with sodium sulphate.

In a preferred embodiment the proline tolerant tripeptidyl peptidase for use in the methods and/or uses of the present invention may be formulated with a carrier comprising (or consisting essentially of; or consisting of) a salt, polyol including sorbitol and glycerol, wheat or a wheat component, sodium acetate, sodium acetate trihydrate, potassium sorbate Talc, polyvinyl alcohol (PVA), benzoate, sorbiate, 1,3-propane diol, glucose, parabens, sodium chloride, citrate, metabisulfite, formate or a combination thereof.

In a preferred embodiment the proline tolerant tripeptidyl peptidase for use in the methods and/or uses of the present invention may be formulated with a carrier comprising (or consisting essentially of; or consisting of) $Na_2SO_4$, $NaH_2PO_4$, $Na_2HPO_4$, $Na_3PO_4$, $(NH_4)H_2PO_4$, $K_2HPO_4$, $KH_2PO_4$, $K_2SO_4$, $KHSO_4$, $ZnSO_4$, $MgSO_4$, $CuSO_4$, $Mg(NO_3)_2$, $(NH_4)_2SO_4$, sodium borate, magnesium acetate, sodium citrate or combinations thereof.

In one embodiment, the proline tolerant tripeptidyl peptidase for use in the methods and/or uses of the present invention may be formulated with $Na_2SO_4$.

The feed additive composition of the present invention suitably comprises the proline tolerant tripeptidyl peptidase formulated with a carrier comprising (or consisting essentially of; or consisting of) a salt, polyol including sorbitol and glycerol, wheat or a wheat component, sodium acetate, sodium acetate trihydrate, potassium sorbate Talc, polyvinyl alcohol (PVA), benzoate, sorbiate, 1,3-propane diol, glucose, parabens, sodium chloride, citrate, metabisulfite, formate or a combination thereof.

In one embodiment the feed additive composition of the present invention suitably comprises the proline tolerant tripeptidyl peptidase formulated with a carrier comprising (or consisting essentially of; or consisting of) $Na_2SO_4$, $NaH_2PO_4$, $Na_2HPO_4$, $Na_3PO_4$, $(NH_4)H_2PO_4$, $K_2HPO_4$, $KH_2PO_4$, $K_2SO_4$, $KHSO_4$, $ZnSO_4$, $MgSO_4$, $CuSO_4$, $Mg(NO_3)_2$, $(NH_4)_2SO_4$, sodium borate, magnesium acetate, sodium citrate or combinations thereof.

In one embodiment the feed additive composition of the present invention suitably comprises the proline tolerant tripeptidyl peptidase formulated with $Na_2SO_4$.

The proline tolerant tripeptidyl peptidase for use in the present invention may be a thermostable proline tolerant tripeptidyl peptidase.

The term "thermostable" means that an enzyme retains its activity when heated to temperatures of up to about 75°C. Suitably "thermostable" may mean that an enzyme retains its activity when heated to about 80 °C, more suitably about 90°C.

Advantageously, a thermostable proline tolerant tripeptidyl peptidase is less prone to being denatured e.g. under the heat treatment of the feed pelleting process and/or will retain its activity for a longer period of time in e.g. an animal when compared to a non-thermostable variant.

The proline tolerant tripeptidyl peptidase may have activity in a range of about pH 2 to about pH 7. Suitably, the proline tolerant tripeptidyl peptidase may have activity in a range of about pH 4 to about pH 7, more suitably in a range of about pH 4.5 to about pH 6.5.

In another embodiment the proline tolerant tripeptidyl peptidase may have activity at an acidic pH (suitably, the proline tolerant tripeptidyl peptidase may have optimum activity at acidic pH). The proline tolerant tripeptidyl peptidase may have activity at a pH of less than about pH 6, more suitably less than about pH 5. Preferably, the proline tolerant tripeptidyl peptidase may have activity at a pH of between about 2.5 to about pH 4.0, more suitably at between about 3.0 to about 3.3. In one embodiment the proline tolerant tripeptidyl peptidase may have activity at a pH around 2.5.

Advantageously, a proline tolerant tripeptidyl peptidase having activity at an acidic pH can be active in the upper gastrointestinal tract of an animal (e.g. in the gizzard, proventriculus or stomach) and/or can digest a peptide and/or protein substrate in combination with endogenous proteases (e.g. pepsin, trypsin or chymotrypsin) that are present in the gastrointestinal tract of the animal.

Many current feeding practices involve administering an alkaline protease active at a high pH (e.g. pH 8) to animals. Alkaline proteases are therefore only active lower down (e.g. later) in the gastrointestinal tract of an animal where the gastrointestinal tract becomes more alkaline, such as in the later part of the small intestine and the large intestine and caecum. Without wishing to be bound by theory, it is believed that producing oligopeptides in the later parts of the gastrointestinal tract increases populations of microbes which utilise the oligopeptides which in turn can lead to enteric disease challenges and/or reduced nutrients available for uptake by the animal. Additionally, later in the gastrointestinal tract (i.e. lower down) the mucosa is less well-protected than in the upper portions (e.g. the gizzard, proventriculus or stomach) and so is more easily damaged leading to inflammation. Advantageously, the use of a proline tolerant tripeptidyl peptidase having activity at an acid pH alleviates this problem as it is capable of digesting its substrate in the upper gastrointestinal tract thereby not substantially increasing populations of microbes and/or increasing the amount of nutrient (e.g. amino acids/peptides) available for uptake by an animal and/or reducing inflammation. Chicken peptide transporter PEPT1 is most highly expressed in the duodenum compared to the jejunum and ileum (Chen et al (2009) J. Anim. Sci. 77:1277-1283 the teaching of which is incorporated herein by reference), therefore the inventors have identified that a proline tolerant tripeptidyl peptidase having activity at an acid pH may facilitate the uptake of peptides by an animal as the digestion of the protein and/or peptide substrate (e.g. present in a feed and/or feedstuff) will be available for uptake by the animal, early in the gastrointestinal tract, in the duodenum. This is in contrast to alkaline proteases which are not active early on in the gastrointestinal tract.

In one embodiment at least one endoprotease may be used in combination with the proline tolerant tripeptidyl peptidase for any of the applications herein. At least one endoprotease may also be comprised in the feed additive composition, feedstuff, kit or premix described herein.

The term "endoprotease" as used herein is synonymous with the term "endopeptidase" and refers to an enzyme which is a proteolytic peptidase capable of cleaving internal peptide bonds of a peptide or protein substrate (e.g. not located towards the C or N-terminus of the peptide or protein substrate). Such endoproteases may be defined as one that tends to act away from the N-terminus or C-terminus.

In one embodiment the endoprotease may be one or more selected from the group consisting of: a serine protease, an aspartic acid protease, a cysteine protease, a metalloprotease, a threonine protease, a glutamic acid protease and a protease selected from the family of ungrouped proteases.

In one embodiment the endoprotease may be one or more selected from the group consisting of: an acid fungal protease, a subtilisin, a chymotrypsin, a trypsin, a pepsin, papain, bromalin, thermostable bacterial neutral metalloendopeptidase, metalloneutral endopeptidase, alkaline serine protease, fungal endoprotease or from the group of commercial protease products Alphalase® AFP, Alphalase® FP2, Alphalase® NP.

Suitably, the endoprotease may be an acid endoprotease.

Preferably the endoprotease may be an acid fungal protease.

Advantageously, the use of an endoprotease in combination with a proline tolerant tripeptidyl peptidase can increase the efficiency of substrate cleavage. Without wishing to be bound by theory, it is believed that an endoprotease is able to cleave a peptide and/or protein substrate at multiple regions away from the C or N-terminus, thereby producing more N-terminal ends for the proline tolerant tripeptidyl peptidase to use as a substrate, thereby advantageously increasing reaction efficiency and/or reducing reaction times.

The use of an acid endoprotease and a proline tolerant tripeptidyl peptidase having activity at an acid pH is highly advantageous as the two enzymes can co-operate to digest a peptide and/or protein substrate in the upper gastrointestinal tract (e.g. gizzard, proventriculus or stomach) of an animal and can be active in combination with other endogenous proteases (e.g. pepsin) present in the animal.

The proline tolerant tripeptidyl peptidase for use in accordance with the present invention may be an "in-feed" proline tolerant tripeptidyl peptidase.

The term "in-feed" as used herein means that the enzyme (e.g. the proline tolerant tripeptidyl peptidase and/or endoprotease) is functional, preferably primarily functional, more preferably solely functional, in the gastrointestinal tract (GIT) of the animal. In other words, the term "in-feed" as used herein means that the enzyme is substantially inactive (or is inactive) in the feed additive composition and/or feedstuff and/or feed and/or feed ingredient and/or premix prior to feeding the feed additive composition and/or feedstuff and/or feed and/or feed ingredient and/or premix to an animal.

There term "primarily functional" means that the enzyme mainly functions on its substrate once it enters the GIT. In other words, prior to entering the GIT the level of enzyme activity defined as the amount of cleavage of peptide and/or protein substrates to tripeptides is less than about 20%, suitably less than about 10%, preferably less than about 5%, of the level of enzyme activity after it enters the GIT (particularly, after it enters the gizzard, proventriculus or stomach of the animal).

Suitably, the proline tolerant tripeptidyl peptidase and/or the endoprotease is/are active in the duodenum and parts of the gastrointestinal tract of the animal preceding the duodenum (e.g. parts of the GIT that feed encounters earlier in the digestion process).

The term "solely functional" as used herein means that the enzyme is inactive before entering the GIT and is activated upon entering the GIT.

The term "inactive" as used herein means that the enzyme is not active. This may mean that the enzyme's activity is somehow inhibited or that the enzyme is in an environment in which it is inactive or that the enzyme is presented to its substrate immediately prior to feeding to the animal such that there is not enough time to be active. The "inactivity" of the enzyme may be in any event reversible once it enters the GIT of an animal.

Therefore, suitably the proline tolerant tripeptidyl peptidase (optionally in combination with an endoprotease) is admixed with the at least one protein or portion thereof immediately prior to feeding the feed additive composition to an animal.

The term "substantially inactive" as used herein means that the enzyme has low activity compared with its activity once it has entered the GIT (e.g. in the gizzard, proventriculus or stomach of the animal). For instance, substantially inactive may mean that the enzyme in the feed additive composition and/or feed and/or feedstuff and/or feed ingredient and/or premix has less than 10% of its activity when compared with its activity in the GIT (particularly, in the gizzard, proventriculus or stomach of the animal).

Maintaining the "in-feed" enzyme in an inactive or substantially inactive state in the feed additive composition and/or feedstuff and/or feed and/or feed ingredient and/or premix can be achieved in a number of ways known to one skilled in the art.

By way of example only maintaining the water content (wt %) of the feed additive composition and/or feedstuff and/or feed and/or feed ingredient and/or premix at less than 15%, preferably less than 10%, is sufficient to ensure that the in-feed enzyme is inactive or substantially inactive in the feed additive composition and/or feedstuff and/or feed and/or feed ingredient and/or premix.

In one embodiment the feed additive composition and/or feedstuff and/or feed and/or feed ingredient and/or premix, post-admixing the in-feed enzyme, are (maintained and/or stored) in a dry state or substantially dry state.

The term "dry state" as used herein means that the in-feed enzyme and/or the feed additive composition contains no or only a very low amount of water. In other words the term "dry state" as used herein may mean that the in-feed enzyme and/or the feed additive composition comprises less than 5%, preferably less than 1%, water content (wt %).

In one embodiment the proline tolerant tripeptidyl peptidase and/or endoprotease for use in the methods and/or uses and/or products of the present invention may in a dry or substantially dry state.

In another embodiment a proline tolerant tripeptidyl peptidase for use in any of the methods and/or uses and/or products of the invention may be admixed with a composition comprising at least one protein or at least a portion of a protein, wherein the composition, the proline tolerant tripeptidyl peptidase or combinations thereof are in a dry or substantially dry state when admixed. Suitably an endoprotease may be further admixed.

The term "dry or substantially dry state" when used herein means that the composition, proline tolerant tripeptidyl peptidase, endoprotease or combinations thereof contains only a very low amount of water. In other words the term "substantially dry state" as used herein may mean that the composition, proline tolerant tripeptidyl peptidase, endoprotease or combinations thereof comprises less than 15%, preferably less than 10%, water content (wt %).

In one embodiment, the composition, proline tolerant tripeptidyl peptidase, endoprotease or combinations thereof may be dried prior to, during or after (preferably prior to) use in the methods and/or uses of the invention.

In another embodiment the composition, proline tolerant tripeptidyl peptidase, endoprotease or combinations thereof either before or after use in the methods and/or uses of the invention comprises less than 15 wt % moisture content.

In another embodiment the composition, proline tolerant tripeptidyl peptidase, endoprotease or combinations thereof either before or after use in the methods and/or uses of the invention comprises less than 10 wt % moisture content. Suitably less than 5 wt % moisture content, more suitably less than 1 wt % moisture content.

The proline tolerant tripeptidyl peptidase (e.g. "in-feed" proline tolerant tripeptidyl peptidase), endoprotease or combinations thereof may be maintained in an inactive or substantially inactive state in the feed additive composition and/or feedstuff and/or feed and/or feed ingredient and/or premix by physically preventing the enzyme from interacting with its substrate. For example the proline tolerant tripeptidyl peptidase (e.g. "in-feed" proline tolerant tripeptidase), endoprotease or combinations thereof may be encapsulated prior to its use in the methods and/or uses feed additive composition and/or feedstuff and/or feed and/or feed ingredient and/or premix of the invention.

When the proline tolerant tripeptidyl peptidase (e.g. "in-feed" proline tolerant tripeptidyl peptidase), endoprotease or combinations thereof is physically prevented from interacting with its substrate in the feed additive composition and/or feedstuff and/or feed and/or feed ingredient and/or premix, then once in the GIT the physical barrier is removed thus allowing the interaction of the proline tolerant tripeptidyl peptidase (e.g. "in-feed" proline tolerant tripeptidyl peptidase), endoprotease or combinations thereof with its substrate.

By way of example only, the encapsulation may be removed by passage of the encapsulated proline tolerant tripeptidyl peptidase (e.g. "in-feed proline tolerant tripeptidyl peptidase), endoprotease or combinations thereof through the gizzard, proventriculus or stomach of an animal. The gizzard, proventriculus or stomach of an animal is at very low (acidic) pH (e.g. pH 2-4). This acidity can be used to activate encapsulated enzymes.

In one embodiment the enzyme may be encapsulated by a polymer, such as chitin or chitosans, gelatin, gum arabic or wax for example. By way of example only the polymer may be a gelatin or gum arabic as taught in Xue et al Food Funct. 2013, Apr 25; 6 Feb (epub); 4 (4) 610-7. Alternatively, the polymer may a chitosan-based hydrogel as taught in Zhang et al Biomacromolecules 2011,12,2894-2901.

In one embodiment the proline tolerant tripeptidyl peptidase (e.g. "in-feed" proline tolerant tripeptidyl peptidase), endoprotease or combinations thereof may be activated by feeding the enzyme to an animal.

The term "inactive" as used herein may mean that the enzyme is presented to its substrate immediately prior to feeding to the animal such that there is not enough time to be active before it enters the GIT of the animal.

In one embodiment the proline tolerant tripeptidyl peptidase for use in the present invention is part of a fermentate.

As used herein the term "fermentate" refers to the mixture of constituents present following (e.g. at the end of) the culturing of a host cell which fermentate includes the tripeptidyl peptidase (e.g. proline tolerant tripeptidyl peptidase), e.g. expressed by the host cell. The fermentate may comprises as well as the tripeptidyl peptidase in accordance with the present invention other components such as particulate matter, solids, substrates not utilised during culturing, debris, media, cell waste, etc. In one aspect, host cells (and particularly any spores) are removed from the fermentate and/or inactivated to provide a cell-free fermentate. In other embodiments the proline tolerant tripeptidyl peptidase for use in the present invention is isolated or purified.

The present enzymes, including combinations of 3PP and an endoprotease, are also useful in a starch conversion process, particularly in a saccharification and fermentation process of gelatinized, raw, and or granular starch that has undergone liquefaction. The desired end-product (often referred to as an "end-of-fermentation" or "EOF" product) may be any product that may be produced by the enzymatic conversion of the starch substrate. For example, the desired product may be a syrup rich in glucose and maltose, which can be used in other processes, such as the preparation of high-fructose corn syrup (HFCS), or which can be converted into a number of other useful products, such as an ascorbic acid intermediates (e.g., gluconate; 2-keto-L-gulonic acid; 5-keto-gluconate; and 2,5-diketogluconate); 1,3-propanediol; amino acids (e.g., tyrosine, serine, lysine, glutamic acid, glycine, phenylalanine and tryptophan); organic acids (e.g., lactate, pyruvate, succinate, citrate, isocitrate, gluconate, itaconate, and oxaloacetate); antibiotics; antimicrobials; enzymes; vitamins; hormones; ethanol, butanol, and other alcohols; glucono delta-lactone; sodium erythorbate; omega-3 fatty acid; isoprene; and other biochemicals and biomaterials. One skilled in the art is aware of various fermentation conditions that may be used in the production of these EOF products.

Those of skill in the art are well aware of available methods that may be used to prepare starch substrates for conversion. Useful starch substrates may be obtained from tubers, roots, stems, legumes, cereals or whole grain. More specifically, the granular starch may be obtained from corn, cobs, wheat, barley, rye, triticale, milo, sago, millet, cassava, tapioca, sorghum, rice, peas, bean, banana, or potatoes. The starch substrate can be a crude starch from milled whole grain, which contains non-starch fractions, e.g., germ residues and fibers. Liquefaction generally involves gelatinization of starch simultaneously with or followed by the addition of an $\alpha$-amylase, although additional liquefaction-inducing enzymes optionally may be added. In some cases, liquefaction of starch is performed at or below the gelatinization temperature, typically requires similar classes of enzymes with different performance criteria. The liquefied starch can be saccharified into a syrup with a lower degree of polymerization (DP) using $\alpha$-amylases, optionally in the presence of other enzymes. The exact composition of the products of saccharification depends on the combination of enzymes used, as well as the type of granular starch processed.

The present enzymes may be added during liquefaction and/or saccharification as an isolated enzyme solution, dried or granular enzyme, clarified broth, ultrafiltrate concentrate, or whole cell broth, optionally as part of a blend. The present enzymes can be also added in the form of a cultured cell material produced by host cells expressing the enzymes. The present enzymes may also be secreted by a host cell into the reaction medium during the fermentation or simultaneous saccharification and fermentation (SSF) process, such that the enzyme is provided continuously into the reaction (see, below). The host cell producing and secreting the present enzymes may also express an additional enzyme, such as a glucoamylase and/or $\alpha$-amylase. The host cell can be engineered to express a broad spectrum of various saccharolytic enzymes.

The soluble starch hydrolysate produced by treatment with amylase can be converted into high fructose starch-based syrup, such as high fructose corn syrup (HFCS). This conversion can be achieved using a glucose isomerase, particularly a glucose isomerase immobilized on a solid support.

Soluble starch hydrolysate, particularly a glucose rich syrup, can be fermented by contacting the starch hydrolysate with a fermenting organism. Ethanologenic microorganisms include yeast, such as *Saccharomyces cerevisiae* and bacteria, *e.g., Zymomonas mobilis,* expressing alcohol dehydrogenase and pyruvate decarboxylase. Commercial sources of yeast include ETHANOL REDO (LeSaffre); THERMOSACCÒ (Lallemand); RED STARÒ (Red Star); FERMIOLÒ (DSM Specialties); and SUPERSTARTÒ (Alltech). Microorganisms that produce other EOF (such as those mentioned, above) are also known in the art. As mentioned, above, the saccharification and fermentation processes may be carried out as an SSF process, wherein the fermenting organism expresses the present enzymes, optionally with one or more additional enzymes, such as a glucoamylase and/or $\alpha$-amylase. Fermentation may comprise subsequent enrichment, purification, and recovery of the EOF. **KITS**

In one aspect there is provided a kit comprising at least one proline tolerant tripeptidyl peptidase predominantly having exopeptidase activity wherein said proline tolerant tripeptidyl peptidase is capable of cleaving tri-peptides from the N-terminus of peptides having:

(i)

(A) Proline at P1; and
(B) An amino acid selected from alanine, arginine, asparagine, aspartic acid, cysteine, glutamine, glutamic acid, glycine, histidine, isoleucine, leucine, lysine, methionine, phenylalanine, serine, threonine, tryptophan, tyrosine, valine or synthetic amino acids at P1; and/or

(ii)

(a') Proline at P1'; and
(b') An amino acid selected from alanine, arginine, asparagine, aspartic acid, cysteine, glutamine, glutamic acid, glycine, histidine, isoleucine, leucine, lysine, methionine, phenylalanine, serine, threonine, tryptophan, tyrosine, valine or synthetic amino acids at P1'; and instructions for administering same to an animal, wherein the at least one proline tolerant tripeptidyl peptidase:

(a) comprises the amino acid sequence SEQ ID No. 29, SEQ ID No. 1, SEQ ID No. 2 or a functional fragment thereof that retains said peptidase activity;
(b) comprises an amino acid having at least 80% or at least 90% identity to SEQ ID No. 29, SEQ ID No. 1, SEQ ID No. 2 or a functional fragment thereof that retains said peptidase activity;
(c) is encoded by a nucleotide sequence comprising the sequence SEQ ID No. 56;
(d) is encoded by a nucleotide sequence comprising at least 80% or at least 90% sequence identity to SEQ ID No. 56;
(e) is encoded by a nucleotide sequence which hybridises to SEQ ID No. 56 under high stringency conditions; or
(f) is encoded by a nucleotide sequence which differs from SEQ ID No. 56 due to degeneracy of the genetic code.

Suitably the proline tolerant tripeptidyl peptidase may be capable of cleaving tri-peptides from the N-terminus of peptides having:

(i)

(A) Proline at P1; and
(B) An amino acid selected from alanine, arginine, asparagine, aspartic acid, cysteine, glutamine, glutamic acid, glycine, histidine, isoleucine, leucine, lysine, methionine, phenylalanine, serine, threonine, tryptophan, tyrosine, valine or synthetic amino acids at P1; and

(ii)

(a') Proline at P1'; and
(b') An amino acid selected from alanine, arginine, asparagine, aspartic acid, cysteine, glutamine, glutamic acid, glycine, histidine, isoleucine, leucine, lysine, methionine, phenylalanine, serine, threonine, tryptophan, tyrosine, valine or synthetic amino acids at P1'.

More suitably, the proline tolerant tripeptidyl peptidase may be capable of cleaving tri-peptides from the N-terminus of peptides having proline at position P1 and at position P1'. Suitably the kit may further comprise at least one endoprotease.

The endoprotease may be compartmentalised separately to the proline tolerant tripeptidyl peptidase or the two enzymes may be mixed.

The proline tolerant tripeptidyl peptidase and/or endoprotease may be formulated in any manner as described herein or known to the person skilled in the art.

Suitably when the kit comprises a proline tolerant tripeptidyl peptidase in combination with at least one endoprotease the instructions may be instructions for co-administering the same. The term "co-administering" as used herein means administering one or more ingredients and/or enzyme either separately (e.g. sequentially) or together (e.g. simultaneously).

## ACTIVITY AND ASSAYS

The proline tolerant tripeptidyl peptidase for use in the present invention predominantly has exopeptidase activity.

The term "exopeptidase" activity as used herein means that the proline tolerant tripeptidyl peptidase is capable of cleaving tri-peptides from the N-terminus of a substrate, such as a protein and/or peptide substrate.

The term "predominantly has exopeptidase activity" as used herein means that the tripeptidyl peptidase has no or substantially no endoprotease activity.

"Substantially no endoprotease activity" means that the proline tolerant tripeptidyl peptidase has less than about 100U endoprotease activity in the "Endoprotease Assay" taught herein when compared to 1000nkat of exopeptidase activity in the "Exopeptidase Broad-Specificity Assay (EBSA)" taught herein. Suitably, "substantially no endoprotease activity" means that the proline tolerant tripeptidyl peptidase has less than about 100U endoprotease activity in the "Endoprotease Assay" taught herein when compared to 1000nkat of exopeptidase activity in the "Exopeptidase Broad-Specificity Assay" taught herein.

Preferably the proline tolerant tripeptidyl peptidase may have less than about 10U endoprotease activity in the "Endoprotease Assay" taught herein when compared to 1000 nkat of exopeptidase activity in the "Exopeptidase Broad-Specificity Assay" taught herein, more preferably less than about 1U endoprotease activity in the "Endoprotease Assay" taught herein when compared to 1000 nkat of exopeptidase activity in the "Exopeptidase Broad-Specificity Assay" taught herein. Even more preferably the proline tolerant tripeptidyl peptidase or exo-tripeptidyl peptidase may have less than about 0.1U endoprotease activity in the "Endoprotease Assay" taught herein when compared to 1000 nkat of exopeptidase activity in the "Exopeptidase Broad-Specificity Assay" taught herein.

## "ENDOPROTEASE ASSAY"

Azoscasein assay for endoprotease activity

A modified version of the endoprotease assay described by Iversen and Jorgensen, 1995 (Biotechnology Techniques 9, 573-576) is used. An enzyme sample of 50 $\mu$l is added to 250 $\mu$l of azocasein (0.25% w/v; from Sigma) in 4 times diluted McIlvaine buffer, pH 5 and incubated for 15 min at 40°C with shaking (800 rpm). The reaction is terminated by adding 50 $\mu$l of 2 M trichloroacetic acid (TCA) (from Sigma Aldrich, Denmark) and centrifugation for 5 min at 20,000 g. To a 195 $\mu$l sample of the supernatant 65 $\mu$l of 1 M NaOH is added and absorbance at 450 nm is measured. One unit of endoprotease activity is defined as the amount which yields an increase in absorbance of 0.1 in 15 min at 40°C at 450 nm.

## "EXOPEPTIDASE ASSAY"

There are two parts to the assay:

## Part 1 - "Exopeptidase Broad-Specificity Assay" (EBSA)

10 $\mu$L of the chromogenic peptide solution (10 mM H-Ala-Ala-Ala-pNA dissolved in dimethyl sulfoxide (DMSO); MW = 387.82; Bachem, Switzerland) were added to 130 $\mu$l Na-acetate (20 mM, adjusted to pH 4.0 with acetic acid) in a microtiter plate and heated for 5 minutes at 40°C. 10 $\mu$L of appropriately diluted enzyme was added and the absorption was measured in a MTP reader (Versa max, Molecular Devices, Denmark) at 405 nm. One katal of proteolytic activity was defined as the amount of enzyme required to release 1 mole of p-nitroaniline per second.

**Part 2 (i) - P1 Proline Assay**

(a) Dissolve the substrate H-Arg-Gly-Pro-Phe-Pro-Ile-Ile-Val (MW = 897.12; from Schafer-N, Copenhagen in 10 times diluted McIlvain buffer, pH=4.5 at 1 mg/ml concentration.
(b) Incubate 1000 ul of the substrate solution with 10 ug of proline tolerant tripeptidyl peptidase solution at 40°C.
(c) Take 100 ul samples at seven time points (0, 30, 60, 120, 720 and 900 min), dilute with 50ul 5% TFA, heat inactivate (10 min at 80°C) and keep at -20°C until LC-MS analysis;
(d) Perform LC-MC/MS analysis using an Agilent 1100 Series Capillary HPLC system (Agilent Technologies, Santa Clara, CA) interfaced to a LTQ Orbitrap Classic hybrid mass spectrometer (Thermo Scientific, Bremen, Germany);
(e) Load samples onto a 50 mm Fortis™ C18 column with an inner diameter of 2.1 mm and a practical size of 1.7 $\mu$m
(f) Perform separation at a flow rate of 200$\mu$L/min using a 14 min gradient of 2-28% Solvent B (H2O/CH3CN/HCOOH (50/950/0.65 v/v/v)) into the IonMAX source- The LTQ Orbitrap Classic instrument was operated in a data-dependent MS/MS mode;
(g) Measure the peptide masses by the Orbitrap (obtain MS scans with a resolution of 60.000 at m/z 400), and select up to 2 of the most intense peptide m/z and subject to fragmentation using CID in the linear ion trap (LTQ). Enable dynamic exclusion with a list size of 500 masses, duration of 40 s, and an exclusion mass width of $\pm$10 ppm relative to masses on the list;
(h) Use the open source program Skyline 1.4.0.4421(available from MacCoss Lab Software, University of Washington, Department of Genome Sciences, 3720 15th Ave NE Seattle, Washington, US) to access the RAW files and extract MS1 intensities to build chromatograms. Set the precursor isotopic import filter to a count of three, (M, M+1, and M+2) at a resolution of 60,000 and use the most intense charge state;
(i) Peptide sequences of the substrate and cleavage products were typed into Skyline and intensities were calculated in each sample (0, 30, 60, 120, 720 and 900 min hydrolysis).
(j) One unit of activity is defined as the amount of enzyme which in this assay will hydrolyse 50% of the substrate within 720 min while releasing Arg-Gly-Pro.

**Part 2 (ii) - P1' Proline Assay**

(a) Dissolve the peptide H-Ala-Ala-Phe-Pro-Ala-NH2 (MW = 474.5; from Schafer-N, Copenhagen) in 10 times diluted McIlvain buffer, pH=4.5 at 0.1 mg/ml concentration.
(b) Incubate 1000 ul of the substrate solution with 10 ug proline tolerant tripeptidyl peptidase solution at 40°C.
(c) Take 100 ul samples at seven time points (0, 30, 60, 120, 720 and 900 min), dilute with 50ul 5% TFA, heat inactivate (10 min at 80°C) and keep at -20°C until LC-MS analysis;
(d) Perform LC-MC/MS analysis using a Agilent 1100 Series Capillary HPLC system (Agilent Technologies, Santa Clara, CA) interfaced to a LTQ Orbitrap Classic hybrid mass spectrometer (Thermo Scientific, Bremen, Germany);
(e) Load samples onto a 50 mm Fortis™ C18 column with an inner diameter of 2.1 mm and a practical size of 1.7 $\mu$m
(f) Perform separation at a flow rate of 200$\mu$L/min using a 14 min gradient of 2-28% Solvent B (H2O/CH3CN/ HCOOH (50/950/0.65 v/v/v)) into the IonMAX source- The LTQ Orbitrap Classic instrument was operated in a data-dependent MS/MS mode;
(g) Measure the peptide masses by the Orbitrap (obtain MS scans with a resolution of 60.000 at m/z 400), and select up to 2 of the most intense peptide m/z and subject to fragmentation using CID in the linear ion trap (LTQ). Enable dynamic exclusion with a list size of 500 masses, duration of 40 s, and an exclusion mass width of $\pm$10 ppm relative to masses on the list.
(h) Use the open source program Skyline 1.4.0.4421(available from MacCoss Lab Software, University of Washington, Department of Genome Sciences, 3720 15th Ave NE Seattle, Washington, US) to access the RAW files and extract MS1 intensities to build chromatograms. Set the precursor isotopic import filter to a count of three, (M, M+1, and M+2) at a resolution of 60,000 and use the most intense charge state;
(i) Peptide sequences of the substrate as well as cleavage products were typed into Skyline and intensities were calculated in each sample.
(j) One unit of activity is defined as the amount of enzyme which in this assay will hydrolyse 50% of the substrate within 720 min while releasing Ala-Ala-Phe.

In one embodiment a proline tolerant tripeptidyl peptidase for use in the present invention has an activity of at least 50 nkat in Part 1 of the activity taught herein and at least 100U activity in Part 2(i) or Part 2(ii) of the assay taught herein per mg of protein.

In one embodiment a proline tolerant tripeptidyl peptidase for use in the present invention has an activity of between about 50-2000 nkat in Part 1 of the activity taught herein and between about 1-500 units activity in Part 2(i) or Part 2(ii) of the assay taught herein per mg of protein. Note the protein measurement is described in Example 2.

## "P1 AND P1' PROLINE ACTIVITY ASSAY"

Suitably the tripeptidyl peptidase for use in the present invention may be able to cleave substrates having proline at position P1 and P1'. This can be assessed using the assay taught below.

In this assay a tripeptidyl peptidase is examined for its ability to hydrolyse a synthetic substrate AAPPA by LC-MS and label free quantification.

(a) Dissolve the peptide H-AAPPA-NH2 (MW=424.3, from Schafer-N, Copenhagen) in 20 mM MES buffer, pH=4.0 (1mg/ml);

(b) Incubate 1000 ul of the H-AAPPA-NH2 solution with 200 ul proline tolerant tripeptidyl peptidase solution (40ug/ml) (substrate/enzyme 100:0.8) at room temperature;

(c) Take 100 ul samples at seven time points (0, 5, 15, 60, 180, 720 and 1440 min), dilute with 50ul 5% TFA, heat inactivate (10 min at 80°C) and keep at -20°C until LC-MS analysis;

(d) Perform Nano LC-MS/MS analyses using an Easy LC system (Thermo Scientific, Odense, DK) interfaced to a LTQ Orbitrap Classic hybrid mass spectrometer (Thermo Scientific, Bremen, Germany);

(e) Load samples onto a custom-made 2 cm trap column (100 $\mu$m i.d., 375 $\mu$m o.d., packed with Reprosil C18, 5 $\mu$m reversed phase particles (Dr. Maisch GmbH, Ammerbuch-Entringen, Germany)) connected to a 10 cm analytical column (75 $\mu$m i.d., 375 $\mu$m o.d., packed with Reprosil C18, 3 $\mu$m reversed phase particles (Dr. Maisch GmbH, Ammerbuch-Entringen, Germany)) with a steel needle;

(f) Perform separation at a flow rate of 300nL/min using a 10 min gradient of 0-34% Solvent B (H2O/CH3CN/TFE/HCOOH (100/800//100/1 v/v/v/v)) into the nanoelectrospray ion source (Thermo Scientific, Odense, DK) - operate the LTQ Orbitrap Classic instrument in a data-dependent MS/MS mode;

(g) Measure the peptide masses by the Orbitrap (obtain MS scans with a resolution of 60 000 at m/z 400), and select up to 2 of the most intense peptide m/z and subject to fragmentation using CID in the linear ion trap (LTQ). Enable dynamic exclusion with a list size of 500 masses, duration of 40 s, and an exclusion mass width of $\pm$10 ppm relative to masses on the list;

(h) Use the open source program Skyline 1.4.0.4421(available from MacCoss Lab Software, University of Washington, Department of Genome Sciences, 3720 15th Ave NE Seattle, Washington, US) to access the RAW files which program can use the MS1 intensities to build chromatograms. Set the precursor isotopic import filter to a count of three, (M, M+1, and M+2) at a resolution of 60,000 and use the most intense charge state;

(i) Peptide sequences of the substrate as cleavage products were typed into Skyline and intensities were calculated in each sample.

(j) One unit of activity is defined as the amount of enzyme which in this assay will hydrolyse 50% of the substrate within 24 h while releasing AAP.

In one embodiment a proline tolerant tripeptidyl peptidase for use in the present invention has an activity of at least 50 nkat in Part 1 of the activity taught herein and at least 100 U activity in Part 2(i) or Part 2(ii) of the assay taught herein per mg of protein.

In one embodiment a proline tolerant tripeptidyl peptidase for use in the present invention has an activity of between about 50-2000 nkat in Part 1 of the activity taught herein and between about 1-500 units activity in Part 2(i) or Part 2(ii) of the assay taught herein per mg of protein (protein concentration is calculated as in Example 2).

In one embodiment a proline tolerant tripeptidyl peptidase for use in the present invention may have at least 10 U activity in the "P1 and P1' Proline Activity Assay" taught herein per mg of protein.

In one embodiment a proline tolerant tripeptidyl peptidase for use in the present invention has an activity of between about 1 U - 500 U activity in the "P1 and P1' Proline Activity Assay" taught herein per mg of protein.

In addition to the above, the proline tolerant tripeptidyl peptidase may also have activity in accordance with Part 1 of the "Exopeptidase Activity Assay" taught above.

In one embodiment the proline tolerant tripeptidyl peptidase for use in the present invention may have at least 10 U activity in the "P1 and P1' Proline Activity Assay" taught herein and at least 50 nkatal in Part 1 of the "Exopeptidase Activity Assay" taught herein per mg of protein. In another embodiment a proline tolerant tripeptidyl peptidase in accordance with the present invention has an activity of between about 1 U - 500 U activity in the "P1 and P1' Proline Activity Assay" taught herein and between about 50 U - 2000 U katal in Part 1 of the "Exopeptidase Activity Assay" taught herein per mg of protein.

## AMINO ACID AND NUCLEOTIDE SEQUENCES

The proline tolerant tripeptidyl peptidase for use in accordance with the present invention may be obtainable (e.g. obtained) from any source so long as it has the activity described in the claims.

In one embodiment the proline tolerant tripeptidyl peptidase for use in accordance with the present invention may be obtainable (e.g. obtained) from *Trichoderma.*

Suitably from *Trichoderma reesei,* more suitably, *Trichoderma reesei* QM6A.

Suitably from *Trichoderma virens,* more suitably, *Trichoderma virens* Gv29-8.

Suitably from *Trichoderma atroviride.* More suitably, *Trichoderma atroviride* IMI 206040.

In one embodiment the proline tolerant tripeptidyl peptidase for use in accordance with the present invention may be obtainable (e.g. obtained) from *Aspergillus.*

Suitably from *Aspergillus fumigatus,* more suitably *Aspergillus fumigatus* CAE17675. Suitably from *Aspergillus kawachii,* more suitably from *Aspergillus kawachii* IFO 4308. Suitably from *Aspergillus nidulans,* more suitably from *Aspergillus nidulans* FGSC A4. Suitably from *Aspergillus oryzae,* more suitably *Aspergillus oryzae* RIB40.

Suitably from *Aspergillus ruber,* more suitably *Aspergillus ruber* CBS135680.

Suitably from *Aspergillus terreus,* more suitably from *Aspergillus terreus* NIH2624.

In one embodiment the proline tolerant tripeptidyl peptidase for use in accordance with the present invention may be obtainable (e.g. obtained) from *Bipolaris,* suitably from *Bipolaris maydis,* more suitably *Bipolaris maydis* C5.

In one embodiment the proline tolerant tripeptidyl peptidase for use in accordance with the present invention may be obtainable (e.g. obtained) from *Togninia,* suitably from *Togninia minima* more suitably *Togninia minima* UCRPA7.

In one embodiment the proline tolerant tripeptidyl peptidase for use in accordance with the present invention may be obtainable (e.g. obtained) from *Talaromyces,* suitably from *Talaromyces stipitatus* more suitably *Talaromyces stipitatus* ATCC 10500.

In one embodiment the proline tolerant tripeptidyl peptidase for use in accordance with the present invention may be obtainable (e.g. obtained) from *Arthroderma,* suitably from *Arthroderma benhamiae* more suitably *Arthroderma benhamiae* CBS 112371.

In one embodiment the proline tolerant tripeptidyl peptidase for use in accordance with the present invention may be obtainable (e.g. obtained) from *Magnaporthe,* suitably from *Magnaporthe oryzae* more suitably *Magnaporthe oryzae* 70-1.

In another embodiment the proline tolerant tripeptidyl peptidase for use in accordance with the present invention may be obtainable (e.g. obtained) from *Fusarium.*

Suitably from *Fusarium oxysporum,* more suitably from *Fusarium oxysporum f. sp.* cubense race 4.

Suitably from *Fusarium graminearum,* more suitably *Fusarium graminearum* PH-1.

In a further embodiment the proline tolerant tripeptidyl peptidase for use in accordance with the present invention may be obtainable (e.g. obtained) from *Phaeosphaeria,* suitably from *Phaeosphaeria nodorum* more suitably *Phaeosphaeria nodorum* SN15.

In a yet further embodiment the proline tolerant tripeptidyl peptidase for use in accordance with the present invention may be obtainable (e.g. obtained) from *Agaricus,* suitably from *Agaricus bisporus* more suitably *Agaricus bisporus var. burnettii* JB137-S8.

In a yet further embodiment the proline tolerant tripeptidyl peptidase for use in accordance with the present invention may be obtainable (e.g. obtained) from *Acremonium,* suitably from *Acremonium alcalophilum.*

In a yet further embodiment the proline tolerant tripeptidyl peptidase for use in accordance with the present invention may be obtainable (e.g. obtained) from *Sodiomyces,* suitably from *Sodiomyces alkalinus.*

In one embodiment the proline tolerant tripeptidyl peptidase for use in accordance with the present invention may be obtainable (e.g. obtained) from *Penicillium.*

Suitably the proline tolerant tripeptidyl peptidase may be obtainable from *Penicillium digitatum,* more suitably from *Penicillium digitatum* Pd1.

Suitably the proline tolerant tripeptidyl peptidase may be obtainable from *Penicillium oxalicum,* more suitably from *Penicillium oxalicum* 114-2.

Suitably the proline tolerant tripeptidyl peptidase may be obtainable from *Penicillium roqueforti,* more suitably from *Penicillium roqueforti* FM164.

Suitably the proline tolerant tripeptidyl peptidase may be obtainable from *Penicillium rubens,* more suitably from *Penicillium rubens* Wisconsin 54-1255.

In another embodiment the proline tolerant tripeptidyl peptidase for use in accordance with the present invention may be obtainable (e.g. obtained) from *Neosartorya.*

Suitably the proline tolerant tripeptidyl peptidase may be obtainable from *Neosartorya fischeri,* more suitably from *Neosartorya fischeri* NRRL181.

In one embodiment the tripeptidyl peptidase (e.g. proline tolerant tripeptidyl peptidase) for use in accordance with the present invention is not obtainable (e.g. obtained) from *Aspergillus niger.*

| SEQ ID No.: | Sequence | Origin |
|---|---|---|
| 1 | MAKLSTLRLASLLSLVSVQVSASVHLLESLEKLPHGWKAAETPSPS SQIVLQVALTQQNIDQLESRLAAVSTPTSSTYGKYLDVDEINSIFAP SDASSSAVESWLQSHGVTSYTKQGSSIWFQTNISTANAMLSTNFH TYSDLTGAKKVRTLKYSIPESLIGHVDLISPTTYFGTTKAMRKLKSS GVSPAADALAARQEPSSCKGTLVFEGETFNVFQPDCLRTEYSVDG YTPSVKSGSRIGFGSFLNESASFADQALFEKHFNIPSQNFSVVLING GTDLPQPPSDANDGEANLDAQTILTIAHPLPITEFITAGSPPYFPDP VEPAGTPNENEPYLQYYEFLLSKSNAEIPQVITNSYGDEEQTVPRS YAVRVCNLIGLLGLRGISVLHSSGDEGVGASCVATNSTTPQFNPIF PATCPYVTSVGGTVSFNPEVAWAGSSGGFSYYFSRPWYQQEAV GTYLEKYVSAETKKYYGPYVDFSGRGFPDVAAHSVSPDYPVFQG GELTPSGGTSAASPVVAAIVALLNDARLREGKPTLGFLNPLIYLHAS KGFTDITSGQSEGCNGNNTQTGSPLPGAGFIAGAHWNATKGWDP TTGFGVPNLKKLLALVRF | Trichoderma reesei QM6a |
| 2 | SVHLLESLEKLPHGWKAAETPSPSSQIVLQVALTQQNIDQLESRLA AVSTPTSSTYGKYLDVDEINSIFAPSDASSSAVESWLQSHGVTSYT KQGSSIWFQTNISTANAMLSTNFHTYSDLTGAKKVRTLKYSIPESLI GHVDLISPTTYFGTTKAMRKLKSSGVSPAADALAARQEPSSCKGT LVFEGETFNVFQPDCLRTEYSVDGYTPSVKSGSRIGFGSFLNESA SFADQALFEKHFNIPSQNFSVVLINGGTDLPQPPSDANDGEANLDA QTILTIAHPLPITEFITAGSPPYFPDPVEPAGTPNENEPYLQYYEFLL SKSNAEIPQVITNSYGDEEQTVPRSYAVRVCNLIGLLGLRGISVLHS SGDEGVGASCVATNSTTPQFNPIFPATCPYVTSVGGTVSFNPEVA WAGSSGGFSYYFSRPWYQQEAVGTYLEKYVSAETKKYYGPYVDF SGRGFPDVAAHSVSPDYPVFQGGELTPSGGTSAASPVVAAIVALL NDARLREGKPTLGFLNPLIYLHASKGFTDITSGQSEGCNGNNTQT GSPLPGAGFIAGAHWNATKGWDPTTGFGVPNLKKLLALVRF | Trichoderma reesei QM6a |
| 3 | EAFEKLSAVPKGWHYSSTPKGNTEVCLKIALAQKDAAGFEKTVLE MSDPDHPSYGQHFTTHDEMKRMLLPRDDTVDAVRQWLENGGVT DFTQDADWINFCTTVDTANKLLNAQFKWYVSDVKHIRRLRTLQYD VPESVTPHINTIQPTTRFGKISPKKAVTHSKPSQLDVTALAAAVVAK NISHCDSIITPTCLKELYNIGDYQADANSGSKIAFASYLEEYARYADL ENFENYLAPWAKGQNFSVTTFNGGLNDQNSSSDSGEANLDLQYIL GVSAPLPVTEFSTGGRGPLVPDLTQPDPNSNSNEPYLEFFQNVLK LDQKDLPQVISTSYGENEQEIPEKYARTVCNLIAQLGSRGVSVLFS SGDSGVGEGCMTNDGTNRTHFPPQFPAACPWVTSVGATFKTTPE RGTYFSSGGFSDYWPRPEWQDEAVSSYLETIGDTFKGLYNSSGR AFPDVAAQGMNFAVYDKGTLGEFDGTSASAPAFSAVIALLNDARL RAGKPTLGFLNPWLYKTGRQGLQDITLGASIGCTGRARFGGAPDG GPVVPYASWNATQGWDPVTGLGTPDFAELKKLALGN | Aspergillus oryzae RIB40 |

(continued)

| SEQ ID No.: | Sequence | Origin |
|---|---|---|
| 4 | EPFEKLFSTPEGWKMQGLATNEQIVKLQIALQQGDVAGFEQHVIDI STPSHPSYGAHYGSHEEMKRMIQPSSETVASVSAWLKAAGINDAE IDSDWVTFKTTVGVANKMLDTKFAWYVSEEAKPRKVLRTLEYSVP DDVAEHINLIQPTTRFAAIRQNHEVAHEIVGLQFAALANNTVNCDAT ITPQCLKTLYKIDYKADPKSGSKVAFASYLEQYARYNDLALFEKAFL PEAVGQNFSVVQFSGGLNDQNTTQDSGEANLDLQYIVGVSAPLPV TEFSTGGRGPWVADLDQPDEADSANEPYLEFLQGVLKLPQSELP QVISTSYGENEQSVPKSYALSVCNLFAQLGSRGVSVIFSSGDSGP GSACQSNDGKNTTKFQPQYPAACPFVTSVGSTRYLNETATGFSS GGFSDYWKRPSYQDDAVKAYFHHLGEKFKPYFNRHGRGFPDVAT QGYGFRVYDQGKLKGLQGTSASAPAFAGVIGLLNDARLKAKKPTL GFLNPLLYSNSDALNDIVLGGSKGCDGHARFNGPPNGSPVIPYAG WNATAGWDPVTGLGTPNFPKLLKAAVPSRYRA | Phaeosphaeria nodorum SN15 |
| 5 | NAAVLLDSLDKVPVGWQAASAPAPSSKITLQVALTQQNIDQLESKL AAVSTPNSSNYGKYLDVDEINQIFAPSSASTAAVESWLKSYGVDYK VQGSSIWFQTDVSTANKMLSTNFHTYTDSVGAKKVRTLQYSVPET LADHIDLISPTTYFGTSKAMRALKIQNAASAVSPLAARQEPSSCKGT IEFENRTFNVFQPDCLRTEYSVNGYKPSAKSGSRIGFGSFLNQSAS SSDLALFEKHFGFASQGFSVELINGGSNPQPPTDANDGEANLDAQ NIVSFVQPLPITEFIAGGTAPYFPDPVEPAGTPDENEPYLEYYEYLL SKSNKELPQVITNSYGDEEQTVPQAYAVRVCNLIGLMGLRGISILES SGDEGVGASCLATNSTTTPQFNPIFPATCPYVTSVGGTVSFNPEV AWDGSSGGFSYYFSRPWYQEAAVGTYLNKYVSEETKEYYKSYVD FSGRGFPDVAAHSVSPDYPVFQGGELTPSGGTSAASPIVASVIALL NDARLRAGKPALGFLNPLIYGYAYKGFTDITSGQAVGCNGNNTQT GGPLPGAGVIPGAFWNATKGWDPTTGFGVPNFKKLLELVRY | Trichoderma atroviride IMI 206040 |
| 6 | KPTPGASHKVIEHLDFVPEGWQMVGAADPAAIIDFWLAIERENPEK LYDTIYDVSTPGRAQYGKHLKREELDDLLRPRAETSESIINWLTNG GVNPQHIRDEGDWVRFSTNVKTAETLMNTRFNVFKDNLNSVSKIR TLEYSVPVAISAHVQMIQPTTLFGRQKPQNSLILNPLTKDLESMSVE EFAASQCRSLVTTACLRELYGLGDRVTQARDDNRIGVSGFLEEYA QYRDLELFLSRFEPSAKGFNFSEGLIAGGKNTQGGPGSSTEANLD MQYVVGLSHKAKVTYYSTAGRGPLIPDLSQPSQASNNNEPYLEQL RYLVKLPKNQLPSVLTTSYGDTEQSLPASYTKATCDLFAQLGTMG VSVIFSSGDTGPGSSCQTNDGKNATRFNPIYPASCPFVTSIGGTVG TGPERAVSFSSGGFSDRFPRPQYQDNAVKDYLKILGNQWSGLFD PNGRAFPDIAAQGSNYAVYDKGRMTGVSGTSASAPAMAAIIAQLN DFRLAKGSPVLGFLNPWIYSKGFSGFTDIVDGGSRGCTGYDIYSGL KAKKVPYASWNATKGWDPVTGFGTPNFQALTKVLP | Arthroderma benhamiae CBS 112371 |

(continued)

| SEQ ID No.: | Sequence | Origin |
|---|---|---|
| 7 | KSYSHHAEAPKGWKVDDTARVASTGKQQVFSIALTMQNVDQLES KLLDLSSPDSKNYGQWMSQKDVTTAFYPSKEAVSSVTKWLKSKG VKHYNVNGGFIDFALDVKGANALLDSDYQYYTKEGQTKLRTLSYSI PDDVAEHVQFVDPSTNFGGTLAFAPVTHPSRTLTERKNKPTKSTV DASCQTSITPSCLKQMYNIGDYTPKVESGSTIGFSSFLGESAIYSDV FLFEEKFGIPTQNFTTVLINNGTDDQNTAHKNFGEADLDAENIVGIA HPLPFTQYITGGSPPFLPNIDQPTAADNQNEPYVPFFRYLLSQKEV PAVVSTSYGDEEDSVPREYATMTCNLIGLLGLRGISVIFSSGDIGVG AGCLGPDHKTVEFNAIFPATCPYLTSVGGTVDVTPEIAWEGSSGG FSKYFPRPSYQDKAVKTYMKTVSKQTKKYYGPYTNWEGRGFPDV AGHSVSPNYEVIYAGKQSASGGTSAAAPVWAAIVGLLNDARFRAG KPSLGWLNPLVYKYGPKVLTDITGGYAIGCDGNNTQSGKPEPAGS GIVPGARWNATAGWDPVTGYGTPDFGKLKDLVLSF | Fusarium graminearum PH-1 |
| 8 | AVVIRAAVLPDAVKLMGKAMPDDIISLQFSLKQQNIDQLETRLRAVS DPSSPEYGQYMSESEVNEFFKPRDDSFAEVIDWVAASGFQDIHLT PQAAAINLAATVETADQLLGANFSWFDVDGTRKLRTLEYTIPDRLA DHVDLISPTTYFGRARLDGPRETPTRLDKRQRDPVADKAYFHLKW DRGTSNCDLVITPPCLEAAYNYKNYMPDPNSGSRVSFTSFLEQAA QQSDLTKFLSLTGLDRLRPPSSKPASFDTVLINGGETHQGTPPNKT SEANLDVQWLAAVIKARLPITQWITGGRPPFVPNLRLRHEKDNTNE PYLEFFEYLVRLPARDLPQVISNSYAEDEQTVPEAYARRVCNLIGIM GLRGVTVLTASGDSGVGAPCRANDGSDRLEFSPQFPTSCPYITAV GGTEGWDPEVAWEASSGGFSHYFLRPWYQANAVEKYLDEELDP ATRAYYDGNGFVQFAGRAYPDLSAHSSSPRYAYIDKLAPGLTGGT SASCPVVAGIVGLLNDARLRRGLPTMGFINPWLYTRGFEALQDVT GGRASGCQGIDLQRGTRVPGAGIIPWASWNATPGWDPATGLGLP DFWAMRGLALGRGT | Acremonium alcalophilum |
| 9 | AVVIRAAPLPESVKLVRKAAAEDGINLQLSLKRQNMDQLEKFLRAV SDPFSPKYGQYMSDAEVHEIFRPTEDSFDQVIDWLTKSGFGNLHIT PQAAAINVATTVETADQLFGANFSWFDVDGTPKLRTGEYTIPDRLV EHVDLVSPTTYFGRMRPPPRGDGVNDWITENSPEQPAPLNKRDT KTESDQARDHPSWDSRTPDCATIITPPCLETAYNYKGYIPDPKSGS RVSFTSFLEQAAQQADLTKFLSLTRLEGFRTPASKKKTFKTVLING GESHEGVHKKSKTSEANLDVQWLAAVTQTKLPITQWITGGRPPFV PNLRIPTPEANTNEPYLEFLEYLFRLPDKDLPQVISNSYAEDEQSVP EAYARRVCGLLGIMGLRGVTVLTASGDSGVGAPCRANDGSGREE FSPQFPSSCPYITTVGGTQAWDPEVAWKGSSGGFSNYFPRPWYQ VAAVEKYLEEQLDPAAREYYEENGFVRFAGRAFPDLSAHSSSPKY AYVDKRVPGLTGGTSASCPVVAGIVGLLNDARLRRGLPTMGFINP WLYAKGYQALEDVTGGAAVGCQGIDIQTGKRVPGAGIIPGASWNA TPDWDPATGLGLPNFWAMRELALED | Sodiomyces alkalinus |

(continued)

| SEQ ID No.: | Sequence | Origin |
|---|---|---|
| 10 | VVHEKLAAVPSGWHHLEDAGSDHQISLSIALARKNLDQLESKLKDL STPGESQYGQWLDQEEVDTLFPVASDKAVISWLRSANITHIARQG SLVNFATTVDKVNKLLNTTFAYYQRGSSQRLRTTEYSIPDDLVDSID LISPTTFFGKEKTSAGLTQRSQKVDNHVAKRSNSSSCADTITLSCL KEMYNFGNYTPSASSGSKLGFASFLNESASYSDLAKFERLFNLPS QNFSVELINGGVNDQNQSTASLTEADLDVELLVGVGHPLPVTEFIT SGEPPFIPDPDEPSAADNENEPYLQYYEYLLSKPNSALPQVISNSY GDDEQTVPEYYAKRVCNLIGLVGLRGISVLESSGDEGIGSGCRTTD GTNSTQFNPIFPATCPYVTAVGGTMSYAPEIAWEASSGGFSNYFE RAWFQKEAVQNYLANHITNETKQYYSQFANFSGRGFPDVSAHSF EPSYEVIFYGARYGSGGTSAACPLFSALVGMLNDARLRAGKSTLG FLNPLLYSKGYKALTDVTAGQSIGCNGIDPQSDEAVAGAGIIPWAH WNATVGWDPVTGLGLPDFEKLRQLVLSL | Aspergillus kawachii IFO 4308 |
| 11 | AAALVGHESLAALPVGWDKVSTPAAGTNIQLSVALALQNIEQLEDH LKSVSTPGSASYGQYLDSDGIAAQYGPSDASVEAVTNWLKEAGVT DIYNNGQSIHFATSVSKANSLLGADFNYYSDGSATKLRTLAYSVPS DLKEAIDLVSPTTYFGKTTASRSIQAYKNKRASTTSKSGSSSVQVS ASCQTSITPACLKQMYNVGNYTPSVAHGSRVGFGSFLNQSAIFDD LFTYEKVNDIPSQNFTKVIIANASNSQDASDGNYGEANLDVQNIVGI SHPLPVTEFLTGGSPPFVASLDTPTNQNEPYIPYYEYLLSQKNEDL PQVISNSYGDDEQSVPYKYAIRACNLIGLTGLRGISVLESSGDLGV GAGCRSNDGKNKTQFDPIFPATCPYVTSVGGTQSVTPEIAWVASS GGFSNYFPRTWYQEPAIQTYLGLLDDETKTYYSQYTNFEGRGFPD VSAHSLTPDYQVVGGGYLQPSGGTSAASPVFAGIIALLNDARLAAG KPTLGFLNPFFYLYGYKGLNDITGGQSVGCNGINGQTGAPVPGGG IVPGAAWNSTTGWDPATGLGTPDFQKLKELVLSF | Talaromyces stipitatus ATCC 10500 |
| 12 | KSFSHHAEAPQGWQVQKTAKVASNTQHVFSLALTMQNVDQLESK LLDLSSPDSANYGNWLSHDELTSTFSPSKEAVASVTKWLKSKGIK HYKVNGAFIDFAADVEKANTLLGGDYQYYTKDGQTKLRTLSYSIPD DVAGHVQFVDPSTNFGGTVAFNPVPHPSRTLQERKVSPSKSTVD ASCQTSITPSCLKQMYNIGDYTPDAKSGSEIGFSSFLGQAAIYSDVF KFEELFGIPKQNYTTILINNGTDDQNTAHGNFGEANLDAENIVGIAH PLPFKQYITGGSPPFVPNIDQPTEKDNQNEPYVPFFRYLLGQKDLP AVISTSYGDEEDSVPREYATLTCNMIGLLGLRGISVIFSSGDIGVGS GCLAPDYKTVEFNAIFPATCPYLTSVGGTVDVTPEIAWEGSSGGFS KYFPRPSYQDKAIKKYMKTVSKETKKYYGPYTNWEGRGFPDVAG HSVAPDYEVIYNGKQARSGGTSAAAPVWAAIVGLLNDARFKAGKK SLGWLNPLIYKHGPKVLTDITGGYAIGCDGNNTQSGKPEPAGSGL VPGARWNATAGWDPTTGYGTPNFQKLKDLVLSL | Fusarium oxysporum f. sp. cubense race 4 |

23

(continued)

| SEQ ID No.: | Sequence | Origin |
|---|---|---|
| 13 | SVLVESLEKLPHGWKAASAPSPSSQITLQVALTQQNIDQLESRLAA VSTPNSKTYGNYLDLDEINEIFAPSDASSAAVESWLHSHGVTKYTK QGSSIWFQTEVSTANAMLSTNFHTYSDAAGVKKLRTLQYSIPESLV GHVDLISPTTYFGTSNAMRALRSKSVASVAQSVAARQEPSSCKGT LVFEGRTFNVFQPDCLRTEYNVNGYTPSAKSGSRIGFGSFLNQSA SFSDLALFEKHFGFSSQNFSVVLINGGTDLPQPPSDDNDGEANLD VQNILTIAHPLPITEFITAGSPPYFPDPVEPAGTPDENEPYLQYFEYL LSKPNRDLPQVITNSYGDEEQTVPQAYAVRVCNLIGLMGLRGISILE SSGDEGVGASCVATNSTTPQFNPIFPATCPYVTSVGGTVNFNPEV AWDGSSGGFSYYFSRPWYQEEAVGNYLEKHVSAETKKYYGPYV DFSGRGFPDVAAHSVSPDYPVFQGGQLTPSGGTSAASPVVASIIA LLNDARLREGKPTLGFLNPLIYQYAYKGFTDITSGQSDGCNGNNTQ TDAPLPGAGVVLGAHWNATKGWDPTTGFGVPNFKKLLELIRYI | Trichoderma virens Gv29-8 |
| 14 | AVLVESLKQVPNGWNAVSTPDPSTSIVLQIALAQQNIDELEWRLAA VSTPNSGNYGKYLDIGEIEGIFAPSNASYKAVASWLQSHGVKNFVK QAGSIWFYTTVSTANKMLSTDFKHYSDPVGIEKLRTLQYSIPEELV GHVDLISPTTYFGNNHPATARTPNMKAINVTYQIFHPDCLKTKYGV DGYAPSPRCGSRIGFGSFLNETASYSDLAQFEKYFDLPNQNLSTLL INGAIDVQPPSNKNDSEANMDVQTILTFVQPLPITEFVVAGIPPYIPD AALPIGDPVQNEPWLEYFEFLMSRTNAELPQVIANSYGDEEQTVP QAYAVRVCNQIGLLGLRGISVIASSGDTGVGMSCMASNSTTPQFN PMFPASCPYITTVGGTQHLDNEIAWELSSGGFSNYFTRPWYQEDA AKTYLERHVSTETKAYYERYANFLGRGFPDVAALSLNPDYPVIIGG ELGPNGGTSAAAPVVASIIALLNDARLCLGKPALGFLNPLIYQYADK GGFTDITSGQSWGCAGNTTQTGPPPPGAGVIPGAHWNATKGWD PVTGFGTPNFKKLLSLALSV | Trichoderma atroviride IMI 206040 |
| 15 | SPLARRWDDFAEKHAWVEVPRGWEMVSEAPSDHTFDLRIGVKSS GMEQLIENLMQTSDPTHSRYGQHLSKEELHDFVQPHPDSTGAVE AWLEDFGISDDFIDRTGSGNWVTVRVSVAQAERMLGTKYNVYRH SESGESVVRTMSYSLPSELHSHIDVVAPTTYFGTMKSMRVTSFLQ PEIEPVDPSAKPSAAPASCLSTTVITPDCLRDLYNTADYVPSATSRN AIGIAGYLDRSNRADLQTFFRRFRPDAVGFNYTTVQLNGGGDDQN DPGVEANLDIQYAAGIAFPTPATYWSTGGSPPFIPDTQTPTNTNEP YLDWINFVLGQDEIPQVISTSYGDDEQTVPEDYATSVCNLFAQLGS RGVTVFFSSGDFGVGGGDCLTNDGSNQVLFQPAFPASCPFVTAV GGTVRLDPEIAVSFSGGGFSRYFSRPSYQNQTVAQFVSNLGNTFN GLYNKNGRAYPDLAAQGNGFQVVIDGIVRSVGGTSASSPTVAGIF ALLNDFKLSRGQSTLGFINPLIYSSATSGFNDIRAGTNPGCGTRGF TAGTGWDPVTGLGTPDFLRLQGLI | Agaricus bisporus var. burnettii JB137-S8 |

(continued)

| SEQ ID No.: | Sequence | Origin |
|---|---|---|
| 16 | RVFDSLPHPPRGWSYSHAAESTEPLTLRIALRQQNAAALEQVVLQ VSNPRHANYGQHLTRDELRSYTAPTPRAVRSVTSWLVDNGVDDY TVEHDWVTLRTTVGAADRLLGADFAWYAGPGETLQLRTLSYGVD DSVAPHVDLVQPTTRFGGPVGQASHIFKQDDFDEQQLKTLSVGFQ VMADLPANGPGSIKAACNESGVTPLCLRTLYRVNYKPATTGNLVA FASFLEQYARYSDQQAFTQRVLGPGVPLQNFSVETVNGGANDQQ SKLDSGEANLDLQYVMAMSHPIPILEYSTGGRGPLVPTLDQPNAN NSSNEPYLEFLTYLLAQPDSAIPQTLSVSYGEEEQSVPRDYAIKVC NMFMQLGARGVSVMFSSGDSGPGNDCVRASDNATFFGSTFPAG CPYVTSVGSTVGFEPERAVSFSSGGFSIYHARPDYQNEVVPKYIES IKASGYEKFFDGNGRGIPDVAAQGARFVVIDKGRVSLISGTSASSP AFAGMVALVNAARKSKDMPALGFLNPMLYQNAAAMTDIVNGAGIG CRKQRTEFPNGARFNATAGWDPVTGLGTPLFDKLLAVGAPGVPN A | Magnaporthe oryzae 70-15 |
| 17 | SDVVLESLREVPQGWKRLRDADPEQSIKLRIALEQPNLDLFEQTLY DISSPDHPKYGQHLKSHELRDIMAPREESTAAVIAWLQDAGLSGS QIEDDSDWINIQTTVAQANDMLNTTFGLFAQEGTEVNRIRALAYSV PEEIVPHVKMIAPIIRFGQLRPQMSHIFSHEKVEETPSIGTIKAAAIPS VDLNVTACNASITPECLRALYNVGDYEADPSKKSLFGVCGYLEQY AKHDQLAKFEQTYAPYAIGADFSVVTINGGGDNQTSTIDDGEANLD MQYAVSMAYKTPITYYSTGGRGPLVPDLDQPDPNDVSNEPYLDFV SYLLKLPDSKLPQTITTSYGEDEQSVPRSYVEKVCTMFGALGARG VSVIFSSGDTGVGSACQTNDGKNTTRFLPIFPAACPYVTSVGGTRY VDPEVAVSFSSGGFSDIFPTPLYQKGAVSGYLKILGDRWKGLYNP HGRGFPDVSGQSVRYHVFDYGKDVMYSGTSASAPMFAALVSLLN NARLAKKLPPMGFLNPWLYTVGFNGLTDIVHGGSTGCTGTDVYSG LPTPFVPYASWNATVGWDPVTGLGTPLFDKLLNLSTPNFHLPHIG GH | Togninia minima UCRPA7 |
| 18 | STTSHVEGEVVERLHGVPEGWSQVGAPNPDQKLRFRIAVRSADS ELFERTLMEVSSPSHPRYGQHLKRHELKDLIKPRAKSTSNILNWLQ ESGIEARDIQNDGEWISFYAPVKRAEQMMSTTFKTYQNEARANIKK IRSLDYSVPKHIRDDIDIIQPTTRFGQIQPERSQVFSQEEVPFSALVV NATCNKKITPDCLANLYNFKDYDASDANVTIGVSGFLEQYARFDDL KQFISTFQPKAAGSTFQVTSVNAGPFDQNSTASSVEANLDIQYTTG LVAPDIETRYFTVPGRGILIPDLDQPTESDNANEPYLDYFTYLNNLE DEELPDVLTTSYGESEQSVPAEYAKKVCNLIGQLGARGVSVIFSSG DTGPGSACQTNDGKNTTRFLPIFPASCPYVTSVGGTVGVEPEKAV SFSSGGFSDLWPRPAYQEKAVSEYLEKLGDRWNGLYNPQGRGF PDVAAQGQGFQVFDKGRLISVGGTSASAPVFASVVALLNNARKAA GMSSLGFLNPWIYEQGYKGLTDIVAGGSTGCTGRSIYSGLPAPLVP YASWNATEGWDPVTGYGTPDFKQLLTLATAPKSGERRVRRGGLG GQA | Bipolaris maydis C5 |

(continued)

| SEQ ID No.: | Sequence | Origin |
|---|---|---|
| 19 | MLSSFLSQGAAVSLALLSLLPSPVAAEIFEKLSGVPNGWRYANNPH GNEVIRLQIALQQHDVAGFEQAVMDMSTPGHADYGKHFRTHDEM KRMLLPSDTAVDSVRDWLESAGVHNIQVDADWVKFHTTVNKANA LLDADFKWYVSEAKHIRRLRTLQYSIPDALVSHINMIQPTTRFGQIQ PNRATMRSKPKHADETFLTAATLAQNTSHCDSIITPHCLKQLYNIG DYQADPKSGSKVGFASYLEEYARYADLERFEQHLAPNAIGQNFSV VQFNGGLNDQLSLSDSGEANLDLQYILGVSAPVPVTEYSTGGRGE LVPDLSSPDPNDNSNEPYLDFLQGILKLDNSDLPQVISTSYGEDEQ TIPVPYARTVCNLYAQLGSRGVSVIFSSGDSGVGAACLTNDGTNR THFPPQFPASCPWVTSVGATSKTSPEQAVSFSSGGFSDLWPRPS YQQAAVQTYLTQHLGNKFSGLFNASGRAFPDVAAQGVNYAVYDK GMLGQFDGTSCSAPTFSGVIALLNDARLRAGLPVMGFLNPFLYGV GSESGALNDIVNGGSLGCDGRNRFGGTPNGSPVVPFASWNATTG WDPVSGLGTPDFAKLRGVALGEAKAYGN | Aspergillus kawachii IFO 4308 |
| 20 | MAATGRFTAFWNVASVPALIGILPLAGSHLRAVLCPVCIWRHSKAV CAPDTLQAMRAFTRVTAISLAGFSCFAAAAAAFESLRAVPDGWIY ESTPDPNQPLRLRIALKQHNVAGFEQALLDMSTPGHSSYGQHFGS YHEMKQLLLPTEEASSSVRDWLSAAGVEFEQDADWINFRTTVDQA NALLDADFLWYTTTGSTGNPTRILRTLSYSVPSELAGYVNMIQPTT RFGGTHANRATVRAKPIFLETNRQLINAISSGSLEHCEKAITPSCLA DLYNTEGYKASNRSGSKVAFASFLEEYARYDDLAEFEETYAPYAIG QNFSVISINGGLNDQDSTADSGEANLDLQYIIGVSSPLPVTEFTTGG RGKLIPDLSSPDPNDNTNEPFLDFLEAVLKLDQKDLPQVISTSYGE DEQTIPEPYARSVCNLYAQLGSRGVSVLFSSGDSGVGAACQTND GKNTTHFPPQFPASCPWVTAVGGTNGTAPESGVYFSSGGFSDYW ARPAYQNAAVESYLRKLGSTQAQYFNRSGRAFPDVAAQAQNFAV VDKGRVGLFDGTSCSSPVFAGIVALLNDVRLKAGLPVLGFLNPWL YQDGLNGLNDIVDGGSTGCDGNNRFNGSPNGSPVIPYAGWNATE GWDPVTGLGTPDFAKLKALVLDA | Aspergillus nidulans FGSC A4 |
| 21 | MLSFVRRGALSLALVSLLTSSVAAEVFEKLHVVPEGWRYASTPNP KQPIRLQIALQQHDVTGFEQSLLEMSTPDHPNYGKHFRTHDEMKR MLLPNENAVHAVREWLQDAGISDIEEDADWVRFHTTVDQANDLLD ANFLWYAHKSHRNTARLRTLEYSIPDSIAPQVNVIQPTTRFGQIRAN RATHSSKPKGGLDELAISQAATADDDSICDQITTPHCLRKLYNVNG YKADPASGSKIGFASFLEEYARYSDLVLFEENLAPFAEGENFTVVM YNGGKNDQNSKSDSGEANLDLQYIVGMSAGAPVTEFSTAGRAPVI PDLDQPDPSAGTNEPYLEFLQNVLHMDQEHLPQVISTSYGENEQT IPEKYARTVCNMYAQLGSRGVSVIFSSGDSGVGSACMTNDGTNRT HFPPQFPASCPWVTSVGATEKMAPEQATYFSSGGFSDLFPRPKY QDAAVSSYLQTLGSRYQGLYNGSNRAFPDVSAQGTNFAVYDKGR LGQFDGTSCSAPAFSGIIALLNDVRLQNNKPVLGFLNPWLYGAGSK GLNDVVHGGSTGCDGQERFAGKANGSPVVPYASWNATQGWDP VTGLGTPDFGKLKDLALSA | Aspergillus ruber CBS 135680 |

(continued)

| SEQ ID No.: | Sequence | Origin |
|---|---|---|
| 22 | MLPSLVNNGALSLAVLSLLTSSVAGEVFEKLSAVPKGWHFSHAAQ ADAPINLKIALKQHDVEGFEQALLDMSTPGHENYGKHFHEHDEMK RMLLPSDSAVDAVQTWLTSAGITDYDLDADWINLRTTVEHANALLD TQFGWYENEVRHITRLRTLQYSIPETVAAHINMVQPTTRFGQIRPD RATFHAHHTSDARILSALAAASNSTSCDSVITPKCLKDLYKVGDYE ADPDSGSQVAFASYLEEYARYADMVKFQNSLAPYAKGQNFSVVL YNGGVNDQSSSADSGEANLDLQTIMGLSAPLPITEYITGGRGKLIP DLSQPNPDNSNEPYLEFLQNILKLDQDELPQVISTSYGEDEQTIP RGYAESVCNMLAQLGSRGVSVVFSSGDSGVGAACQTNDGRNQT HFNPQFPASCPWVTSVGATTKTNPEQAVYFSSGGFSDFWKRPKY QDEAVAAYLDTLGDKFAGLFNKGGRAFPDVAAQGMNYAIYDKGTL GRLDGTSCSAPAFSAIISLLNDARLREGKPTMGFLNPWLYGEGRE ALNDVVVGGSKGCDGRDRFGGKPNGSPVVPFASWNATQGWDPV TGLGTPNFAKMLELAP | Aspergillus terreus NIH2624 |
| 23 | MIASLFNRRALTLALLSLFASSATADVFESLSAVPQGWRYSRTPSA NQPLKLQIALAQGDVAGFEAAVIDMSTPDHPSYGNHFNTHEEMKR MLQPSAESVDSIRNWLESAGISKIEQDADWMTFYTTVKTANELLAA NFQFYINGVKKIERLRTLKYSVPDALVSHINMIQPTTRFGQLRAQRA ILHTEVKDNDEAFRSNAMSANPDCNSIITPQCLKDLYSIGDYEADPT NGNKVAFASYLEEYARYSDLALFEKNIAPFAKGQNFSVVQYNGGG NDQQSSSGSSEANLDLQYIVGVSSPVPVTEFSTGGRGELVPDLDQ PNPNDNNNEPYLEFLQNVLKLHKKDLPQVISTSYGEDEQSVPEKY ARAVCNLYSQLGSRGVSVIFSSGDSGVGAACQTNDGRNATHFPP QFPAACPWVTSVGATTHTAPERAVYFSSGGFSDLWDRPTWQEDA VSEYLENLGDRWSGLFNKGRAFPDVAAQGENYAIYDKGSLISVD GTSCSAPAFAGVIALLNDARIKANRPPMGFLNPWLYSEGRSGLNDI VNGGSTGCDGHGRFSGPTNGGTSIPGASWNATKGWDPVSGLGS PNFAAMRKLANAE | Penicillium digitatum Pd1 |
| 24 | MHVPLLNQGALSLAVVSLLASTVSAEVFDKLVAVPEGWRFSRTPS GDQPIRLQVALTQGDVEGFEKAVLDMSTPDHPNYGKHFKSHEEVK RMLQPAGESVEAIHQWLEKAGITHIQQDADWMTFYTTVEKANNLL DANFQYYLNENKQVERLRTLEYSVPDELVSHINLVTPTTRFGQLHA EGVTLHGKSKDVDEQFRQAATSPSSDCNSAITPQCLKDLYKVGDY KASASNGNKVAFTSYLEQYARYSDLALFEQNIAPYAQGQNFTVIQY NGGLNDQSSPADSSEANLDLQYIIGTSSPVPVTEFSTGGRGPLVP DLDQPDINDNNNEPYLDFLQNVIKMSDKDLPQVISTSYGEDEQSVP ASYARSVCNLIAQLGGRGVSVIFSSGDSGVGSACQTNDGKNTTRF PAQFPAACPWVTSVGATTGISPERGVFFSSGGFSDLWSRPSWQS HAVKAYLHKLGKRQDGLFNREGRAFPDVSAQGENYAIYAKGRLGK VDGTSCSAPAFAGLVSLLNDARIKAGKSSLGFLNPWLYSHPDALN DITVGGSTGCDGNARFGGRPNGSPVVPYASWNATEGWDPVTGL GTPNFQKLLKSAVKQK | Penicillium oxalicum 114-2 |

(continued)

| SEQ ID No.: | Sequence | Origin |
|---|---|---|
| 25 | MIASLFSRGALSLAVLSLLASSAAADVFESLSAVPQGWRYSRRPRA DQPLKLQIALTQGDTAGFEEAVMEMSTPDHPSYGHHFTTHEEMKR MLQPSAESAESIRDWLEGAGITRIEQDADWMTFYTTVETANELLAA NFQFYVSNVRHIERLRTLKYSVPKALVPHINMIQPTTRFGQLRAHR GILHGQVKESDEAFRSNAVSAQPDCNSIITPQCLKDIYNIGDYQAN DTNGNKVGFASYLEEYARYSDLALFEKNIAPSAKGQNFSVTRYNG GLNDQSSSGSSSEANLDLQYIVGVSSPVPVTEFSVGGRGELVPDL DQPDPNDNNNEPYLEFLQNVLKLDKKDLPQVISTSYGEDEQSIPEK YARSVCNLYSQLGSRGVSVIFSSGDSGVGSACLTNDGRNATRFPP QFPAACPWVTSVGATTHTAPEQAVYFSSGGFSDLWARPKWQEE AVSEYLEILGNRWSGLFNPKGRAFPDVTAQGRNYAIYDKGSLTSV DGTSCSAPAFAGVVALLNDARLKVNKPPMGFLNPWLYSTGRAGL KDIVDGGSTGCDGKSRFGGANNGGPSIPGASWNATKGWDPVSG LGSPNFATMRKLANAE | Penicillium roqueforti FM164 |
| 26 | MIASLFNRGALSLAVLSLLASSASADVFESLSAVPQGWRYSRRPR ADQPLKLQIALAQGDTAGFEEAVMDMSTPDHPSYGNHFHTHEEM KRMLQPSAESADSIRDWLESAGINRIEQDADWMTFYTTVETANELL AANFQFYANSAKHIERLRTLQYSVPEALMPHINMIQPTTRFGQLRV QGAILHTQVKETDEAFRSNAVSTSPDCNSIITPQCLKNMYNVGDYQ ADDDNGNKVGFASYLEEYARYSDLELFEKNVAPFAKGQNFSVIQY NGGLNDQHSSASSSEANLDLQYIVGVSSPVPVTEFSVGGRGELVP DLDQPDPNDNNNEPYLEFLQNVLKMEQQDLPQVISTSYGENEQSV PEKYARTVCNLFSQLGSRGVSVIFASGDSGVGAACQTNDGRNAT RFPAQFPAACPWVTSVGATTHTAPEKAVYFSSGGFSDLWDRPKW QEDAVSDYLDTLGDRWSGLFNPKGRAFPDVSAQGGQNYAIYDKGS LTSVDGTSCSAPAFAGVIALLNDARLKANKPPMGFLNPWLYSTGR DGLNDIVHGGSTGCDGNARFGGPGNGSPRVPGASWNATKGWDP VSGLGSPNFATMRKLANGE | Penicillium rubens Wisconsin 54-1255 |
| 27 | MLSSTLYAGLLCSLAAPALGVVHEKLSAVPSGWTLVEDASESDTTT LSIALARQNLDQLESKLTTLATPGNAEYGKWLDQSDIESLFPTASD DAVIQWLKDAGVTQVSRQGSLVNFATTVGTANKLFDTKFSYYRNG ASQKLRTTQYSIPDSLTESIDLIAPTVFFGKEQDSALPPHAVKLPAL PRRAATNSSCANLITPDCLVEMYNLGDYKPDASSGSRVGFGSFLN QSANYADLAAYEQLFNIPPQNFSVELINGGANDQNWATASLGEAN LDVELIVAVSHALPVVEFITGGSPPFVPNVDEPTAADNQNEPYLQY YEYLLSKPNSHLPQVISNSYGDDEQTVPEYYARRVCNLIGLMGLR GITVLESSGDTGIGSACMSNDGTNTPQFTPTFPGTCPFITAVGGTQ SYAPEVAWDASSGGFSNYFSRPWYQYFAVENYLNNHITKDTKKY YSQYTNFKGRGFPDVSAHSLTPDYEVVLTGKHYKSGGTSAACPVF AGIVGLLNDARLRAGKSTLGFLNPLLYSILAEGFTDITAGSSIGCNGI NPQTGKPVPGGGIIPYAHWNATAGWDPVTGLGVPDFMKLKELVLS L | Neosartorya fischeri NRRL 181 |

(continued)

| SEQ ID No.: | Sequence | Origin |
|---|---|---|
| 28 | <u>MLSSTLYAGWLLSLAAPALC</u>VVQEKLSAVPSGWTLIEDASESDTIT LSIALARQNLDQLESKLTTLATPGNPEYGKWLDQSDIESLFPTASD DAVLQWLKAAGITQVSRQGSLVNFATTVGTANKLFDTKFSYYRNG ASQKLRTTQYSIPDHLTESIDLIAPTVFFGKEQNSALSSHAVKLPAL PRRAATNSSCANLITPDCLVEMYNLGDYKPDASSGSRVGFGSFLN ESANYADLAAYEQLFNIPPQNFSVELINRGVNDQNWATASLGEAN LDVELIVAVSHPLPVVEFITGALPPVLRVLALQTQLPSSSGDFQLTV PEYYARRVCNLIGLMGLRGITVLESSGDTGIGSACMSNDGTNKPQ FTPTFPGTCPFITAVGGTQSYAPEVAWDGSSGGFSNYFSRPWYQ SFAVDNYLNNHITKDTKKYYSQYTNFKGRGFPDVSAHSLTPYYEV VLTGKHYKSGGTSAASPVFAGIVGLLNDARLRAGKSTLGFLNPLLY SILAEGFTDITAGSSIGCNGINPQTGKPVPGGGIIPYAHWNATAGW DPVTGLGVPDFMKLKELVLSL | Aspergillus fumigatus CAE17675 |
| 29 | QEPSSCKGTLVFEGETFNVFQPDCLRTEYSVDGYTPSVKSGSRIG FGSFLNESASFADQALFEKHFNIPSQNFSVVLINGGTDLPQPPSDA NDGEANLDAQTILTIAHPLPITEFITAGSPPYFPDPVEPAGTPNENE PYLQYYEFLLSKSNAEIPQVITNSYGDEEQTVPRSYAVRVCNLIGLL GLRGISVLHSSGDEGVGASCVATNSTTPQFNPIFPATCPYVTSVG GTVSFNPEVAWAGSSGGFSYYFSRPWYQQEAVGTYLEKYVSAET KKYYGPYVDFSGRGFPDVAAHSVSPDYPVFQGGELTPSGGTSAA SPVVAAIVALLNDARLREGKPTLGFLNPLIYLHASKGFTDITSGQSE GCNGNNTQTGSPLPGAGFIAGAHWNATKGWDPTTGFGVPNLKKL LALVRF | Trichoderma reesei QM6a |
| 30 | CDSIITPTCLKELYNIGDYQADANSGSKIAFASYLEEYARYADLENF ENYLAPWAKGQNFSVTTFNGGLNDQNSSSDSGEANLDLQYILGVS APLPVTEFSTGGRGPLVPDLTQPDPNSNSNEPYLEFFQNVLKLDQ KDLPQVISTSYGENEQEIPEKYARTVCNLIAQLGSRGVSVLFSSGD SGVGEGCMTNDGTNRTHFPPQFPAACPWVTSVGATFKTTPERGT YFSSGGFSDYWPRPEWQDEAVSSYLETIGDTFKGLYNSSGRAFP DVAAQGMNFAVYDKGTLGEFDGTSASAPAFSAVIALLNDARLRAG KPTLGFLNPWLYKTGRQGLQDITLGASIGCTGRARFGGAPDGGPV VPYASWNATQGWDPVTGLTPDFAELKKLA | Aspergillus oryzae RIB40 |
| 31 | CDATITPQCLKTLYKIDYKADPKSGSKVAFASYLEQYARYNDLALFE KAFLPEAVGQNFSVVQFSGGLNDQNTTQDSGEANLDLQYIVGVSA PLPVTEFSTGGRGPWVADLDQPDEADSANEPYLEFLQGVLKLPQS ELPQVISTSYGENEQSVPKSYALSVCNLFAQLGSRGVSVIFSSGDS GPGSACQSNDGKNTTKFQPQYPAACPFVTSVGSTRYLNETATGF SSGGFSDYWKRPSYQDDAVKAYFHHLGEKFKPYFNRHGRGFPDV ATQGYGFRVYDQGKLKGLQGTSASAPAFAGVIGLLNDARLKAKKP TLGELNPLLYSNSDALNDIVLGGSKGCDGHARENGPPNGSPVIPYA GWNATAGWDPVTGLTPNFPKLLKAA | Phaeosphaeria nodorum SN15 |

(continued)

| SEQ ID No.: | Sequence | Origin |
|---|---|---|
| 32 | VFQPDCLRTEYSVNGYKPSAKSGSRIGFGSFLNQSASSSDLALFE KHFGFASQGFSVELINGGSNPQPPTDANDGEANLDAQNIVSFVQP LPITEFIAGGTAPYFPDPVEPAGTPDENEPYLEYYEYLLSKSNKELP QVITNSYGDEEQTVPQAYAVRVCNLIGLMGLRGISILESSGDEGVG ASCLATNSTTTPQFNPIFPATCPYVTSVGGTVSFNPEVAWDGSSG GFSYYFSRPWYQEAAVGTYLNKYVSEETKEYYKSYVDFSGRGFP DVAAHSVSPDYPVFQGGELTPSGGTSAASPIVASVIALLNDARLRA GKPALGFLNPLIYGYAYKGFTDITSGQAVGCNGNNTQTGGPLPGA GVIPGAFWNATKGWDPTTGFGVPNFKKLLELV | Trichoderma atroviride IMI 206040 |
| 33 | CRSLVTTACLRELYGLGDRVTQARDDNRIGVSGFLEEYAQYRDLE LFLSRFEPSAKGFNFSEGLIAGGKNTQGGPGSSTEANLDMQYVVG LSHKAKVTYYSTAGRGPLIPDLSQPSQASNNNEPYLEQLRYLVKLP KNQLPSVLTTSYGDTEQSLPASYTKATCDLFAQLGTMGVSVIFSSG DTGPGSSCQTNDGKNATRFNPIYPASCPFVTSIGGTVGTGPERAV SFSSGGFSDRFPRPQYQDNAVKDYLKILGNQWSGLFDPNGRAFP DIAAQGSNYAVYDKGRMTGVSGTSASAPAMAAIIAQLNDFRLAKG SPVLGFLNPWIYSKGFSGFTDIVDGGSRGCTGYDIYSGLKAKKVPY ASWNATKGWDPVTGFGTPNFQALTKVL | Arthroderma benhamiae CBS 112371 |
| 34 | CQTSITPSCLKQMYNIGDYTPKVESGSTIGFSSFLGESAIYSDVFLF EEKFGIPTQNFTTVLINNGTDDQNTAHKNFGEADLDAENIVGIAHPL PFTQYITGGSPPFLPNIDQPTAADNQNEPYVPFFRYLLSQKEVPAV VSTSYGDEEDSVPREYATMTCNLIGLLGLRGISVIFSSGDIGVGAG CLGPDHKTVEFNAIFPATCPYLTSVGGTVDVTPEIAWEGSSGGFSK YFPRPSYQDKAVKTYMKTVSKQTKKYYGPYTNWEGRGFPDVAGH SVSPNYEVIYAGKQSASGGTSAAAPVWAAIVGLLNDARFRAGKPS LGWLNPLVYKYGPKVLTDITGGYAIGCDGNNTQSGKPEPAGSGIV PGARWNATAGWDPVTGYGTPDFGKLKDLVLS | Fusarium graminearum PH-1 |
| 35 | CDLVITPPCLEAAYNYKNYMPDPNSGSRVSFTSFLEQAAQQSDLT KFLSLTGLDRLRPPSSKPASFDTVLINGGETHQGTPPNKTSEANLD VQWLAAVIKARLPITQWITGGRPPFVPNLRLRHEKDNTNEPYLEFF EYLVRLPARDLPQVISNSYAEDEQTVPEAYARRVCNLIGIMGLRGV TVLTASGDSGVGAPCRANDGSDRLEFSPQFPTSCPYITAVGGTEG WDPEVAWEASSGGFSHYFLRPWYQANAVEKYLDEELDPATRAYY DGNGFVQFAGRAYPDLSAHSSSPRYAYIDKLAPGLTGGTSASCPV VAGIVGLLNDARLRRGLPTMGFINPWLYTRGFEALQDVTGGRASG CQGIDLQRGTRVPGAGIIPWASWNATPGWDPATGLGLPDFWAMR GL | Acremonium alcalophilum |
| 36 | CATIITPPCLETAYNYKGYIPDPKSGSRVSFTSFLEQAAQQADLTKF LSLTRLEGFRTPASKKKTFKTVLINGGESHEGVHKKSKTSEANLDV QWLAAVTQTKLPITQWITGGRPPFVPNLRIPTPEANTNEPYLEFLE YLFRLPDKDLPQVISNSYAEDEQSVPEAYARRVCGLLGIMGLRGVT VLTASGDSGVGAPCRANDGSGREEFSPQFPSSCPYITTVGGTQA WDPEVAWKGSSGGFSNYFPRPWYQVAAVEKYLEEQLDPAAREY YEENGFVRFAGRAFPDLSAHSSSPKYAYVDKRVPGLTGGTSASCP VVAGIVGLLNDARLRRGLPTMGFINPWLYAKGYQALEDVTGGAAV GCQGIDIQTGKRVPGAGIIPGASWNATPDWDPATGLGLPNFWAMR ELA | Sodiomyces alkalinus |

(continued)

| SEQ ID No.: | Sequence | Origin |
|---|---|---|
| 37 | CADTITLSCLKEMYNFGNYTPSASSGSKLGFASFLNESASYSDLAK FERLFNLPSQNFSVELINGGVNDQNQSTASLTEADLDVELLVGVG HPLPVTEFITSGEPPFIPDPDEPSAADNENEPYLQYYEYLLSKPNSA LPQVISNSYGDDEQTVPEYYAKRVCNLIGLVGLRGISVLESSGDEGI GSGCRTTDGTNSTQFNPIFPATCPYVTAVGGTMSYAPEIAWEASS GGFSNYFERAWFQKEAVQNYLANHITNETKQYYSQFANFSGRGF PDVSAHSFEPSYEVIFYGARYGSGGTSAACPLFSALVGMLNDARL RAGKSTLGFLNPLLYSKGYKALTDVTAGQSIGCNGIDPQSDEAVAG AGIIPWAHWNATVGWDPVTGLGLPDFEKLRQLVLS | Aspergillus kawachii IFO 4308 |
| 38 | CQTSITPACLKQMYNVGNYTPSVAHGSRVGFGSFLNQSAIFDDLF TYEKVNDIPSQNFTKVIIANASNSQDASDGNYGEANLDVQNIVGISH PLPVTEFLTGGSPPFVASLDTPTNQNEPYIPYYEYLLSQKNEDLPQ VISNSYGDDEQSVPYKYAIRACNLIGLTGLRGISVLESSGDLGVGA GCRSNDGKNKTQFDPIFPATCPYVTSVGGTQSVTPEIAWVASSGG FSNYFPRTWYQEPAIQTYLGLLDDETKTYYSQYTNFEGRGFPDVS AHSLTPDYQVVGGGYLQPSGGTSAASPVFAGIIALLNDARLAAGKP TLGFLNPFFYLYGYKGLNDITGGQSVGCNGINGQTGAPVPGGGIV PGAAWNSTTGWDPATGLGTPDFQKLKELVLS | Talaromyces stipitatus ATCC 10500 |
| 39 | CQTSITPSCLKQMYNIGDYTPDAKSGSEIGFSSFLGQAAIYSDVFKF EELFGIPKQNYTTILINNGTDDQNTAHGNFGEANLDAENIVGIAHPL PFKQYITGGSPPFVPNIDQPTEKDNQNEPYVPFFRYLLGQKDLPAV ISTSYGDEEDSVPREYATLTCNMIGLLGLRGISVIFSSGDIGVGSGC LAPDYKTVEFNAIFPATCPYLTSVGGTVDVTPEIAWEGSSGGFSKY FPRPSYQDKAIKKYMKTVSKETKKYYGPYTNWEGRGFPDVAGHS VAPDYEVIYNGKQARSGGTSAAAPVWAAIVGLLNDARFKAGKKSL GWLNPLIYKHGPKVLTDITGGYAIGCDGNNTQSGKPEPAGSGLVP GARWNATAGWDPTTGYGTPNFQKLKDLVLS | Fusarium oxysporum f. sp. cubense race 4 |
| 40 | VFQPDCLRTEYNVNGYTPSAKSGSRIGFGSFLNQSASFSDLALFE KHFGFSSQNFSVVLINGGTDLPQPPSDDNDGEANLDVQNILTIAHP LPITEFITAGSPPYFPDPVEPAGTPDENEPYLQYFEYLLSKPNRDLP QVITNSYGDEEQTVPQAYAVRVCNLIGLMGLRGISILESSGDEGVG ASCVATNSTTPQFNPIFPATCPYVTSVGGTVNFNPEVAWDGSSGG FSYYFSRPWYQEEAVGNYLEKHVSAETKKYYGPYVDFSGRGFPD VAAHSVSPDYPVFQGGQLTPSGGTSAASPVVASIIALLNDARLREG KPTLGFLNPLIYQYAYKGFTDITSGQSDGCNGNNTQTDAPLPGAG VVLGAHWNATKGWDPTTGFGVPNFKKLLELI | Trichoderma virens Gv29-8 |
| 41 | QIFHPDCLKTKYGVDGYAPSPRCGSRIGFGSFLNETASYSDLAQFE KYFDLPNQNLSTLLINGAIDVQPPSNKNDSEANMDVQTILTFVQPLP ITEFVVAGIPPYIPDAALPIGDPVQNEPWLEYFEFLMSRTNAELPQVI ANSYGDEEQTVPQAYAVRVCNQIGLLGLRGISVIASSGDTGVGMS CMASNSTTPQFNPMFPASCPYITTVGGTQHLDNEIAWELSSGGFS NYFTRPWYQEDAAKTYLERHVSTETKAYYERYANFLGRGFPDVAA LSLNPDYPVIGGELGPNGGTSAAAPVVASIIALLNDARLCLGKPAL GFLNPLIYQYADKGGFTDITSGQSWGCAGNTTQTGPPPPGAGVIP GAHWNATKGWDPVTGFGTPNFKKLLSLALS | Trichoderma atroviride IMI 206040 |

(continued)

| SEQ ID No.: | Sequence | Origin |
|---|---|---|
| 42 | TVITPDCLRDLYNTADYVPSATSRNAIGIAGYLDRSNRADLQTFFRR FRPDAVGFNYTTVQLNGGGDDQNDPGVEANLDIQYAAGIAFPTPA TYWSTGGSPPFIPDTQTPTNTNEPYLDWINFVLGQDEIPQVISTSY GDDEQTVPEDYATSVCNLFAQLGSRGVTVFFSSGDFGVGGGDCL TNDGSNQVLFQPAFPASCPFTAVGGTVRLDPEIAVSFSGGGFSR YFSRPSYQNQTVAQFVSNLGNTFNGLYNKNGRAYPDLAAQGNGF QVVIDGIVRSVGGTSASSPTVAGIFALLNDFKLSRGQSTLGFINPLIY SSATSGFNDIRAGTNPCGTRGFTAGTGWDPVTGLGTPDFLRLQ | Agaricus bisporus var. burnettii JB137-S8 |
| 43 | GVTPLCLRTLYRVNYKPATTGNLVAFASFLEQYARYSDQQAFTQR VLGPGVPLQNFSVETVNGGANDQQSKLDSGEANLDLQYVMAMSH PIPILEYSTGGRGPLVPTLDQPNANNSSNEPYLEFLTYLLAQPDSAI PQTLSVSYGEEEQSVPRDYAIKVCNMFMQLGARGVSVMFSSGDS GPGNDCVRASDNATFFGSTFPAGCPYVTSVGSTVGFEPERAVSF SSGGFSIYHARPDYQNEVVPKYIESIKASGYEKFFDGNGRGIPDVA AQGARFVVIDKGRVSLISGTSASSPAFAGMVALVNAARKSKDMPA LGFLNPMLYQNAAAMTDIVNGAGIGCRKQRTEFPNGARFNATAG WDPVTGLGTPLFDKLLA | Magnaporthe oryzae 70-15 |
| 44 | CNASITPECLRALYNVGDYEADPSKKSLFGVCGYLEQYAKHDQLA KFEQTYAPYAIGADFSVVTINGGGDNQTSTIDDGEANLDMQYAVS MAYKTPITYYSTGGRGPLVPDLDQPDPNDVSNEPYLDFVSYLLKLP DSKLPQTITTSYGEDEQSVPRSYVEKVCTMFGALGARGVSVIFSS GDTGVGSACQTNDGKNTTRFLPIFPAACPYVTSVGGTRYVDPEVA VSFSSGGFSDIFPTPLYQKGAVSGYLKILGDRWKGLYNPHGRGFP DVSGQSVRYHVFDYGKDVMYSGTSASAPMFAALVSLLNNARLAK KLPPMGFLNPWLYTVGFNGLTDIVHGGSTGCTGTDVYSGLPTPFV PYASWNATVGWDPVTGLGTPLFDKLLNL | Togninia minima UCRPA7 |
| 45 | CNKKITPDCLANLYNFKDYDASDANVTIGVSGFLEQYARFDDLKQF ISTFQPKAAGSTFQVTSVNAGPFDQNSTASSVEANLDIQYTTGLVA PDIETRYFTVPGRGILIPDLDQPTESDNANEPYLDYFTYLNNLEDEE LPDVLTTSYGESEQSVPAEYAKKVCNLIGQLGARGVSVIFSSGDTG PGSACQTNDGKNTTRFLPIFPASCPYVTSVGGTVGVEPEKAVSFS SGGFSDLWPRPAYQEKAVSEYLEKLGDRWNGLYNPQGRGFPDV AAQGQGFQVFDKGRLISVGGTSASAPVFASVVALLNNARKAAGMS SLGFLNPWIYEQGYKGLTDIVAGGSTGCTGRSIYSGLPAPLVPYAS WNATEGWDPVTGYGTPDFKQLLTLAT | Bipolaris maydis C5 |
| 46 | CDSIITPHCLKQLYNIGDYQADPKSGSKVGFASYLEEYARYADLER FEQHLAPNAIGQNFSVVQFNGGLNDQLSLSDSGEANLDLQYILGV SAPVPVTEYSTGGRGELVPDLSSPDPNDNSNEPYLDFLQGILKLD NSDLPQVISTSYGEDEQTIPVPYARTVCNLYAQLGSRGVSVIFSSG DSGVGAACLTNDGTNRTHFPPQFPASCPWVTSVGATSKTSPEQA VSFSSGGFSDLWPRPSYQQAAVQTYLTQHLGNKFSGLFNASGRA FPDVAAQGVNYAVYDKGMLGQFDGTSCSAPTFSGVIALLNDARLR AGLPVMGFLNPFLYGVGSESGALNDIVNGGSLGCDGRNRFGGTP NGSPVVPFASWNATTGWDPVSGLGTPDFAKLRGV | Aspergillus kawachii IFO 4308 |

(continued)

| SEQ ID No.: | Sequence | Origin |
|---|---|---|
| 47 | CEKAITPSCLADLYNTEGYKASNRSGSKVAFASFLEEYARYDDLAE FEETYAPYAIGQNFSVISINGGLNDQDSTADSGEANLDLQYIIGVSS PLPVTEFTTGGRGKLIPDLSSPDPNDNTNEPFLDFLEAVLKLDQKD LPQVISTSYGEDEQTIPEPYARSVCNLYAQLGSRGVSVLFSSGDSG VGAACQTNDGKNTTHFPPQFPASCPWVTAVGGTNGTAPESGVYF SSGGFSDYWARPAYQNAAVESYLRKLGSTQAQYFNRSGRAFPDV AAQAQNFAVVDKGRVGLFDGTSCSSPVFAGIVALLNDVRLKAGLP VLGFLNPWLYQDGLNGLNDIVDGGSTGCDGNNRFNGSPNGSPVI PYAGWNATEGWDPVTGLGTPDFAKLKALVL | Aspergillus nidulans FGSC A4 |
| 48 | CDQITTPHCLRKLYNVNGYKADPASGSKIGFASFLEEYARYSDLVL FEENLAPFAEGENFTVVMYNGGKNDQNSKSDSGEANLDLQYIVG MSAGAPVTEFSTAGRAPVIPDLDQPDPSAGTNEPYLEFLQNVLHM DQEHLPQVISTSYGENEQTIPEKYARTVCNMYAQLGSRGVSVIFSS GDSGVGSACMTNDGTNRTHFPPQFPASCPWVTSVGATEKMAPE QATYFSSGGFSDLFPRPKYQDAAVSSYLQTLGSRYQGLYNGSNR AFPDVSAQGTNFAVYDKGRLGQFDGTSCSAPAFSGIIALLNDVRLQ NNKPVLGFLNPWLYGAGSKGLNDVVHGGSTGCDGQERFAGKAN GSPVVPYASWNATQGWDPVTGLGTPDFGKLKDLAL | Aspergillus ruber CBS 135680 |
| 49 | CDSVITPKCLKDLYKVGDYEADPDSGSQVAFASYLEEYARYADMV KFQNSLAPYAKGQNFSVVLYNGGVNDQSSSADSGEANLDLQTIM GLSAPLPITEYITGGRGKLIPDLSQPNPNDNSNEPYLEFLQNILKLD QDELPQVISTSYGEDEQTIPRGYAESVCNMLAQLGSRGVSVVFSS GDSGVGAACQTNDGRNQTHFNPQFPASCPWVTSVGATTKTNPE QAVYFSSGGFSDFWKRPKYQDEAVAAYLDTLGDKFAGLFNKGGR AFPDVAAQGMNYAIYDKGTLGRLDGTSCSAPAFSAIISLLNDARLR EGKPTMGFLNPWLYGEGREALNDVVVGGSKGCDGRDRFGGKPN GSPVVPFASWNATQGWDPVTGLGTPNFAKMLELA | Aspergillus terreus NIH2624 |
| 50 | CNSIITPQCLKDLYSIGDYEADPTNGNKVAFASYLEEYARYSDLALF EKNIAPFAKGQNFSVVQYNGGGNDQQSSSGSSEANLDLQYIVGVS SPVPVTEFSTGGRGELVPDLDQPNPNDNNNEPYLEFLQNVLKLHK KDLPQVISTSYGEDEQSVPEKYARAVCNLYSQLGSRGVSVIFSSG DSGVGAACQTNDGRNATHFPPQFPAACPWVTSVGATTHTAPERA VYFSSGGFSDLWDRPTWQEDAVSEYLENLGDRWSGLFNPKGRA FPDVAAQGENYAIYDKGSLISVDGTSCSAPAFAGVIALLNDARIKAN RPPMGFLNPWLYSEGRSGLNDIVNGGSTGCDGHGRFSGPTNGG TSIPGASWNATKGWDPVSGLGSPNFAAMRKLA | Penicillium digitatum Pd1 |
| 51 | CNSAITPQCLKDLYKVGDYKASASNGNKVAFTSYLEQYARYSDLAL FEQNIAPYAQGQNFTVIQYNGGLNDQSSPADSSEANLDLQYIIGTS SPVPVTEFSTGGRGPLVPDLDQPDINDNNNEPYLDFLQNVIKMSD KDLPQVISTSYGEDEQSVPASYARSVCNLIAQLGGRGVSVIFSSGD SGVGSACQTNDGKNTTRFPAQFPAACPWVTSVGATTGISPERGV FFSSGGFSDLWSRPSWQSHAVKAYLHKLGKRQDGLFNREGRAFP DVSAQGENYAIYAKGRLGKVDGTSCSAPAFAGLVSLLNDARIKAG KSSLGFLNPWLYSHPDALNDITVGGSTGCDGNARFGGRPNGSPV VPYASWNATEGWDPVTGLGTPNFQKLLKSAV | Penicillium oxalicum 114-2 |

(continued)

| SEQ ID No.: | Sequence | Origin |
|---|---|---|
| 52 | CNSIITPQCLKDIYNIGDYQANDTNGNKVGFASYLEEYARYSDLALF EKNIAPSAKGQNFSVTRYNGGLNDQSSSGSSSEANLDLQYIVGVS SPVPVTEFSVGGRGELVPDLDQPDPNDNNNEPYLEFLQNVLKLDK KDLPQVISTSYGEDEQSIPEKYARSVCNLYSQLGSRGVSVIFSSGD SGVGSACLTNDGRNATRFPPQFPAACPWVTSVGATTHTAPEQAV YFSSGGFSDLWARPKWQEEAVSEYLEILGNRWSGLFNPKGRAFP DVTAQGRNYAIYDKGSLTSVDGTSCSAPAFAGVVALLNDARLKVN KPPMGFLNPWLYSTGRAGLKDIVDGGSTGCDGKSRFGGANNGG PSIPGASWNATKGWDPVSGLGSPNFATMRKLA | Penicillium roqueforti FM164 |
| 53 | CNSIITPQCLKNMYNVGDYQADDDNGNKVGFASYLEEYARYSDLE LFEKNVAPFAKGQNFSVIQYNGGLNDQHSSASSSEANLDLQYIVG VSSPVPVTEFSVGGRGELVPDLDQPDPNDNNNEPYLEFLQNVLK MEQQDLPQVISTSYGENEQSVPEKYARTVCNLFSQLGSRGVSVIF ASGDSGVGAACQTNDGRNATRFPAQFPAACPWVTSVGATTHTAP EKAVYFSSGGFSDLWDRPKWQEDAVSDYLDTLGDRWSGLFNPK GRAFPDVSAQGQNYAIYDKGSLTSVDGTSCSAPAFAGVIALLNDA RLKANKPPMGFLNPWLYSTGRDGLNDIVHGGSTGCDGNARFGGP GNGSPRVPGASWNATKGWDPVSGLGSPNFATMRKLA | Penicillium rubens Wisconsin 54-1255 |
| 54 | CANLITPDCLVEMYNLGDYKPDASSGSRVGFGSFLNQSANYADLA AYEQLFNIPPQNFSVELINGGANDQNWATASLGEANLDVELIVAVS HALPVVEFITGGSPPFVPNVDEPTAADNQNEPYLQYYEYLLSKPNS HLPQVISNSYGDDEQTVPEYYARRVCNLIGLMGLRGITVLESSGDT GIGSACMSNDGTNTPQFTPTFPGTCPFITAVGGTQSYAPEVAWDA SSGGFSNYFSRPWYQYFAVENYLNNHITKDTKKYYSQYTNFKGR GFPDVSAHSLTPDYEVVLTGKHYKSGGTSAACPVFAGIVGLLNDA RLRAGKSTLGFLNPLLYSILAEGFTDITAGSSIGCNGINPQTGKPVP GGGIIPYAHWNATAGWDPVTGLGPDFMKLKELVLS | Neosartorya fischeri NRRL 181 |
| 55 | CANLITPDCLVEMYNLGDYKPDASSGSRVGFGSFLNESANYADLA AYEQLFNIPPQNFSVELINRGVNDQNWATASLGEANLDVELIVAVS HPLPVVEFITGALPPVLRVLALQTQLPSSSGDFQLTVPEYYARRVC NLIGLMGLRGITVLESSGDTGIGSACMSNDGTNKPQFTPTFPGTCP FITAVGGTQSYAPEVAWDGSSGGFSNYFSRPWYQSFAVDNYLNN HITKDTKKYYSQYTNFKGRGFPDVSAHSLTPYYEVVLTGKHYKSG GTSAASPVFAGIVGLLNDARLRAGKSTLGFLNPLLYSILAEGFTDIT AGSSIGCNGINPQTGKPVPGGGIIPYAHWNATAGWDPVTGLGVPD FMKLKELVLS | Aspergillus fumigatus CAE17675 |

(continued)

| SEQ ID No.: | Sequence | Origin |
|---|---|---|
| 56 | ATGGCAAAGTTGAGCACTCTCCGGCTTGCGAGCCTTCTTTCCCT TGTCAGTGTGCAGGTATCTGCCTCTGTCCATCTATTGGAGAGTC TGGAGAAGCTGCCTCATGGATGGAAAGCAGCTGAAACCCCGAG CCCTTCGTCTCAAATCGTCTTGCAGGTTGCTCTGACGCAGCAGA ACATTGACCAGCTTGAATCGAGGCTCGCAGCTGTATCCACACC CACTTCTAGCACCTACGGCAAATACTTGGATGTAGACGAGATCA ACAGCATCTTCGCTCCAAGTGATGCTAGCAGTTCTGCCGTCGA GTCTTGGCTTCAGTCCCACGGAGTGACGAGTTACACCAAGCAA GGCAGCAGCATTTGGTTTCAAACAAACATCTCCACTGCAAATGC GATGCTCAGCACCAATTTCCACACGTACAGCGATCTCACCGGC GCGAAGAAGGTGCGCACTCTCAAGTACTCGATCCCGGAGAGCC TCATCGGCCATGTCGATCTCATCTCTCCCACGACCTATTTTGGC ACGACAAAGGCCATGAGGAAGTTGAAATCCAGTGGCGTGAGCC CAGCCGCTGATGCTCTAGCCGCTCGCCAAGAACCTTCCAGCTG CAAAGGAACTCTAGTCTTTGAGGGAGAAACGTTCAATGTCTTTC AGCCAGACTGTCTCAGGACCGAGTATAGTGTTGATGGATACAC CCCGTCTGTCAAGTCTGGCAGCAGAATTGGGTTTGGTTCCTTTC TCAATGAGAGCGCAAGCTTCGCAGATCAAGCACTCTTTGAGAA GCACTTCAACATCCCCAGTCAAAACTTCTCCGTTGTCCTGATCA ACGGTGGAACGGATCTCCCTCAGCCGCCTTCTGACGCCAACGA TGGCGAAGCCAACCTGGACGCTCAAACCATTTTGACCATCGCA CATCCTCTCCCCATCACCGAATTCATCACCGCCGGCAGTCCGC CATACTTCCCCGATCCAGTTGAACCTGCGGGAACACCCAACGA GAACGAGCCTTATTTACAGTATTACGAATTTCTGTTGTCCAAGTC CAACGCTGAAATTCCGCAAGTCATTACCAACTCCTACGGCGAC GAGGAGCAAACTGTGCCGCGGTCATATGCCGTTCGAGTTTGCA ATCTGATTGGTCTGCTAGGACTACGCGGTATCTCTGTCCTTCAT TCCTCGGGCGACGAGGGTGTGGGCGCCTCTTGCGTTGCTACC AACAGCACCACGCCTCAGTTTAACCCCATCTTTCCTGTAGGTCT TCTACGTCAACACTTCCAGACAACCATTTTCTCCTACTAACCACT CTACCCTACTCTCTGTTCACATAGGCTACATGTCCTTATGTTACA AGTGTTGGCGGAACCGTGAGCTTCAATCCCGAGGTTGCCTGGG CTGGTTCATCTGGAGGTTTCAGCTACTACTTCTCTAGACCCTGG TACCAGCAGGAAGCTGTGGGTACTTACCTTGAGAAATATGTCAG TGCTGAGACAAAGAAATACTATGGACCTTATGTCGATTTCTCCG GACGAGGTTTCCCCGATGTTGCAGCCCACAGCGTCAGCCCCGA GTGAGTTCTATTCCTACCTATGCAAATCATAGAATGTATGCTAAC TCGCCATGAAGCTATCCTGTGTTTCAGGGCGGTGAACTCACCC CAAGCGGAGGCACTTCAGCAGCCTCTCCTGTCGTAGCAGCCAT CGTGGCGCTGTTGAACGATGCCCGTCTCCGCGAAGGAAAACCC ACGCTTGGATTTCTCAATCCGCTGATTTACCTACACGCCTCCAA AGGGTTCACCGACATCACCTCGGGCCAATCTGAAGGGTGCAAC GGCAATAACACCCAGACGGGCAGTCCTCTCCCAGGAGTATGCA GAACATCAAGAAGCCTTCTATCAGACGCCAATGCTAACTTGTGG ATAGGCCGGCTTCATTGCAGGCGCACACTGGAACGCGACCAAG GGATGGGACCCGACGACTGGATTTGGTGTTCCAAACCTCAAAA AGCTCCTCGCACTTGTCCGGTTCTAA | Trichoderma reesei QM6a |

(continued)

| SEQ ID No.: | Sequence | Origin |
|---|---|---|
| 57 | ATGTTCTTCAGTCGTGGAGCGCTTTCGCTCGCAGTGCTTTCACT GCTCAGCTCCTCCGCCGCAGGGGAGGCTTTTGAGAAGCTGTCT GCCGTTCCAAAGGGATGGCACTATTCTAGTACCCCTAAAGGCA ACACTGAGGTTTGTCTGAAGATCGCCCTCGCGCAGAAGGATGC TGCTGGGTTCGAAAAGACCGTCTTGGAGATGTCGGATCCCGAC CACCCCAGCTACGGCCAGCACTTCACCACCCACGACGAGATGA AGCGCATGCTTCTTCCCAGAGATGACACCGTTGATGCCGTTCG ACAATGGCTCGAAAACGGCGGCGTGACCGACTTTACCCAGGAT GCCGACTGGATCAACTTCTGTACTACCGTCGATACCGCGAACA AACTCTTGAATGCCCAGTTCAAATGGTACGTCAGCGATGTGAAG CACATCCGCCGTCTCAGAACACTGCAGTACGACGTCCCCGAGT CGGTCACCCCTCACATCAACACCATCCAACCGACCACCCGTTTT GGCAAGATTAGCCCCAAGAAGGCCGTTACCCACAGCAAGCCCT CCCAGTTGGACGTGACCGCCCTTGCTGCCGCTGTCGTTGCAAA GAACATCTCGCACTGTGATTCTATCATTACCCCCACCTGTCTGA AGGAGCTTTACAACATTGGTGATTACCAGGCCGATGCAAACTCG GGCAGCAAGATCGCCTTCGCCAGCTATCTGGAGGAGTACGCGC GCTACGCTGACCTGGAGAACTTTGAGAACTACCTTGCTCCCTG GGCTAAGGGCCAGAACTTCTCCGTTACCACCTTCAACGGCGGT CTCAATGATCAGAACTCCTCGTCCGATAGCGGTGAGGCCAACC TGGACCTGCAGTACATTCTTGGTGTCAGCGCTCCACTGCCCGT TACTGAATTCAGCACCGGAGGCCGTGGTCCCCTCGTTCCTGAT CTGACCCAGCCGGATCCCAACTCTAACAGCAATGAGCCGTACC TTGAGTTCTTCCAGAATGTGTTGAAGCTCGACCAGAAGGACCTC CCCCAGGTCATCTCGACCTCCTATGGAGAGAACGAACAGGAAA TCCCCGAAAGTACGCTCGCACCGTCTGCAACCTGATCGCTCA GCTTGGCAGCCGCGGTGTCTCCGTTCTCTTCTCCTCCGGTGAC TCTGGTGTTGGCGAGGGCTGCATGACCAACGACGGCACCAACC GGACTCACTTCCCACCCCAGTTCCCCGCCGCTTGCCCGTGGGT CACCTCCGTCGGCGCCACCTTCAAGACCACTCCCGAGCGCGG CACCTACTTCTCCTCGGGCGGTTTCTCCGACTACTGGCCCCGT CCCGAATGGCAGGATGAGGCCGTGAGCAGCTACCTCGAGACG ATCGGCGACACTTTCAAGGGCCTCTACAACTCCTCCGGCCGTG CTTTCCCCGACGTCGCAGCCCAGGGCATGAACTTCGCCGTCTA CGACAAGGGCACCTTGGGCGAGTTCGACGGCACCTCCGCCTC CGCCCCGGCCTTCAGCGCCGTCATCGCTCTCCTGAACGATGCC CGTCTCCGCGCCGGCAAGCCCACTCTCGGCTTCCTGAACCCCT GGTTGTACAAGACCGGCCGCCAGGGTCTGCAAGATATCACCCT CGGTGCTAGCATTGGCTGCACCGGTCGCGCTCGCTTCGGCGG CGCCCCTGACGGTGGTCCCGTCGTGCCTTACGCTAGCTGGAAC GCTACCCAGGGCTGGGATCCCGTCACTGGTCTCGGAACTCCCG ATTTCGCCGAGCTCAAGAAGCTTGCCCTTGGCAACTAA | Aspergillus oryzae RIB40 |

(continued)

| SEQ ID No.: | Sequence | Origin |
|---|---|---|
| 58 | ATGGCGCCCATCCTCTCGTTCCTTGTTGGCTCTCTCCTGGCGG TTCGCGCTCTTGCTGAGCCATTTGAGAAGCTGTTCAGCACCCC GGAAGGATGGAAGATGCAAGGTCTTGCTACCAATGAGCAGATC GTCAAGCTCCAGATTGCTCTTCAGCAAGGCGATGTTGCAGGTTT CGAGCAACATGTGATTGACATCTCAACGCCTAGCCACCCGAGC TATGGTGCTCACTATGGCTCGCATGAGGAGATGAAGAGGATGA TCCAGCCAAGCAGCGAGACAGTCGCTTCTGTGTCTGCATGGCT GAAGGCCGCCGGTATCAACGACGCTGAGATTGACAGCGACTG GGTCACCTTCAAGACGACCGTTGGCGTTGCCAACAAGATGCTC GACACCAAGTTCGCTTGGTACGTGAGCGAGGAGGCCAAGCCC CGCAAGGTCCTTCGCACACTCGAGTACTCTGTACCAGATGATGT TGCAGAACACATCAACTTGATCCAGCCCACTACTCGGTTTGCTG CGATCCGCCAAAACCACGAGGTTGCGCACGAGATTGTTGGTCT TCAGTTCGCTGCTCTTGCCAACAACACCGTTAACTGCGATGCCA CCATCACTCCCCAGTGCTTGAAGACTCTTTACAAGATTGACTAC AAGGCCGATCCCAAGAGTGGTTCCAAGGTCGCTTTTGCTTCGT ATTTGGAGCAGTACGCGCGTTACAATGACCTCGCCCTCTTCGA GAAGGCCTTCCTCCCCGAAGCAGTTGGCCAGAACTTCTCTGTC GTCCAGTTCAGCGGCGGTCTCAACGACCAGAACACCACGCAAG ACAGTGGCGAGGCCAACTTGGACTTGCAGTACATTGTCGGTGT CAGCGCTCCTCTTCCCGTCACCGAGTTCAGCACCGGTGGTCGC GGCCCATGGGTCGCTGACCTAGACCAACCTGACGAGGCGGAC AGCGCCAACGAGCCCTACCTTGAATTCCTTCAGGGTGTGCTCA AACTTCCCCAGTCTGAGCTACCTCAGGTCATCTCCACATCCTAT GGCGAGAATGAGCAGAGTGTACCTAAGTCATACGCTCTCTCCG TCTGCAACTTGTTCGCCCAACTCGGTTCCCGTGGCGTCTCCGT CATCTTCTCTTCTGGTGACAGCGGCCCTGGATCCGCATGCCAG AGCAACGACGGCAAGAACACGACCAAGTTCCAGCCTCAGTACC CCGCTGCCTGCCCCTTTGTCACCTCGGTTGGATCGACTCGCTA CCTCAACGAGACCGCAACCGGCTTCTCATCTGGTGGTTTCTCC GACTACTGGAAGCGCCCATCGTACCAGGACGATGCTGTTAAGG CGTATTTCCACCACCTCGGTGAGAAATTCAAGCCATACTTCAAC CGCCACGGCCGTGGATTCCCCGACGTTGCAACCCAGGGATATG GCTTCCGCGTCTACGACCAGGGCAAGCTCAAGGGTCTCCAAGG TACTTCTGCCTCCGCGCCTGCATTCGCCGGTGTGATTGGTCTC CTCAACGACGCGCGATTGAAGGCGAAGAAGCCTACCTTGGGAT TCCTAAACCCACTGCTTTACTCTAACTCAGACGCGCTAAATGAC ATTGTTCTCGGTGGAAGCAAGGGATGCGATGGTCATGCTCGCT TTAACGGGCCGCCAAATGGCAGCCCAGTAATCCCATATGCGGG ATGGAACGCGACTGCTGGGTGGGATCCAGTGACTGGTCTTGGA ACGCCGAACTTCCCCAAGCTTCTTAAGGCTGCGGTGCCTAGCC GGTACAGGGCGTGA | Phaeosphaeria nodorum SN15 |

(continued)

| SEQ ID No.: | Sequence | Origin |
|---|---|---|
| 59 | ATGGCGAAACTGACAGCTCTTGCCGGTCTCCTGACCCTTGCCA GCGTGCAGGCAAATGCCGCCGTGCTCTTGGACAGCCTCGACAA GGTGCCTGTTGGATGGCAGGCTGCTTCGGCCCCGGCCCCGTC ATCCAAGATCACCCTCCAAGTTGCCCTCACGCAGCAGAACATTG ATCAGTTGGAATCAAAGCTCGCTGCCGTCTCCACGCCCAACTC CAGCAACTATGGAAAGTACCTGGATGTCGATGAGATTAACCAAA TCTTCGCTCCCAGCAGCGCCAGCACCGCTGCTGTTGAGTCCTG GCTCAAGTCGTACGGCGTGGACTACAAGGTGCAGGGCAGCAG CATCTGGTTCCAGACGGATGTCTCCACGGCCAACAAGATGCTC AGCACAAACTTCCACACTTACACCGACTCGGTTGGTGCCAAGAA AGTGCGAACTCTCCAGTACTCGGTCCCCGAGACCCTGGCCGAC CACATCGATCTGATTTCGCCCACAACCTACTTTGGCACGTCCAA GGCCATGCGGGCGTTGAAGATCCAGAACGCGGCCTCTGCCGT CTCGCCCCTGGCTGCTCGTCAGGAGCCCTCCAGCTGCAAGGG CACAATTGAGTTTGAGAACCGCACATTCAACGTCTTCCAGCCCG ACTGTCTCAGGACCGAGTACAGCGTCAACGGATACAAGCCCTC AGCCAAGTCCGGTAGCAGGATTGGCTTCGGCTCTTTCCTGAAC CAGAGCGCCAGCTCCTCAGATCTCGCTCTGTTCGAGAAGCACT TTGGCTTTGCCAGCCAGGGCTTCTCCGTCGAGCTCATCAATGG CGGATCAAACCCCCAGCCGCCCACAGACGCCAATGACGGCGA GGCCAACCTGGACGCCCAGAACATTGTGTCGTTTGTGCAGCCT CTGCCCATCACCGAGTTTATTGCTGGAGGAACTGCGCCGTACT TCCCAGACCCCGTTGAGCCGGCTGGAACTCCCGATGAGAACGA GCCTTACCTCGAGTACTACGAGTACCTGCTCTCCAAGTCAAACA AGGAGCTTCCCCAAGTCATCACCAACTCCTACGGTGATGAGGA GCAGACTGTTCCCCAGGCATATGCCGTCCGCGTGTGCAACCTC ATTGGATTGATGGGCCTTCGTGGTATCTCTATCCTCGAGTCATC CGGTGATGAGGGTGTTGGTGCCTCTTGTCTCGCTACCAACAGC ACCACCACTCCCCAGTTCAACCCCATCTTCCCGGCTACATGCC CCTATGTCACCAGTGTTGGTGGAACCGTCAGCTTCAACCCCGA GGTTGCCTGGGACGGCTCATCCGGAGGCTTCAGCTACTACTTC TCAAGACCTTGGTACCAGGAGGCCGCAGTCGGCACATACCTTA ACAAGTATGTCAGCGAGGAGACCAAGGAATACTACAAGTCGTAT GTCGACTTTTCCGGACGTGGCTTCCCCGATGTTGCAGCTCACA GCGTGAGCCCCGATTACCCCGTGTTCCAAGGCGGCGAGCTTAC CCCCAGCGGCGGTACTTCTGCGGCCTCTCCCATCGTGGCCAGT GTTATTGCCCTCCTGAACGATGCTCGTCTCCGTGCAGGCAAGC CTGCTCTCGGATTCTTGAACCCTCTGATCTACGGATATGCCTAC AAGGGCTTTACCGATATCACGAGTGGCCAAGCTGTCGGCTGCA ACGGCAACAACACTCAAACTGGAGGCCCTCTTCCTGGTGCGGG TGTTATTCCAGGTGCTTTCTGGAACGCGACCAAGGGCTGGGAT CCTACAACTGGATTCGGTGTCCCCAACTTCAAGAAGCTGCTTGA GCTTGTCCGATACATTTAG | Trichoderma atroviride IMI 206040 |

(continued)

| SEQ ID No.: | Sequence | Origin |
|---|---|---|
| 60 | ATGCGTCTTCTCAAATTTGTGTGCCTGTTGGCATCAGTTGCCGC CGCAAAGCCTACTCCAGGGGCGTCACACAAGGTCATTGAACAT CTTGACTTTGTTCCAGAAGGATGGCAGATGGTTGGTGCCGCGG ACCCTGCTGCTATCATTGATTTCTGGCTTGCCATCGAGCGCGAA AACCCAGAAAAGCTCTACGACACCATCTATGACGTCTCCACCCC TGGACGCGCACAATATGGCAAACATTTGAAGCGTGAGGAATTG GATGACTTACTACGCCCAAGGGCAGAGACGAGTGAGAGCATCA TCAACTGGCTCACCAATGGTGGAGTCAACCCACAACATATTCGG GATGAAGGGGACTGGGTCAGATTCTCTACCAATGTCAAGACTG CCGAAACGTTGATGAATACCCGCTTCAACGTCTTCAAGGACAAC CTAAATTCCGTTTCAAAAATTCGAACTTTGGAGTATTCCGTCCCT GTAGCTATATCAGCTCATGTCCAAATGATCCAGCCAACTACCTT ATTTGGACGACAGAAGCCACAGAACAGTTTGATCCTAAACCCCT TGACCAAGGATCTAGAATCCATGTCCGTTGAAGAATTTGCTGCT TCTCAGTGCAGGTCCTTAGTGACTACTGCCTGCCTTCGAGAATT GTACGGACTTGGTGACCGTGTCACTCAGGCTAGGGATGACAAC CGTATTGGAGTATCCGGCTTTTTGGAGGAGTACGCCCAATACC GCGATCTTGAGCTCTTCCTCTCGCTTTGAGCCATCCGCCAAA GGATTTAATTTCAGTGAAGGCCTTATTGCCGGAGGAAAGAACAC TCAGGGTGGTCCTGGAAGCTCTACTGAGGCCAACCTTGATATG CAATATGTCGTCGGTCTGTCCCACAAGGCAAAGGTCACCTATTA CTCCACCGCTGGCCGTGGCCCATTAATTCCCGATCTATCTCAG CCAAGCCAAGCTTCAAACAACAACGAACCATACCTTGAACAGCT GCGGTACCTCGTAAAGCTCCCCAAGAACCAGCTTCCATCTGTAT TGACAACTTCCTATGGAGACACAGAACAGAGCTTGCCCGCCAG CTATACCAAAGCCACTTGCGACCTCTTTGCTCAGCTAGGAACTA TGGGTGTGTCTGTTATCTTCAGCAGTGGTGATACCGGGCCCGG AAGCTCATGCCAGACCAACGATGGCAAGAATGCGACTCGCTTC AACCCTATCTACCCAGCTTCTTGCCCGTTTGTGACCTCCATCGG TGGAACCGTTGGTACCGGTCCTGAGCGTGCAGTTTCATTCTCCT CTGGTGGCTTCTCAGACAGGTTCCCCCGCCCACAATATCAGGA TAACGCTGTTAAAGACTACCTGAAAATTTTGGGCAACCAGTGGA GCGGATTGTTTGACCCCAACGGCCGTGCTTTCCCAGATATCGC AGCTCAGGGATCAAATTATGCTGTCTATGACAAGGGAAGGATGA CTGGAGTCTCCGGCACCAGTGCATCCGCCCCTGCCATGGCTGC CATCATTGCCCAGCTTAACGATTTCCGACTGGCAAAGGGCTCTC CTGTGCTGGGATTCTTGAACCCATGGATATATTCCAAGGGTTTC TCTGGCTTTACAGATATTGTTGATGGCGGTTCCAGGGGTTGCAC TGGTTACGATATATACAGCGGCTTGAAAGCGAAGAAGGTTCCCT ACGCAAGCTGGAATGCAACTAAGGGATGGGACCCAGTAACGGG ATTTGGTACTCCCAACTTCCAAGCTCTCACTAAAGTGCTGCCCT AA | Arthroderma benhamiae CBS 112371 |

(continued)

| SEQ ID No.: | Sequence | Origin |
|---|---|---|
| 61 | ATGTATATCACCTCATCCCGCCTCGTGCTGGCCTTAGCGGCACT TCCGACAGCATTTGGTAAATCATACTCCCACCATGCCGAAGCAC CAAAGGGATGGAAGGTCGACGACACCGCTCGTGTTGCCTCCAC CGGTAAACAACAGGTCTTCAGCATCGCACTGACCATGCAAAATG TTGATCAGCTCGAGTCCAAGCTCCTTGACCTCTCCAGCCCCGA CAGCAAGAACTATGGCCAGTGGATGTCTCAAAAGGACGTAACA ACTGCTTTCTATCCTTCGAAAGAAGCTGTTTCCAGTGTGACAAA GTGGCTCAAGTCCAAGGGTGTCAAGCACTACAACGTCAACGGT GGTTTCATTGACTTTGCTCTCGATGTCAAGGGTGCCAATGCGCT ACTTGATAGTGACTATCAATACTACACCAAAGAGGGCCAGACCA AGTTGCGAACTCTGTCTTACTCTATCCCTGATGATGTAGCCGAA CACGTTCAGTTCGTCGACCCAAGCACCAACTTTGGCGGCACAC TGGCTTTCGCCCCTGTCACTCACCCATCGCGTACTCTAACCGA GCGCAAGAACAAGCCCACCAAGAGCACAGTCGATGCTTCATGC CAAACCAGCATCACACCCTCATGCTTGAAGCAGATGTACAACAT TGGTGACTACACTCCCAAGGTCGAGTCTGGAAGCACTATTGGTT TCAGCAGCTTCCTTGGCGAGTCCGCCATCTACTCCGATGTTTTC CTGTTTGAGGAGAAGTTTGGAATTCCCACGCAGAACTTTACCAC TGTTCTCATCAACAACGGCACTGATGACCAGAACACTGCTCACA AGAACTTTGGCGAGGCTGACTTGGATGCCGAGAACATTGTTGG AATTGCCCACCCTCTTCCCTTCACCCAGTACATCACTGGCGGTT CACCACCTTTTCTTCCCAACATCGATCAGCCAACTGCTGCCGAT AACCAGAACGAGCCTTATGTGCCTTTCTTCCGCTACCTTCTATC GCAGAAGGAAGTCCCTGCAGTTGTCTCTACCTCGTATGGTGAC GAAGAAGATAGCGTCCCTCGCGAATATGCTACCATGACCTGCA ACCTGATTGGTCTTCTCGGACTTCGAGGAATCAGTGTCATCTTC TCCTCTGGCGATATCGGCGTTGGTGCTGGATGTCTCGGCCCTG ACCACAAGACTGTCGAGTTCAACGCCATCTTCCCTGCCACCTG CCCTTACCTCACCTCCGTCGGCGGTACCGTTGATGTCACCCCC GAAATCGCCTGGGAAGGTTCTTCTGGTGGTTTCAGCAAGTACTT CCCCCGACCCAGCTACCAGGACAAGGCTGTCAAGACGTACATG AAGACTGTCTCCAAGCAGACAAAGAAGTACTACGGCCCTTACAC CAACTGGGAAGGCCGAGGCTTCCCTGATGTTGCTGGCCACAGT GTCTCTCCCAACTATGAGGTTATCTATGCTGGTAAGCAGAGTGC AAGCGGAGGTACCAGTGCTGCTGCTCCTGTTTGGGCTGCCATT GTCGGTCTGCTCAACGATGCCCGTTTCAGAGCTGGGAAGCCAA GCTTGGGATGGTTGAACCCTCTTGTTTACAAGTATGGACCAAAG GTGTTGACTGACATCACTGGTGGTTACGCCATTGGATGTGATG GCAACAACACCCAGTCCGGAAAGCCTGAGCCTGCAGGATCCG GTATTGTGCCCGGTGCCAGATGGAATGCCACTGCCGGATGGGA TCCTGTCACTGGTTATGGTACACCCGACTTTGGAAAGTTGAAGG ATTTGGTTCTTAGCTTCTAA | Fusarium graminearum PH-1 |

(continued)

| SEQ ID No.: | Sequence | Origin |
|---|---|---|
| 62 | ATGCGTTCCTCCGGTCTTTACGCAGCACTGCTGTGCTCTCTGG CCGCATCGACCAACGCAGTTGTTCATGAGAAGCTCGCCGCGGT CCCCTCGGGCTGGCACCATCTCGAAGATGCTGGCTCCGATCAC CAGATTAGCCTGTCGATCGCATTGGCACGCAAGAACCTCGATC AGCTTGAATCCAAGCTGAAAGACTTGTCCACACCAGGTGAATCG CAGTATGGCCAGTGGCTGGATCAAGAGGAAGTCGACACACTGT TCCCAGTGGCCAGCGACAAGGCCGTGATCAGCTGGTTGCGCA GCGCCAACATCACCCATATTGCCCGGCAGGGCAGCTTGGTGAA CTTTGCGACCACCGTCGACAAGGTGAACAAGCTTCTCAACACC ACTTTTGCTTACTACCAAAGAGGTTCTTCCCAGAGACTGCGCAC GACAGAGTACTCCATTCCCGATGATCTGGTCGACTCGATCGAC CTCATCTCCCCGACAACCTTTTTCGGCAAGGAAAAGACCAGTGC TGGCCTGACCCAGCGGTCGCAGAAAGTCGACAACCATGTGGCC AAACGCTCCAACAGCTCGTCCTGCGCCGATACCATCACGTTATC CTGCCTGAAGGAGATGTACAACTTTGGCAACTACACTCCCAGC GCCTCGTCAGGAAGCAAGCTGGGATTCGCCAGCTTCCTGAACG AGTCCGCCTCGTATTCCGATCTTGCCAAGTTCGAGAGACTGTTC AACTTGCCGTCTCAGAACTTCTCCGTGGAGCTGATCAACGGCG GCGTCAATGACCAGAACCAATCGACGGCTTCTCTGACCGAGGC TGACCTCGATGTGGAATTGCTCGTTGGCGTAGGTCATCCTCTTC CGGTGACCGAGTTTATCACTTCTGGCGAACCTCCTTTCATTCCC GACCCCGATGAGCCGAGTGCCGCCGATAATGAGAATGAGCCTT ACCTTCAGTACTACGAGTACCTCCTCTCCAAGCCCAACTCGGCC CTGCCCCAAGTGATTTCCAACTCCTACGGTGACGACGAACAGA CCGTTCCAGAATACTACGCCAAGCGAGTCTGCAACCTGATCGG ACTGGTCGGCCTGCGCGGCATCAGCGTCCTGGAATCATCCGGT GACGAAGGAATTGGATCTGGCTGCCGCACCACCGACGGCACTA ACAGCACCCAATTCAATCCCATCTTCCCCGCCACCTGTCCCTAC GTGACCGCCGTAGGAGGCACCATGTCCTACGCGCCCGAAATTG CCTGGGAAGCCAGTTCCGGTGGTTTCAGCAACTACTTCGAGCG AGCCTGGTTCCAGAAGGAAGCCGTGCAGAACTACCTGGCGAAC CACATCACCAACGAGACGAAGCAGTATTACTCACAATTCGCTAA CTTTAGCGGTCGCGGATTTCCCGATGTTTCGGCCCATAGCTTTG AGCCTTCGTACGAAGTTATCTTCTACGGCGCCCGTTACGGCTC CGGCGGTACTTCCGCCGCATGTCCTCTGTTCTCTGCGCTAGTG GGCATGTTGAACGATGCTCGTCTGCGGGCGGGCAAGTCCACG CTTGGTTTCTTGAACCCCCTGCTGTACAGTAAGGGGTACAAGG CGCTGACAGATGTCACGGCGGGACAATCGATCGGGTGCAATG GCATTGATCCGCAGAGTGATGAGGCTGTTGCGGGCGCGGGCA TTATCCCGTGGGCGCATTGGAATGCCACAGTCGGATGGGATCC GGTGACGGGATTGGGACTTCCTGATTTTGAGAAGTTGAGGCAG TTGGTGCTGTCGTTGTAG | Aspergillus kawachii IFO 4308 |

(continued)

| SEQ ID No.: | Sequence | Origin |
|---|---|---|
| 63 | ATGAGTCGAAATCTCCTCGTTGGTGCTGGCCTGTTGGCCCTCG CCCAATTGAGCGGTCAAGCTCTCGCTGCCGCTGCCCTCGTCGG CCATGAATCCCTAGCTGCGCTGCCAGTTGGCTGGGATAAGGTC AGCACGCCAGCTGCAGGGACGAACATTCAATTGTCCGTCGCCC TCGCTCTGCAAAACATCGAGCAGCTGGAAGACCACTTGAAGTC TGTGTCAACCCCCGGTTCTGCCAGCTACGGTCAGTACCTGGAT TCCGACGGTATTGCCGCTCAATACGGTCCCAGCGACGCATCCG TTGAGGCTGTCACCAACTGGCTGAAGGAGGCCGGTGTCACTGA CATCTACAACAACGGCCAGTCGATTCACTTCGCAACCAGTGTCA GCAAGGCCAACAGCTTGCTCGGGGCCGATTTCAACTACTATTCT GATGGTAGTGCGACCAAGTTGCGTACCTTAGCTTATTCCGTTCC CAGTGACCTCAAAGAGGCCATCGACCTTGTCTCGCCCACCACC TATTTCGGCAAGACCACTGCTTCTCGTAGCATCCAGGCTTACAA GAACAAGCGCGCCTCTACTACTTCCAAGTCTGGATCGAGCTCT GTGCAAGTATCTGCTTCCTGCCAGACCAGCATCACTCCTGCCT GCTTGAAACAGATGTACAATGTTGGCAACTACACACCCAGCGTC GCTCACGGCAGTCGTGTCGGATTCGGTAGCTTCTTGAATCAATC TGCCATCTTTGACGACTTGTTCACCTACGAAAAGGTCAATGATA TTCCATCACAGAATTTCACTAAGGTGATTATTGCAAATGCATCCA ACAGCCAAGATGCCAGCGATGGCAACTACGGCGAAGCCAACCT TGACGTGCAAAACATTGTCGGCATCTCTCATCCTCTCCCCGTGA CTGAATTCCTCACTGGTGGCTCACCTCCCTTCGTTGCTAGCCTC GACACCCCTACCAACCAGAACGAGCCATATATTCCTTACTACGA ATATCTTTTGTCTCAGAAGAACGAGGATCTCCCCCAGGTCATTT CCAACTCTTACGGAGACGACGAGCAGTCTGTGCCGTACAAGTA TGCCATCCGTGCATGCAACCTGATCGGCCTGACAGGTTTACGA GGTATCTCGGTCTTGGAATCCAGCGGTGATCTCGGCGTTGGAG CCGGCTGTCGCAGCAACGATGGCAAGAACAAGACTCAATTTGA CCCCATCTTCCCTGCCACTTGCCCCTACGTTACCTCTGTTGGTG GTACCCAATCCGTTACCCCTGAAATTGCCTGGGTCGCCAGCTC CGGTGGTTTCAGCAACTACTTCCCTCGTACCTGGTACCAGGAA CCCGCAATTCAGACCTATCTCGGACTCCTTGACGATGAGACCAA GACATACTATTCTCAATACACCAACTTTGAAGGCCGTGGTTTCC CCGATGTTTCCGCCCACAGCTTGACCCCTGATTACCAGGTCGT CGGTGGTGGCTATCTCCAGCCAAGCGGTGGTACTTCCGCTGCT TCTCCTGTCTTTGCCGGCATCATTGCGCTTTTGAACGACGCTCG TCTCGCTGCTGGCAAGCCCACTCTTGGCTTCTTGAACCCGTTCT TCTACCTTTATGGATACAAGGGTTTAAACGATATCACTGGAGGA CAGTCAGTGGGTTGCAACGGTATCAACGGCCAAACTGGGGCTC CTGTTCCCGGTGGTGGCATTGTTCCTGGAGCGGCCTGGAACTC TACTACTGGCTGGGACCCAGCCACTGGTCTCGGAACACCCGAC TTCCAGAAGTTGAAAGAACTCGTACTTAGCTTTTAA | Talaromyces stipitatus ATCC 10500 |

(continued)

| SEQ ID No.: | Sequence | Origin |
|---|---|---|
| 64 | ATGTATATCTCCTCCCAAAATCTGGTACTCGCCTTATCGGCGCT GCCTTCAGCATTTGGCAAATCCTTCTCTCACCATGCTGAAGCTC CTCAAGGCTGGCAAGTCCAAAAGACTGCCAAAGTCGCTTCCAA CACGCAGCATGTCTTCAGTCTTGCACTAACCATGCAAAACGTGG ATCAGCTCGAATCCAAGCTTCTTGACCTCTCCAGCCCCGACAG CGCCAACTACGGTAACTGGCTCTCCCACGATGAGCTCACAAGC ACTTTCTCTCCTTCCAAGGAGGCGGTGGCTAGTGTGACAAAGT GGCTCAAGTCAAAGGGCATCAAGCACTACAAGGTCAACGGTGC TTTCATTGACTTTGCTGCTGATGTTGAGAAGGCCAATACGCTTC TCGGAGGTGATTACCAGTACTACACTAAGGATGGTCAGACGAA GCTGAGAACGCTGTCTTACTCCATTCCTGATGATGTCGCCGGTC ACGTTCAATTTGTTGATCCTAGCACAAACTTCGGTGGCACCGTT GCGTTCAACCCTGTGCCTCACCCCTCGCGCACCCTCCAAGAGC GCAAGGTCTCTCCCTCCAAGAGCACCGTTGATGCTTCATGCCA GACAAGCATCACCCCTTCTTGCCTCAAGCAGATGTACAACATTG GAGACTACACTCCCGATGCCAAGTCTGGAAGTGAGATTGGTTT CAGCAGCTTTCTCGGCCAGGCTGCTATTTACTCTGATGTCTTCA AGTTTGAGGAGCTGTTTGGTATTCCTAAGCAGAACTACACCACT ATTCTGATCAACAATGGCACCGATGATCAGAATACTGCGCATGG AAACTTTGGAGAGGCTAACCTTGATGCTGAGAACATTGTTGGAA TCGCTCATCCTCTTCCTTTCAAGCAGTACATTACTGGAGGTTCA CCACCTTTCGTTCCCAACATCGATCAGCCCACCGAGAAGGATAA CCAGAACGAGCCCTACGTGCCTTTCTTCCGTTACCTCTTGGGC CAGAAGGATCTCCCAGCCGTCATCTCCACTTCCTACGGCGATG AAGAAGACAGCGTTCCTCGTGAGTATGCTACACTCACCTGCAAC ATGATCGGTCTTCTCGGTCTCCGTGGCATCAGTGTCATCTTCTC TTCCGGTGACATCGGTGTCGGTTCCGGCTGCCTTGCTCCCGAC TACAAGACCGTCGAGTTCAATGCCATCTTCCCCGCCACATGCC CCTACCTCACCTCCGTCGGCGGTACCGTCGACGTCACCCCCGA GATCGCCTGGGAGGGATCCTCCGGCGGATTCAGCAAGTACTTC CCCCGACCCAGCTACCAGGACAAGGCCATCAAGAAGTACATGA AGACAGTCTCCAAGGAGACCAAGAAGTACTACGGCCCTTACAC CAACTGGGAGGGCCGAGGTTTCCCTGATGTCGCTGGACACAGT GTTGCGCCTGACTACGAGGTTATCTACAATGGTAAGCAGGCTC GAAGTGGAGGTACCAGCGCTGCTGCCCCTGTTTGGGCTGCTAT CGTTGGTCTGTTGAACGATGCCCGCTTCAAGGCTGGTAAGAAG AGCTTGGGATGGTTGAACCCTCTTATCTACAAGCATGGACCCAA GGTCTTGACTGACATCACCGGTGGCTATGCTATTGGATGTGAC GGTAACAACACTCAGTCTGGAAAGCCCGAGCCCGCTGGATCTG GTCTTGTTCCCGGTGCTCGATGGAACGCCACAGCTGGATGGGA TCCTACCACTGGCTATGGAACTCCCAACTTCCAGAAGTTGAAGG ACTTGGTTCTCAGCTTGTAA | Fusarium oxysporum f. sp.cubense race 4 |

(continued)

| SEQ ID No.: | Sequence | Origin |
|---|---|---|
| 65 | ATGCCTAAGTCCACAGCGCTTCGGCTTGTTAGCCTCCTTTCCCT GGCCAGTGTGCCGATATCTGCCTCCGTCCTTGTGGAAAGTCTC GAAAAGCTGCCTCACGGATGGAAAGCTGCTTCGGCTCCTAGCC CTTCCTCCCAGATAACCCTACAAGTCGCTCTTACGCAGCAGAAC ATCGATCAGCTGGAATCGAGGCTCGCGGCTGTATCCACACCAA ATTCCAAGACATACGGAAATTATCTGGATCTTGATGAGATCAAT GAGATCTTCGCGCCAAGCGATGCCAGCAGCGCAGCCGTGGAG TCTTGGCTCCATTCTCACGGTGTGACAAAATACACGAAGCAAGG CAGCAGTATCTGGTTCCAAACCGAAGTTTCTACAGCAAATGCAA TGTTGAGCACAAACTTCCACACTTACAGTGATGCTGCTGGCGTT AAGAAGTTGCGAACTCTTCAGTATTCAATTCCGGAGAGTCTTGT GGGCCATGTCGATCTCATCTCACCCACGACCTACTTTGGCACCT CTAACGCTATGAGAGCTTTGAGATCTAAAAGCGTGGCTTCAGTT GCTCAAAGTGTGGCAGCCCGCCAAGAACCTTCTAGCTGCAAGG GAACTCTGGTTTTCGAAGGAAGAACGTTCAATGTCTTCCAACCA GATTGTCTTAGGACAGAGTACAATGTCAATGGATACACTCCATC AGCCAAGTCTGGTAGTAGAATAGGATTTGGTTCCTTCTTAAACC AAAGTGCAAGCTTTTCAGACCTCGCACTCTTTGAAAAACACTTT GGGTTTTCCAGCCAAAATTTCTCCGTCGTTCTGATCAATGGTGG AACGGACCTGCCCCAACCACCCTCTGACGACAACGATGGCGAG GCCAATTTGGATGTCCAAAACATTTTGACAATCGCACACCCTCT GCCCATCACTGAATTCATCACTGCCGGAAGCCCGCCGTACTTC CCAGATCCCGTTGAACCTGCAGGAACTCCCGATGAGAACGAGC CTTACTTGCAGTACTTTGAGTATCTGTTGTCGAAGCCCAACAGA GATCTTCCTCAGGTCATTACCAACTCTTACGGTGATGAGGAGCA AACAGTACCTCAGGCTTATGCTGTCCGAGTGTGCAACCTAATTG GATTGATGGGACTGCGTGGTATCAGTATCCTCGAGTCCTCCGG CGATGAGGGAGTGGGTGCTTCCTGCGTTGCTACCAACAGCACC ACTCCTCAATTTAACCCCATTTTCCCGGCAACATGCCCCTATGT CACTAGCGTAGGTGGAACTGTGAACTTCAACCCAGAAGTTGCC TGGGACGGTTCATCTGGAGGTTTCAGCTACTATTTCTCCAGGCC ATGGTACCAAGAGGAAGCAGTTGGAAACTACCTAGAGAAGCAT GTCAGCGCCGAAACAAAGAAGTACTACGGGCCTTATGTCGATTT CTCTGGACGTGGCTTCCCTGATGTTGCAGCTCACAGCGTGAGC CCCGATTATCCTGTGTTCCAAGGCGGCCAGCTCACTCCTAGCG GAGGCACTTCTGCGGCTTCTCCCGTCGTAGCCAGTATCATTGC CCTTCTGAACGATGCACGCCTCCGTGAAGGCAAGCCCACACTT GGGTTCCTGAACCCGCTGATTTACCAATATGCTTACAAGGGTTT CACGGATATCACATCCGGCCAGTCTGATGGCTGCAATGGCAAC AACACCCAAACGGATGCCCCTCTTCCTGGAGCTGGCGTTGTCC TAGGAGCACACTGGAATGCGACCAAAGGATGGGATCCTACGAC AGGATTTGGTGTCCCTAACTTTAAGAAGCTACTCGAGCTGATCC GATATATATAG | Trichoderma virens Gv29-8 |

(continued)

| SEQ ID No.: | Sequence | Origin |
|---|---|---|
| 66 | ATGGCTAAACTGACGGCACTTCGGCTCGTCAGCCTTCTTTGCCT TGCGGCTGCGCAGGCCTCTGCTGCTGTGCTCGTGGAAAGCCTC AAACAAGTGCCCAACGGGTGGAATGCAGTCTCGACCCCAGACC CTTCGACATCGATTGTCTTGCAAATCGCCCTCGCGCAACAGAAT ATCGATGAATTGGAATGGCGTCTCGCGGCTGTATCCACGCCCA ACTCTGGCAATTATGGCAAATACCTGGATATTGGAGAGATTGAA GGAATTTTCGCCCCAAGCAATGCCTCTTACAAAGCCGTGGCATC GTGGCTCCAGTCTCATGGGGTGAAGAACTTCGTCAAACAAGCC GGCAGTATTTGGTTCTACACTACTGTCTCTACCGCAAACAAGAT GCTTAGCACAGATTTCAAACACTATAGCGATCCTGTTGGCATTG AGAAGCTGCGTACTCTTCAGTACTCGATCCCAGAAGAACTAGTC GGCCATGTTGATCTCATCTCGCCTACAACATATTTTGGAAACAA CCACCCCGCGACAGCGAGAACACCCAACATGAAGGCCATTAAC GTAACCTACCAAATCTTTCACCCAGACTGCCTTAAAACGAAATA CGGCGTTGATGGCTATGCCCCATCTCCAAGATGTGGCAGCAGG ATTGGTTTTGGCTCATTCCTCAACGAAACTGCCAGTTATTCGGA TCTTGCGCAGTTTGAGAAGTACTTTGACCTTCCCAACCAAAACC TTTCCACCTTATTGATCAATGGCGCAATCGACGTTCAGCCACCT TCCAACAAAAACGACAGCGAGGCCAACATGGACGTTCAGACCA TCTTGACCTTTGTCCAACCTCTTCCTATTACTGAGTTTGTTGTTG CCGGAATCCCGCCGTATATTCCTGATGCGGCTTTGCCGATCGG CGACCCTGTCCAAAACGAGCCGTGGCTGGAATACTTTGAGTTTT TGATGTCCAGGACCAACGCAGAGCTTCCCCAGGTCATTGCCAA CTCATACGGTGACGAGGAACAAACGGTACCACAGGCGTATGCC GTCCGAGTATGCAACCAGATTGGGCTGTTGGGCCTTCGCGGTA TATCCGTTATCGCATCATCTGGCGATACGGGTGTTGGAATGTCT TGTATGGCTTCGAACAGCACTACTCCTCAGTTTAACCCCATGTT CCCGGCTTCGTGTCCTTATATCACCACTGTCGGTGGAACTCAG CACCTTGATAATGAGATTGCTTGGGAGCTTTCATCGGGAGGCTT CAGTAACTATTTCACAAGGCCATGGTATCAAGAAGACGCAGCCA AAACATATCTTGAACGTCATGTCAGCACCGAGACAAAGGCATAT TACGAACGTTACGCCAATTTCTTGGGACGCGGCTTTCCCGACG TTGCAGCACTTAGTCTCAACCCCGATTATCCAGTGATTATTGGC GGAGAACTTGGTCCCAATGGAGGCACTTCTGCGGCCGCACCC GTCGTCGCTAGTATTATTGCACTCTTGAACGATGCACGCCTTTG CCTAGGCAAACCTGCCCTTGGGTTCTTGAACCCCCTGATCTATC AATATGCTGATAAGGGTGGCTTCACGGATATCACGTCCGGCCA GTCTTGGGGCTGTGCCGGAAATACCACTCAGACGGGGCCTCCT CCCCCTGGAGCTGGTGTCATTCCGGGGGCACACTGGAATGCG ACCAAGGGATGGGATCCTGTAACAGGATTTGGAACCCCGAACT TCAAGAAATTACTCTCACTGGCCCTGTCCGTCTAA | Trichoderma atroviride IMI 206040 |

(continued)

| SEQ ID No.: | Sequence | Origin |
|---|---|---|
| 67 | ATGTTTTGGCGTCCAGCTTTTGTCCTTCTTCTCGCTCAGCTTGT CACTGCTAGTCCTTTAGCTCGACGCTGGGATGATTTCGCAGAAA AACATGCCTGGGTTGAAGTTCCTCGCGGGTGGGAAATGGTCTC CGAGGCTCCCAGTGACCATACCTTTGATCTTCGCATTGGAGTAA AGTCAAGTGGCATGGAGCAGCTCATTGAAAACTTGATGCAAACC AGCGATCCTACTCATTCCAGATATGGTCAACATCTTAGTAAAGA AGAGCTCCATGATTTCGTTCAGCCTCATCCTGATTCTACCGGAG CGGTCGAAGCATGGCTTGAAGATTTCGGTATCTCCGATGATTTC ATTGATCGTACTGGAAGTGGCAACTGGGTTACTGTTCGAGTTTC AGTAGCCCAGGCTGAACGTATGCTTGGTACCAAGTATAACGTCT ACCGCCATTCTGAATCAGGGGAATCGGTTGTACGAACAATGTCT TATTCGCTTCCCAGCGAACTTCACTCCCACATAGATGTTGTCGC ACCCACCACTTATTTCGGCACGATGAAAAGCATGCGGGTGACC AGCTTCTTACAGCCGGAAATAGAGCCTGTTGACCCAAGCGCTA AACCATCGGCTGCTCCAGCTTCCTGTTTGAGTACCACTGTCATA ACCCCCGATTGCCTCCGTGACCTTTATAATACGGCTGACTACGT TCCTTCCGCCACTTCACGGAATGCCATTGGTATTGCTGGGTACT TGGATCGTTCAAATCGTGCAGATCTTCAGACTTTCTTCCGACGC TTCCGGCCCGATGCCGTTGGCTTCAATTACACGACTGTCCAACT AAATGGCGGAGGAGACGACCAGAATGATCCCGGTGTAGAGGC CAACCTCGATATTCAATACGCCGCTGGTATTGCTTTCCCCACAC CAGCTACATACTGGAGTACTGGCGGCTCTCCACCTTTCATTCCA GATACTCAAACCCCGACAAACACCAATGAGCCCTACCTGGATTG GATCAATTTTGTCCTAGGCCAGGACGAGATTCCACAGGTGATTT CAACGTCCTATGGTGACGACGAGCAAACAGTTCCTGAAGATTAC GCTACTAGCGTGTGTAATCTCTTCGCGCAACTCGGCAGCCGTG GCGTTACAGTATTCTTCTCCAGCGGTGACTTTGGTGTTGGTGGT GGAGATTGCCTCACGAATGATGGCTCAAACCAAGTCCTTTTCCA GCCGGCTTTCCCCGCTTCCTGCCCATTCGTAACAGCTGTTGGC GGAACTGTCAGGCTTGATCCTGAGATTGCTGTCAGTTTCTCTGG AGGAGGCTTTTCCCGTTACTTCTCCAGGCCATCGTACCAGAATC AAACTGTGGCTCAATTTGTTTCTAATCTTGGGAATACATTCAACG GACTCTACAATAAAAATGGAAGGGCCTACCCAGATCTTGCAGCA CAGGGCAATGGCTTCCAAGTTGTTATAGACGGCATCGTCCGTT CGGTTGGAGGGACCAGCGCCAGCTCTCCGACGGTTGCCGGTA TCTTTGCGCTTTTGAATGACTTCAAGCTCTCAAGAGGCCAGTCG ACACTCGGATTTATCAACCCACTTATATACTCCTCCGCTACATCC GGCTTCAATGACATCAGGGCGGGTACAAACCCTGGTTGTGGTA CTCGCGGATTTACCGCTGGTACTGGTTGGGATCCGGTCACTGG TCTGGGCACTCCCGATTTTTTTGAGGCTTCAGGGACTTATTTAA | Agaricus bisporus var. burnettii JB137-S8 |

(continued)

| SEQ ID No.: | Sequence | Origin |
|---|---|---|
| 68 | ATGCTCGACCGTATCCTTCTCCCCCTCGGCCTCCTGGCCTCCC TTGCCACCGCTCGTGTCTTTGACAGCCTACCCCACCCTCCCCG AGGCTGGTCATACTCGCACGCGGCGGAATCGACGGAGCCGCT GACCCTGCGCATCGCCCTCCGCCAGCAAAATGCCGCCGCCCT GGAGCAGGTGGTGCTGCAGGTCTCGAACCCCAGGCACGCCAA TTACGGCCAGCACCTGACGCGCGACGAGCTGCGCAGCTACAC GGCGCCCACGCCGCGGGCCGTCCGCAGCGTGACGTCGTGGCT GGTCGACAACGGCGTCGACGACTACACGGTCGAGCACGACTG GGTGACGCTGCGCACGACGGTCGGGGCCGCGGACAGGCTGCT CGGCGCAGACTTTGCCTGGTATGCCGGCCCGGGCGAGACGCT GCAGCTGCGGACGCTCTCGTACGGCGTCGACGACTCGGTGGC GCCGCACGTCGACCTCGTGCAGCCCACGACGCGGTTTGGCGG TCCCGTCGGGCAGGCGTCGCACATCTTCAAGCAGGACGACTTT GACGAGCAGCAGCTCAAGACCTTGTCGGTGGGGTTCCAGGTCA TGGCTGACCTGCCGGCCAACGGGCCTGGGTCGATCAAGGCGG CATGTAACGAGTCTGGCGTGACGCCCCTGTGCCTGCGAACTCT GTACAGGGTCAACTACAAGCCGGCAACCACGGGGAACCTGGTC GCTTTCGCGTCGTTCCTGGAGCAGTACGCCAGGTACAGTGATC AGCAGGCATTCACTCAGCGGGTCCTTGGCCCTGGTGTTCCGTT GCAGAACTTTTCGGTCGAAACGGTCAACGGTGGAGCCAATGAC CAGCAGAGCAAACTTGACAGCGGCGAGGCGAACCTCGATCTGC AGTACGTCATGGCAATGAGCCACCCTATTCCAATTTTGGAGTAC AGCACTGGAGGCAGAGGACCCCTCGTCCCAACTCTGGACCAG CCCAACGCCAACAACAGCAGCAATGAGCCTTACCTGGAGTTCC TGACGTACCTCCTGGCCCAACCCGACTCAGCCATCCCTCAGAC CCTGTCGGTGTCGTATGGCGAGGAGGAACAGTCGGTGCCGCG CGACTACGCCATCAAGGTTTGCAACATGTTCATGCAGCTCGGC GCCCGCGGCGTGTCGGTTATGTTTTCGTCGGGCGACTCGGGC CCGGGTAATGACTGTGTTCGAGCCTCGGACAACGCAACCTTTTT TGGCTCAACATTCCCCGCAGGCTGCCCCTACGTCACGTCGGTG GGCTCCACCGTCGGCTTCGAGCCGGAGCGCGCCGTCTCCTTTT CCTCGGGCGGCTTCAGCATTTACCACGCTCGCCCCGACTACCA AAACGAAGTGGTCCCCAAGTACATTGAATCGATCAAGGCTTCG GGCTACGAAAAGTTCTTTGACGGCAACGGCCGCGGAATTCCCG ACGTGGCTGCCCAGGGCGCCCGCTTCGTCGTCATCGACAAGG GCCGCGTTTCTCTAATCTCGGGGACCAGCGCCAGCTCACCTGC GTTTGCTGGCATGGTGGCGCTCGTCAACGCCGCCCGCAAGTCA AAGGACATGCCGGCCTTGGGCTTCCTCAACCCCATGCTGTACC AGAACGCCGCGGCCATGACGGACATTGTCAACGGCGCTGGCA TCGGCTGCAGGAAGCAACGTACAGAATTCCCGAATGGCGCCAG GTTCAACGCCACGGCCGGCTGGGATCCCGTCACAGGGCTGGG GACGCCGTTGTTTGACAAGCTGCTGGCTGTTGGCGCACCTGGA GTTCCCAACGCGTGA | Magnaporthe oryzae 70-15 |

(continued)

| SEQ ID No.: | Sequence | Origin |
|---|---|---|
| 69 | ATGCGTAGCCAGTTGCTCTTCTGCACAGCATTTGCTGCTCTCCA GTCGCTTGTGGAGGGCAGCGATGTGGTGTTGGAGTCATTGCGA GAGGTCCCTCAGGGCTGGAAGAGGCTTCGAGATGCGGACCCC GAGCAGTCCATCAAGCTGCGCATTGCGCTTGAGCAGCCTAACC TGGACCTGTTCGAGCAGACCCTCTACGACATCTCGTCACCGGA TCACCCAAAATATGGCCAGCATCTCAAGAGCCACGAGTTACGG GATATTATGGCACCTCGCGAGGAGTCAACTGCTGCTGTCATCG CTTGGCTGCAAGACGCTGGGCTTTCTGGCTCGCAGATTGAGGA CGACAGCGACTGGATCAACATCCAGACGACAGTCGCCCAAGCC AACGACATGCTGAACACGACTTTCGGTCTCTTCGCCCAGGAAG GCACCGAGGTCAATCGAATTCGAGCTCTGGCATATTCCGTGCC TGAGGAGATCGTCCCTCACGTCAAGATGATTGCTCCCATCATCC GCTTCGGTCAGTTGAGACCTCAGATGAGCCACATCTTCTCGCAT GAGAAAGTCGAGGAGACCCCGTCTATTGGCACCATCAAGGCCG CCGCTATCCCATCTGTGGATCTTAACGTCACCGCTTGCAATGCC AGCATCACCCCCGAGTGCCTCCGAGCGCTTTACAACGTTGGTG ATTACGAGGCGGACCCATCGAAGAAGTCTCTTTTCGGAGTCTGT GGCTACTTGGAGCAATATGCCAAGCACGATCAGCTGGCCAAGT TTGAGCAGACCTACGCTCCGTATGCTATCGGTGCCGACTTCAG CGTCGTGACCATCAATGGCGGAGGCGACAACCAGACCAGTACG ATCGATGATGGAGAAGCCAACCTGGATATGCAGTATGCTGTCA GCATGGCATACAAGACGCCAATCACATACTATTCAACTGGGGGT CGAGGACCTCTTGTTCCAGATCTCGACCAACCTGATCCCAACG ACGTCTCAAACGAGCCGTACCTTGATTTTGTGAGCTACCTTCTC AAGCTGCCCGACTCCAAATTGCCGCAGACCATCACAACTTCGTA CGGAGAGGATGAGCAATCCGTTCCACGCTCCTACGTGGAGAAG GTCTGCACCATGTTCGGCGCGCTCGGTGCCCGAGGCGTGTCT GTGATCTTCTCCTCTGGTGATACCGGTGTCGGCTCAGCGTGCC AGACCAACGACGGCAAGAACACCACCCGCTTCCTGCCTATATT CCCTGCTGCGTGCCCTTATGTGACCTCGGTTGGAGGCACTCGC TATGTCGACCCGGAAGTCGCTGTGTCCTTCTCGTCTGGAGGCT TCTCGGACATCTTCCCTACGCCACTCTACCAGAAGGGCGCTGT CTCTGGCTACCTGAAGATCCTCGGCGATCGCTGGAAGGGCCTC TATAACCCTCACGGCCGCGGTTTCCCTGACGTCTCCGGACAGA GTGTCAGATACCACGTCTTCGACTACGGCAAGGACGTCATGTA CTCTGGCACAAGTGCCTCTGCACCGATGTTCGCCGCGCTTGTC TCGCTGCTGAACAACGCCCGTCTCGCAAAGAAGTTGCCGCCCA TGGGATTCCTGAATCCCTGGCTGTATACCGTTGGTTTTAACGGG CTGACGGATATTGTGCACGGTGGATCTACTGGGTGCACTGGCA CAGACGTGTACAGCGGCCTGCCCACACCTTTCGTTCCGTATGC GTCTTGGAACGCAACCGTGGGATGGGACCCCGTTACTGGACTT GGCACGCCTCTCTTTGATAAGCTGCTCAATTTGAGCACGCCAAA CTTCCACTTGCCGCACATTGGCGGTCACTAG | Togninia minima UCRPA7 |

(continued)

| SEQ ID No.: | Sequence | Origin |
|---|---|---|
| 70 | ATGAAGTACAACACACTCCTCACCGGCCTGCTGGCTGTTGCCC ATGGCAGTGCCGTTTCCGCTTCAACTACTTCACATGTCGAGGGT GAAGTTGTCGAGCGACTTCATGGCGTTCCTGAGGGTTGGAGTC AAGTGGGCGCCCCAATCCAGACCAGAAGCTGCGCTTTCGCAT CGCAGTACGCTCGGTGAGTAATTGCTTTTGTGAACCCATGTTTG AATCTTGCGGTGCTTTTTACTGAACATAACAGGCGGATAGCGAG CTGTTTGAGAGGACGCTTATGGAGGTTTCTTCTCCCAGCCATCC TCGCTACGGACAGCACCTAAAGCGACACGAACTCAAGGACCTC ATCAAACCGCGCGCCAAGTCAACTTCAAACATCCTGAACTGGCT GCAAGAGTCTGGAATTGAGGCCAGAGATATCCAGAACGATGGC GAGTGGATCAGCTTCTATGCTCCGGTTAAACGTGCCGAGCAAA TGATGAGCACTACATTCAAGACCTATCAGAACGAGGCCCGAGC GAATATCAAGAAGATCCGCTCTCTAGACTACTCGGTGCCGAAG CACATTCGAGATGACATCGACATCATCCAGCCTACGACTCGCTT CGGCCAGATCCAACCGGAGCGTAGCCAAGTCTTTAGTCAAGAA GAGGTCCCATTCTCAGCGCTTGTTGTCAATGCGACGTGTAACAA GAAAATCACTCCCGACTGCCTCGCCAACCTCTACAACTTCAAAG ACTATGATGCCAGCGATGCCAATGTCACTATCGGAGTCAGCGG CTTCCTGGAGCAATATGCTCGCTTTGACGACTTGAAGCAATTCA TCAGCACTTTCCAACCAAAGCAGCTGGTTCCACATTCCAAGTT ACATCTGTCAATGCAGGGCCTTTTGACCAGAACTCGACAGCCA GCAGTGTTGAAGCCAATCTTGACATTCAGTACACAACAGGTCTT GTTGCGCCCGACATTGAAACCCGCTACTTCACTGTTCCCGGTC GCGGTATCCTGATCCCTGATCTGGACCAGCCTACGGAGAGCGA CAACGCTAATGAGCCGTATCTGGATTACTTTACATATCTTAATAA CCTCGAAGACGAAGAACTCCCCGACGTGCTGACCACATCTTAC GGCGAGAGCGAGCAGAGTGTACCCGCCGAATATGCAAAAAAG GTGTGCAATTTGATCGGCCAGTTGGGTGCTCGTGGTGTGTCCG TCATCTTCTCCAGCGGTGATACTGGCCCTGGCTCTGCATGCCA AACCAATGATGGAAAAAACACGACACGTTTCTTGCCCATCTTCC CTGCTTCTTGCCCCTACGTCACTTCGGTTGGCGGCACTGTTGG TGTTGAGCCCGAAAAGGCTGTCAGCTTCTCTTCGGGCGGCTTT TCTGACCTATGGCCTCGACCCGCTTATCAAGAGAAGGCCGTAT CAGAATATCTTGAAAGCTCGGAGACCGCTGGAACGGGCTTTA CAACCCTCAAGGACGCGGATTTCCTGATGTAGCTGCTCAGGGC CAAGGCTTCCAGGTGTTTGACAAGGGCAGGCTGATTTCGGTCG GAGGAACGAGCGCTTCAGCTCCTGTTTTCGCATCCGTAGTCGC ACTCCTGAACAATGCTCGCAAGGCTGCCGGCATGTCTTCACTC GGCTTCTTGAACCCATGGATCTACGAGCAAGGCTACAAGGGCT TGACCGATATCGTTGCTGGAGGCTCGACAGGATGCACAGGAAG ATCCATCTATTCAGGCCTCCCAGCACCACTCGTGCCGTATGCTT CTTGGAATGCCACCGAAGGATGGGATCCGGTGACGGGCTATG GTACACCTGATTTCAAGCAATTGCTCACCCTCGCGACGGCACC CAAGTCTGGCGAGCGTCGCGTTCGTCGTGGCGGTCTCGGTGG CCAGGCTTAG | Bipolaris maydis C5 |

(continued)

| SEQ ID No.: | Sequence | Origin |
|---|---|---|
| 71 | ATGTTATCTTCCTTCCTTAGCCAGGGAGCAGCCGTATCCCTCGC GTTATTGTCGCTGCTCCCTTCGCCTGTAGCCGCGGAGATCTTC GAGAAGCTGTCCGGCGTCCCCAATGGCTGGAGATACGCCAACA ATCCTCACGGCAACGAGGTCATTCGCCTTCAAATCGCCCTTCAG CAGCACGATGTTGCCGGTTTCGAACAAGCCGTGATGGACATGT CCACCCCCGGTCACGCCGACTATGGAAAGCATTTCCGCACACA TGATGAGATGAAGCGCATGCTGCTCCCCAGCGACACTGCCGTC GACTCAGTTCGCGACTGGCTGGAATCCGCCGGAGTCCACAATA TCCAGGTCGACGCCGACTGGGTCAAGTTCCATACCACCGTCAA CAAGGCCAATGCCCTGCTGGATGCCGACTTCAAGTGGTATGTC AGCGAGGCCAAGCACATTCGTCGTCTACGCACCCTGCAATACT CCATCCCCGACGCCCTGGTCTCGCACATCAACATGATCCAGCC CACCACTCGCTTTGGCCAGATCCAGCCGAACCGTGCCACCATG CGCAGCAAGCCCAAGCACGCCGACGAGACATTCCTGACCGCA GCCACCTTGGCCCAGAACACCTCCCACTGCGACTCCATCATCA CGCCGCACTGTCTGAAGCAGCTCTACAACATCGGTGACTACCA GGCCGACCCCAAGTCCGGTAGCAAGGTCGGCTTCGCCAGCTA CCTCGAAGAATACGCCCGGTATGCCGATCTCGAAAGGTTCGAG CAGCACCTGGCTCCCAACGCCATCGGCCAGAACTTCAGCGTCG TTCAATTCAACGGCGGCCTCAACGACCAGCTTTCATTGAGCGAC AGCGGCGAAGCCAACCTCGACCTGCAGTACATCCTGGGCGTCA GCGCTCCCGTCCCGGTCACTGAATACAGCACTGGCGGACGCG GCGAACTGGTCCCCGACCTGAGCTCCCCGGACCCCAACGACA ACAGCAACGAGCCCTACCTCGACTTCCTCCAGGGTATTCTCAAA CTCGACAATTCCGACCTCCCCCAAGTCATCTCTACCTCCTACGG CGAAGACGAACAGACCATCCCCGTCCCCTACGCCCGCACAGTC TGCAATCTCTACGCCCAACTCGGCAGCCGCGGTGTCTCCGTGA TCTTCTCGAGCGGCGACTCCGGCGTCGGCGCCGCCTGCCTCA CCAACGACGGCACCAACCGCACCCACTTCCCTCCTCAATTCCC GGCCTCCTGCCCCTGGGTAACCTCCGTCGGTGCCACCAGCAAA ACCTCCCCGGAGCAAGCCGTCTCCTTCTCCTCAGGAGGCTTCT CCGACCTCTGGCCCCGCCCCTCCTACCAACAGGCTGCCGTCCA AACCTACCTCACCCAGCACCTGGGCAACAAGTTCTCAGGCCTC TTCAACGCCTCCGGCCGCGCCTTCCCCGACGTCGCCGCGCAG GGCGTCAACTACGCCGTCTACGACAAGGGCATGCTTGGCCAGT TCGATGGAACCAGTTGCTCCGCGCCGACGTTCAGTGGTGTCAT TGCCTTGTTGAATGACGCCAGACTGAGGGCGGGTTTGCCCGTT ATGGGATTCCTGAACCCGTTCCTCTATGGAGTTGGTAGTGAGA GTGGCGCGTTGAATGATATTGTCAACGGCGGGAGCCTGGGTTG TGATGGTAGGAATCGATTTGGAGGCACGCCCAATGGAAGTCCC GTTGTGCCGTTTGCTAGTTGGAATGCGACCACCGGGTGGGATC CGGTTTCTGGGCTGGGAACGCCGGATTTTGCGAAGTTGAGGGG TGTGGCGTTGGGTGAAGCTAAGGCGTATGGTAATTAA | Aspergillus kawachii IFO 4308 |

(continued)

| SEQ ID No.: | Sequence | Origin |
|---|---|---|
| 72 | AUGGCAGCGACUGGACGAUUCACUGCCUUCUGGAAUGUCGC GAGCGUGCCCGCCUUGAUUGGCAUUCUCCCCCUUGCUGGAU CUCAUUUAAGAGCUGUCCUUUGCCCGUCUGUAUCUGGCGUC ACUCGAAGGCCGUUUGUGCACCAGACACUUUGCAAGCCAUGC GCGCCUUCACCCGUGUAACGGCCAUCUCCCUGGCCGGUUUC UCCUGCUUCGCUGCUGCGGCGGCUGCGGCUUUUGAGAGCCU GCGAGCUGUCCCUGACGGCUGGAUCUACGAGAGCACCCCCG ACCCUAACCAACCGCUGCGUCUACGCAUCGCGCUGAAACAGC ACAAUGUCGCCGGCUUCGAGCAGGCACUGCUGGAUAUGUCCA CACCCGGUCACUCCAGCUACGGGCAGCAUUUCGGCUCCUACC ACGAGAUGAAGCAGCUGCUUCUCCCUACCGAGGAGGCGUCCU CCUCGGUGCGAGACUGGCUCUCGGCGGCGGGCGUUGAGUUC GAACAGGACGCCGACUGGAUCAACUUCCGCACGACCGUCGAC CAGGCUAACGCCCUCCUCGACGCCGAUUUCCUCUGGUACACA ACGACCGGCUCGACGGGCAACCCGACGCGGAUCCUCCGAACC CUCUCCUACAGCGUUCCCAGCGAGCUCGCUGGAUACGUCAAC AUGAUCCAGCCGACUACGCGUUUCGGCGGCACGCAUGCCAAC CGGGCCACCGUUCGCGCGAAGCCGAUCUUCCUCGAGACCAAC CGGCAGCUCAUCAACGCCAUCUCCUCUGGCUCGCUCGAGCAC UGCGAGAAGGCCAUCACCCCAUCGUGCCUGGCGGAUCUGUAC AACACUGAAGGGUACAAGGCGUCCAACCGCAGCGGGAGCAAG GUGGCCUUUGCCUCCUUCCUCGAAGAGUACGCGCGCUACGA CGAUCUCGCCGAGUUCGAGGAGACCUACGCUCCCUAUGCGAU CGGGCAGAACUUCUCGGUUAUCUCCAUCAACGGCGGCCUCAA CGACCAGGACUCCACGGCCGACAGCGGCGAGGCGAACCUCGA CCUGCAGUACAUCAUCGGCGUCUCGUCGCCGCUACCUGUGAC CGAGUUCACAACCGGUGGCCGCGGCAAGCUCAUUCCUGACCU CUCCUCCCCCGACCCGAAUGACAACACCAACGAGCCUUUCCU UGACUUCCUUGAGGCCGUCCUCAAGCUCGAUCAGAAAGACCU GCCCCAGGUCAUCUCGACCUCCUACGGCGAGGACGAGCAGAC AAUCCCUGAGCCGUACGCCCGCUCCGUCUGCAACCUGUACGC UCAGCUCGGUUCCCGCGGCGUGUCUGUGCUCUUCUCCUCGG GUGACUCUGGCGUCGGCGCCGCCUGCCAGACCAACGAUGGC AAAAACACGACGCACUUCCCGCCGCAGUUCCCGGCCUCUUGC CCCUGGGUGACCGCCGUCGGCGGCACGAACGGCACAGCGCC CGAAUCCGGUGUAUACUUCUCCAGCGGCGGGUUCUCCGACUA CUGGGCGCGCCCGGCGUACCAGAACGCCGCGGUUGAGUCAU ACCUGCGCAAACUCGGUAGCACACAGGCGCAGUACUUCAACC GCAGCGGACGCGCCUUCCCGGACGUCGCAGCGCAGGCGCAG AACUUCGCUGUCGUCGACAAGGGCCGUGUCGGUCUCUUCGA CGGAACGAGCUGCAGUUCGCCUGUAUUUGCGGGCAUCGUGG CGUUGCUCAACGACGUGCGUCUGAAGGCAGGCCUGCCCGUG CUGGGAUUCCUCAACCCUUGGCUCUACCAGGAUGGCCUGAAC GGGCUCAACGAUAUCGUGGAUGGAGGGAGCACCGGCUGCGA CGGGAACAACCGGUUUAACGGAUCGCCAAAUGGGAGCCCCGU AAUCCCGUAUGCGGGGUUGGAACGCGACGGAGGGGUGGGAUC CUGUGACGGGGCUGGGAACGCCGGAUUUCGCGAAGCUGAAA GCGCUCGUGCUUGAUGCUUAG | Aspergillus nidulans FGSC A4 |

| SEQ ID No.: | Sequence | Origin |
|---|---|---|
| 73 | ATGTTGTCATTTGTTCGTCGGGGAGCTCTCTCCCTCGCTCTCGT TTCGCTGTTGACCTCGTCTGTCGCCGCCGAGGTCTTCGAGAAG CTGCATGTTGTGCCCGAAGGTTGGAGATATGCCTCCACTCCTAA CCCCAAACAACCCATTCGTCTTCAGATCGCTCTGCAGCAGCAC GATGTCACCGGTTTCGAACAGTCCCTCTTGGAGATGTCGACTC CCGACCATCCCAACTACGGAAAACACTTCCGCACCCACGATGA GATGAAGCGCATGCTTCTCCCCAATGAAAATGCCGTTCACGCC GTCCGCGAATGGCTGCAAGACGCCGGAATCAGCGACATCGAA GAAGACGCCGATTGGGTCCGTTTCCACACCACCGTGGACCAGG CCAACGACCTCCTCGACGCCAACTTCCTCTGGTACGCGCACAA GAGCCATCGTAACACGGCGCGTCTCCGCACTCTCGAGTACTCG ATCCCAGACTCTATTGCGCCGCAGGTCAACGTGATCCAGCCAA CCACGCGATTCGGACAGATCCGTGCCAACCGGGCTACGCATAG CAGCAAGCCCAAGGGTGGGCTTGACGAGTTGGCTATCTCGCAG GCAGCTACGGCGGATGATGATAGCATTTGTGACCAGATCACCA CCCCACACTGTCTGCGGAAGCTGTACAATGTCAATGGCTACAA GGCCGATCCCGCTAGTGGTAGCAAGATCGGTTTTGCTAGTTTC CTGGAGGAATACGCGCGGTACTCTGATCTGGTACTGTTCGAGG AGAACCTGGCACCGTTTGCGGAGGGTGAGAACTTTACTGTCGT CATGTACAACGGCGGCAAGAATGACCAGAACTCCAAGAGCGAC AGCGGCGAGGCCAACCTCGATCTGCAGTACATCGTGGGAATGA GCGCGGGCGCGCCCGTGACCGAGTTCAGCACCGCCGGTCGC GCACCCGTCATCCCGGACCTGGACCAGCCCGACCCCAGCGCC GGTACCAACGAGCCGTACCTCGAGTTCCTGCAGAACGTGCTAC ACATGGACCAGGAGCACCTGCCGCAGGTGATCTCTACTTCCTA CGGTGAGAACGAACAGACCATCCCCGAAAAGTACGCCCGCACC GTTTGCAACATGTACGCGCAGCTGGGCAGCCGCGGTGTGTCG GTGATTTTCTCGTCGGGCGACTCCGGCGTCGGCTCTGCCTGTA TGACCAACGACGGTACAAACCGCACCCACTTCCCCCCGCAGTT CCCGGCGTCCTGCCCCTGGGTGACATCGGTCGGGGCCACTGA GAAGATGGCCCCCGAGCAAGCGACATATTTCTCCTCGGGCGGC TTCTCTGACCTCTTCCCGCGCCCAAAGTACCAGGACGCTGCTG TCAGCAGCTACCTTCAGACCCTCGGATCCCGGTACCAGGGCTT GTACAACGGTTCCAACCGTGCATTCCCTGACGTCTCGGCGCAG GGTACCAACTTTGCTGTGTACGACAAGGGCCGTCTAGGCCAGT TCGATGGTACTTCTTGCTCTGCTCCCGCGTTTAGCGGTATCATC GCCTTGCTCAACGACGTCCGTCTCCAGAACAACAAGCCCGTCC TGGGCTTCTTGAACCCCTGGTTGTATGGCGCTGGGAGCAAGGG CCTGAACGACGTCGTGCACGGTGGCAGTACAGGATGCGATGG ACAGGAGCGGTTTGCAGGAAAGGCCAATGGAAGCCCCGTCGT GCCGTACGCTAGCTGGAATGCTACGCAAGGCTGGGATCCAGTC ACTGGCCTTGGAACGCCGGATTTCGGCAAGTTGAAGGATTTGG CTCTGTCGGCTTAA | Aspergillus ruber CBS 135680 |

(continued)

| SEQ ID No.: | Sequence | Origin |
|---|---|---|
| 74 | AUGUUGCCCUCUCUUGUAAACAACGGGGCGCUGUCCCUGGC UGUGCUUUCGCUGCUCACCUCGUCCGUCGCCGGCGAGGUGU UUGAGAAGCUGUCGGCCGUGCCGAAAGGAUGGCACUUCUCC CACGCUGCCCAGGCCGACGCCCCCAUCAACCUGAAGAUCGCC CUGAAGCAGCAUGAUGUCGAGGGCUUCGAGCAGGCCCUGCU GGACAUGUCCACCCCGGGCCACGAGAACUACGGCAAGCACUU CCACGAGCACGACGAGAUGAAACGCAUGCUGCUCCCCAGCGA CUCCGCCGUCGACGCCGUCCAGACCUGGCUGACCUCCGCCG GCAUCACCGACUACGACCUCGACGCCGACUGGAUCAACCUGC GCACCACCGUCGAGCACGCCAACGCCCUGCUGGACACGCAGU UCGGCUGGUACGAGAACGAAGUGCGCCACAUCACGCGCCUGC GCACCCUGCAAUACUCCAUCCCCGAGACCGUCGCCGCGCACA UCAACAUGGUGCAGCCGACCACGCGCUUUGGCCAGAUCCGGC CCGACCGCGCGACCUUCCACGCGCACCACACCUCCGACGCGC GCAUCCUGUCCGCCCUGGCCGCCGCCAGCAACAGCACCAGCU GCGACUCAGUCAUCACCCCCAAGUGCCUCAAGGACCUCUACA AGGUCGGCGACUACGAGGCCGACCCGGACUCGGGCAGCCAG GUCGCCUUCGCCAGCUACCUCGAGGAAUACGCCCGCUACGCC GACAUGGUCAAGUUCCAGAACUCGCUCGCCCCCUACGCCAAG GGCCAGAACUUCUCGGUCGUCCUGUACAACGGCGGCGUCAAC GACCAGUCGUCCAGCGCCGACUCCGGCGAGGCCAACCUCGAC CUGCAGACCAUCAUGGGCCUCAGCGCGCCGCUCCCCAUCACC GAGUACAUCACCGGCGGCCGCGGCAAGCUCAUCCCCGAUCUC AGCCAGCCCAACCCCAACGACAACAGCAACGAGCCCUACCUC GAGUUCCUCCAGAACAUCCUCAAGCUGGACCAGGACGAGCUG CCGCAGGUGAUCUCGACCUCCUACGGCGAGGACGAGCAGACA AUCCCCCGUGGCUACGCCGAAUCCGUCUGCAACAUGCUGGCC CAGCUCGGCAGCCGCGGCGUGUCGGUGGUCUUCUCGUCAGG CGAUUCGGGCGUCGGCGCCGCCUGCCAGACCAACGACGGCC GCAACCAAACCCACUUCAACCCGCAGUUCCCGGCCAGCUGCC CGUGGGUGACGUCGGUCGGGGCCACGACCAAGACCAACCCG GAGCAGGCGGUGUACUUCUCGUCGGGCGGGUUCUCGGACUU CUGGAAGCGCCCGAAGUACCAGGACGAGGCGGUGGCCGCGU ACCUGGACACGCUGGGCGACAAGUUCGCGGGGCUGUUCAAC AAGGGCGGGCGCGCGUUCCGGACGUCGCGGCGCAGGGCAU GAACUACGCCAUCUACGACAAGGGCACGCUGGGCCGGCUGGA CGGCACCUCGUGCUCGGCGCCGGCCUUCUCGGCCAUCAUCU CGCUGCUGAACGAUGCGCGCCUGCGCGAGGGUAAGCCGACC AUGGGCUUCUUGAACCCGUGGCUGUAUGGUGAGGGCCGCGA GGCGCUGAAUGAUGUUGUCGUGGGUGGGAGCAAGGGCUGUG AUGGGCGCGACCGGUUUGGCGGCAAGCCCAAUGGGAGCCCU GUCGUGCCUUUUGCUAGCUGGAAUGCUACGCAGGGCUGGGA CCCGGUUACUGGGCUGGGGACGCCGAACUUUGCGAAGAUGU UGGAGCUGGCGCCAUAG | Aspergillus terreus NIH2624 |

(continued)

| SEQ ID No.: | Sequence | Origin |
|---|---|---|
| 75 | ATGATTGCATCATTATTCAACCGTAGGGCATTGACGCTCGCTTT ATTGTCACTTTTTGCATCCTCTGCCACAGCCGATGTTTTTGAGA GTTTGTCTGCTGTTCCTCAGGGATGGAGATATTCTCGCACACCG AGTGCTAATCAGCCCTTGAAGCTACAGATTGCTCTGGCTCAGG GAGATGTTGCTGGGTTCGAGGCAGCTGTGATCGATATGTCAAC CCCCGACCACCCCAGTTACGGGAACCACTTCAACACCCACGAG GAAATGAAGCGGATGCTGCAGCCTAGCGCGGAGTCCGTAGACT CGATCCGTAACTGGCTCGAAAGTGCCGGTATTTCCAAGATCGA ACAGGACGCTGACTGGATGACCTTCTATACCACCGTGAAGACA GCGAATGAGCTGCTGGCAGCCAACTTCCAGTTCTACATCAATG GAGTCAAGAAAATAGAGCGTCTCCGCACACTCAAGTACTCTGTC CCGGACGCTTTGGTGTCCCACATTAACATGATCCAGCCAACCA CCCGTTTCGGCCAGCTGCGCGCCCAGCGCGCCATTTTACACAC CGAGGTCAAGGATAACGACGAGGCTTTCCGCTCAAATGCCATG TCCGCTAATCCGGACTGCAACAGCATCATCACTCCCCAGTGTCT CAAGGATTTGTACAGTATCGGTGACTATGAGGCCGACCCCACC AATGGGAACAAGGTCGCGTTTGCCAGCTACCTAGAGGAGTATG CCCGATACTCCGATCTCGCATTATTTGAGAAAAACATCGCCCCC TTTGCCAAGGGACAGAATTTCTCCGTTGTCCAGTATAACGGCGG TGGTAATGATCAACAATCGAGCAGTGGCAGTAGTGAGGCGAAT CTTGACTTGCAGTACATCGTTGGAGTCAGCTCTCCTGTTCCCGT TACAGAGTTTAGCACTGGAGGTCGCGGTGAACTTGTTCCGGAT CTCGACCAGCCGAATCCCAATGACAACAACAACGAGCCATACC TTGAATTCCTCCAGAACGTGCTCAAGTTGCACAAGAAGGACCTC CCCCAGGTGATTTCCACCTCTTATGGCGAGGACGAGCAGAGCG TTCCAGAGAAGTACGCCCGCGCCGTTTGCAACCTGTACTCCCA ACTCGGTAGCCGTGGTGTGTCCGTAATCTTTTCATCCGGCGACT CTGGCGTTGGCGCCGCGTGTCAGACGAACGACGGCCGGAACG CGACCCACTTCCCACCCCAGTTCCCGGCCGCCTGCCCCTGGGT GACATCAGTCGGTGCGACAACCCACACTGCGCCCGAACGAGC CGTTTACTTCTCATCTGGCGGTTTCTCCGATCTCTGGGATCGCC CTACGTGGCAAGAAGATGCTGTGAGTGAGTACCTCGAGAACCT GGGCGACCGCTGGTCTGGCCTCTTCAACCCTAAGGGCCGTGC CTTCCCCGACGTCGCAGCCCAGGGTGAAAACTACGCCATCTAC GATAAGGGTTCTTTGATCAGCGTCGATGGCACCTCTTGCTCGG CACCTGCGTTTGCCGGAGTCATCGCCCTCCTCAACGACGCCCG CATCAAGGCCAATAGACCACCCATGGGCTTCCTCAACCCTTGG CTGTACTCTGAAGGCCGCAGCGGCCTAAACGACATTGTCAACG GCGGTAGCACTGGCTGCGACGGTCATGGCCGCTTCTCCGGCC CCACTAACGGTGGTACGTCGATTCCAGGTGCCAGCTGGAACGC TACTAAGGGCTGGGACCCTGTCTCCGGTCTTGGATCGCCCAAC TTTGCTGCCATGCGCAAACTCGCCAACGCTGAGTAG | Penicillium digitatum Pd1 |

(continued)

| SEQ ID No.: | Sequence | Origin |
|---|---|---|
| 76 | ATGCATGTTCCTCTGTTGAACCAAGGCGCGCTGTCGCTGGCCG TCGTCTCGCTGTTGGCCTCCACGGTCTCGGCCGAAGTATTCGA CAAGCTTGTCGCTGTCCCTGAAGGATGGCGATTCTCCCGCACT CCCAGTGGAGACCAGCCCATCCGACTGCAGGTTGCCCTCACAC AGGGTGACGTTGAGGGCTTCGAGAAGGCCGTTCTGGACATGTC AACTCCCGACCACCCCAACTATGGCAAGCACTTCAAGTCACAC GAGGAAGTTAAGCGCATGCTGCAGCCTGCAGGCGAGTCCGTC GAAGCCATCCACCAGTGGCTCGAGAAGGCCGGCATCACCCACA TTCAACAGGATGCCGACTGGATGACCTTCTACACCACCGTTGA GAAGGCCAACAACCTGCTGGATGCCAACTTCCAGTACTACCTC AACGAGAACAAGCAGGTCGAGCGTCTGCGCACCTTGGAGTACT CGGTTCCTGACGAGCTCGTCTCGCACATTAACCTTGTCACCCCC GACCACTCGCTTCGGCCAGCTGCACGCCGAGGGTGTGACGCT GCACGGCAAGTCTAAGGACGTCGACGAGCAATTCCGCCAGGCT GCTACTTCCCCTAGCAGCGACTGCAACAGTGCTATCACCCCGC AGTGCCTCAAGGACCTGTACAAGGTCGGCGACTACAAGGCCAG TGCCTCCAATGGCAACAAGGTCGCCTTCACCAGCTACCTGGAG CAGTACGCCCGGTACTCGGACCTGGCTCTGTTTGAGCAGAACA TTGCCCCCTATGCTCAGGGCCAGAACTTCACCGTTATCCAGTAC AACGGTGGTCTGAACGACCAGAGCTCGCCTGCGGACAGCAGC GAGGCCAACCTGGATCTCCAGTACATTATCGGAACGAGCTCTC CCGTCCCCGTGACTGAGTTCAGCACCGGTGGTCGTGGTCCCTT GGTCCCCGACTTGGACCAGCCTGACATCAACGACAACAACAAC GAGCCTTACCTCGACTTCTTGCAGAATGTCATCAAGATGAGCGA CAAGGATCTTCCCCAGGTTATCTCCACCTCGTACGGTGAGGAC GAGCAGAGCGTCCCCGCAAGCTACGCTCGTAGCGTCTGCAACC TCATCGCTCAGCTCGGCGGCCGTGGTGTCTCCGTGATCTTCTC ATCTGGTGATTCCGGTGTGGGCTCTGCCTGTCAGACCAACGAC GGCAAGAACACCACTCGCTTCCCCGCTCAGTTCCCCGCCGCCT GCCCCTGGGTGACCTCTGTTGGTGCTACTACCGGTATCTCCCC CGAGCGCGGTGTCTTCTTCTCCTCCGGTGGCTTCTCCGACCTC TGGAGCCGCCCCTCGTGGCAAAGCCACGCCGTCAAGGCCTAC CTTCACAAGCTTGGCAAGCGTCAAGACGGTCTCTTCAACCGCG AAGGCCGTGCGTTCCCCGACGTGTCAGCCCAGGGTGAGAACTA CGCTATCTACGCGAAGGGTCGTCTCGGCAAGGTTGACGGCACT TCCTGCTCGGCTCCCGCTTTCGCCGGTCTGGTTTCTCTGCTGA ACGACGCTCGCATCAAGGCGGGCAAGTCCAGCCTCGGCTTCCT GAACCCCTGGTTGTACTCGCACCCCGATGCCTTGAACGACATC ACCGTCGGTGGAAGCACCGGCTGCGACGGCAACGCTCGCTTC GGTGGTCGTCCCAACGGCAGTCCCGTCGTCCCTTACGCTAGCT GGAACGCTACTGAGGGCTGGGACCCCGTCACCGGTCTGGGTA CTCCCAACTTCCAGAAGCTGCTCAAGTCTGCCGTTAAGCAGAA GTAA | Penicillium oxalicum 114-2 |

(continued)

| SEQ ID No.: | Sequence | Origin |
|---|---|---|
| 77 | ATGATTGCATCCCTATTTAGTCGTGGAGCATTGTCGCTCGCGGT CTTGTCGCTTCTCGCGTCCTCTGCTGCAGCCGATGTATTTGAGA GTTTGTCTGCTGTTCCTCAAGGATGGAGATATTCTCGCAGGCCG CGTGCTGATCAGCCCTTGAAGTTACAGATCGCTCTGACACAGG GGGATACTGCCGGCTTCGAAGAGGCTGTGATGGAGATGTCAAC CCCCGATCACCCTAGCTACGGGCACCACTTCACCACCCACGAA GAAATGAAGCGGATGCTACAGCCCAGTGCGGAGTCCGCGGAG TCAATCCGTGACTGGCTCGAAGGCGCGGGTATTACCAGGATCG AACAGGATGCAGATTGGATGACCTTCTACACCACCGTGGAGAC GGCAAATGAGCTGCTGGCAGCCAATTTCCAGTTCTACGTCAGTA ATGTCAGGCACATTGAGCGTCTTCGCACACTCAAGTACTCAGTC CCGAAGGCTCTGGTGCCACACATCAACATGATCCAGCCAACCA CCCGTTTCGGCCAGCTGCGCGCCCATCGGGGCATATTACACGG CCAGGTCAAGGAATCCGACGAGGCTTTCCGCTCAAACGCCGTG TCCGCTCAGCCGGATTGCAACAGTATCATCACTCCTCAGTGTCT CAAGGATATATATAATATCGGTGATTACCAGGCCAATGATACCA ATGGGAACAAGGTCGGGTTTGCCAGCTACCTAGAGGAGTATGC ACGATACTCCGATCTGGCACTATTTGAGAAAAATATCGCGCCCT CTGCCAAGGGCCAGAACTTCTCCGTCACCAGGTACAACGGCGG TCTTAATGATCAAAGTTCCAGCGGTAGCAGCAGCGAGGCGAAC CTGGACTTGCAGTACATTGTTGGAGTCAGCTCTCCTGTTCCCGT CACCGAATTTAGCGTTGGCGGCCGTGGTGAACTTGTTCCCGAT CTCGACCAGCCTGATCCCAATGATAACAACAACGAGCCATACCT TGAATTCCTCCAGAACGTGCTCAAGCTGGACAAAAAGGACCTTC CCCAGGTGATTTCTACCTCCTATGGTGAGGACGAGCAGAGCAT TCCCGAGAAGTACGCCCGCAGTGTTTGCAACTTGTACTCGCAG CTCGGTAGCCGTGGTGTATCCGTCATTTTCTCATCTGGCGACTC CGGCGTTGGGTCCGCGTGCCTGACGAACGACGGCAGGAACGC GACCCGCTTCCCACCCCAGTTCCCCGCCGCCTGCCCGTGGGT GACATCAGTCGGCGCGACAACCCATACCGCGCCCGAACAGGC CGTGTACTTCTCGTCCGGCGGCTTTTCCGATCTCTGGGCTCGC CCGAAATGGCAAGAGGAGGCCGTGAGTGAGTACCTCGAGATCC TGGGTAACCGCTGGTCTGGCCTCTTCAACCCTAAGGGTCGTGC CTTCCCCGATGTCACAGCCCAAGGTCGCAATTACGCTATATACG ATAAGGGCTCGTTGACCAGCGTCGACGGCACCTCCTGCTCGGC ACCTGCCTTCGCCGGAGTCGTCGCCCTCCTCAACGACGCTCGC CTCAAAGTCAACAAACCACCAATGGGCTTCCTTAATCCTTGGCT GTACTCGACAGGGCGCGCCGGCCTAAAGGACATTGTCGATGG CGGCAGCACGGGTTGCGATGGCAAGAGCCGCTTCGGTGGTGC CAATAACGGTGGTCCGTCGATCCCAGGTGCTAGCTGGAACGCT ACTAAGGGTTGGGACCCTGTTTCTGGTCTCGGGTCGCCCAACT TTGCTACCATGCGCAAGCTTGCGAACGCTGAGTAG | Penicillium roqueforti FM164 |

| SEQ ID No.: | Sequence | Origin |
|---|---|---|
| 78 | AUGAUUGCAUCUCUAUUUAACCGUGGAGCAUUGUCGCUCGCG GUAUUGUCGCUUCUCGCGUCUUCGGCUUCCGCUGAUGUAUU UGAGAGUUUGUCUGCUGUUCCUCAAGGAUGGAGAUAUUCUCG CAGACCGCGUGCUGAUCAGCCCCUGAAGCUACAGAUUGCUCU GGCACAAGGGGAUACUGCCGGAUUCGAAGAGGCUGUGAUGG ACAUGUCAACCCCUGAUCACCCCAGCUACGGGAACCACUUCC ACACCCACGAGGAAAUGAAGCGGAUGCUGCAGCCCAGCGCGG AGUCCGCAGACUCGAUCCGUGACUGGCUUGAAAGUGCGGGU AUCAAUAGAAUUGAACAGGAUGCCGACUGGAUGACAUUCUAC ACCACCGUCGAGACGGCAAAUGAGCUGCUGGCAGCCAAUUUC CAGUUCUAUGCCAACAGUGCCAAGCACAUUGAGCGUCUUCGC ACACUCCAGUACUCCGUCCCGGAGGCUCUGAUGCCACACAUC AACAUGAUCCAGCCAACCACUCGUUUCGGCCAGCUGCGCGUC CAGGGGGCCAUAUUGCACACCCAGGUCAAGGAAACCGACGAG GCUUUCCGCUCAAACGCCGUGUCCACUUCACCGGACUGCAAC AGUAUCAUCACUCCUCAGUGUCUCAAGAAUAUGUACAAUGUG GGUGACUACCAGGCCGACGACGACAAUGGGAACAAGGUCGGA UUUGCCAGCUACCUAGAGGAGUAUGCACGGUACUCCGAUUUG GAACUAUUUGAGAAAAAUGUCGCACCCUUCGCCAAGGGCCAG AACUUCUCCGUCAUCCAGUAUAACGGCGGUCUUAACGAUCAA CACUCGAGUGCUAGCAGCAGCGAGGCGAACCUUGACUUACAG UACAUUGUUGGAGUUAGCUCUCCUGUUCCAGUUACAGAGUUU AGCGUUGGCGGUCGUGGUGAACUUGUUCCCGAUCUUGACCA GCCUGAUCCCAAUGAUAACAACAACGAGCCAUACCUUGAAUU CCUCCAGAACGUGCUCAAGAUGGAACAACAGGACCUCCCCCA GGUGAUUUCCACCUCUUAUGGCGAGAACGAGCAGAGUGUUCC CGAGAAAUACGCCCGCACCGUAUGCAACUUGUUCUCGCAGCU UGGCAGCCGUGGUGUGUCCGUCAUCUUCGCAUCUGGCGACU CCGGCGUUGGCGCCGCGUGCCAGACGAAUGACGGCAGGAAC GCGACCCGCUUCCCGGCCCAGUUCCCUGCUGCCUGCCCAUG GGUGACAUCGGUCGGCGCGACAACCCACACCGCGCCCGAGAA GGCCGUGUACUUCUCGUCCGGUGGCUUCUCCGAUCUUUGGG AUCGCCCGAAAUGGCAAGAAGACGCCGUGAGUGACUACCUCG ACACCCUGGGCGACCGCUGGUCCGGCCUCUUCAAUCCUAAGG GCCGUGCCUUCCCCGACGUCUCAGCCCAAGGUCAAAACUACG CCAUAUACGAUAAGGGCUCGUUGACCAGCGUCGACGGCACCU CGUGCUCGGCACCCGCCUUCGCCGGUGUCAUCGCCCUCCUC AACGACGCCCGCCUCAAGGCCAACAAACCACCCAUGGGCUUC CUCAAUCCCUGGCUGUACUCGACAGGCCGUGACGGCCUGAAC GACAUUGUUCAUGGCGGCAGCACUGGCUGUGAUGGCAACGC CCGCUUCGGCGGCCCCGGUAACGGCAGUCCGAGGGUUCCAG GUGCCAGCUGGAACGCUACUAAGGGCUGGGACCCUGUUUCU GGUCUUGGAUCACCCAACUUUGCUACCAUGCGCAAGCUCGCG AACGGUGAGUAG | Penicillium rubens Wisconsin 54-1255 |

(continued)

| SEQ ID No.: | Sequence | Origin |
|---|---|---|
| 79 | AUGCUGUCCUCGACUCUCUACGCAGGGUUGCUCUGCUCCCU CGCAGCCCCAGCCCUUGGUGUGGUGCACGAGAAGCUCUCAG CUGUUCCUAGUGGCUGGACACUCGUCGAGGAUGCAUCGGAG AGCGACACGACCACUCUCUCAAUUGCCCUUGCUCGGCAGAAC CUCGACCAGCUCGAGUCCAAGUUGACCACACUGGCGACCCCA GGGAACGCGGAGUACGGCAAGUGGCUGGACCAGUCCGACAU UGAGUCCCUAUUUCCUACUGCAAGCGAUGACGCUGUUAUCCA AUGGCUCAAGGAUGCCGGGGUCACCCAAGUGUCUCGUCAGG GCAGCUUGGUGAACUUUGCCACCACUGUGGGAACGGCGAACA AGCUCUUUGACACCAAGUUCUCCUACUACCGCAAUGGUGCUU CCCAGAAACUGCGUACCACGCAGUACUCCAUUCCCGAUAGCC UGACAGAGUCGAUCGAUCUGAUUGCCCCCACUGUCUUCUUUG GCAAGGAGCAAGACAGCGCACUGCCACCUCACGCAGUGAAGC UUCCAGCCCUUCCCAGGAGGGCAGCCACCAACAGUUCUUGCG CCAACCUGAUCACUCCCGACUGCCUAGUGGAGAUGUACAACC UCGGCGACUACAAGCCUGAUGCAUCUUCGGGCAGUCGAGUC GGCUUUGGUAGCUUCUUGAAUCAGUCAGCCAACUAUGCAGAU CUGGCUGCUUAUGAGCAACUGUUCAACAUCCCACCCCAGAAU UUCUCAGUCGAAUUGAUUAACGGAGGCGCCAAUGAUCAGAAU UGGGCCACUGCUUCCCUCGGCGAGGCCAAUCUGGACGUGGA GUUGAUUGUAGCCGUCAGCCACGCCCUGCCAGUAGUGGAGU UUAUCACUGGCGGUUCACCUCCGUUUGUUCCCAAUGUCGACG AGCCAACCGCUGCGGACAACCAGAAUGAGCCCUACCUCCAGU ACUACGAGUACUUGCUCUCCAAACCCAACUCCCAUCUUCCUC AGGUGAUUUCCAACUCGUAUGGUGACGAUGAACAGACUGUUC CCGAGUACUACGCCAGGAGAGUUUGCAACUUGAUCGGCUUGA UGGGUCUUCGUGGUAUCACUGUGCUCGAGUCCUCUGGUGAU ACCGGAAUCGGCUCGGCGUGCAUGUCCAAUGACGGCACCAAC ACGCCUCAGUUCACUCCUACAUUCCCUGGCACCUGCCCCUUC AUCACCGCAGUUGGUGGUACACAGUCCUAUGCUCCUGAAGUU GCCUGGGACGCCAGCUCGGGUGGAUUCAGCAACUACUUCAG CCGUCCCUGGUACCAGUAUUUCGCGGUGGAGAACUACCUCAA UAAUCACAUUACCAAGGACACCAAGAAGUACUAUUCGCAGUAC ACCAACUUCAAGGGCCGUGGAUUCCCUGAUGUUUCUGCCCAU AGCUUGACCCCUGACUACGAGGUCGUCCUAACUGGCAAACAU UACAAGUCCGGUGGCACAUCGGCCGCCUGCCCCGUCUUUGC UGGUAUCGUCGGCCUGUUGAAUGACGCCCGUCUGCGCGCCG GCAAGUCCACCCUUGGCUUCCUGAACCCAUUGCUGUAUAGCA UACUCGCGGAAGGAUUCACCGAUAUCACUGCCGGAAGUUCUA UCGGUUGUAAUGGUAUCAACCCACAGACCGGAAAGCCAGUCC CCGGUGGUGGUAUCAUCCCCUACGCUCACUGGAACGCUACUG CCGGCUGGGAUCCUGUUACAGGUCUUGGGGUUCCUGAUUUC AUGAAGUUGAAGGAGUUGGUUUUGUCGUUGUAA | Neosartorya fischeri NRRL 181 |

(continued)

| SEQ ID No.: | Sequence | Origin |
|---|---|---|
| 80 | AUGCUGUCCUCGACUCUCUACGCAGGGUGGCUCCUCUCCCU CGCAGCCCCAGCCCUUUGUGUGGUGCAGGAGAAGCUCUCAG CUGUUCCUAGUGGCUGGACACUCAUCGAGGAUGCAUCGGAGA GCGACACGAUCACUCUCUCAAUUGCCCUUGCUCGGCAGAACC UCGACCAGCUUGAGUCCAAGCUGACCACGCUGGCGACCCCAG GGAACCCGGAGUACGGCAAGUGGCUGGACCAGUCCGACAUU GAGUCCCUAUUUCCUACUGCAAGCGAUGAUGCUGUUCUCCAA UGGCUCAAGGCGGCCGGGAUUACCCAAGUGUCUCGUCAGGG CAGCUUGGUGAACUUCGCCACCACUGUGGGAACAGCGAACAA GCUCUUUGACACCAAGUUCUCUUACUACCGCAAUGGUGCUUC CCAGAAACUGCGUACCACGCAGUACUCCAUCCCCGAUCACCU GACAGAGUCGAUCGAUCUGAUUGCCCCCACUGUCUUCUUUGG CAAGGAGCAGAACAGCGCACUGUCAUCUCACGCAGUGAAGCU UCCAGCUCUUCCUAGGAGGGCAGCCACCAACAGUUCUUGCGC CAACCUGAUCACCCCCGACUGCCUAGUGGAGAUGUACAACCU CGGCGACUACAAACCUGAUGCAUCUUCGGGAAGUCGAGUCGG CUUCGGUAGCUUCUUGAAUGAGUCGGCCAACUAUGCAGAUUU GGCUGCGUAUGAGCAACUCUUCAACAUCCCACCCCAGAAUUU CUCAGUCGAAUUGAUCAACAGAGGCGUCAAUGAUCAGAAUUG GGCCACUGCUUCCUCGGCGAGGCCAAUCUGGACGUGGAGU UGAUUGUAGCCGUCAGCCACCCCCUGCCAGUAGUGGAGUUUA UCACUGGCGCCCUACCUCCAGUACUACGAGUACUUGCUCUCC AAACCCAACUCCCAUCUUCCUCAGGUGAUUUCCAACUCACUG UUCCCGAGUACUACGCCAGGAGAGUUUGCAACUUGAUCGGCU UGAUGGGUCUUCGUGGCAUCACGGUGCUCGAGUCCUCUGGU GAUACCGGAAUCGGCUCGGCAUGCAUGUCCAAUGACGGCACC AACAAGCCCCAAUUCACUCCUACAUUCCCUGGCACCUGCCCC UUCAUCACCGCAGUUGGUGGUACUCAGUCCUAUGCUCCUGAA GUUGCUUGGGACGGCAGUUCCGGCGGAUUCAGCAACUACUU CAGCCGUCCUGGUACCAGUCUUUCGCGGUGGACAACUACCU CAACAACCACAUUACCAAGGAUACCAAGAAGUACUAUUCGCAG UACACCAACUUCAAGGGCCGUGGAUUCCCUGAUGUUUCCGCC CAUAGUUUGACCCCUUACUACGAGGUCGUCUUGACUGGCAAA CACUACAAGUCUGGCGGCACAUCCGCCGCCAGCCCCGUCUUU GCCGGUAUUGUCGGUCUGCUGAACGACGCCCGUCUGCGCGC CGGCAAGUCCACUCUUGGCUUCCUGAACCCAUUGCUGUAUAG CAUCCUGGCCGAAGGAUUCACCGAUAUCACUGCCGGAAGUUC AAUCGGUUGUAAUGGUAUCAACCCACAGACCGGAAAGCCAGU UCCUGGUGGUGGUAUUAUCCCCUACGCUCACUGGAACGCUAC UGCCGGCUGGGAUCCUGUUACUGGCCUUGGGGUUCCUGAUU UCAUGAAAUUGAAGGAGUUGGUUCUGUCGUUGUAA | Aspergillus fumigatus CAE17675 |

(continued)

| SEQ ID No.: | Sequence | Origin |
|---|---|---|
| 81 | <u>ATGCAGACCTTCGGTGCTTTTCTCGTTTCCTTCCTCGCCGCCAG CGGCCTGGCCGCGGCC</u>GAGCCCTTCGAGAAGCTCTTTAGCAC CCCCGAGGGCTGGAAGATGCAGGGCCTCGCCACCAACGAGCA GATCGTCAAGCTCCAGATCGCCCTCCAGCAGGGCGACGTGGC CGGCTTTGAGCAGCACGTCATCGACATCAGCACCCCCAGCCAC CCCAGCTACGGCGCTCACTACGGCAGCCACGAAGAGATGAAG CGCATGATCCAGCCCAGCAGCGAGACTGTCGCCAGCGTCAGC GCCTGGCTCAAGGCCGCTGGCATCAACGACGCCGAGATCGAC AGCGACTGGGTCACCTTCAAGACCACCGTCGGCGTCGCCAACA AGATGCTCGACACCAAGTTCGCCTGGTACGTCAGCGAGGAAGC CAAGCCCCGCAAGGTCCTCCGCACCCTTGAGTACAGCGTCCCC GACGACGTCGCCGAGCACATCAACCTCATCCAGCCCACCACCC GCTTCGCCGCCATCCGCCAGAACCACGAGGTCGCCCACGAGA TCGTCGGCCTCCAGTTTGCCGCCCTCGCCAACAACACCGTCAA CTGCGACGCCACCATCACCCCCCAGTGCCTCAAGACCCTCTAC AAGATCGACTACAAGGCCGACCCCAAGAGCGGCAGCAAGGTC GCCTTCGCCAGCTACCTTGAGCAGTACGCCCGCTACAACGACC TCGCCCTCTTCGAGAAGGCCTTCCTGCCTGAGGCCGTCGGCCA GAACTTCAGCGTCGTCCAGTTCTCTGGCGGCCTCAACGACCAG AACACCACCCAGGATAGCGGCGAGGCCAACCTCGACCTCCAGT ACATCGTCGGCGTCAGCGCCCTCTGCCCGTCACCGAGTTTAG CACTGGCGGCCGAGGCCCTTGGGTCGCCGATCTCGATCAGCC TGACGAGGCCGACAGCGCCAACGAGCCCTACCTTGAGTTCCTC CAGGGCGTCCTCAAGCTCCCCCAGAGCGAGCTGCCCCAGGTC ATCAGCACCTCGTACGGCGAGAACGAGCAGAGCGTCCCCAAGA GCTACGCCCTCAGCGTCTGCAACCTCTTCGCCCAGCTTGGCTC TCGCGGCGTCAGCGTCATCTTCAGCAGCGGCGATAGCGGCCC TGGCAGCGCCTGCCAGTCTAACGACGGCAAGAACACCACCAAG TTCCAGCCCCAGTACCCTGCCGCCTGCCCCTTCGTCACTAGCG TCGGCTCTACCCGCTACCTCAACGAGACTGCCACCGGCTTCAG CTCCGGCGGCTTCAGCGACTACTGGAAGCGCCCCAGCTACCA GGACGACGCCGTCAAGGCCTACTTCCACCACCTCGGCGAGAA GTTCAAGCCCTACTTCAACCGCCACGGCCGAGGCTTCCCTGAC GTCGCCACTCAGGGCTACGGCTTCCGCGTCTACGACCAGGGC AAGCTCAAGGGCCTCCAGGGCACTTCTGCCAGCGCCCCTGCCT TCGCCGGCGTCATTGGCCTGCTCAACGACGCCCGCCTCAAGG CCAAGAAGCCCACCCTCGGCTTTCTCAACCCCCTGCTCTACAG CAACAGCGACGCCCTCAACGACATCGTCCTCGGCGGCTCCAAG GGCTGCGACGGCCACGCTAGGTTTAACGGCCCTCCCAACGGC AGCCCCGTCATCCCTTACGCCGGCTGGAACGCCACTGCCGGCT GGGACCCTGTTACCGGCCTCGGCACCCCCAACTTCCCCAAGCT CCTCAAGGCCGCCGTCCCCTCTCGATACCGCGCTTAA | Phaeosphaeria nodorum SN15 |

(continued)

| SEQ ID No.: | Sequence | Origin |
|---|---|---|
| 82 | <u>ATGCAGACCTTCGGTGCTTTTCTCGTTTCCTTCCTCGCCGCCAG CGGCCTGGCCGCGGCC</u>AACGCTGCTGTCCTCCTCGACAGCCT CGACAAGGTCCCCGTCGGCTGGCAGGCTGCTTCTGCCCCTGCT CCCAGCAGCAAGATCACCCTCCAGGTCGCCCTCACCCAGCAGA ACATCGACCAGCTTGAGAGCAAGCTCGCCGCCGTCAGCACCCC CAACAGCAGCAACTACGGCAAGTACCTCGACGTCGACGAGATC AACCAGATCTTCGCCCCCAGCAGCGCCAGCACTGCCGCTGTCG AGAGCTGGCTCAAGAGCTACGGCGTCGACTACAAGGTCCAGG GCAGCAGCATCTGGTTCCAGACCGACGTCAGCACGGCCAACAA GATGCTCAGCACCAACTTCCACACCTACACCGACAGCGTCGGC GCCAAGAAGGTCCGCACCCTCCAGTACAGCGTCCCCGAGACTC TCGCCGACCACATCGACCTCATCAGCCCCACCACCTACTTCGG CACCAGCAAGGCCATGCGAGCCCTCAAGATCCAGAACGCCGC CAGCGCCGTCAGCCCTCTCGCTGCTCGACAAGAGCCCAGCAG CTGCAAGGGCACCATCGAGTTCGAGAACCGCACCTTCAACGTC TTTCAGCCCGACTGCCTCCGCACCGAGTACAGCGTCAACGGCT ACAAGCCCAGCGCCAAGAGCGGCAGCCGAATCGGCTTCGGCA GCTTCCTCAACCAGAGCGCCAGCAGCAGCGACCTCGCCCTCTT CGAGAAGCACTTCGGCTTCGCCAGCCAGGGCTTCAGCGTCGA GCTGATCAACGGCGGCAGCAACCCCCAGCCTCCCACCGATGCT AACGACGGCGAGGCCAACCTCGACGCCCAGAACATCGTCAGCT TCGTCCAGCCCCTGCCCATCACCGAGTTTATCGCTGGCGGCAC CGCCCCCTACTTCCCCGATCCTGTTGAGCCTGCCGGCACCCCC GACGAGAACGAGCCCTACCTTGAGTACTACGAGTACCTCCTCA GCAAGAGCAACAAGGAACTCCCCCAGGTCATCACCAACAGCTA CGGCGACGAGGAACAGACCGTCCCCCAGGCCTACGCCGTCCG CGTCTGCAACCTCATCGGCCTCATGGGCCTCCGCGGCATCAGC ATCCTTGAGAGCAGCGGCGACGAGGGCGTCGGCGCTTCTTGC CTCGCCACCAACAGCACCACCACCCCCCAGTTCAACCCCATCT TCCCCGCCACGTGCCCCTACGTCACTAGCGTCGGCGGCACCG TCAGCTTCAACCCCGAGGTCGCTTGGGACGGCAGCAGCGGCG GCTTCAGCTACTACTTCAGCCGCCCCTGGTATCAAGAGGCCGC CGTCGGCACCTACCTCAACAAGTACGTCAGCGAGGAAACGAAG GAATATTACAAGAGCTACGTCGACTTCAGCGGCCGAGGCTTCC CTGACGTCGCCGCTCACTCTGTCAGCCCCGACTACCCGTCTT TCAGGGCGGCGAGCTGACTCCTTCTGGCGGCACTTCTGCCGC CAGCCCCATCGTCGCCAGCGTCATTGCCCTGCTCAACGACGCC CGACTCCGAGCCGGCAAGCCTGCCCTCGGCTTTCTCAACCCCC TCATCTACGGCTACGCCTACAAGGGCTTCACCGACATCACCTC CGGCCAGGCCGTTGGCTGCAACGGCAACAACACCCAGACCGG CGGACCCCTTCCTGGCGCTGGCGTTATCCCTGGCGCCTTCTGG AACGCCACCAAGGGCTGGGACCCCACCACCGGCTTTGGCGTC CCCAACTTCAAGAAGCTCCTTGAGCTGGTCCGCTACATC | Trichoderma atroviride IMI 206040 |

(continued)

| SEQ ID No.: | Sequence | Origin |
|---|---|---|
| 83 | <u>ATGCAGACCTTCGGTGCTTTTCTCGTTTCCTTCCTCGCCGCCAG CGGCCTGGCCGCGGCC</u>AAGCCTACTCCTGGCGCTTCCCACAA GGTCATCGAGCACCTCGACTTCGTCCCCGAGGGCTGGCAGATG GTCGGCGCTGCTGACCCTGCCGCCATCATCGACTTTTGGCTCG CCATCGAGCGCGAGAACCCCGAGAAGCTCTACGACACCATCTA CGACGTCAGCACCCCCGGACGCGCCCAGTACGGCAAGCACCT CAAGCGCGAGGAACTCGACGACCTCCTCCGCCCTCGCGCCGA GACTAGCGAGAGCATCATCAACTGGCTCACCAACGGCGGCGTC AACCCCCAGCACATTCGCGACGAGGGCGACTGGGTCCGCTTCA GCACCAACGTCAAGACCGCCGAGACTCTCATGAACACCCGCTT CAACGTCTTTAAGGACAACCTCAACAGCGTCAGCAAGATCCGC ACCCTTGAGTACAGCGTCCCCGTCGCCATCAGCGCCCACGTCC AGATGATCCAGCCCACCACCCTCTTCGGCCGCCAGAAGCCCCA GAACAGCCTCATCCTCAACCCCCTCACCAAGGACCTTGAGAGC ATGAGCGTCGAAGAGTTCGCCGCCAGCCAGTGCCGCAGCCTC GTCACTACTGCCTGCCTCCGCGAGCTGTACGGCCTCGGCGATC GAGTCACCCAGGCCCGCGACGACAACCGAATTGGCGTCAGCG GCTTCCTCGAAGAGTACGCCCAGTACCGCGACCTTGAGCTGTT CCTCAGCCGCTTCGAGCCCAGCGCCAAGGGCTTCAACTTCAGC GAGGGCCTGATCGCTGGCGGCAAGAACACCCAGGGTGGCCCT GGCTCTAGCACCGAGGCCAACCTCGACATGCAGTACGTCGTCG GCCTCAGCCACAAGGCCAAGGTCACCTACTACAGCACTGCCGG CCGAGGCCCCCTCATCCCTGATCTCTCACAGCCCAGCCAGGCC AGCAACAACAACGAGCCCTACCTTGAGCAGCTCCGCTACCTCG TCAAGCTCCCCAAGAACCAGCTCCCCAGCGTCCTCACCACCAG CTACGGCGACACCGAGCAGAGCCTCCCCGCCAGCTACACCAA GGCCACGTGCGACCTCTTCGCCCAGCTCGGCACTATGGGCGT CAGCGTCATCTTCAGCAGCGGCGACACTGGCCCTGGCAGCTC GTGCCAGACCAACGACGGCAAGAACGCCACGCGCTTCAACCCC CATCTACCCCGCCAGCTGCCCCTTCGTCACCAGCATTGGCGGC ACCGTCGGCACCGGCCCTGAGCGAGCTGTCAGCTTTAGCAGC GGCGGCTTCAGCGACCGCTTCCCTCGCCCTCAGTACCAGGACA ACGCCGTCAAGGACTACCTCAAGATCCTCGGCAACCAGTGGTC CGGCCTCTTCGACCCTAACGGCCGAGCCTTCCCCGACATTGCC GCCCAGGGCAGCAACTACGCCGTCTACGACAAGGGCCGCATG ACCGGCGTTAGCGGCACTTCTGCTTCCGCCCCTGCTATGGCCG CCATCATTGCCCAGCTCAACGACTTCCGCCTCGCCAAGGGCAG CCCCGTCCTCGGCTTTCTCAACCCCTGGATCTACAGCAAGGGC TTCAGCGGCTTCACCGACATCGTCGACGGCGGCTCTAGGGGCT GCACCGGCTACGACATCTACAGCGGCCTCAAGGCCAAGAAGGT CCCCTACGCCAGCTGGAACGCCACCAAGGGCTGGGACCCCGT CACCGGCTTTGGCACCCCCAACTTCCAGGCCCTGACCAAGGTC CTGCCCTAA | Arthroderma benhamiae CBS 112371 |

(continued)

| SEQ ID No.: | Sequence | Origin |
|---|---|---|
| 84 | <u>ATGCAGACCTTCGGTGCTTTTCTCGTTTCCTTCCTCGCCGCCAG CGGCCTGGCCGCGGCC</u>AAGAGCTACTCTCACCACGCCGAGGC CCCCAAGGGCTGGAAGGTCGACGATACTGCCCGCGTCGCCAG CACCGGCAAGCAGCAGGTCTTTTCGATCGCCCTGACCATGCAG AACGTCGACCAGCTTGAGAGCAAGCTCCTCGACCTCAGCAGCC CCGACAGCAAGAACTACGGCCAGTGGATGAGCCAGAAGGACG TCACCACCGCCTTCTACCCCAGCAAGGAAGCCGTCAGCAGCGT CACCAAGTGGCTCAAGAGCAAGGGCGTCAAGCACTACAACGTC AACGGCGGCTTCATCGACTTCGCCCTCGACGTGAAGGGCGCCA ACGCCCTCCTCGACAGCGACTACCAGTACTACACCAAGGAAGG CCAGACCAAGCTCCGCACCCTCAGCTACAGCATCCCCGACGAC GTCGCCGAGCACGTCCAGTTCGTCGACCCCAGCACCAACTTCG GCGGCACCCTCGCCTTTGCCCCCGTCACTCACCCTAGCCGCAC CCTCACCGAGCGCAAGAACAAGCCCACCAAGAGCACCGTCGAC GCCAGCTGCCAGACCAGCATCACCCCCAGCTGCCTCAAGCAGA TGTACAACATCGGCGACTACACCCCCAAGGTCGAGAGCGGCAG CACGATCGGCTTCAGCAGCTTCCTCGGCGAGAGCGCTATCTAC AGCGACGTCTTTCTGTTCGAGGAAAAGTTCGGCATCCCCACCC AGAACTTCACCACCGTCCTCATCAACAACGGCACCGACGACCA GAACACCGCCCACAAGAACTTCGGCGAGGCCGACCTCGACGC CGAGAACATCGTCGGCATTGCCCACCCCCTGCCCTTCACCCAG TACATCACTGGCGGCAGCCCCCCCTTCCTGCCCAACATCGATC AGCCCACTGCCGCCGACAACCAGAACGAGCCCTACGTCCCCTT CTTCCGCTACCTCCTCAGCCAGAAGGAAGTCCCCGCCGTCGTC AGCACCAGCTACGGCGACGAAGAGGACAGCGTCCCCCGCGAG TACGCCACCATGACCTGCAACCTCATCGGCCTGCTCGGCCTCC GCGGCATCAGCGTCATCTTCAGCAGCGGCGACATCGGCGTCG GCGCTGGCTGTCTTGGCCCCGACCACAAGACCGTCGAGTTCAA CGCCATCTTCCCCGCCACGTGCCCCTACCTCACTAGCGTCGGC GGCACGGTCGACGTCACCCCCGAGATTGCTTGGGAGGGCAGC AGCGGCGGCTTCAGCAAGTACTTCCCTCGCCCCAGCTACCAGG ACAAGGCCGTCAAGACCTACATGAAGACCGTCAGCAAGCAGAC CAAGAAGTACTACGGCCCCTACACCAACTGGGAGGGCCGAGG CTTTCCTGACGTCGCCGGCCACAGCGTCAGCCCCAACTACGAG GTCATCTACGCCGGCAAGCAGAGCGCCTCTGGCGGCACTTCTG CTGCCGCCCTGTCTGGGCTGCCATCGTCGGCCTGCTCAACGA CGCCCGATTCCGAGCCGGCAAGCCTAGCCTCGGCTGGCTCAA CCCCCTCGTCTACAAGTACGGCCCCAAGGTCCTCACCGACATC ACCGGCGGCTACGCCATTGGCTGCGACGGCAACAACACCCAG AGCGGCAAGCCCGAGCCTGCCGGCTCTGGCATTGTCCCTGGC GCCCGATGGAACGCCACTGCCGGATGGGACCCTGTCACCGGC TACGGCACCCCCGACTTCGGCAAGCTCAAGGACCTCGTCCTCA GCTTCTAA | Fusarium graminearum PH-1 |

| SEQ ID No.: | Sequence | Origin |
|---|---|---|
| 85 | <u>ATGCAGACCTTCGGTGCTTTTCTCGTTTCCTTCCTCGCCGCCAG</u> <u>CGGCCTGGCCGCGGCC</u>GCCGTCGTCATTCGCGCCGCCGTCCT CCCCGACGCCGTCAAGCTGATGGGCAAGGCCATGCCCGACGA CATTATTTCCCTCCAGTTTTCCCTGAAGCAGCAGAACATCGACC AGCTGGAGACCCGCCTCCGCGCCGTCTCGGACCCCAGCTCCC CCGAGTACGGCCAGTACATGAGCGAGTCCGAGGTCAACGAGTT CTTTAAGCCCCGCGACGACTCGTTCGCCGAGGTCATTGACTGG GTCGCCGCCAGCGGCTTTCAGGACATCCACCTGACGCCCCAG GCTGCCGCCATTAACCTCGCCGCCACCGTCGAGACGGCCGAC CAGCTCCTGGGCGCCAACTTCAGCTGGTTTGACGTCGACGGCA CCCGCAAGCTCCGCACCCTGGAGTACACGATCCCCGACCGCCT CGCCGACCACGTCGACCTGATTTCCCCCACCACGTACTTCGGC CGCGCCCGACTGGACGGCCCCCGCGAGACCCCCACGCGCCTC GACAAGCGCCAGCGCGACCCCGTCGCCGACAAGGCCTACTTC CACCTCAAGTGGGACCGCGGCACCAGCAACTGCGACCTGGTC ATCACGCCCCCTGCCTGGAGGCCGCCTACAACTACAAGAACT ACATGCCCGACCCCAACTCGGGCAGCCGCGTCTCGTTCACCAG CTTTCTGGAGCAGGCCGCCCAGCAGAGCGACCTCACCAAGTTC CTCTCCCTGACGGGCCTCGACCGCCTGCGCCCCCCCAGCAGC AAGCCCGCCAGCTTCGACACGGTCCTGATCAACGGCGGCGAG ACCCACCAGGGCACGCCCCCCAACAAGACCTCCGAGGCCAAC CTCGACGTCCAGTGGCTGGCCGCCGTCATTAAGGCCCGACTCC CCATCACCCAGTGGATTACGGGCGGCCGCCCCCCCTTCGTCCC CAACCTCCGCCTGCGCCACGAGAAGGACAACACGAACGAGCC CTACCTGGAGTTCTTTGAGTACCTCGTCCGCCTGCCCGCCCGC GACCTCCCCCAGGTCATCTCCAACTCGTACGCCGAGGACGAGC AGACCGTCCCCGAGGCCTACGCCCGACGCGTCTGCAACCTCAT CGGCATTATGGGCCTGCGCGGCGTCACCGTCCTCACGGCCTC CGGCGACTCGGGCGTCGGCGCCCCCTGCCGCGCCAACGACG GCAGCGACCGCCTGGAGTTCTCCCCCCAGTTTCCCACCTCGTG CCCCTACATCACCGCCGTCGGCGGCACGGAGGGCTGGGACCC CGAGGTCGCCTGGGAGGCCTCCTCGGGCGGCTTCAGCCACTA CTTTCTCCGCCCCTGGTACCAGGCCAACGCCGTCGAGAAGTAC CTCGACGAGGAGCTGGACCCCGCCACCCGCGCCTACTACGAC GGCAACGGCTTCGTCCAGTTTGCCGGCCGAGCCTACCCCGAC CTGTCCGCCCACAGCTCCTCGCCCCGCTACGCCTACATCGACA AGCTCGCCCCCGGCCTGACCGGCGGCACGAGCGCCTCCTGCC CCGTCGTCGCCGGCATCGTCGGCCTCCTGAACGACGCCCGAC TCCGCCGCGGCCTGCCCACGATGGGCTTCATTAACCCCTGGCT GTACACGCGCGGCTTTGAGGCCCTCCAGGACGTCACCGGCGG CCGCGCCTCGGGCTGCCAGGGCATCGACCTCCAGCGCGGCAC CCGCGTCCCCGGCGCCGGCATCATTCCCTGGGCCTCCTGGAA CGCCACCCCCGGCTGGGACCCCGCCACGGGCCTCGGCCTGCC CGACTTCTGGGCCATGCGCGGCCTCGCCCTGGGCCGCGGCAC CTAA | Acremonium alcalophilum |

(continued)

| SEQ ID No.: | Sequence | Origin |
|---|---|---|
| 86 | <u>ATGCAGACCTTCGGTGCTTTTCTCGTTTCCTTCCTCGCCGCCAG</u><br><u>CGGCCTGGCCGCGGCC</u>GCCGTCGTCATTCGCGCCGCCCCCCT<br>CCCCGAGAGCGTCAAGCTCGTCCGCAAGGCCGCCGCCGAGGA<br>CGGCATTAACCTCCAGCTCTCCCTGAAGCGCCAGAACATGGAC<br>CAGCTGGAGAAGTTCCTCCGCGCCGTCAGCGACCCCTTTTCCC<br>CCAAGTACGGCCAGTACATGTCGGACGCCGAGGTCCACGAGAT<br>CTTCCGCCCCACCGAGGACTCCTTTGACCAGGTCATTGACTGG<br>CTCACCAAGTCGGGCTTCGGCAACCTGCACATCACGCCCCAGG<br>CTGCCGCCATTAACGTCGCCACCACGGTCGAGACCGCCGACCA<br>GCTGTTTGGCGCCAACTTCTCCTGGTTTGACGTCGACGGCACG<br>CCCAAGCTCCGCACCGGCGAGTACACGATCCCCGACCGCCTC<br>GTCGAGCACGTCGACCTGGTCAGCCCCACCACGTACTTCGGCC<br>GCATGCGCCCCCCCCTCGCGGCGACGGCGTCAACGACTGGA<br>TCACCGAGAACTCGCCCGAGCAGCCCGCCCCCCTGAACAAGC<br>GCGACACCAAGACGGAGAGCGACCAGGCCCGCGACCACCCCT<br>CCTGGGACTCGCGCACCCCCGACTGCGCCACCATCATTACGCC<br>CCCCTGCCTGGAGACGGCCTACAACTACAAGGGCTACATCCCC<br>GACCCCAAGTCCGGCTCGCGCGTCAGCTTCACCAGCTTCCTGG<br>AGCAGGCCGCCCAGCAGGCCGACCTGACCAAGTTCCTCAGCC<br>TGACGCGCCTGGAGGGCTTTCGCACCCCCGCCAGCAAGAAGA<br>AGACCTTCAAGACGGTCCTGATCAACGGCGGCGAGTCCCACGA<br>GGGCGTCCACAAGAAGTCGAAGACCAGCGAGGCCAACCTCGA<br>CGTCCAGTGGCTGGCCGCCGTCACCCAGACGAAGCTGCCCAT<br>CACCCAGTGGATTACGGGCGGCCGCCCCCCCTTCGTCCCCAA<br>CCTCCGCATCCCCACCCCCGAGGCCAACACGAACGAGCCCTAC<br>CTGGAGTTCCTGGAGTACCTCTTTCGCCTGCCCGACAAGGACC<br>TCCCCCAGGTCATCAGCAACTCCTACGCCGAGGACGAGCAGAG<br>CGTCCCCGAGGCCTACGCCCGACGCGTCTGCGGCCTCCTGGG<br>CATTATGGGCCTCCGCGGCGTCACCGTCCTGACGGCCTCCGG<br>CGACTCGGGCGTCGGCGCCCCTGCCGCGCCAACGACGGCTC<br>GGGCCGCGAGGAGTTCAGCCCCCAGTTTCCCAGCTCCTGCCC<br>CTACATCACCACGGTCGGCGGCACCCAGGCCTGGGACCCCGA<br>GGTCGCCTGGAAGGGCAGCAGCGGCGGCTTCTCCAACTACTTT<br>CCCCGCCCCTGGTACCAGGTCGCCGCCGTCGAGAAGTACCTG<br>GAGGAGCAGCTGGACCCCGCCGCCCGCGAGTACTACGAGGAG<br>AACGGCTTCGTCCGCTTTGCCGGCCGAGCCTTCCCCGACCTGA<br>GCGCCCACAGCAGCAGCCCCAAGTACGCCTACGTCGACAAGC<br>GCGTCCCCGGCCTCACCGGCGGCACGTCGGCCAGCTGCCCCG<br>TCGTCGCCGGCATCGTCGGCCTCCTGAACGACGCCCGACTCC<br>GCCGCGGCCTGCCCACGATGGGCTTCATTAACCCCTGGCTCTA<br>CGCCAAGGGCTACCAGGCCCTGGAGGACGTCACCGGCGGCGC<br>CGCCGTCGGCTGCCAGGGCATCGACATTCAGACGGGCAAGCG<br>CGTCCCCGGCGCCGGCATCATTCCCGGCGCCAGCTGGAACGC<br>CACCCCCGACTGGGACCCCGCCACGGGCCTCGGCCTGCCCAA<br>CTTCTGGGCCATGCGCGAGCTCGCCCTGGAGGACTAA | Sodiomyces alkalinus |

(continued)

| SEQ ID No.: | Sequence | Origin |
|---|---|---|
| 87 | ATGCAGACCTTCGGTGCTTTTCTCGTTTCCTTCCTCGCCGCCAG CGGCCTGGCCGCGGCCGTCGTCCATGAGAAGCTCGCTGCTGT CCCCAGCGGCTGGCACCACCTTGAGGATGCCGGCAGCGACCA CCAGATCAGCCTCTCGATTGCCCTCGCCCGCAAGAACCTCGAC CAGCTTGAGAGCAAGCTCAAGGACCTCAGCACCCCTGGCGAGA GCCAGTACGGCCAGTGGCTCGACCAAGAGGAAGTCGACACCC TGTTCCCCGTCGCCAGCGACAAGGCCGTCATCAGCTGGCTCCG CAGCGCCAACATCACCCACATTGCCCGCCAGGGCAGCCTCGTC AACTTCGCCACCACCGTCGACAAGGTCAACAAGCTCCTCAACA CCACCTTCGCCTACTACCAGCGCGGCAGCTCTCAGCGCCTCCG CACCACCGAGTACAGCATCCCCGACGACCTCGTCGACAGCATC GACCTGATCAGCCCCACCACGTTCTTCGGCAAGGAAAAGACCT CTGCCGGCCTCACCCAGCGCAGCCAGAAGGTCGATAACCACGT CGCCAAGCGCAGCAACAGCAGCAGCTGCGCCGACACCATCAC CCTCAGCTGCCTCAAGGAAATGTACAACTTCGGCAACTACACCC CCAGCGCCAGCAGCGGCAGCAAGCTCGGCTTCGCCAGCTTCC TCAACGAGAGCGCCAGCTACAGCGACCTCGCCAAGTTCGAGCG CCTCTTCAACCTCCCCAGCCAGAACTTCAGCGTCGAGCTGATC AACGGCGGCGTCAACGACCAGAACCAGAGCACCGCCAGCCTC ACCGAGGCCGACCTCGATGTCGAGCTGCTTGTCGGCGTCGGC CACCCCCTGCCCGTCACCGAGTTTATCACCAGCGGCGAGCCCC CCTTCATCCCCGACCCTGATGAGCCTTCTGCCGCCGACAACGA GAACGAGCCCTACCTCCAGTACTACGAGTACCTCCTCAGCAAG CCCAACAGCGCCCTGCCCCAGGTCATCAGCAACAGCTACGGC GACGACGAGCAGACCGTCCCCGAGTACTACGCCAAGCGCGTC TGCAACCTCATCGGCCTCGTCGGCCTCCGCGGCATCAGCGTCC TTGAGTCTAGCGGCGACGAGGGCATCGGCTCTGGCTGCCGAA CCACCGACGGCACCAACAGCACCCAGTTCAACCCCATCTTCCC CGCCACGTGCCCCTACGTCACTGCCGTCGGCGGCACCATGAG CTACGCCCCCGAGATTGCTTGGGAGGCCAGCTCCGGCGGCTT CAGCAACTACTTCGAGCGAGCCTGGTTCCAGAAGGAAGCCGTC CAGAACTACCTCGCCAACCACATCACCAACGAGACTAAGCAGT ACTACAGCCAGTTCGCCAACTTCAGCGGCCGAGGCTTCCCCGA CGTCAGCGCCCACAGCTTCGAGCCCAGCTACGAGGTCATCTTC TACGGCGCTCGCTACGGCAGCGGCGGCACTTCTGCTGCCTGC CCCCTGTTTTCTGCCCTCGTCGGCATGCTCAACGACGCCCGAC TCCGAGCCGGCAAGTCGACCCTCGGCTTCCTCAACCCCCTGCT CTACAGCAAGGGCTACAAGGCCCTCACCGACGTCACCGCTGGC CAGAGCATTGGCTGCAACGGCATCGACCCCCAGAGCGACGAG GCTGTCGCTGGCGCTGGCATCATTCCCTGGGCCCACTGGAACG CCACCGTCGGCTGGGACCCTGTCACTGGCCTTGGCCTCCCCG ACTTCGAGAAGCTCCGCCAGCTCGTCCTCAGCCTCTAA | Aspergillus kawachii IFO 4308 |

(continued)

| SEQ ID No.: | Sequence | Origin |
|---|---|---|
| 88 | <u>ATGCAGACCTTCGGTGCTTTTCTCGTTTCCTTCCTCGCCGCCAG</u><br><u>CGGCCTGGCCGCGGCC</u>GCTGCTGCTCTTGTTGGCCACGAGTC<br>TCTCGCCGCCCTCCCTGTCGGCTGGGACAAGGTCAGCACTCCT<br>GCCGCTGGCACCAACATCCAGCTCAGCGTCGCCCTCGCCCTCC<br>AGAACATCGAGCAGCTTGAGGACCACCTCAAGAGCGTCAGCAC<br>CCCCGGCTCTGCCAGCTACGGCCAGTACCTCGACAGCGACGG<br>CATTGCCGCCCAGTACGGCCCTTCTGACGCCAGCGTCGAGGC<br>CGTCACCAACTGGCTCAAGGAAGCCGGCGTCACCGACATCTAC<br>AACAACGGCCAGAGCATCCACTTCGCCACCAGCGTCAGCAAGG<br>CCAACAGCCTCCTCGGCGCCGACTTCAACTACTACAGCGACGG<br>CTCCGCCACCAAGCTCCGCACCCTCGCTTACAGCGTCCCCAGC<br>GACCTGAAGGAAGCCATCGACCTCGTCAGCCCCACCACCTACT<br>TCGGCAAGACCACCGCCAGCCGCAGCATCCAGGCCTACAAGAA<br>CAAGCGAGCCAGCACCACCAGCAAGAGCGGCAGCAGCAGCGT<br>CCAGGTCAGCGCCTCTTGCCAGACCAGCATCACCCCCGCCTGC<br>CTCAAGCAGATGTACAACGTCGGCAACTACACCCCCAGCGTCG<br>CCCACGGCTCTCGCGTTGGCTTCGGCAGCTTCCTCAACCAGAG<br>CGCCATCTTCGACGACCTCTTCACCTACGAGAAGGTCAACGAC<br>ATCCCCAGCCAGAACTTCACCAAGGTCATCATTGCCAACGCCA<br>GCAACAGCCAGGACGCCAGCGACGGCAACTACGGCGAGGCCA<br>ACCTCGACGTCCAGAACATTGTCGGCATCAGCCACCCCCTGCC<br>CGTCACCGAGTTTCTCACTGGCGGCAGCCCACCCTTCGTCGCC<br>AGCCTCGACACCCCCACCAACCAGAACGAGCCCTACATCCCCT<br>ACTACGAGTACCTCCTCAGCCAGAAGAACGAGGACCTCCCCCA<br>GGTCATCAGCAACAGCTACGGCGACGACGAGCAGAGCGTCCC<br>CTACAAGTACGCCATCCGCGCCTGCAACCTCATCGGCCTCACT<br>GGCCTCCGCGGCATCAGCGTCCTTGAGAGCAGCGGCGATCTC<br>GGCGTTGGCGCTGGCTGCCGATCCAACGACGGCAAGAACAAG<br>ACCCAGTTCGACCCCATCTTCCCCGCCACGTGCCCCTACGTCA<br>CTAGCGTCGGCGGCACCCAGAGCGTCACCCCCGAGATTGCTT<br>GGGTCGCTTCCAGCGGCGGCTTCAGCAACTACTTCCCCCGCAC<br>CTGGTATCAAGAGCCCGCCATCCAGACCTACCTCGGCCTCCTC<br>GACGACGAGACTAAGACCTACTACAGCCAGTACACCAACTTCG<br>AGGGCCGAGGCTTCCCCGACGTCAGCGCCCATTCTCTCACCCC<br>CGACTACCAGGTCGTCGGCGGAGGCTACCTTCAGCCTTCTGGC<br>GGCACTTCTGCCGCCAGCCCTGTCTTTGCCGGCATCATTGCCC<br>TGCTCAACGACGCCCGACTCGCCGCTGGCAAGCCCACCCTCG<br>GCTTTCTCAACCCCTTCTTCTACCTCTACGGCTACAAGGGCCTC<br>AACGACATCACTGGCGGCCAGAGCGTCGGCTGCAACGGCATC<br>AACGGCCAGACTGGCGCCCTGTTCCCGGCGGAGGAATTGTC<br>CCTGGCGCCGCTTGGAACAGCACCACCGGATGGGACCCTGCC<br>ACCGGCCTTGGCACCCCCGACTTTCAGAAGCTCAAGGAACTCG<br>TCCTCAGCTTCTAA | Talaromyces stipitatus ATCC 10500 |

(continued)

| SEQ ID No.: | Sequence | Origin |
|---|---|---|
| 89 | <u>ATGCAGACCTTCGGTGCTTTTCTCGTTTCCTTCCTCGCCGCCAG CGGCCTGGCCGCGGCC</u>AAGTCGTTTTCCCACCACGCCGAGGC CCCCCAGGGCTGGCAGGTCCAGAAGACCGCCAAGGTCGCCTC CAACACGCAGCACGTCTTTAGCCTCGCCCTGACCATGCAGAAC GTCGACCAGCTGGAGTCGAAGCTCCTGGACCTGAGCTCCCCC GACAGCGCCAACTACGGCAACTGGCTCAGCCACGACGAGCTG ACCTCCACGTTCTCGCCCAGCAAGGAGGCCGTCGCCTCGGTCA CCAAGTGGCTGAAGAGCAAGGGCATCAAGCACTACAAGGTCAA CGGCGCCTTCATTGACTTTGCCGCCGACGTCGAGAAGGCCAAC ACCCTCCTGGGCGGCGACTACCAGTACTACACGAAGGACGGC CAGACCAAGCTGCGCACGCTCTCCTACTCGATCCCCGACGACG TCGCCGGCCACGTCCAGTTCGTCGACCCCAGCACCAACTTCGG CGGCACGGTCGCCTTTAACCCCGTCCCCCACCCCTCCCGCACC CTCCAGGAGCGCAAGGTCTCCCCCTCCAAGTCGACGGTCGAC GCCTCCTGCCAGACCTCGATCACGCCCAGCTGCCTGAAGCAGA TGTACAACATTGGCGACTACACCCCCGACGCCAAGAGCGGCTC CGAGATCGGCTTCAGCAGCTTCCTCGGCCAGGCCGCCATTTAC AGCGACGTCTTCAAGTTTGAGGAGCTCTTCGGCATCCCCAAGC AGAACTACACCACGATCCTGATTAACAACGGCACCGACGACCA GAACACGGCCCACGGCAACTTTGGCGAGGCCAACCTCGACGC CGAGAACATCGTCGGCATTGCCCACCCCCTGCCCTTCAAGCAG TACATCACCGGCGGCAGCCCCCCCTTTGTCCCCAACATTGACC AGCCCACGGAGAAGGACAACCAGAACGAGCCCTACGTCCCCTT CTTTCGCTACCTCCTGGGCCAGAAGGACCTGCCCGCCGTCATC TCGACCAGCTACGGCGACGAGGAGGACTCCGTCCCCCGCGAG TACGCCACCCTCACGTGCAACATGATCGGCCTCCTGGGCCTGC GCGGCATCTCCGTCATTTTCTCCTCGGGCGACATTGGCGTCGG CTCGGGCTGCCTCGCCCCCGACTACAAGACCGTCGAGTTCAAC GCCATCTTTCCCGCCACCTGCCCCTACCTGACGTCCGTCGGCG GCACCGTCGACGTCACGCCCGAGATTGCCTGGGAGGGCAGCT CCGGCGGCTTCTCCAAGTACTTTCCCCGCCCCTCGTACCAGGA CAAGGCCATCAAGAAGTACATGAAGACCGTCTCGAAGGAGACG AAGAAGTACTACGGCCCCTACACCAACTGGGAGGGCCGCGGC TTCCCCGACGTCGCCGGCCACTCCGTCGCCCCCGACTACGAG GTCATCTACAACGGCAAGCAGGCCCGATCCGGCGGCACCAGC GCCGCCGCCCCCGTCTGGGCCGCCATCGTCGGCCTCCTGAAC GACGCCCGATTCAAGGCCGGCAAGAAGAGCCTGGGCTGGCTC AACCCCCTGATCTACAAGCACGGCCCCAAGGTCCTCACCGACA TCACGGGCGGCTACGCCATTGGCTGCGACGGCAACAACACCC AGAGCGGCAAGCCCGAGCCCGCCGGCTCCGGCCTGGTCCCCG GCGCCCGATGGAACGCCACCGCCGGCTGGGACCCCACCACGG GCTACGGCACGCCCAACTTCCAGAAGCTCAAGGACCTCGTCCT GTCCCTCTAA | Fusarium oxysporum f. sp.cubense race 4 |

(continued)

| SEQ ID No.: | Sequence | Origin |
|---|---|---|
| 90 | <u>ATGCAGACCTTCGGTGCTTTTCTCGTTTCCTTCCTCGCCGCCAG</u><br><u>CGGCCTGGCCGCGGCC</u>GTCGTCCATGAGAAGCTCGCTGCTGT<br>CCCCAGCGGCTGGCACCACCTTGAGGATGCCGGCAGCGACCA<br>CCAGATCAGCCTCTCGATTGCCCTCGCCCGCAAGAACCTCGAC<br>CAGCTTGAGAGCAAGCTCAAGGACCTCAGCACCCCTGGCGAGA<br>GCCAGTACGGCCAGTGGCTCGACCAAGAGGAAGTCGACACCC<br>TGTTCCCCGTCGCCAGCGACAAGGCCGTCATCAGCTGGCTCCG<br>CAGCGCCAACATCACCCACATTGCCCGCCAGGGCAGCCTCGTC<br>AACTTCGCCACCACCGTCGACAAGGTCAACAAGCTCCTCAACA<br>CCACCTTCGCCTACTACCAGCGCGGCAGCTCTCAGCGCCTCCG<br>CACCACCGAGTACAGCATCCCCGACGACCTCGTCGACAGCATC<br>GACCTGATCAGCCCCACCACGTTCTTCGGCAAGGAAAAGACCT<br>CTGCCGGCCTCACCCAGCGCAGCCAGAAGGTCGATAACCACGT<br>CGCCAAGCGCAGCAACAGCAGCAGCTGCGCCGACACCATCAC<br>CCTCAGCTGCCTCAAGGAAATGTACAACTTCGGCAACTACACCC<br>CCAGCGCCAGCAGCGGCAGCAAGCTCGGCTTCGCCAGCTTCC<br>TCAACGAGAGCGCCAGCTACAGCGACCTCGCCAAGTTCGAGCG<br>CCTCTTCAACCTCCCCAGCCAGAACTTCAGCGTCGAGCTGATC<br>AACGGCGGCGTCAACGACCAGAACCAGAGCACCGCCAGCCTC<br>ACCGAGGCCGACCTCGATGTCGAGCTGCTTGTCGGCGTCGGC<br>CACCCCCTGCCCGTCACCGAGTTTATCACCAGCGGCGAGCCCC<br>CCTTCATCCCCGACCCTGATGAGCCTTCTGCCGCCGACAACGA<br>GAACGAGCCCTACCTCCAGTACTACGAGTACCTCCTCAGCAAG<br>CCCAACAGCGCCCTGCCCCAGGTCATCAGCAACAGCTACGGC<br>GACGACGAGCAGACCGTCCCCGAGTACTACGCCAAGCGCGTC<br>TGCAACCTCATCGGCCTCGTCGGCCTCCGCGGCATCAGCGTCC<br>TTGAGTCTAGCGGCGACGAGGGCATCGGCTCTGGCTGCCGAA<br>CCACCGACGGCACCAACAGCACCCAGTTCAACCCCATCTTCCC<br>CGCCACGTGCCCCTACGTCACTGCCGTCGGCGGCACCATGAG<br>CTACGCCCCCGAGATTGCTTGGGAGGCCAGCTCCGGCGGCTT<br>CAGCAACTACTTCGAGCGAGCCTGGTTCCAGAAGGAAGCCGTC<br>CAGAACTACCTCGCCAACCACATCACCAACGAGACTAAGCAGT<br>ACTACAGCCAGTTCGCCAACTTCAGCGGCCGAGGCTTCCCCGA<br>CGTCAGCGCCCACAGCTTCGAGCCCAGCTACGAGGTCATCTTC<br>TACGGCGCTCGCTACGGCAGCGGCGGCACTTCTGCTGCCTGC<br>CCCCTGTTTTCTGCCCTCGTCGGCATGCTCAACGACGCCCGAC<br>TCCGAGCCGGCAAGTCGACCCTCGGCTTCCTCAACCCCCTGCT<br>CTACAGCAAGGGCTACAAGGCCCTCACCGACGTCACCGCTGGC<br>CAGAGCATTGGCTGCAACGGCATCGACCCCCAGAGCGACGAG<br>GCTGTCGCTGGCGCTGGCATCATTCCCTGGGCCCACTGGAACG<br>CCACCGTCGGCTGGGACCCTGTCACTGGCCTTGGCCTCCCCG<br>ACTTCGAGAAGCTCCGCCAGCTCGTCCTCAGCCTCTAA | Trichoderma virens Gv29-8 |

(continued)

| SEQ ID No.: | Sequence | Origin |
|---|---|---|
| 91 | <u>ATGCAGACCTTCGGTGCTTTTCTCGTTTCCTTCCTCGCCGCCAG</u><br><u>CGGCCTGGCCGCGGCC</u>GCTGTCCTTGTCGAGTCTCTCAAGCA<br>GGTCCCCAACGGCTGGAACGCCGTCAGCACCCCTGACCCCAG<br>CACCAGCATCGTCCTCCAGATCGCCCTCGCCCAGCAGAACATC<br>GACGAGCTTGAGTGGCGCCTCGCCGCCGTGTCTACCCCCAACT<br>CTGGCAACTACGGCAAGTACCTCGACATCGGCGAGATCGAGGG<br>CATCTTCGCCCCCAGCAACGCCAGCTACAAGGCCGTCGCTTCC<br>TGGCTCCAGAGCCACGGCGTCAAGAACTTCGTCAAGCAGGCCG<br>GCAGCATCTGGTTCTACACCACCGTCAGCACCGCCAACAAGAT<br>GCTCAGCACCGACTTCAAGCACTACAGCGACCCCGTCGGCATC<br>GAGAAGCTCCGCACCCTCCAGTACAGCATCCCCGAGGAACTCG<br>TCGGCCACGTCGACCTCATCAGCCCCACCACCTACTTCGGCAA<br>CAACCACCCTGCCACCGCCCGCACCCCCAACATGAAGGCCATC<br>AACGTCACCTACCAGATCTTCCACCCCGACTGCCTCAAGACCAA<br>GTACGGCGTCGACGGCTACGCCCCCTCACCTCGATGCGGCAG<br>CCGAATCGGCTTCGGCAGCTTCCTCAACGAGACTGCCAGCTAC<br>AGCGACCTCGCCCAGTTCGAGAAGTACTTCGACCTCCCCAACC<br>AGAACCTCAGCACCCTCCTCATCAACGGCGCCATCGACGTCCA<br>GCCCCCCAGCAACAAGAACGACAGCGAGGCCAACATGGACGT<br>CCAGACCATCCTCACCTTCGTCCAGCCCCTGCCCATCACCGAG<br>TTCGTCGTCGCCGGCATCCCCCCCTACATTCCCGATGCCGCCC<br>TCCCCATTGGCGACCCCGTTCAGAACGAGCCCTGGCTTGAGTA<br>CTTCGAGTTCCTCATGAGCCGCACCAACGCCGAGCTGCCCCAG<br>GTCATTGCCAACAGCTACGGCGACGAGGAACAGACCGTCCCCC<br>AGGCCTACGCCGTCCGCGTCTGCAACCAGATTGGCCTCCTCGG<br>CCTCCGCGGCATCAGCGTCATTGCCTCTAGCGGCGACACCGG<br>CGTCGGCATGTCTTGCATGGCCAGCAACAGCACCACCCCCCAG<br>TTCAACCCCATGTTCCCCGCCAGCTGCCCCTACATCACCACCG<br>TCGGCGGCACCCAGCACCTCGACAACGAGATCGCCTGGGAGC<br>TGAGCAGCGGCGGCTTCAGCAACTACTTCACCCGCCCCTGGTA<br>TCAAGAGGACGCCGCCAAGACCTACCTTGAGCGCCACGTCAGC<br>ACCGAGACTAAGGCCTACTACGAGCGCTACGCCAACTTCCTGG<br>GCCGAGGCTTTCCTGACGTCGCCGCCCTCAGCCTCAACCCCGA<br>CTACCCCGTCATCATCGGCGGCGAGCTTGGCCCTAACGGCGG<br>CACTTCTGCTGCCGCCCCTGTCGTCGCCAGCATCATTGCCCTG<br>CTCAACGACGCCCGCCTCTGCCTCGGCAAGCCTGCCCTCGGCT<br>TTCTCAACCCCCTCATCTACCAGTACGCCGACAAGGGCGGCTT<br>CACCGACATCACCAGCGGCCAGTCTTGGGGCTGCGCCGGCAA<br>CACCACTCAGACTGGACCTCCCCCTCCTGGCGCTGGCGTCATT<br>CCTGGCGCTCACTGGAACGCCACCAAGGGCTGGGACCCCGTC<br>ACCGGCTTTGGCACCCCCAACTTCAAGAAGCTCCTCAGCCTCG<br>CCCTCAGCGTCTAA | Trichoderma atroviride IMI 206040 |

(continued)

| SEQ ID No.: | Sequence | Origin |
|---|---|---|
| 92 | <u>ATGCAGACCTTCGGTGCTTTTCTCGTTTCCTTCCTCGCCGCCAG CGGCCTGGCCGCGGCC</u>TCTCCTCTTGCTCGACGCTGGGACGA CTTCGCCGAGAAGCACGCCTGGGTCGAGGTTCCTCGCGGCTG GGAGATGGTCAGCGAGGCCCCTAGCGACCACACCTTCGACCTC CGCATCGGCGTCAAGAGCAGCGGCATGGAACAGCTCATCGAG AACCTCATGCAGACCAGCGACCCCACCCACAGCCGCTACGGCC AGCACCTCAGCAAGGAAGAACTCCACGACTTCGTCCAGCCCCA CCCCGACTCTACTGGCGCCGTCGAGGCCTGGCTTGAGGACTTC GGCATCAGCGACGACTTCATCGACCGCACCGGCAGCGGCAAC TGGGTCACCGTCCGAGTCTCTGTCGCCCAGGCCGAGCGAATG CTCGGCACCAAGTACAACGTCTACCGCCACAGCGAGAGCGGC GAGTCCGTCGTCCGCACCATGAGCTACAGCCTCCCCAGCGAGC TGCACAGCCACATCGACGTCGTCGCCCCCACCACCTACTTCGG CACCATGAAGTCGATGCGCGTCACCTCGTTCCTCCAGCCCGAG ATCGAGCCCGTCGACCCCTCTGCCAAGCCTTCTGCTGCTCCCG CCAGCTGCCTCAGCACCACCGTCATTACCCCCGACTGCCTCCG CGACCTCTACAACACCGCCGACTACGTCCCCAGCGCCACCAGC CGCAACGCCATTGGCATTGCCGGCTACCTCGACCGCAGCAACC GAGCCGACCTCCAGACCTTCTTCCGCCGCTTTCGCCCTGACGC CGTCGGCTTCAACTACACCACCGTCCAGCTCAACGGCGGAGGC GACGACCAGAACGACCCTGGCGTCGAGGCCAACCTCGACATC CAGTACGCCGCTGGCATTGCCTTCCCCACCCCCGCCACCTACT GGTCTACTGGCGGCAGCCCCCCCTTCATCCCCGACACCCAGAC CCCCACCAACACCAACGAGCCCTACCTCGACTGGATCAACTTC GTCCTCGGCCAGGATGAGATCCCCCAGGTCATCAGCACCAGCT ACGGCGACGACGAGCAGACCGTCCCCGAGGACTACGCCACCA GCGTCTGCAACCTCTTCGCCCAGCTTGGCTCTCGCGGCGTCAC CGTCTTTTTCAGCAGCGGCGACTTCGGCGTCGGCGGTGGCGA CTGCCTCACTAACGACGGCAGCAACCAGGTCCTCTTCCAGCCC GCCTTCCCTGCCAGCTGCCCCTTTGTCACTGCCGTCGGCGGCA CCGTCCGACTCGACCCTGAGATCGCCGTCAGCTTCAGCGGCG GTGGCTTCAGCCGCTACTTCAGCCGCCCCAGCTACCAGAACCA GACCGTCGCCCAGTTCGTCAGCAACCTCGGCAACACCTTCAAC GGCCTCTACAACAAGAACGGCCGAGCCTACCCCGACCTCGCC GCTCAGGGCAACGGCTTCCAGGTCGTCATCGACGGCATCGTCC GATCGGTCGGCGGCACTTCTGCCAGCAGCCCTACCGTCGCCG GCATCTTCGCCCTGCTCAACGACTTCAAGCTCTCTCGCGGCCA GAGCACCCTCGGCTTCATCAACCCCCTCATCTACAGCAGCGCC ACCTCCGGCTTCAACGACATCCGAGCCGGCACCAACCCTGGCT GTGGCACCCGAGGCTTTACCGCCGGCACTGGCTGGGACCCTG TCACCGGACTCGGCACCCCTGACTTTCTCCGCCTCCAGGGCCT CATCTAA | Agaricus bisporus var. burnettii JB137-S8 |

(continued)

| SEQ ID No.: | Sequence | Origin |
|---|---|---|
| 93 | <u>ATGCAGACCTTCGGTGCTTTTCTCGTTTCCTTCCTCGCCGCCAG</u> <u>CGGCCTGGCCGCGGCC</u>CGCGTCTTTGATTCTCTCCCTCACCCC CCTCGCGGCTGGTCCTACTCTCACGCCGCTGAGAGCACCGAG CCCCTCACCCTCCGAATTGCCCTCCGCCAGCAGAACGCCGCTG CCCTTGAGCAGGTCGTCCTCCAGGTCAGCAACCCCCGCCACGC CAACTACGGCCAGCACCTCACCCGAGATGAGCTGCGCTCTTAC ACCGCCCCTACCCCTCGCGCTGTCCGCTCTGTCACTAGCTGGC TCGTCGACAACGGCGTCGACGACTACACCGTCGAGCACGACTG GGTCACCCTCCGCACCACTGTCGGCGCTGCCGATCGACTCCTC GGCGCCGACTTTGCCTGGTACGCTGGCCCTGGCGAGACTCTC CAGCTCCGCACTCTCAGCTACGGCGTGGACGACAGCGTCGCC CCTCACGTCGATCTCGTCCAGCCCACCACCCGCTTTGGCGGCC CTGTTGGCCAGGCCAGCCACATCTTCAAGCAGGACGACTTCGA CGAGCAGCAGCTCAAGACCCTCAGCGTCGGCTTCCAGGTCATG GCCGACCTCCCTGCTAACGGCCCTGGCAGCATTAAGGCCGCCT GCAACGAGAGCGGCGTCACCCCTCTCTGCCTCCGCACCCTCTA CCGCGTCAACTACAAGCCCGCCACCACCGGCAACCTCGTCGCC TTCGCCAGCTTCCTTGAGCAGTACGCCCGCTACAGCGACCAGC AGGCCTTCACCCAGCGAGTCCTTGGCCCTGGCGTCCCGCTCCA GAACTTCAGCGTCGAGACTGTCAACGGCGGAGCCAACGACCAG CAGAGCAAGCTCGATAGCGGCGAGGCCAACCTCGACCTCCAGT ACGTCATGGCCATGTCCCACCCCATCCCCATCCTTGAGTACAG CACTGGCGGCCGAGGCCCCCTCGTCCCTACTCTCGATCAGCCC AACGCCAACAACAGCAGCAACGAGCCCTACCTTGAGTTCCTCA CCTACCTGCTCGCCCAGCCCGACAGCGCCATTCCCCAGACTCT CAGCGTGAGCTACGGCGAGGAAGAACAGAGCGTCCCCCGCGA CTACGCCATCAAGGTCTGCAACATGTTCATGCAGCTCGGCGCT CGCGGCGTCAGCGTCATGTTTAGCAGCGGCGATAGCGGCCCT GGCAACGACTGCGTCCGAGCCTCTGACAACGCCACCTTCTTCG GCAGCACCTTCCCTGCCGGCTGCCCCTACGTCACTAGCGTCGG CAGCACCGTCGGCTTCGAGCCTGAGCGAGCCGTCAGCTTTAGC TCCGGCGGCTTCAGCATCTACCACGCCCGACCCGACTACCAGA ACGAGGTCGTCCCCAAGTACATCGAGAGCATCAAGGCCAGCGG CTACGAGAAGTTCTTCGACGGCAACGGCCGAGGCATCCCCGAT GTCGCTGCTCAGGGCGCTCGCTTCGTCGTCATCGACAAGGGCC GCGTCAGCCTCATCAGCGGCACTAGCGCTTCCAGCCCCGCCTT CGCTGGCATGGTCGCCCTCGTCAACGCCGCTCGCAAGAGCAA GGATATGCCCGCCCTCGGCTTCCTCAACCCCATGCTCTACCAG AACGCTGCCGCCATGACCGACATCGTCAACGGCGCTGGCATCG GCTGCCGCAAGCAGCGCACCGAGTTTCCCAACGGTGCCCGCTT CAACGCCACCGCCGGATGGGACCCTGTCACTGGCCTTGGCAC CCCCCTGTTCGACAAGCTCCTCGCCGTTGGCGCTCCCGGCGTC CCTAACGCCTAA | Magnaporthe oryzae 70-15 |

(continued)

| SEQ ID No.: | Sequence | Origin |
|---|---|---|
| 94 | <u>ATGCAGACCTTCGGTGCTTTTCTCGTTTCCTTCCTCGCCGCCAG CGGCCTGGCCGCGGCC</u>TCCGATGTCGTCCTTGAGTCTCTCCGC GAGGTCCCCCAGGGCTGGAAGCGACTCCGAGATGCCGACCCC GAGCAGAGCATCAAGCTCCGCATTGCCCTTGAGCAGCCCAACC TCGACCTCTTCGAGCAGACCCTCTACGACATCAGCAGCCCCGA CCACCCCAAGTACGGCCAGCACCTCAAGAGCCACGAGCTGCG CGACATCATGGCCCCTCGCGAGGAATCCACTGCCGCCGTCATT GCCTGGCTCCAGGATGCTGGCCTCAGCGGCAGCCAGATCGAG GACGACAGCGACTGGATCAACATCCAGACCACCGTCGCCCAGG CCAACGACATGCTCAACACCACCTTCGGCCTCTTCGCCCAAGA GGGCACCGAGGTCAACCGCATTCGCGCCCTCGCCTACAGCGT CCCCGAGGAAATTGTCCCCCACGTCAAGATGATCGCCCCCATC ATCCGCTTCGGCCAGCTCCGCCCTCAGATGAGCCACATCTTCA GCCACGAGAAGGTCGAGGAAACCCCCAGCATCGGCACCATCAA GGCCGCTGCCATCCCCAGCGTCGACCTCAACGTCACCGCCTG CAACGCCAGCATCACCCCCGAGTGCCTCCGCGCCCTCTACAAC GTCGGCGACTACGAGGCCGACCCCAGCAAGAAGTCCCTCTTCG GCGTCTGCGGCTACCTTGAGCAGTACGCCAAGCACGACCAGCT CGCCAAGTTCGAGCAGACGTACGCCCCCTACGCCATCGGCGC CGACTTCAGCGTCGTCACCATCAACGGCGGAGGCGACAACCAG ACCAGCACCATCGACGACGGCGAGGCCAACCTCGACATGCAGT ACGCCGTCAGCATGGCCTACAAGACCCCCATCACCTACTACAG CACTGGCGGCCGAGGCCCCCTCGTCCCTGATCTCGATCAGCC CGACCCCAACGACGTCAGCAACGAGCCCTACCTCGACTTCGTC AGCTACCTCCTCAAGCTCCCCGACAGCAAGCTCCCCCAGACCA TCACCACCAGCTACGGCGAGGACGAGCAGAGCGTCCCCCGCA GCTACGTCGAGAAGGTCTGCACCATGTTCGGCGCCCTTGGCGC CGAGGCGTCAGCGTCATTTTCAGCTCTGGCGACACCGGCGTC GGCAGCGCCTGCCAGACTAACGACGGCAAGAACACCACCCGC TTTCTGCCCATCTTCCCTGCCGCCTGCCCCTACGTCACTAGCGT CGGCGGCACCCGCTACGTCGATCCTGAGGTCGCCGTCAGCTT CAGCAGCGGCGGCTTCAGCGACATCTTCCCCACCCCCCTGTAC CAGAAGGGCGCCGTCAGCGGCTACCTCAAGATCCTCGGCGAC CGCTGGAAGGGCCTCTACAACCCTCACGGCCGAGGCTTCCCTG ACGTCAGCGGCCAGTCTGTCCGCTACCACGTCTTTGACTACGG CAAGGACGTCATGTACAGCGGCACCAGCGCCAGCGCCCCCAT GTTTGCTGCTCTCGTCAGCCTCCTCAACAACGCCCGCCTCGCC AAGAAGCTCCCCCCTATGGGCTTCCTCAACCCCTGGCTCTACA CCGTCGGCTTCAACGGCCTCACCGACATCGTCCACGGCGGCTC TACTGGCTGCACCGGCACCGATGTCTACAGCGGCCTGCCTACC CCCTTCGTCCCCTACGCCTCTTGGAACGCCACCGTCGGCTGGG ACCCTGTCACTGGCCTTGGCACCCCCCTGTTCGACAAGCTCCT CAACCTCAGCACCCCCAACTTCCACCTCCCCCACATCGGCGGC CACTAA | Togninia minima UCRPA7 |

(continued)

| SEQ ID No.: | Sequence | Origin |
|---|---|---|
| 95 | <u>ATGCAGACCTTCGGTGCTTTTCTCGTTTCCTTCCTCGCCGCCAG</u><br><u>CGGCCTGGCCGCGGCC</u>TCTACCACTTCTCACGTCGAGGGCGA<br>GGTCGTCGAGCGCCTTCATGGCGTCCCTGAGGGCTGGTCACA<br>GGTCGGCGCTCCCAACCCCGACCAGAAGCTCCGCTTCCGCATT<br>GCCGTCCGCAGCGCCGACAGCGAGCTGTTCGAGCGCACCCTC<br>ATGGAAGTCAGCAGCCCCAGCCACCCCGCTACGGCCAGCAC<br>CTCAAGCGCCACGAGCTGAAGGACCTCATCAAGCCTCGCGCCA<br>AGAGCACCAGCAACATCCTCAACTGGCTCCAAGAGAGCGGCAT<br>CGAGGCCCGCGACATCCAGAACGACGGCGAGTGGATCAGCTT<br>CTACGCCCCCGTCAAGCGAGCCGAGCAGATGATGAGCACCAC<br>CTTCAAGACCTACCAGAACGAGGCCCGAGCCAACATCAAGAAG<br>ATCCGCAGCCTCGACTACAGCGTCCCCAAGCACATCCGCGACG<br>ACATCGACATCATCCAGCCCACCACGCGCTTCGGCCAGATCCA<br>GCCTGAGCGCAGCCAGGTCTTTAGCCAAGAGGAAGTCCCCTTC<br>AGCGCCCTCGTCGTCAACGCCACGTGCAACAAGAAGATCACCC<br>CCGACTGCCTCGCCAACCTCTACAACTTCAAGGACTACGACGC<br>CAGCGACGCCAACGTCACGATCGGCGTCAGCGGCTTCCTTGAG<br>CAGTACGCCCGCTTCGACGACCTCAAGCAGTTCATCAGCACCT<br>TCCAGCCCAAGGCCGCTGGCTCCACCTTCCAGGTCACCAGCGT<br>CAACGCTGGCCCCTTCGACCAGAACAGCACCGCCTCTAGCGTC<br>GAGGCCAACCTCGACATCCAGTACACCACCGGCCTCGTCGCCC<br>CCGACATCGAGACTCGCTACTTCACCGTCCCCGGACGCGGCAT<br>CCTCATCCCCGACCTCGACCAGCCTACCGAGAGCGACAACGCC<br>AACGAGCCCTACCTCGACTACTTCACCTACCTCAACAACCTTGA<br>GGACGAGGAACTCCCCGACGTCCTCACCACCAGCTACGGCGA<br>GAGCGAGCAGAGCGTCCCTGCCGAGTACGCCAAGAAGGTCTG<br>CAACCTCATCGGCCAGCTCGGCGCTCGCGGCGTCAGCGTCATT<br>TTCAGCAGCGGCGACACCGGCCCTGGCAGCGCCTGCCAGACT<br>AACGACGGCAAGAACACCACCCGCTTTCTGCCCATCTTCCCCG<br>CCAGCTGCCCCTACGTCACTAGCGTCGGCGGCACTGTCGGCG<br>TCGAGCCTGAGAAGGCCGTCAGCTTTAGCAGCGGCGGCTTCAG<br>CGACCTCTGGCCCCGACCTGCCTACCAAGAGAAGGCCGTGAG<br>CGAGTACCTTGAGAAGCTCGGCGACCGCTGGAACGGCCTCTAC<br>AACCCTCAGGGCCGAGGCTTCCCTGACGTCGCTGCTCAGGGC<br>CAGGGCTTCCAGGTCTTTGACAAGGGCCGCCTCATCTCGGTCG<br>GCGGCACATCTGCTTCCGCCCCTGTCTTTGCCAGCGTCGTCGC<br>CCTCCTCAACAACGCCCGAAAGGCTGCCGGAATGAGCAGCCTC<br>GGCTTCCTCAACCCCTGGATCTACGAGCAGGGCTACAAGGGCC<br>TCACCGACATCGTCGCTGGCGGCTCTACTGGCTGCACCGGCC<br>GCTCTATCTACAGCGGCCTCCCTGCCCCCCTGGTCCCTTACGC<br>TTCTTGGAACGCCACCGAGGGCTGGGACCCCGTCACTGGCTAT<br>GGCACCCCCGACTTCAAGCAGCTCCTCACCCTCGCCACCGCCC<br>CCAAGTCTGGCGAGCGACGAGTTCGACGAGGCGGCCTTGGAG<br>GCCAGGCTTAA | Bipolaris maydis C5 |

| SEQ ID No.: | Description | Sequence | Origin |
|---|---|---|---|
| 96 | TRI045 Genomic sequence CDS | ATGCGTCTTCTCAAATTTGTGTGCCTGTTGGCATC AGTTGCCGCCGCAAAGCCTACTCCAGGGGCGTC ACACAAGGTCATTGAACATCTTGACTTTGTTCCAG AAGGATGGCAGATGGTTGGTGCCGCGGACCCTG CTGCTATCATTGATTTCTGGCTTGCCATCGAGCGC GAAAACCCAGAAAAGCTCTACGACACCATCTATG ACGTCTCCACCCCTGGACGCGCACAATATGGCAA ACATTTGAAGCGTGAGGAATTGGATGACTTACTAC GCCCAAGGGCAGAGACGAGTGAGAGCATCATCA ACTGGCTCACCAATGGTGGAGTCAACCCACAACA TATTCGGGATGAAGGGGACTGGGTCAGATTCTCT ACCAATGTCAAGACTGCCGAAACGTTGATGAATA CCCGCTTCAACGTCTTCAAGGACAACCTAAATTCC GTTTCAAAAATTCGAACTTTGGAGTATTCCGTCCC TGTAGCTATATCAGCTCATGTCCAAATGATCCAGC CAACTACCTTATTTGGACGACAGAAGCCACAGAA CAGTTTGATCCTAAACCCCTTGACCAAGGATCTAG AATCCATGTCCGTTGAAGAATTTGCTGCTTCTCAG TGCAGGTCCTTAGTGACTACTGCCTGCCTTCGAG AATTGTACGGACTTGGTGACCGTGTCACTCAGGC TAGGGATGACAACCGTATTGGAGTATCCGGCTTT TTGGAGGAGTACGCCCAATACCGCGATCTTGAGC TCTTCCTCTCTCGCTTTGAGCCATCCGCCAAAGG ATTTAATTTCAGTGAAGGCCTTATTGCCGGAGGAA AGAACACTCAGGGTGGTCCTGGAAGCTCTACTGA GGCCAACCTTGATATGCAATATGTCGTCGGTCTG TCCCACAAGGCAAAGGTCACCTATTACTCCACCG CTGGCCGTGGCCCATTAATTCCCGATCTATCTCA GCCAAGCCAAGCTTCAAACAACAACGAACCATAC CTTGAACAGCTGCGGTACCTCGTAAAGCTCCCCA AGAACCAGCTTCCATCTGTATTGACAACTTCCTAT GGAGACACAGAACAGAGCTTGCCCGCCAGCTATA CCAAAGCCACTTGCGACCTCTTTGCTCAGCTAGG AACTATGGGTGTGTCTGTTATCTTCAGCAGTGGTG ATACCGGGCCCGGAAGCTCATGCCAGACCAACG ATGGCAAGAATGCGACTCGCTTCAACCCTATCTA CCCAGCTTCTTGCCCGTTTGTGACCTCCATCGGT GGAACCGTTGGTACCGGTCCTGAGCGTGCAGTTT CATTCTCCTCTGGTGGCTTCTCAGACAGGTTCCC CCGCCCACAATATCAGGATAACGCTGTTAAAGAC TACCTGAAAATTTTGGGCAACCAGTGGAGCGGAT TGTTTGACCCCAACGGCCGTGCTTTCCCAGATAT CGCAGCTCAGGGATCAAATTATGCTGTCTATGAC AAGGGAAGGATGACTGGAGTCTCCGGCACCAGT GCATCCGCCCCTGCCATGGCTGCCATCATTGCCC AGCTTAACGATTTCCGACTGGCAAAGGGCTCTCC TGTGCTGGGATTCTTGAACCCATGGATATATTCCA AGGGTTTCTCTGGCTTTACAGATATTGTTGATGGC GGTTCCAGGGGGTTGCACTGGTTACGATATATACA GCGGCTTGAAAGCGAAGAAGGTTCCCTACGCAAG CTGGAATGCAACTAAGGGATGGGACCCAGTAACG GGATTTGGTACTCCCAACTTCCAAGCTCTCACTAA AGTGCTGCCCTAA | Human skin fungus *Arthroderma benhamiae* |

(continued)

| SEQ ID No.: | Description | Sequence | Origin |
|---|---|---|---|
| 97 | TRI045Synthetic Gene optimized for expression in trichoderma with trichoderma signal sequence underlined | <u>ATGCAGACCTTCGGTGCTTTTCTCGTTTCCTTCCT CGCCGCCAGCGGCCTGGCCGCGGCC</u>AAGCCTAC TCCTGGCGCTTCCCACAAGGTCATCGAGCACCTC GACTTCGTCCCCGAGGGCTGGCAGATGGTCGGC GCTGCTGACCCTGCCGCCATCATCGACTTTTGGC TCGCCATCGAGCGCGAGAACCCCGAGAAGCTCTA CGACACCATCTACGACGTCAGCACCCCCGGACG CGCCCAGTACGGCAAGCACCTCAAGCGCGAGGA ACTCGACGACCTCCTCCGCCCTCGCGCCGAGACT AGCGAGAGCATCATCAACTGGCTCACCAACGGCG GCGTCAACCCCCAGCACATTCGCGACGAGGGCG ACTGGGTCCGCTTCAGCACCAACGTCAAGACCGC CGAGACTCTCATGAACACCCGCTTCAACGTCTTTA AGGACAACCTCAACAGCGTCAGCAAGATCCGCAC CCTTGAGTACAGCGTCCCCGTCGCCATCAGCGCC CACGTCCAGATGATCCAGCCCACCACCCTCTTCG GCCGCCAGAAGCCCCAGAACAGCCTCATCCTCAA CCCCCTCACCAAGGACCTTGAGAGCATGAGCGTC GAAGAGTTCGCCGCCAGCCAGTGCCGCAGCCTC GTCACTACTGCCTGCCTCCGCGAGCTGTACGGCC TCGGCGATCGAGTCACCCAGGCCCGCGACGACA ACCGAATTGGCGTCAGCGGCTTCCTCGAAGAGTA CGCCCAGTACCGCGACCTTGAGCTGTTCCTCAGC CGCTTCGAGCCCAGCGCCAAGGGCTTCAACTTCA GCGAGGGCCTGATCGCTGGCGGCAAGAACACCC AGGGTGGCCCTGGCTCTAGCACCGAGGCCAACC TCGACATGCAGTACGTCGTCGGCCTCAGCCACAA GGCCAAGGTCACCTACTACAGCACTGCCGGCCG AGGCCCCCTCATCCCTGATCTCTCACAGCCCAGC CAGGCCAGCAACAACAACGAGCCCTACCTTGAGC AGCTCCGCTACCTCGTCAAGCTCCCCAAGAACCA GCTCCCCAGCGTCCTCACCACCAGCTACGGCGA CACCGAGCAGAGCCTCCCCGCCAGCTACACCAA GGCCACGTGCGACCTCTTCGCCCAGCTCGGCAC TATGGGCGTCAGCGTCATCTTCAGCAGCGGCGAC ACTGGCCCTGGCAGCTCGTGCCAGACCAACGAC GGCAAGAACGCCACGCGCTTCAACCCCATCTACC CCGCCAGCTGCCCCTTCGTCACCAGCATTGGCG GCACCGTCGGCACCGGCCCTGAGCGAGCTGTCA GCTTTAGCAGCGGCGGCTTCAGCGACCGCTTCCC TCGCCCTCAGTACCAGGACAACGCCGTCAAGGAC TACCTCAAGATCCTCGGCAACCAGTGGTCCGGCC TCTTCGACCCTAACGGCCGAGCCTTCCCCGACAT TGCCGCCCAGGGCAGCAACTACGCCGTCTACGA CAAGGGCCGCATGACCGGCGTTAGCGGCACTTC TGCTTCCGCCCCTGCTATGGCCGCCATCATTGCC CAGCTCAACGACTTCCGCCTCGCCAAGGGCAGC CCCGTCCTCGGCTTTCTCAACCCCTGGATCTACA GCAAGGGCTTCAGCGGCTTCACCGACATCGTCGA CGGCGGCTCTAGGGGCTGCACCGGCTACGACAT CTACAGCGGCCTCAAGGCCAAGAAGGTCCCCTAC GCCAGCTGGAACGCCACCAAGGGCTGGGACCCC GTCACCGGCTTTGGCACCCCCAACTTCCAGGCCC<br><br>TGACCAAGGTCCTGCCCTAA | Human skin fungus *Arthroderma benhamiae* |

(continued)

| SEQ ID No.: | Description | Sequence | Origin |
|---|---|---|---|
| 98 | TRI045 pre_pro amino acid sesquence | KPTPGASHKVIEHLDFVPEGWQMVGAADPAAIIDFW LAIERENPEKLYDTIYDVSTPGRAQYGKHLKREELD DLLRPRAETSESIINWLTNGGVNPQHIRDEGDWVRF STNVKTAETLMNTRFNVFKDNLNSVSKIRTLEYSVP VAISAHVQMIQPTTLFGRQKPQNSLILNPLTKDLESM SVEEFAASQCRSLVTTACLRELYGLGDRVTQARDD NRIGVSGFLEEYAQYRDLELFLSRFEPSAKGFNFSE GLIAGGKNTQGGPGSSTEANLDMQYVVGLSHKAKV TYYSTAGRGPLIPDLSQPSQASNNNEPYLEQLRYLV KLPKNQLPSVLTTSYGDTEQSLPASYTKATCDLFAQ LGTMGVSVIFSSGDTGPGSSCQTNDGKNATRFNPIY PASCPFVTSIGGTVGTGPERAVSFSSGGFSDRFPR PQYQDNAVKDYLKILGNQWSGLFDPNGRAFPDIAA QGSNYAVYDKGRMTGVSGTSASAPAMAAIIAQLND FRLAKGSPVLGFLNPWIYSKGFSGFTDIVDGGSRGC TGYDIYSGLKAKKVPYASWNATKGWDPVTGFGTPN FQALTKVLP | Human skin fungus *Arthroderma benhamiae* |
| 99 | TRI045 mature Interpro domain IPR000209 Peptidase S8/S53 dom | CRSLVTTACLRELYGLGDRVTQARDDNRIGVSGFLE EYAQYRDLELFLSRFEPSAKGFNFSEGLIAGGKNTQ GGPGSSTEANLDMQYVVGLSHKAKVTYYSTAGRGP LIPDLSQPSQASNNNEPYLEQLRYLVKLPKNQLPSV LTTSYGDTEQSLPASYTKATCDLFAQLGTMGVSVIF SSGDTGPGSSCQTNDGKNATRFNPIYPASCPFVTSI GGTVGTGPERAVSFSSGGFSDRFPRPQYQDNAVK DYLKILGNQWSGLFDPNGRAFPDIAAQGSNYAVYD KGRMTGVSGTSASAPAMAAIIAQLNDFRLAKGSPVL GFLNPWIYSKGFSGFTDIVDGGSRGCTGYDIYSGLK AKKVPYASWNATKGWDPVTGFGTPNFQALTKVLP | Human skin fungus *Arthroderma benhamiae* |

Described herein, the at least one proline tolerant tripeptidyl peptidase may:

(a) comprise the amino acid sequence SEQ ID No. 29, SEQ ID No. 1, SEQ ID No. 2, SEQ ID No. 3, SEQ ID No. 4, SEQ ID No. 5, SEQ ID No.6, SEQ ID No. 7, SEQ ID No. 8, SEQ ID No. 9, SEQ ID No. 10, SEQ ID No. 11, SEQ ID No. 12, SEQ ID No. 13, SEQ ID No. 14, SEQ ID No. 15, SEQ ID No. 16, SEQ ID No. 17, SEQ ID No. 18, SEQ ID No. 19, SEQ ID No. 20, SEQ ID No. 21, SEQ ID No. 22, SEQ ID No. 23, SEQ ID No. 24, SEQ ID No. 25, SEQ ID No. 26, SEQ ID No. 27, SEQ ID No. 28, SEQ ID No. 30, SEQ ID No. 31, SEQ ID No. 32, SEQ ID No. 33, SEQ ID No. 34, SEQ ID No. 35, SEQ ID No. 36, SEQ ID No. 37, SEQ ID No. 38, SEQ ID No. 39, SEQ ID No. 40, SEQ ID No. 41, SEQ ID No. 42, SEQ ID No. 43, SEQ ID No. 44, SEQ ID No. 45, SEQ ID No. 46, SEQ ID No. 47, SEQ ID No. 48, SEQ ID No. 49, SEQ ID No. 50, SEQ ID No. 51, SEQ ID No. 52, SEQ ID No. 53, SEQ ID No. 54, SEQ ID No. 55, SEQ ID No. 98, SEQ ID No. 99 or a functional fragment thereof;
(b) comprise an amino acid having at least 70% identity to SEQ ID No. 29, SEQ ID No. 1, SEQ ID No. 2, SEQ ID No. 3, SEQ ID No. 4, SEQ ID No. 5, SEQ ID No.6, SEQ ID No. 7, SEQ ID No. 8, SEQ ID No. 9, SEQ ID No. 10, SEQ ID No. 11, SEQ ID No. 12, SEQ ID No. 13, SEQ ID No. 14, SEQ ID No. 15, SEQ ID No. 16, SEQ ID No. 17, SEQ ID No. 18, SEQ ID No. 19, SEQ ID No. 20, SEQ ID No. 21, SEQ ID No. 22, SEQ ID No. 23, SEQ ID No. 24, SEQ ID No. 25, SEQ ID No. 26, SEQ ID No. 27, SEQ ID No. 28, SEQ ID No. 30, SEQ ID No. 31, SEQ ID No. 32, SEQ ID No. 33, SEQ ID No. 34, SEQ ID No. 35, SEQ ID No. 36, SEQ ID No. 37, SEQ ID No. 38, SEQ ID No. 39, SEQ ID No. 40, SEQ ID No. 41, SEQ ID No. 42, SEQ ID No. 43, SEQ ID No. 44, SEQ ID No. 45, SEQ ID No. 46, SEQ ID No. 47, SEQ ID No. 48, SEQ ID No. 49, SEQ ID No. 50, SEQ ID No. 51, SEQ ID No. 52, SEQ ID No. 53, SEQ ID No. 54, SEQ ID No. 55, SEQ ID No. 98, SEQ ID No. 99 or a functional fragment thereof;

(c) be encoded by a nucleotide sequence comprising the sequence SEQ ID No. 56, SEQ ID No. 57, SEQ ID No. 58, SEQ ID No. 59, SEQ ID No. 60, SEQ ID No. 61, SEQ ID No. 62, SEQ ID No. 63, SEQ ID No. 64, SEQ ID No. 65, SEQ ID No. 66, SEQ ID No. 67, SEQ ID No. 68, SEQ ID No. 69, SEQ ID No. 70, SEQ ID No. 71, SEQ ID No. 72, SEQ ID No. 73, SEQ ID No. 74, SEQ ID No. 75, SEQ ID No. 76, SEQ ID No. 77, SEQ ID No. 78, SEQ ID No. 79, SEQ ID No. 80, SEQ ID No. 81, SEQ ID No. 82, SEQ ID No. 83, SEQ ID No. 84, SEQ ID No. 85, SEQ ID No. 86, SEQ ID No. 87, SEQ ID No. 88, SEQ ID No. 89, SEQ ID No. 90, SEQ ID No. 91, SEQ ID No. 92, SEQ ID No. 93, SEQ ID No. 94,SEQ ID No. 95, SEQ ID No. 96 or SEQ ID No. 97;

(d) be encoded by a nucleotide sequence comprising at least about 70% sequence identity to SEQ ID No. 56, SEQ ID No. 57, SEQ ID No. 58, SEQ ID No. 59, SEQ ID No. 60, SEQ ID No. 61, SEQ ID No. 62, SEQ ID No. 63, SEQ ID No. 64, SEQ ID No. 65, SEQ ID No. 66, SEQ ID No. 67, SEQ ID No. 68, SEQ ID No. 69, SEQ ID No. 70, SEQ ID No. 71, SEQ ID No. 72, SEQ ID No. 73, SEQ ID No. 74, SEQ ID No. 75, SEQ ID No. 76, SEQ ID No. 77, SEQ ID No. 78, SEQ ID No. 79, SEQ ID No. 80, SEQ ID No. 81, SEQ ID No. 82, SEQ ID No. 83, SEQ ID No. 84, SEQ ID No. 85, SEQ ID No. 86, SEQ ID No. 87, SEQ ID No. 88, SEQ ID No. 89, SEQ ID No. 90, SEQ ID No. 91, SEQ ID No. 92, SEQ ID No. 93, SEQ ID No. 94, SEQ ID No. 95, SEQ ID No. 96 or SEQ ID No. 97;

(e) be encoded by a nucleotide sequence which hybridises to SEQ ID No. 56, SEQ ID No. 57, SEQ ID No. 58, SEQ ID No. 59, SEQ ID No. 60, SEQ ID No. 61, SEQ ID No. 62, SEQ ID No. 63, SEQ ID No. 64, SEQ ID No. 65, SEQ ID No. 66, SEQ ID No. 67, SEQ ID No. 68, SEQ ID No. 69, SEQ ID No. 70, SEQ ID No. 71, SEQ ID No. 72, SEQ ID No. 73, SEQ ID No. 74, SEQ ID No. 75, SEQ ID No. 76, SEQ ID No. 77, SEQ ID No. 78, SEQ ID No. 79, SEQ ID No. 80, SEQ ID No. 81, SEQ ID No. 82, SEQ ID No. 83, SEQ ID No. 84, SEQ ID No. 85, SEQ ID No. 86, SEQ ID No. 87, SEQ ID No. 88, SEQ ID No. 89, SEQ ID No. 90, SEQ ID No. 91, SEQ ID No. 92, SEQ ID No. 93, SEQ ID No. 94, SEQ ID No. 95, SEQ ID No. 96 or SEQ ID No. 97 under medium stringency conditions; or

(f) be encoded by a nucleotide sequence which differs from SEQ ID No. 56, SEQ ID No. 57, SEQ ID No. 58, SEQ ID No. 59, SEQ ID No. 60, SEQ ID No. 61, SEQ ID No. 62, SEQ ID No. 63, SEQ ID No. 64, SEQ ID No. 65, SEQ ID No. 66, SEQ ID No. 67, SEQ ID No. 68, SEQ ID No. 69, SEQ ID No. 70, SEQ ID No. 71, SEQ ID No. 72, SEQ ID No. 73, SEQ ID No. 74, SEQ ID No. 75, SEQ ID No. 76, SEQ ID No. 77, SEQ ID No. 78, SEQ ID No. 79, SEQ ID No. 80, SEQ ID No. 81, SEQ ID No. 82, SEQ ID No. 83, SEQ ID No. 84, SEQ ID No. 85, SEQ ID No. 86, SEQ ID No. 87, SEQ ID No. 88, SEQ ID No. 89, SEQ ID No. 90, SEQ ID No. 91, SEQ ID No. 92, SEQ ID No. 93, SEQ ID No. 94, SEQ ID No. 95, SEQ ID No. 96 or SEQ ID No. 97 due to degeneracy of the genetic code.

The proline tolerant tripeptidyl peptidase may be expressed as a polypeptide sequence which undergoes further post-transcriptional and/or post-translational modification.

Described herein, the proline tolerant tripeptidyl peptidase may comprise the amino acid sequence SEQ ID No. 1, SEQ ID No. 2, SEQ ID No. 3, SEQ ID No. 4, SEQ ID No. 5, SEQ ID No.6, SEQ ID No. 7, SEQ ID No. 8, SEQ ID No. 9, SEQ ID No. 10, SEQ ID No. 11, SEQ ID No. 12, SEQ ID No. 13, SEQ ID No. 14, SEQ ID No. 15, SEQ ID No. 16, SEQ ID No. 17, SEQ ID No. 18, SEQ ID No. 19, SEQ ID No. 20, SEQ ID No. 21, SEQ ID No. 22, SEQ ID No. 23, SEQ ID No. 24, SEQ ID No. 25, SEQ ID No. 26, SEQ ID No. 27, SEQ ID No. 28, SEQ ID No. 98 or a functional fragment thereof.

Described herein, the proline tolerant tripeptidyl peptidase comprise an amino acid having at least 70% identity to SEQ ID No. 1, SEQ ID No. 2, SEQ ID No. 3, SEQ ID No. 4, SEQ ID No. 5, SEQ ID No.6, SEQ ID No. 7, SEQ ID No. 8, SEQ ID No. 9, SEQ ID No. 10, SEQ ID No. 11, SEQ ID No. 12, SEQ ID No. 13, SEQ ID No. 14, SEQ ID No. 15, SEQ ID No. 16, SEQ ID No. 17, SEQ ID No. 18, SEQ ID No. 19, SEQ ID No. 20, SEQ ID No. 21, SEQ ID No. 22, SEQ ID No. 23, SEQ ID No. 24, SEQ ID No. 25, SEQ ID No. 26, SEQ ID No. 27, SEQ ID No. 28, SEQ ID No. 98 or a functional fragment thereof.

Described herein, the proline tolerant tripeptidyl peptidase may comprise the amino acid sequence SEQ ID No. 1, SEQ ID No. 2, SEQ ID No. 3, SEQ ID No. 4, SEQ ID No. 5, SEQ ID No.6, SEQ ID No. 7, SEQ ID No. 8, SEQ ID No. 9, SEQ ID No. 10, SEQ ID No. 11, SEQ ID No. 12, SEQ ID No. 13, SEQ ID No. 14, SEQ ID No. 15, SEQ ID No. 16, SEQ ID No. 17, SEQ ID No. 18, SEQ ID No. 98 or a functional fragment thereof.

Described herein, the proline tolerant tripeptidyl peptidase comprise an amino acid having at least 70% identity to SEQ ID No. 1, SEQ ID No. 2, SEQ ID No. 3, SEQ ID No. 4, SEQ ID No. 5, SEQ ID No.6, SEQ ID No. 7, SEQ ID No. 8, SEQ ID No. 9, SEQ ID No. 10, SEQ ID No. 11, SEQ ID No. 12, SEQ ID No. 13, SEQ ID No. 14, SEQ ID No. 15, SEQ ID No. 16, SEQ ID No. 17, SEQ ID No. 18, SEQ ID No. 98 or a functional fragment thereof.

In another embodiment the proline tolerant tripeptidyl peptidase may be a "mature" proline tolerant tripeptidyl peptidase which has undergone post-transcriptional and/or post-translational modification (e.g. post-translational cleavage). Suitably such modification may lead to an activation of the enzyme.

Described herein, the proline tolerant tripeptidyl peptidase may comprise the amino acid sequence SEQ ID No. 29, SEQ ID No. 30, SEQ ID No. 31, SEQ ID No. 32, SEQ ID No. 33, SEQ ID No. 34, SEQ ID No. 35, SEQ ID No. 36, SEQ ID No. 37, SEQ ID No. 38, SEQ ID No. 39, SEQ ID No. 40, SEQ ID No. 41, SEQ ID No. 42, SEQ ID No. 43, SEQ ID No. 44, SEQ ID No. 45, SEQ ID No. 46, SEQ ID No. 47, SEQ ID No. 48, SEQ ID No. 49, SEQ ID No. 50, SEQ ID No. 51, SEQ ID No. 52, SEQ ID No. 53, SEQ ID No. 54, SEQ ID No. 55, SEQ ID No. 99 or a functional fragment thereof.

Described herein, the proline tolerant tripeptidyl peptidase comprise an amino acid having at least 70% identity to SEQ ID No. 29, SEQ ID No. 30, SEQ ID No. 31, SEQ ID No. 32, SEQ ID No. 33, SEQ ID No. 34, SEQ ID No. 35, SEQ ID No. 36, SEQ ID No. 37, SEQ ID No. 38, SEQ ID No. 39, SEQ ID No. 40, SEQ ID No. 41, SEQ ID No. 42, SEQ ID No. 43, SEQ ID No. 44, SEQ ID No. 45, SEQ ID No. 46, SEQ ID No. 47, SEQ ID No. 48, SEQ ID No. 49, SEQ ID No. 50, SEQ ID No. 51, SEQ ID No. 52, SEQ ID No. 53, SEQ ID No. 54, SEQ ID No. 55, SEQ ID No. 99 or a functional fragment thereof.

Described herein, the proline tolerant tripeptidyl peptidase may comprise the amino acid sequence SEQ ID No. 29, SEQ ID No. 30, SEQ ID No. 31, SEQ ID No. 32, SEQ ID No. 33, SEQ ID No. 34, SEQ ID No. 35, SEQ ID No. 36, SEQ ID No. 37, SEQ ID No. 38, SEQ ID No. 39, SEQ ID No. 40, SEQ ID No. 41, SEQ ID No. 42, SEQ ID No. 43, SEQ ID No. 44, SEQ ID No. 45, SEQ ID No. 99 or a functional fragment thereof.

Described herein, the proline tolerant tripeptidyl peptidase comprise an amino acid having at least 70% identity to SEQ ID No. 29, SEQ ID No. 30, SEQ ID No. 31, SEQ ID No. 32, SEQ ID No. 33, SEQ ID No. 34, SEQ ID No. 35, SEQ ID No. 36, SEQ ID No. 37, SEQ ID No. 38, SEQ ID No. 39, SEQ ID No. 40, SEQ ID No. 41, SEQ ID No. 42, SEQ ID No. 43, SEQ ID No. 44, SEQ ID No. 45, SEQ ID No. 99 or a functional fragment thereof.

The term "functional fragment" is a portion of an amino acid sequence that retains its peptidase enzyme activity. Therefore, a functional fragment of a proline tolerant tripeptidyl peptidase is a portion of a proline tolerant tripeptidyl peptidase predominantly having exopeptidase activity wherein said proline tolerant tripeptidyl peptidase is capable of cleaving tri-peptides from the N-terminus of peptides having

(i)

(A) Proline at P1; and
(B) An amino acid selected from alanine, arginine, asparagine, aspartic acid, cysteine, glutamine, glutamic acid, glycine, histidine, isoleucine, leucine, lysine, methionine, phenylalanine, serine, threonine, tryptophan, tyrosine, valine or synthetic amino acids at P1; and/or

(ii)

(a') Proline at P1'; and
(b') An amino acid selected from alanine, arginine, asparagine, aspartic acid, cysteine, glutamine, glutamic acid, glycine, histidine, isoleucine, leucine, lysine, methionine, phenylalanine, serine, threonine, tryptophan, tyrosine, valine or synthetic amino acids at P1'. Alternatively or additionally a functional fragment of a proline tolerant tripeptidyl peptidase is a portion of a proline tolerant tripeptidyl peptidase predominantly having exopeptidase activity and capable of cleaving tri-peptides from the N-terminus of peptides having proline at P1 and P1'.

The "portion" is any portion that still has the activity as defined above, suitably a portion may be at least 50 amino acids in length, more suitably at least 100. In other embodiments the portion may be about 150 or about 200 amino acids in length.

In one embodiment the functional fragment may be portion of a proline tolerant tripeptidyl peptidase following post transcriptional and/or post-translational modification (e.g. cleavage). Described herein, the functional fragment may comprise a sequence shown as: SEQ ID No. 29, SEQ ID No. 30, SEQ ID No. 31, SEQ ID No. 32, SEQ ID No. 33, SEQ ID No. 34, SEQ ID No. 35, SEQ ID No. 36, SEQ ID No. 37, SEQ ID No. 38, SEQ ID No. 39, SEQ ID No. 40, SEQ ID No. 41, SEQ ID No. 42, SEQ ID No. 43, SEQ ID No. 44, SEQ ID No. 45, SEQ ID No. 46, SEQ ID No. 47, SEQ ID No. 48, SEQ ID No. 49, SEQ ID No. 50, SEQ ID No. 51, SEQ ID No. 52, SEQ ID No. 53, SEQ ID No. 54, SEQ ID No. 55 or SEQ ID NO: 98.

The proline tolerant tripeptidyl peptidase may comprise one or more amino acid sequence selected from SEQ ID No. 1, or a functional fragment thereof.

Described herein, the proline tolerant tripeptidyl peptidase may comprise an amino acid having at least 70% identity to SEQ ID No. 1 or a functional fragment thereof.

The proline tolerant tripeptidyl peptidase may comprise one or more amino acid sequence selected from SEQ ID No. 2, or a functional fragment thereof.

Described herein, the proline tolerant tripeptidyl peptidase may comprise an amino acid having at least 70% identity to SEQ ID No. 2 or a functional fragment thereof.

Described herein, the proline tolerant tripeptidyl peptidase may comprise one or more amino acid sequence selected from SEQ ID No. 3 or a functional fragment thereof.

Described herein, the proline tolerant tripeptidyl peptidase may comprise an amino acid having at least 70% identity to SEQ ID No. 3 or a functional fragment thereof.

Described herein, the proline tolerant tripeptidyl peptidase may comprise one or more amino acid sequence selected from SEQ ID No. 4 or a functional fragment thereof.

Described herein, the proline tolerant tripeptidyl peptidase may comprise an amino acid having at least 70% identity to SEQ ID No. 4 or a functional fragment thereof.

Described herein, the proline tolerant tripeptidyl peptidase may comprise one or more amino acid sequence selected from SEQ ID No. 5 or a functional fragment thereof.

Described herein, the proline tolerant tripeptidyl peptidase may comprise an amino acid having at least 70% identity to SEQ ID No. 5 or a functional fragment thereof.

Described herein, the proline tolerant tripeptidyl peptidase may comprise one or more amino acid sequence selected from SEQ ID No.6 or a functional fragment thereof.

Described herein, the proline tolerant tripeptidyl peptidase may comprise an amino acid having at least 70% identity to SEQ ID No.6 or a functional fragment thereof.

Described herein, the proline tolerant tripeptidyl peptidase may comprise one or more amino acid sequence selected from SEQ ID No. 7 or a functional fragment thereof.

Described herein, the proline tolerant tripeptidyl peptidase may comprise an amino acid having at least 70% identity to SEQ ID No. 7 or a functional fragment thereof.

Described herein, the proline tolerant tripeptidyl peptidase may comprise one or more amino acid sequence selected from SEQ ID No. 8 or a functional fragment thereof.

Described herein, the proline tolerant tripeptidyl peptidase may comprise an amino acid having at least 70% identity to SEQ ID No. 8 or a functional fragment thereof.

Described herein, the proline tolerant tripeptidyl peptidase may comprise one or more amino acid sequence selected from SEQ ID No. 9 or a functional fragment thereof.

Described herein, the proline tolerant tripeptidyl peptidase may comprise an amino acid having at least 70% identity to SEQ ID No. 9 or a functional fragment thereof.

Described herein, the proline tolerant tripeptidyl peptidase may comprise one or more amino acid sequence selected from SEQ ID No. 10 or a functional fragment thereof.

Described herein, the proline tolerant tripeptidyl peptidase may comprise an amino acid having at least 70% identity to SEQ ID No. 10 or a functional fragment thereof.

Described herein, the proline tolerant tripeptidyl peptidase may comprise one or more amino acid sequence selected from SEQ ID No. 11 or a functional fragment thereof.

Described herein, the proline tolerant tripeptidyl peptidase may comprise an amino acid having at least 70% identity to SEQ ID No. 11 or a functional fragment thereof.

Described herein, the proline tolerant tripeptidyl peptidase may comprise one or more amino acid sequence selected from SEQ ID No. 12 or a functional fragment thereof.

Described herein, the proline tolerant tripeptidyl peptidase may comprise an amino acid having at least 70% identity to SEQ ID No. 12 or a functional fragment thereof.

Described herein, the proline tolerant tripeptidyl peptidase may comprise one or more amino acid sequence selected from SEQ ID No. 13 or a functional fragment thereof.

Described herein, the proline tolerant tripeptidyl peptidase may comprise an amino acid having at least 70% identity to SEQ ID No. 13 or a functional fragment thereof.

Described herein, the proline tolerant tripeptidyl peptidase may comprise one or more amino acid sequence selected from SEQ ID No. 14 or a functional fragment thereof.

Described herein, the proline tolerant tripeptidyl peptidase may comprise an amino acid having at least 70% identity to SEQ ID No. 14 or a functional fragment thereof.

Described herein, the proline tolerant tripeptidyl peptidase may comprise one or more amino acid sequence selected from SEQ ID No. 15 or a functional fragment thereof.

Described herein, the proline tolerant tripeptidyl peptidase may comprise an amino acid having at least 70% identity to SEQ ID No. 15 or a functional fragment thereof.

Described herein, the proline tolerant tripeptidyl peptidase may comprise one or more amino acid sequence selected from SEQ ID No. 16 or a functional fragment thereof.

Described herein, the proline tolerant tripeptidyl peptidase may comprise an amino acid having at least 70% identity to SEQ ID No. 16 or a functional fragment thereof.

Described herein, the proline tolerant tripeptidyl peptidase may comprise one or more amino acid sequence selected from SEQ ID No. 17 or a functional fragment thereof.

Described herein, the proline tolerant tripeptidyl peptidase may comprise an amino acid having at least 70% identity to SEQ ID No. 17 or a functional fragment thereof.

Described herein, the proline tolerant tripeptidyl peptidase may comprise one or more amino acid sequence selected from SEQ ID No. 18 or a functional fragment thereof.

Described herein, the proline tolerant tripeptidyl peptidase may comprise an amino acid having at least 70% identity to SEQ ID No. 18 or a functional fragment thereof.

Described herein, the proline tolerant tripeptidyl peptidase may comprise one or more amino acid sequence selected from SEQ ID No. 19 or a functional fragment thereof.

Described herein, the proline tolerant tripeptidyl peptidase may comprise an amino acid having at least 70% identity to SEQ ID No. 19 or a functional fragment thereof.

Described herein, the proline tolerant tripeptidyl peptidase may comprise one or more amino acid sequence selected from SEQ ID No. 20 or a functional fragment thereof.

Described herein, the proline tolerant tripeptidyl peptidase may comprise an amino acid having at least 70% identity to SEQ ID No. 20 or a functional fragment thereof.

Described herein, the proline tolerant tripeptidyl peptidase may comprise one or more amino acid sequence selected from SEQ ID No. 21 or a functional fragment thereof.

Described herein, the proline tolerant tripeptidyl peptidase may comprise an amino acid having at least 70% identity to SEQ ID No. 21 or a functional fragment thereof.

Described herein, the proline tolerant tripeptidyl peptidase may comprise one or more amino acid sequence selected from SEQ ID No. 22 or a functional fragment thereof.

Described herein, the proline tolerant tripeptidyl peptidase may comprise an amino acid having at least 70% identity to SEQ ID No. 22 or a functional fragment thereof.

Described herein, the proline tolerant tripeptidyl peptidase may comprise one or more amino acid sequence selected from SEQ ID No. 23 or a functional fragment thereof.

Described herein, the proline tolerant tripeptidyl peptidase may comprise an amino acid having at least 70% identity to SEQ ID No. 23 or a functional fragment thereof.

Described herein, the proline tolerant tripeptidyl peptidase may comprise one or more amino acid sequence selected from SEQ ID No. 24 or a functional fragment thereof.

Described herein, the proline tolerant tripeptidyl peptidase may comprise an amino acid having at least 70% identity to SEQ ID No. 24 or a functional fragment thereof.

Described herein, the proline tolerant tripeptidyl peptidase may comprise one or more amino acid sequence selected from SEQ ID No. 25 or a functional fragment thereof.

Described herein, the proline tolerant tripeptidyl peptidase may comprise an amino acid having at least 70% identity to SEQ ID No. 25 or a functional fragment thereof.

Described herein, the proline tolerant tripeptidyl peptidase may comprise one or more amino acid sequence selected from SEQ ID No. 26 or a functional fragment thereof.

Described herein, the proline tolerant tripeptidyl peptidase may comprise an amino acid having at least 70% identity to SEQ ID No. 26 or a functional fragment thereof.

Described herein, the proline tolerant tripeptidyl peptidase may comprise one or more amino acid sequence selected from SEQ ID No. 27 or a functional fragment thereof.

Described herein, the proline tolerant tripeptidyl peptidase may comprise an amino acid having at least 70% identity to SEQ ID No. 27 or a functional fragment thereof.

Described herein, the proline tolerant tripeptidyl peptidase may comprise one or more amino acid sequence selected from SEQ ID No. 28, or a functional fragment thereof.

Described herein, the proline tolerant tripeptidyl peptidase may comprise an amino acid having at least 70% identity to SEQ ID No. 28 or a functional fragment thereof.

The proline tolerant tripeptidyl peptidase may comprise one or more amino acid sequence selected from SEQ ID No. 29, or a functional fragment thereof.

Described herein, the proline tolerant tripeptidyl peptidase may comprise an amino acid having at least 70% identity to SEQ ID No. 29 or a functional fragment thereof.

Described herein, the proline tolerant tripeptidyl peptidase may comprise one or more amino acid sequence selected from SEQ ID No. 30 or a functional fragment thereof.

Described herein, the proline tolerant tripeptidyl peptidase may comprise an amino acid having at least 70% identity to SEQ ID No. 30 or a functional fragment thereof.

Described herein, the proline tolerant tripeptidyl peptidase may comprise one or more amino acid sequence selected from SEQ ID No. 31 or a functional fragment thereof.

Described herein, the proline tolerant tripeptidyl peptidase may comprise an amino acid having at least 70% identity to SEQ ID No. 31 or a functional fragment thereof.

Described herein, the proline tolerant tripeptidyl peptidase may comprise one or more amino acid sequence selected from SEQ ID No. 32 or a functional fragment thereof.

Described herein, the proline tolerant tripeptidyl peptidase may comprise an amino acid having at least 70% identity to SEQ ID No. 32 or a functional fragment thereof.

Described herein, the proline tolerant tripeptidyl peptidase may comprise one or more amino acid sequence selected from SEQ ID No.33 or a functional fragment thereof.

Described herein, the proline tolerant tripeptidyl peptidase may comprise an amino acid having at least 70% identity to SEQ ID No.33 or a functional fragment thereof.

Described herein, the proline tolerant tripeptidyl peptidase may comprise one or more amino acid sequence selected from SEQ ID No. 34 or a functional fragment thereof.

Described herein, the proline tolerant tripeptidyl peptidase may comprise an amino acid having at least 70% identity to SEQ ID No. 34 or a functional fragment thereof.

Described herein, the proline tolerant tripeptidyl peptidase may comprise one or more amino acid sequence selected from SEQ ID No. 35 or a functional fragment thereof.

Described herein, the proline tolerant tripeptidyl peptidase may comprise an amino acid having at least 70% identity to SEQ ID No. 35 or a functional fragment thereof.

Described herein, the proline tolerant tripeptidyl peptidase may comprise one or more amino acid sequence selected from SEQ ID No. 36 or a functional fragment thereof.

Described herein, the proline tolerant tripeptidyl peptidase may comprise an amino acid having at least 70% identity to SEQ ID No. 36 or a functional fragment thereof.

Described herein, the proline tolerant tripeptidyl peptidase may comprise one or more amino acid sequence selected from SEQ ID No. 37 or a functional fragment thereof.

Described herein, the proline tolerant tripeptidyl peptidase may comprise an amino acid having at least 70% identity to SEQ ID No. 37 or a functional fragment thereof.

Described herein, the proline tolerant tripeptidyl peptidase may comprise one or more amino acid sequence selected from SEQ ID No. 38 or a functional fragment thereof.

Described herein, the proline tolerant tripeptidyl peptidase may comprise an amino acid having at least 70% identity to SEQ ID No. 38 or a functional fragment thereof.

Described herein, the proline tolerant tripeptidyl peptidase may comprise one or more amino acid sequence selected from SEQ ID No. 39 or a functional fragment thereof.

Described herein, the proline tolerant tripeptidyl peptidase may comprise an amino acid having at least 70% identity to SEQ ID No. 39 or a functional fragment thereof.

Described herein, the proline tolerant tripeptidyl peptidase may comprise one or more amino acid sequence selected from SEQ ID No. 40 or a functional fragment thereof.

Described herein, the proline tolerant tripeptidyl peptidase may comprise an amino acid having at least 70% identity to SEQ ID No. 40 or a functional fragment thereof.

Described herein, the proline tolerant tripeptidyl peptidase may comprise one or more amino acid sequence selected from SEQ ID No. 41 or a functional fragment thereof.

Described herein, the proline tolerant tripeptidyl peptidase may comprise an amino acid having at least 70% identity to SEQ ID No. 41 or a functional fragment thereof.

Described herein, the proline tolerant tripeptidyl peptidase may comprise one or more amino acid sequence selected from SEQ ID No. 42 or a functional fragment thereof.

Described herein, the proline tolerant tripeptidyl peptidase may comprise an amino acid having at least 70% identity to SEQ ID No.42 or a functional fragment thereof.

Described herein, the proline tolerant tripeptidyl peptidase may comprise one or more amino acid sequence selected from SEQ ID No. 43 or a functional fragment thereof.

Described herein, the proline tolerant tripeptidyl peptidase may comprise an amino acid having at least 70% identity to SEQ ID No. 43 or a functional fragment thereof.

Described herein, the proline tolerant tripeptidyl peptidase may comprise one or more amino acid sequence selected from SEQ ID No. 44 or a functional fragment thereof.

Described herein, the proline tolerant tripeptidyl peptidase may comprise an amino acid having at least 70% identity to SEQ ID No. 44 or a functional fragment thereof.

Described herein, the proline tolerant tripeptidyl peptidase may comprise one or more amino acid sequence selected from SEQ ID No. 45 or a functional fragment thereof.

Described herein, the proline tolerant tripeptidyl peptidase may comprise an amino acid having at least 70% identity to SEQ ID No. 45 or a functional fragment thereof.

Described herein, the proline tolerant tripeptidyl peptidase may comprise one or more amino acid sequence selected from SEQ ID No. 46 or a functional fragment thereof.

Described herein, the proline tolerant tripeptidyl peptidase may comprise an amino acid having at least 70% identity to SEQ ID No. 46 or a functional fragment thereof.

Described herein, the proline tolerant tripeptidyl peptidase may comprise one or more amino acid sequence selected from SEQ ID No. 47 or a functional fragment thereof.

Described herein, the proline tolerant tripeptidyl peptidase may comprise an amino acid having at least 70% identity to SEQ ID No. 47 or a functional fragment thereof.

Described herein, the proline tolerant tripeptidyl peptidase may comprise one or more amino acid sequence selected from SEQ ID No. 48 or a functional fragment thereof.

Described herein, the proline tolerant tripeptidyl peptidase may comprise an amino acid having at least 70% identity to SEQ ID No. 48 or a functional fragment thereof.

Described herein, the proline tolerant tripeptidyl peptidase may comprise one or more amino acid sequence selected from SEQ ID No. 49 or a functional fragment thereof.

Described herein, the proline tolerant tripeptidyl peptidase may comprise an amino acid having at least 70% identity to SEQ ID No. 49 or a functional fragment thereof.

Described herein, the proline tolerant tripeptidyl peptidase may comprise one or more amino acid sequence selected from SEQ ID No. 50 or a functional fragment thereof.

Described herein, the proline tolerant tripeptidyl peptidase may comprise an amino acid having at least 70% identity to SEQ ID No. 50 or a functional fragment thereof.

Described herein, the proline tolerant tripeptidyl peptidase may comprise one or more amino acid sequence selected from SEQ ID No. 51 or a functional fragment thereof.

Described herein, the proline tolerant tripeptidyl peptidase may comprise an amino acid having at least 70% identity to SEQ ID No. 51 or a functional fragment thereof.

Described herein, the proline tolerant tripeptidyl peptidase may comprise one or more amino acid sequence selected from SEQ ID No. 52 or a functional fragment thereof.

Described herein, the proline tolerant tripeptidyl peptidase may comprise an amino acid having at least 70% identity to SEQ ID No. 52 or a functional fragment thereof.

Described herein, the proline tolerant tripeptidyl peptidase may comprise one or more amino acid sequence selected from SEQ ID No. 53 or a functional fragment thereof.

Described herein, the proline tolerant tripeptidyl peptidase may comprise an amino acid having at least 70% identity to SEQ ID No. 53 or a functional fragment thereof.

Described herein, the proline tolerant tripeptidyl peptidase may comprise one or more amino acid sequence selected from SEQ ID No. 54 or a functional fragment thereof.

Described herein, the proline tolerant tripeptidyl peptidase may comprise an amino acid having at least 70% identity to SEQ ID No. 54 or a functional fragment thereof.

Described herein, the proline tolerant tripeptidyl peptidase may comprise one or more amino acid sequence selected from SEQ ID No. 55 or a functional fragment thereof.

Described herein, the proline tolerant tripeptidyl peptidase may comprise an amino acid having at least 70% identity to SEQ ID No. 55 or a functional fragment thereof.

Described herein, the proline tolerant tripeptidyl peptidase may comprise one or more amino acid sequence selected from SEQ ID No. 98 or a functional fragment thereof.

Described herein, the proline tolerant tripeptidyl peptidase may comprise an amino acid having at least 70% identity to SEQ ID No. 98 or a functional fragment thereof.

Described herein, the proline tolerant tripeptidyl peptidase may comprise one or more amino acid sequence selected from SEQ ID No. 99 or a functional fragment thereof.

Described herein, the proline tolerant tripeptidyl peptidase may comprise an amino acid having at least 70% identity to SEQ ID No. 99 or a functional fragment thereof.

Described herein, the proline tolerant tripeptidyl peptidase may comprise an amino acid having at least 80% identity to SEQ ID No. 29, SEQ ID No. 1, SEQ ID No. 2, SEQ ID No. 3, SEQ ID No. 4, SEQ ID No. 5, SEQ ID No.6, SEQ ID No. 7, SEQ ID No. 8, SEQ ID No. 9, SEQ ID No. 10, SEQ ID No. 11, SEQ ID No. 12, SEQ ID No. 13, SEQ ID No. 14, SEQ ID No. 15, SEQ ID No. 16, SEQ ID No. 17, SEQ ID No. 18, SEQ ID No. 19, SEQ ID No. 20, SEQ ID No. 21, SEQ ID No. 22, SEQ ID No. 23, SEQ ID No. 24, SEQ ID No. 25, SEQ ID No. 26, SEQ ID No. 27, SEQ ID No. 28, SEQ ID No. 30, SEQ ID No. 31, SEQ ID No. 32, SEQ ID No. 33, SEQ ID No. 34, SEQ ID No. 35, SEQ ID No. 36, SEQ ID No. 37, SEQ ID No. 38, SEQ ID No. 39, SEQ ID No. 40, SEQ ID No. 41, SEQ ID No. 42, SEQ ID No. 43, SEQ ID No. 44, SEQ ID No. 45, SEQ ID No. 46, SEQ ID No. 47, SEQ ID No. 48, SEQ ID No. 49, SEQ ID No. 50, SEQ ID No. 51, SEQ ID No. 52, SEQ ID No. 53, SEQ ID No. 54, SEQ ID No. 55, SEQ ID No. 98, SEQ ID No. 99 or a functional fragment thereof.

Described herein, the proline tolerant tripeptidyl peptidase may comprise an amino acid having at least 85% identity to SEQ ID No. 29, SEQ ID No. 1, SEQ ID No. 2, SEQ ID No. 3, SEQ ID No. 4, SEQ ID No. 5, SEQ ID No.6, SEQ ID No. 7, SEQ ID No. 8, SEQ ID No. 9, SEQ ID No. 10, SEQ ID No. 11, SEQ ID No. 12, SEQ ID No. 13, SEQ ID No. 14, SEQ ID No. 15, SEQ ID No. 16, SEQ ID No. 17, SEQ ID No. 18, SEQ ID No. 19, SEQ ID No. 20, SEQ ID No. 21, SEQ ID No. 22, SEQ ID No. 23, SEQ ID No. 24, SEQ ID No. 25, SEQ ID No. 26, SEQ ID No. 27, SEQ ID No. 28, SEQ ID No. 30, SEQ ID No. 31, SEQ ID No. 32, SEQ ID No. 33, SEQ ID No. 34, SEQ ID No. 35, SEQ ID No. 36, SEQ ID No. 37, SEQ ID No. 38, SEQ ID No. 39, SEQ ID No. 40, SEQ ID No. 41, SEQ ID No. 42, SEQ ID No. 43, SEQ ID No. 44, SEQ ID No. 45, SEQ ID No. 46, SEQ ID No. 47, SEQ ID No. 48, SEQ ID No. 49, SEQ ID No. 50, SEQ ID No. 51, SEQ ID No. 52, SEQ ID No. 53, SEQ ID No. 54, SEQ ID No. 55, SEQ ID No. 98, SEQ ID No. 99 or a functional fragment thereof.

Described herein, the proline tolerant tripeptidyl peptidase may comprise an amino acid having at least 90% identity to SEQ ID No. 29, SEQ ID No. 1, SEQ ID No. 2, SEQ ID No. 3, SEQ ID No. 4, SEQ ID No. 5, SEQ ID No.6, SEQ ID No. 7, SEQ ID No. 8, SEQ ID No. 9, SEQ ID No. 10, SEQ ID No. 11, SEQ ID No. 12, SEQ ID No. 13, SEQ ID No. 14, SEQ ID No. 15, SEQ ID No. 16, SEQ ID No. 17, SEQ ID No. 18, SEQ ID No. 19, SEQ ID No. 20, SEQ ID No. 21, SEQ ID No. 22, SEQ ID No. 23, SEQ ID No. 24, SEQ ID No. 25, SEQ ID No. 26, SEQ ID No. 27, SEQ ID No. 28, SEQ ID No. 30, SEQ ID No. 31, SEQ ID No. 32, SEQ ID No 33, SEQ ID No. 34, SEQ ID No. 35, SEQ ID No. 36, SEQ ID No. 37, SEQ ID No. 38, SEQ ID No. 39, SEQ ID No. 40, SEQ ID No. 41, SEQ ID No. 42, SEQ ID No. 43, SEQ ID No. 44, SEQ ID No. 45, SEQ ID No. 46, SEQ ID No. 47, SEQ ID No. 48, SEQ ID No. 49, SEQ ID No. 50, SEQ ID No. 51, SEQ ID No. 52, SEQ ID No. 53, SEQ ID No. 54, SEQ ID No. 55, SEQ ID No. 98, SEQ ID No. 99 or a functional fragment thereof.

Described herein, the proline tolerant tripeptidyl peptidase may comprise an amino acid having at least 95% identity to SEQ ID No. 29, SEQ ID No. 1, SEQ ID No. 2, SEQ ID No. 3, SEQ ID No. 4, SEQ ID No. 5, SEQ ID No.6, SEQ ID No. 7, SEQ ID No. 8, SEQ ID No. 9, SEQ ID No. 10, SEQ ID No. 11, SEQ ID No. 12, SEQ ID No. 13, SEQ ID No. 14, SEQ ID No. 15, SEQ ID No. 16, SEQ ID No. 17, SEQ ID No. 18, SEQ ID No. 19, SEQ ID No. 20, SEQ ID No. 21, SEQ ID No. 22, SEQ ID No. 23, SEQ ID No. 24, SEQ ID No. 25, SEQ ID No. 26, SEQ ID No. 27, SEQ ID No. 28, SEQ ID No. 30, SEQ ID No. 31, SEQ ID No. 32, SEQ ID No. 33, SEQ ID No. 34, SEQ ID No. 35, SEQ ID No. 36, SEQ ID No. 37, SEQ ID No. 38, SEQ ID No. 39, SEQ ID No. 40, SEQ ID No. 41, SEQ ID No. 42, SEQ ID No. 43, SEQ ID No. 44, SEQ ID No. 45, SEQ ID No. 46, SEQ ID No. 47, SEQ ID No. 48, SEQ ID No. 49, SEQ ID No. 50, SEQ ID No. 51, SEQ ID No. 52, SEQ ID No. 53, SEQ ID No. 54, SEQ ID No. 55, SEQ ID No. 98, SEQ ID No. 99 or a functional fragment thereof.

Described herein, the proline tolerant tripeptidyl peptidase may comprise an amino acid having at least 97% identity to SEQ ID No. 29, SEQ ID No. 1, SEQ ID No. 2, SEQ ID No. 3, SEQ ID No. 4, SEQ ID No. 5, SEQ ID No.6, SEQ ID No. 7, SEQ ID No. 8, SEQ ID No. 9, SEQ ID No. 10, SEQ ID No. 11, SEQ ID No. 12, SEQ ID No. 13, SEQ ID No. 14, SEQ ID No. 15, SEQ ID No. 16, SEQ ID No. 17, SEQ ID No. 18, SEQ ID No. 19, SEQ ID No. 20, SEQ ID No. 21, SEQ ID No. 22, SEQ ID No. 23, SEQ ID No. 24, SEQ ID No. 25, SEQ ID No. 26, SEQ ID No. 27, SEQ ID No. 28, SEQ ID No. 30, SEQ ID No. 31, SEQ ID No. 32, SEQ ID No. 33, SEQ ID No. 34, SEQ ID No. 35, SEQ ID No. 36, SEQ ID No. 37, SEQ ID No. 38, SEQ ID No. 39, SEQ ID No. 40, SEQ ID No. 41, SEQ ID No. 42, SEQ ID No. 43, SEQ ID No. 44, SEQ ID No. 45, SEQ ID No. 46, SEQ ID No. 47, SEQ ID No. 48, SEQ ID No. 49, SEQ ID No. 50, SEQ ID No. 51, SEQ ID No. 52, SEQ ID No. 53, SEQ ID No. 54, SEQ ID No. 55, SEQ ID No. 98, SEQ ID No. 99 or a functional fragment thereof.

Described herein, the proline tolerant tripeptidyl peptidase may comprise an amino acid having at least 99% identity to SEQ ID No. 29, SEQ ID No. 1, SEQ ID No. 2, SEQ ID No. 3, SEQ ID No. 4, SEQ ID No. 5, SEQ ID No.6, SEQ ID No. 7, SEQ ID No. 8, SEQ ID No. 9, SEQ ID No. 10, SEQ ID No. 11, SEQ ID No. 12, SEQ ID No. 13, SEQ ID No. 14, SEQ ID No. 15, SEQ ID No. 16, SEQ ID No. 17, SEQ ID No. 18, SEQ ID No. 19, SEQ ID No. 20, SEQ ID No. 21, SEQ ID No. 22, SEQ ID No. 23, SEQ ID No. 24, SEQ ID No. 25, SEQ ID No. 26, SEQ ID No. 27, SEQ ID No. 28, SEQ ID No. 30, SEQ ID No. 31, SEQ ID No. 32, SEQ ID No. 33, SEQ ID No. 34, SEQ ID No. 35, SEQ ID No. 36, SEQ ID No. 37, SEQ ID No. 38, SEQ ID No. 39, SEQ ID No. 40, SEQ ID No. 41, SEQ ID No. 42, SEQ ID No. 43, SEQ ID No. 44, SEQ ID No. 45, SEQ ID No. 46, SEQ ID No. 47, SEQ ID No. 48, SEQ ID No. 49, SEQ ID No. 50, SEQ ID No. 51, SEQ ID No. 52, SEQ ID No. 53, SEQ ID No. 54, SEQ ID No. 55, SEQ ID No. 98, SEQ ID No. 99 or a functional fragment thereof.

Described herein, the proline tolerant tripeptidyl peptidase may comprise an amino acid sequence selected from one more of the group consisting of: SEQ ID No. 29, SEQ ID No. 1, SEQ ID No. 2, SEQ ID No. 3, SEQ ID No. 4, SEQ ID No. 5, SEQ ID No.6, SEQ ID No. 7, SEQ ID No. 8, SEQ ID No. 9, SEQ ID No. 10, SEQ ID No. 11, SEQ ID No. 12, SEQ ID No. 13, SEQ ID No. 14, SEQ ID No. 15, SEQ ID No. 16, SEQ ID No. 17, SEQ ID No. 18, SEQ ID No. 19, SEQ ID No. 20, SEQ ID No. 21, SEQ ID No. 22, SEQ ID No. 23, SEQ ID No. 24, SEQ ID No. 25, SEQ ID No. 26, SEQ ID No. 27, SEQ ID No. 28, SEQ ID No. 30, SEQ ID No. 31, SEQ ID No. 32, SEQ ID No. 33, SEQ ID No. 34, SEQ ID No. 35, SEQ ID No. 36, SEQ ID No. 37, SEQ ID No. 38, SEQ ID No. 39, SEQ ID No. 40, SEQ ID No. 41, SEQ ID No. 42, SEQ ID No. 43, SEQ ID No. 44, SEQ ID No. 45, SEQ ID No. 46, SEQ ID No. 47, SEQ ID No. 48, SEQ ID No. 49, SEQ ID No. 50, SEQ ID No. 51, SEQ ID No. 52, SEQ ID No. 53, SEQ ID No. 54, SEQ ID No. 55, SEQ ID No. 98 and SEQ ID No. 99.

Described herein, the proline tolerant tripeptidyl peptidase may comprise an amino acid sequence selected from one more of the group consisting of: SEQ ID No. 1, SEQ ID No. 2, SEQ ID No. 3, SEQ ID No. 29, SEQ ID No. 30, SEQ ID No. 98, and SEQ ID No. 99 or a sequence having at least 70% identity thereto,suitably a sequence having at least 80% thereto or at least 90% thereto.

Described herein, it may be suitable that the proline tolerant tripeptidyl peptidase may comprise an amino acid sequence selected from the group consisting of SEQ ID No. 3, SEQ ID No. 4, SEQ ID No. 30 and SEQ ID No. 31, or a sequence having at least 70% identity thereto,suitably a sequence having at least 80% thereto or at least 90% thereto.

Described herein, it may be suitable that the proline tolerant tripeptidyl peptidase may comprise an amino acid sequence selected from the group consisting of SEQ ID No. 98 and SEQ ID No. 99, or a sequence having at least 70% identity thereto, suitably a sequence having at least 80% thereto or at least 90% thereto.

Advantageously these particular amino acid sequences may be particularly suited to cleaving peptide and/or protein substrates enriched in lysine, arginine and/or glycine. Particularly where lysine, arginine and/or glycine are present at the P1 position.

Described herein, the proline tolerant tripeptidyl peptidase may comprise an amino acid sequence selected from one more of the group consisting of: SEQ ID No. 1, SEQ ID No. 2, SEQ ID No. 5, SEQ ID No. 6, SEQ ID No. 7, SEQ ID No. 29, SEQ ID No. 32, SEQ ID No. 33 and SEQ ID No. 34, or a sequence having at least 70% identity thereto,suitably a sequence having at least 80% thereto or at least 90% thereto.

Suitably the proline tolerant tripeptidyl peptidase may have the sequence SEQ ID No. 1, SEQ ID No. 2 or SEQ ID No. 29.

The proline tolerant tripeptidyl peptidase may comprise one or more of the sequence motifs selected from the group consisting of: xEANLD, y'Tzx'G and QNFSV.

Suitably, the proline tolerant tripeptidyl peptidase may comprise xEANLD.

x may be one or more amino acid selected from the group consisting of: G, T, S and V.

In another embodiment the proline tolerant tripeptidyl peptidase may comprise y'Tzx'G.

y' may be one or more amino acid selected from the group consisting of: I, L and V.

z may be one or more amino acid selected from the group consisting of: S and T.

x' may be one or more amino acid selected from the group consisting of: I and V.

In another embodiment the proline tolerant tripeptidyl peptidase may comprise the sequence motif QNFSV.

In a further embodiment the proline tolerant tripeptidyl peptidase may comprise the sequence motifs xEANLD and y'Tzx'G or xEANLD and QNFSV.

In a yet further embodiment the proline tolerant tripeptidyl peptidase may comprise the sequence motifs y'Tzx'G and QNFSV.

Suitably the proline tolerant tripeptidyl peptidase may comprise the sequence motifs xEANLD, y'Tzx'G and QNFSV.

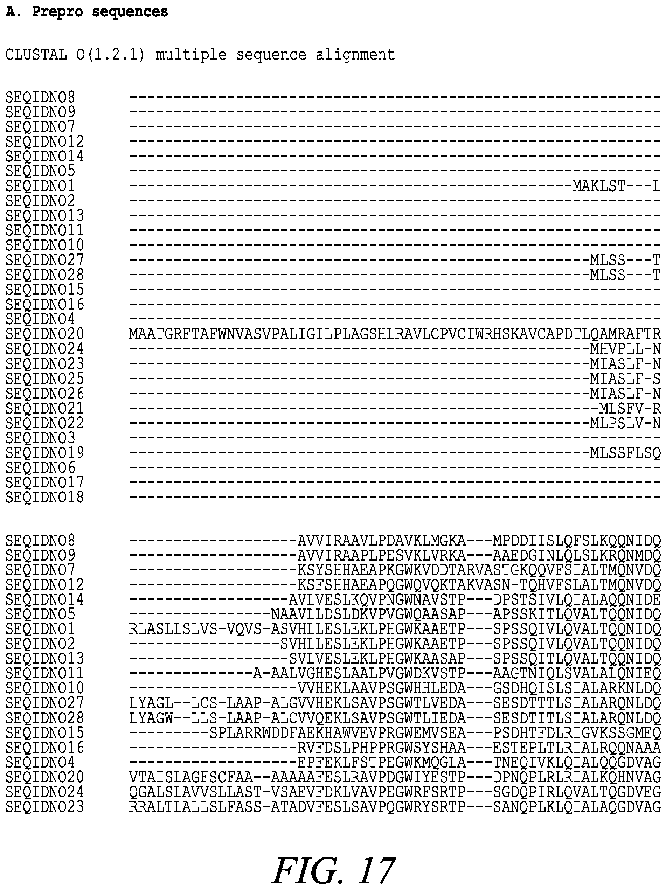

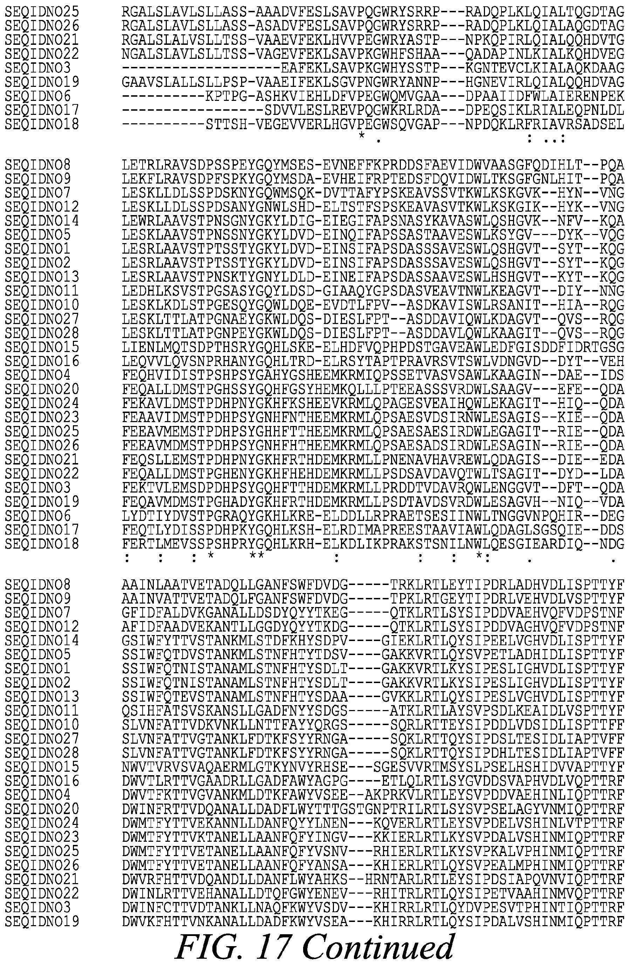

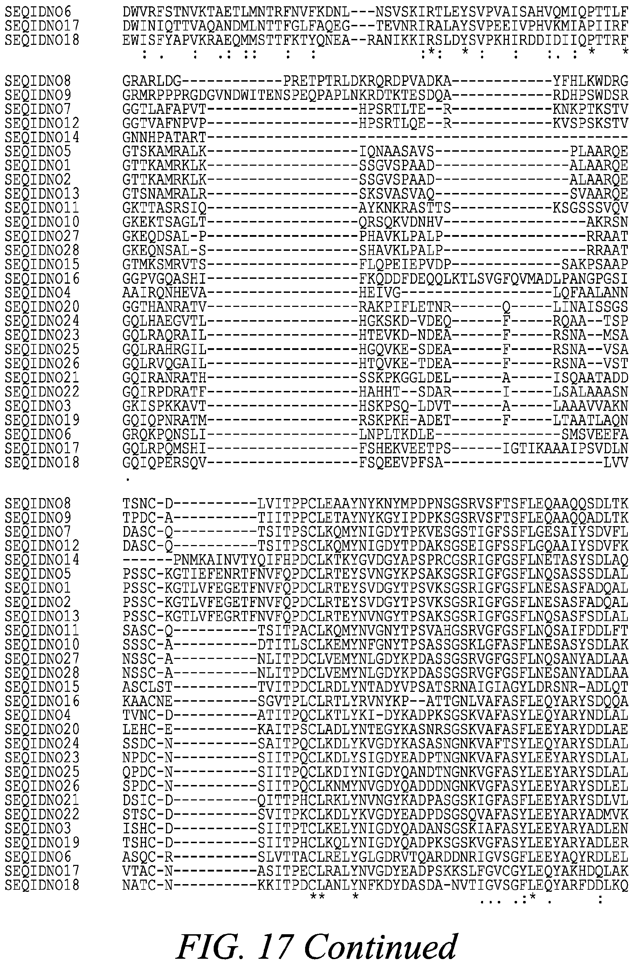

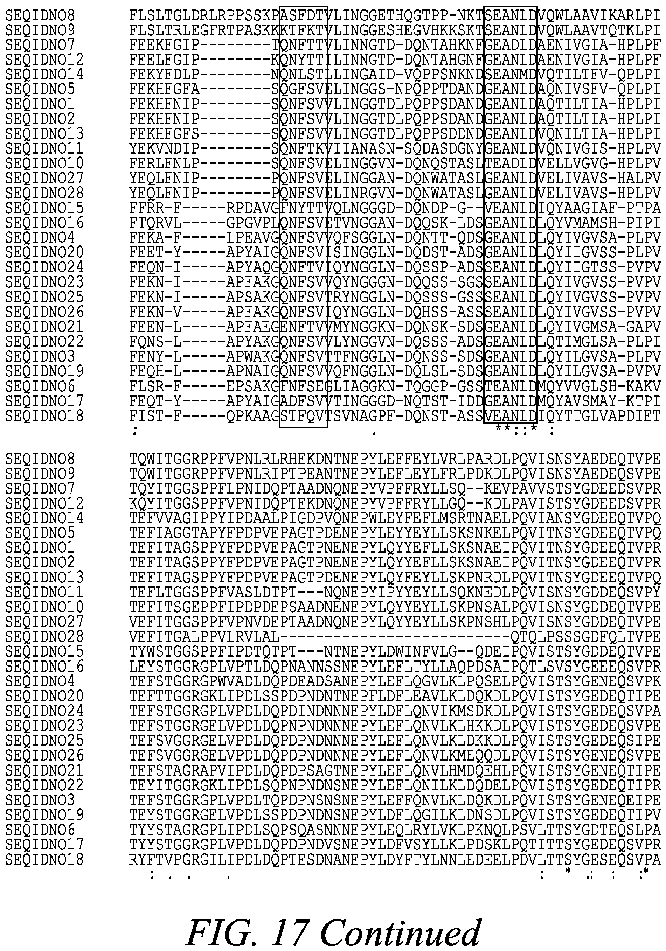

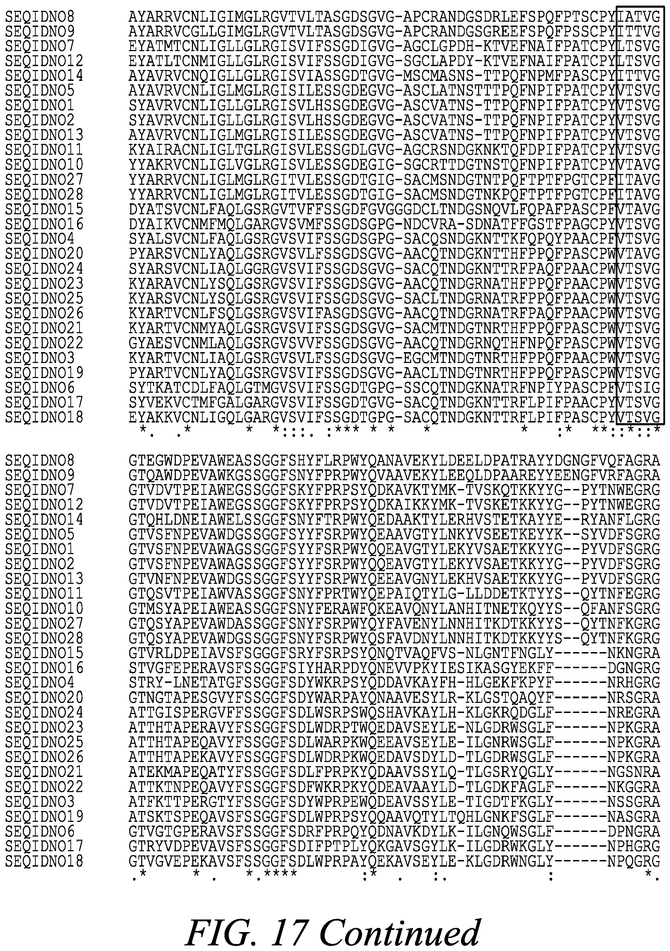

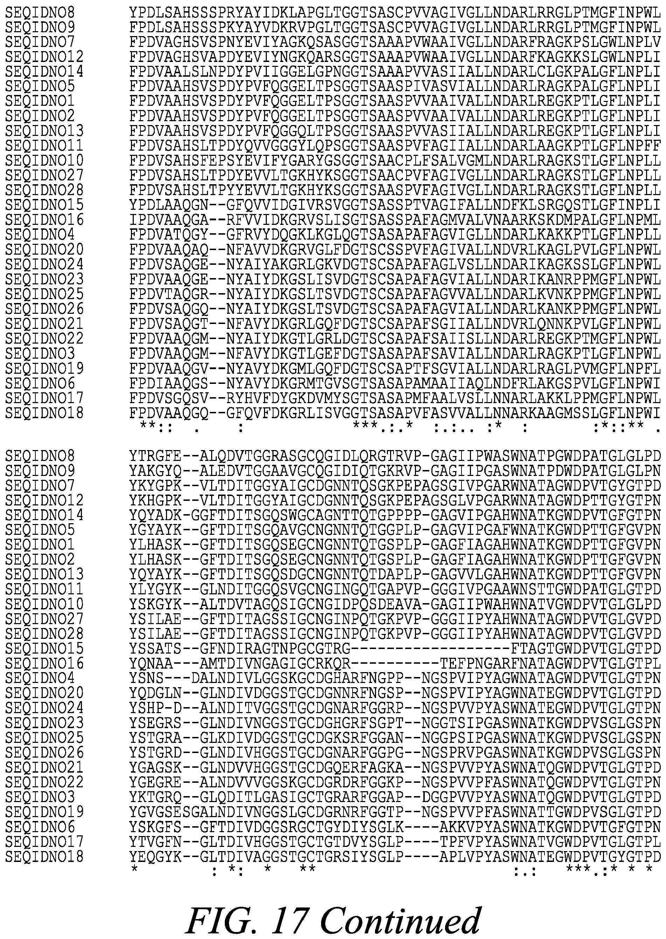

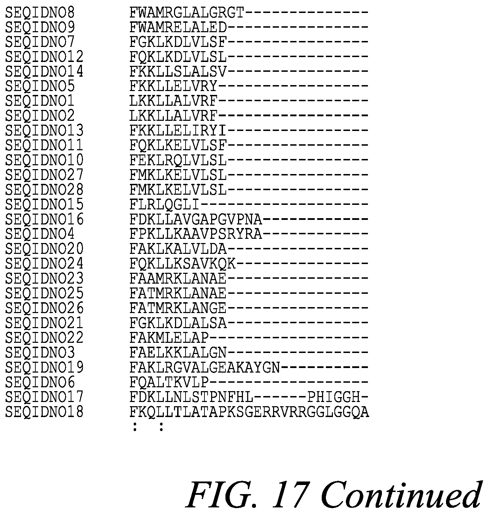

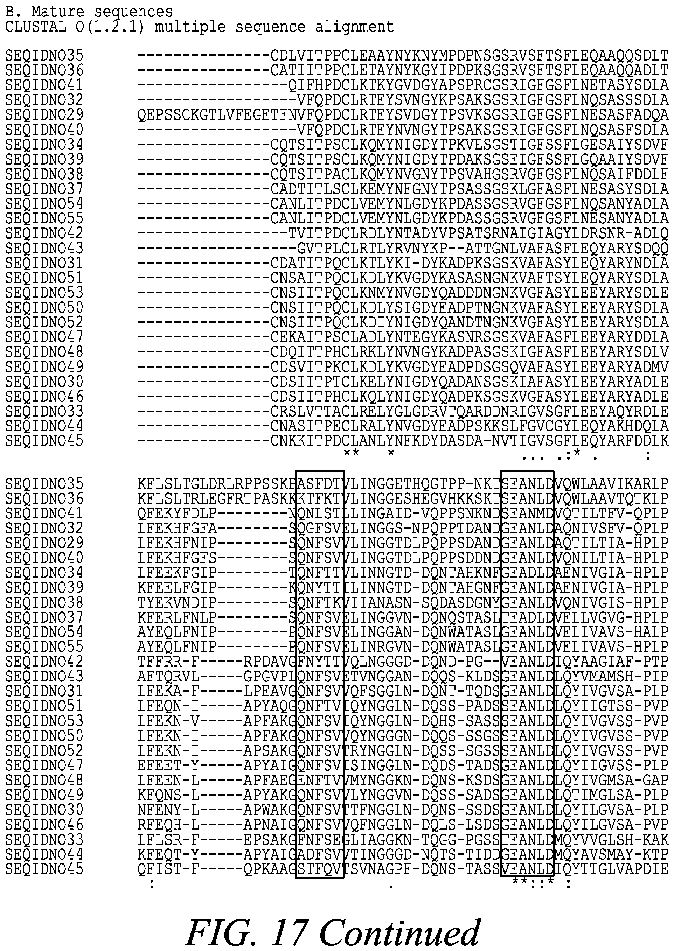

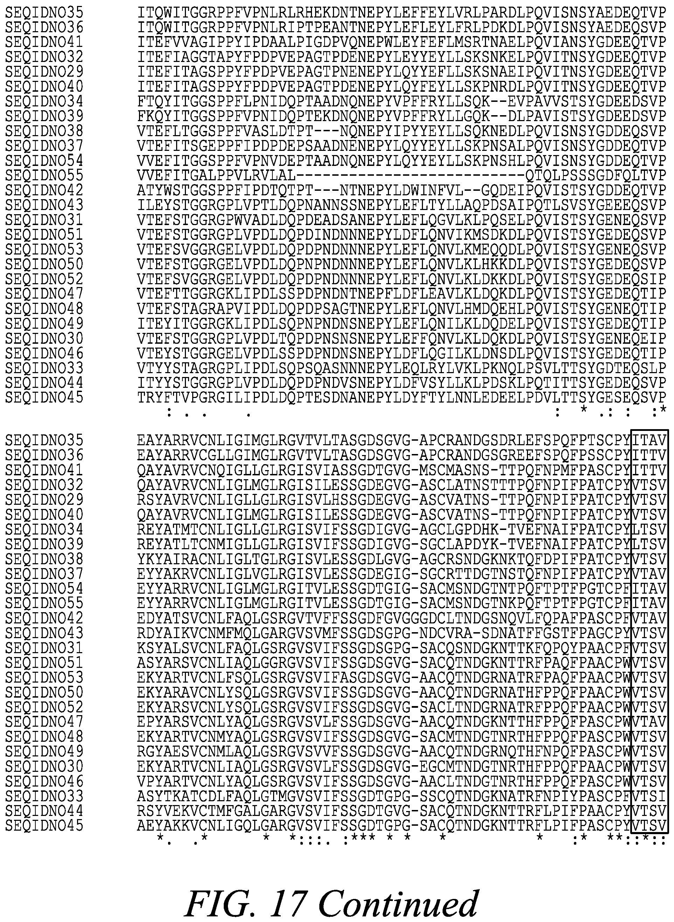

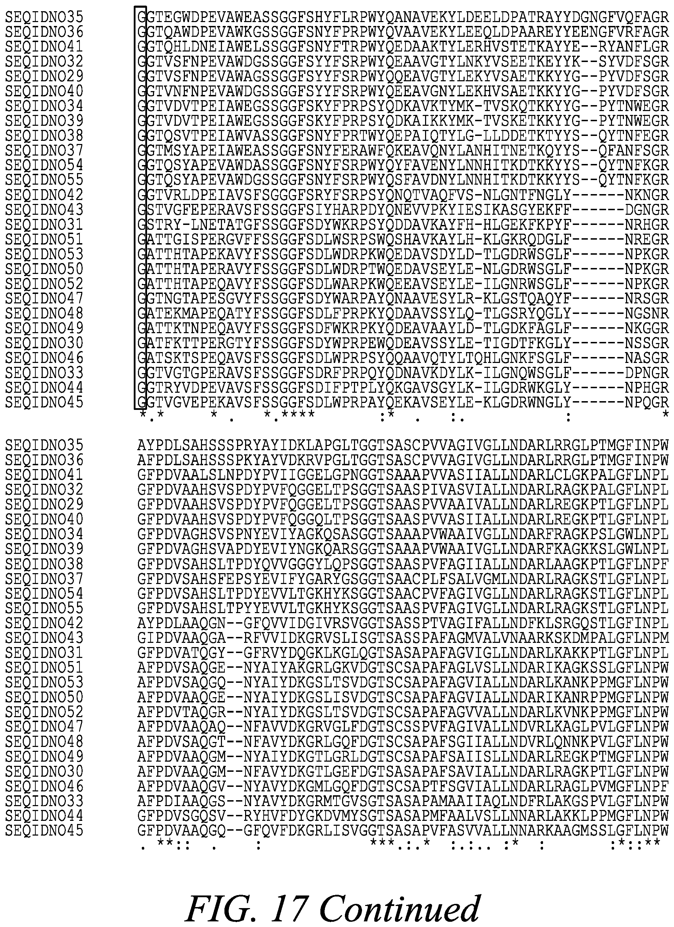

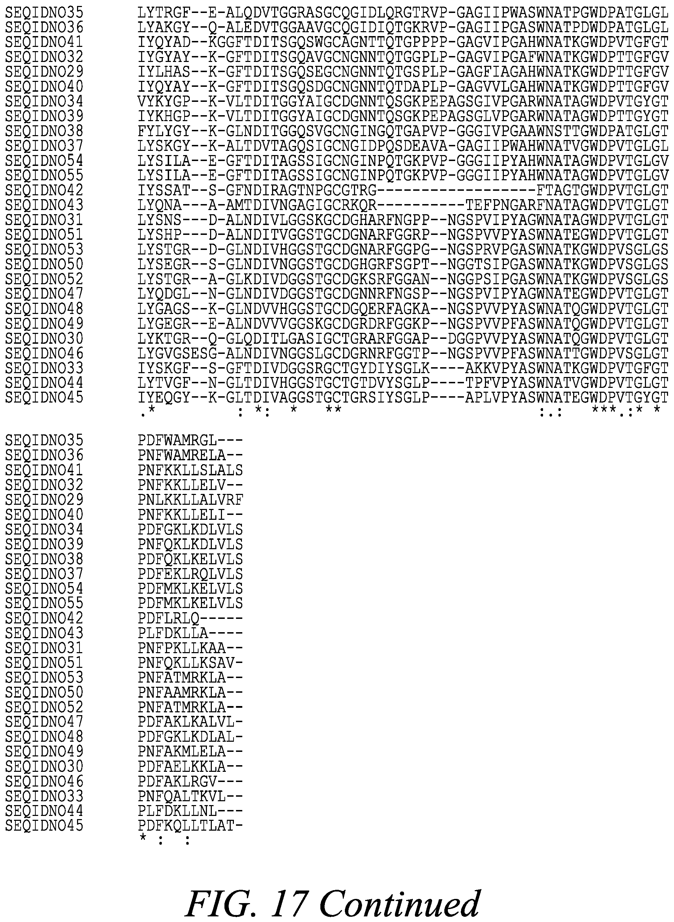

One or more of the motifs are present in the proline tolerant tripeptidyl peptidases for use in the present invention. Figure 17 indicates the positioning of these motifs.

Described herein, the proline tolerant tripeptidyl peptidase may be encoded by a nucleotide sequence shown as SEQ ID No. 56, SEQ ID No. 57, SEQ ID No. 58, SEQ ID No. 59, SEQ ID No. 60, SEQ ID No. 61, SEQ ID No. 62, SEQ ID No. 63, SEQ ID No. 64, SEQ ID No. 65, SEQ ID No. 66, SEQ ID No. 67, SEQ ID No. 68, SEQ ID No. 69, SEQ ID No. 70, SEQ ID No. 71, SEQ ID No. 72, SEQ ID No. 73, SEQ ID No. 74, SEQ ID No. 75, SEQ ID No. 76, SEQ ID No. 77, SEQ ID No. 78, SEQ ID No. 79, SEQ ID No. 80, SEQ ID No. 96, SEQ ID No. 97 or a nucleotide sequence having at least 70% identity thereto, suitably a sequence having at least 80% thereto or at least 90% thereto.

Described herein, the proline tolerant tripeptidyl peptidase may be encoded by a nucleotide sequence having at least 95% sequence identity to SEQ ID No. 56, SEQ ID No. 57, SEQ ID No. 58, SEQ ID No. 59, SEQ ID No. 60, SEQ ID No. 61, SEQ ID No. 62, SEQ ID No. 63, SEQ ID No. 64, SEQ ID No. 65, SEQ ID No. 66, SEQ ID No. 67, SEQ ID No. 68, SEQ ID No. 69, SEQ ID No. 70, SEQ ID No. 71, SEQ ID No. 72, SEQ ID No. 73, SEQ ID No. 74, SEQ ID No. 75, SEQ ID No. 76, SEQ ID No. 77, SEQ ID No. 78, SEQ ID No. 79, SEQ ID No. 80, SEQ ID No. 96 or SEQ ID No. 97, more preferably at least 99% identity to SEQ ID No. 56, SEQ ID No. 57, SEQ ID No. 58, SEQ ID No. 59, SEQ ID No. 60, SEQ ID No. 61, SEQ ID No. 62, SEQ ID No. 63, SEQ ID No. 64, SEQ ID No. 65, SEQ ID No. 66, SEQ ID No. 67, SEQ ID No. 68, SEQ ID No. 69, SEQ ID No. 70, SEQ ID No. 71, SEQ ID No. 72, SEQ ID No. 73, SEQ ID No. 74, SEQ ID No. 75, SEQ ID No. 76, SEQ ID No. 77, SEQ ID No. 78, SEQ ID No. 79, SEQ ID No. 80, SEQ ID No. 96 or SEQ ID No. 97.

Described herein, the proline tolerant tripeptidyl peptidase may be encoded by a nucleotide sequence which hybridises to SEQ ID No. 56, SEQ ID No. 57, SEQ ID No. 58, SEQ ID No. 59, SEQ ID No. 60, SEQ ID No. 61, SEQ ID No. 62, SEQ ID No. 63, SEQ ID No. 64, SEQ ID No. 65, SEQ ID No. 66, SEQ ID No. 67, SEQ ID No. 68, SEQ ID No. 69, SEQ ID No. 70, SEQ ID No. 71, SEQ ID No. 72, SEQ ID No. 73, SEQ ID No. 74, SEQ ID No. 75, SEQ ID No. 76, SEQ ID No. 77, SEQ ID No. 78, SEQ ID No. 79, SEQ ID No. 80, SEQ ID No. 96 or SEQ ID No. 97 under medium stringency conditions. Suitably, a nucleotide sequence which hybridises to SEQ ID No. 56, SEQ ID No. 57, SEQ ID No. 58, SEQ ID No. 59, SEQ ID No. 60, SEQ ID No. 61, SEQ ID No. 62, SEQ ID No. 63, SEQ ID No. 64, SEQ ID No. 65, SEQ ID No. 66, SEQ ID No. 67, SEQ ID No. 68, SEQ ID No. 69, SEQ ID No. 70, SEQ ID No. 71, SEQ ID No. 72, SEQ ID No. 73, SEQ ID No. 74, SEQ ID No. 75, SEQ ID No. 76, SEQ ID No. 77, SEQ ID No. 78, SEQ ID No. 79, SEQ ID No. 80, SEQ ID No. 96 or SEQ ID No. 97 under high stringency conditions.

Described herein, the proline tolerant tripeptidyl peptidase may be encoded by a nucleotide sequence which differs from SEQ ID No. 56, SEQ ID No. 57, SEQ ID No. 58, SEQ ID No. 59, SEQ ID No. 60, SEQ ID No. 61, SEQ ID No. 62, SEQ ID No. 63, SEQ ID No. 64, SEQ ID No. 65, SEQ ID No. 66, SEQ ID No. 67, SEQ ID No. 68, SEQ ID No. 69, SEQ ID No. 70, SEQ ID No. 71, SEQ ID No. 72, SEQ ID No. 73, SEQ ID No. 74, SEQ ID No. 75, SEQ ID No. 76, SEQ ID No. 77, SEQ ID No. 78, SEQ ID No. 79, SEQ ID No. 80, SEQ ID No. 96 or SEQ ID No. 97 due to degeneracy of the genetic code.

Described herein, the nucleotide sequence comprising a nucleotide sequence shown as SEQ ID No. 56, SEQ ID No. 57, SEQ ID No. 58, SEQ ID No. 59, SEQ ID No. 60, SEQ ID No. 61, SEQ ID No. 62, SEQ ID No. 63, SEQ ID No. 64, SEQ ID No. 65, SEQ ID No. 66, SEQ ID No.

**[0324]** 67, SEQ ID No. 68, SEQ ID No. 69, SEQ ID No. 70, SEQ ID No. 71, SEQ ID No. 72, SEQ ID No. 73, SEQ ID No. 74, SEQ ID No. 75, SEQ ID No. 76, SEQ ID No. 77, SEQ ID No. 78, SEQ ID No. 79, SEQ ID No. 80, SEQ ID No. 81, SEQ ID No. 82, SEQ ID No. 83, SEQ ID No. 84, SEQ ID No. 85, SEQ ID No. 86, SEQ ID No. 87, SEQ ID No. 88, SEQ ID No. 89, SEQ ID No. 90, SEQ ID No. 91, SEQ ID No. 92, SEQ ID No. 93, SEQ ID No. 94, SEQ ID No. 95, SEQ ID No. 96 or SEQ ID No. 97 may be a DNA, cDNA, synthetic DNA and/or RNA sequence.

Preferably the sequence is a DNA sequence, more preferably a cDNA sequence coding for the proline tolerant tripeptidyl peptidase for use in the present invention.

In one aspect, preferably the amino acid and/or nucleotide sequence for use in the present invention is in an isolated form. The term "isolated" means that the sequence is at least substantially free from at least one other component with which the sequence is naturally associated in nature and as found in nature. The amino acid and/or nucleotide sequence for use in the present invention may be provided in a form that is substantially free of one or more contaminants with which the substance might otherwise be associated. Thus, for example it may be substantially free of one or more potentially contaminating polypeptides and/or nucleic acid molecules.

In one aspect, preferably the amino acid and/or nucleotide sequence for use in the present invention is in a purified form. The term "purified" means that a given component is present at a high level. The component is desirably the predominant component present in a composition. Preferably, it is present at a level of at least about 90%, or at least about 95% or at least about 98%, said level being determined on a dry weight/dry weight basis with respect to the total composition under consideration.

## ENZYMES

In one embodiment the enzyme for use in the present invention may be a tripeptidyl peptidase comprising SEQ ID No. 1, a functional fragment thereof or a sequence having at least 80% identity to SEQ ID No. 1. Suitably the enzyme may have at least 85%, preferably at least 90%, preferably at least 95%, preferably at least 97%, suitably at least 99% identity to SEQ ID No. 1.

In one embodiment the enzyme for use in the present invention may be a tripeptidyl peptidase comprising SEQ ID No. 2, a functional fragment thereof or a sequence having at least 80% identity to SEQ ID No. 2. Suitably the enzyme may have at least 85%, preferably at least 90%, preferably at least 95%, preferably at least 97%, suitably at least 99% identity to SEQ ID

Described herein, the enzyme may be a tripeptidyl peptidase comprising SEQ ID No. 3, a functional fragment thereof or a sequence having at least 70% identity to SEQ ID No. 3. Suitably the enzyme may have at least 80%, suitably at least 85%, preferably at least 90%, preferably at least 95%, preferably at least 97%, suitably at least 99% identity to SEQ ID No. 3.

Described herein, the enzyme may be a tripeptidyl peptidase comprising SEQ ID No. 4, a functional fragment thereof or a sequence having at least 70% identity to SEQ ID No. 4. Suitably the enzyme may have at least 80%, suitably at least 85%, preferably at least 90%, preferably at least 95%, preferably at least 97%, suitably at least 99% identity to SEQ ID No. 4.

Described herein, the enzyme may be a tripeptidyl peptidase comprising SEQ ID No. 5, a functional fragment thereof or a sequence having at least 70% identity to SEQ ID No. 5. Suitably the enzyme may have at least 80%, suitably at least 85%, preferably at least 90%, preferably at least 95%, preferably at least 97%, suitably at least 99% identity to SEQ ID No. 5.

Described herein, the enzyme may be a tripeptidyl peptidase comprising SEQ ID No. 6, a functional fragment thereof or a sequence having at least 70% identity to SEQ ID No. 6. Suitably the enzyme may have at least 80%, suitably at least 85%, preferably at least 90%, preferably at least 95%, preferably at least 97%, suitably at least 99% identity to SEQ ID No. 6.

Described herein, the enzyme may be a tripeptidyl peptidase comprising SEQ ID No. 7, a functional fragment thereof or a sequence having at least 70% identity to SEQ ID No. 7. Suitably the enzyme may have at least 80%, suitably at least 85%, preferably at least 90%, preferably at least 95%, preferably at least 97%, suitably at least 99% identity to SEQ ID No. 7.

Described herein, the enzyme may be a tripeptidyl peptidase comprising SEQ ID No. 8, a functional fragment thereof or a sequence having at least 70% identity to SEQ ID No. 8. Suitably the enzyme may have at least 80%, suitably at least 85%, preferably at least 90%, preferably at least 95%, preferably at least 97%, suitably at least 99% identity to SEQ ID No. 8.

Described herein, the enzyme may be a tripeptidyl peptidase comprising SEQ ID No. 9, a functional fragment thereof or a sequence having at least 70% identity to SEQ ID No. 9. Suitably the enzyme may have at least 80%, suitably at least 85%, preferably at least 90%, preferably at least 95%, preferably at least 97%, suitably at least 99% identity to SEQ ID No. 9.

Described herein, the enzyme may be a tripeptidyl peptidase comprising SEQ ID No. 10, a functional fragment thereof or a sequence having at least 70% identity to SEQ ID No. 10. Suitably the enzyme may have at least 80%, suitably at least 85%, preferably at least 90%, preferably at least 95%, preferably at least 97%, suitably at least 99% identity to SEQ ID No. 10.

Described herein, the enzyme may be a tripeptidyl peptidase comprising SEQ ID No. 11, a functional fragment thereof or a sequence having at least 70% identity to SEQ ID No. 11. Suitably the enzyme may have at least 80%, suitably at least 85%, preferably at least 90%, preferably at least 95%, preferably at least 97%, suitably at least 99% identity to SEQ ID No. 11.

Described herein, the enzyme may be a tripeptidyl peptidase comprising SEQ ID No. 12, a functional fragment thereof or a sequence having at least 70% identity to SEQ ID No. 12. Suitably the enzyme may have at least 80%, suitably at least 85%, preferably at least 90%, preferably at least 95%, preferably at least 97%, suitably at least 99% identity to SEQ ID No. 12.

Described herein, the enzyme may be a tripeptidyl peptidase comprising SEQ ID No. 13, a functional fragment thereof or a sequence having at least 70% identity to SEQ ID No. 13. Suitably the enzyme may have at least 80%, suitably at least 85%, preferably at least 90%, preferably at least 95%, preferably at least 97%, suitably at least 99% identity to SEQ ID No. 13.

Described herein, the enzyme may be a tripeptidyl peptidase comprising SEQ ID No. 14, a functional fragment thereof or a sequence having at least 70% identity to SEQ ID No. 14. Suitably the enzyme may have at least 80%, suitably at least 85%, preferably at least 90%, preferably at least 95%, preferably at least 97%, suitably at least 99% identity to SEQ ID No. 14.

Described herein, the enzyme may be a tripeptidyl peptidase comprising SEQ ID No. 15, a functional fragment thereof or a sequence having at least 70% identity to SEQ ID No. 15. Suitably the enzyme may have at least 80%, suitably at least 85%, preferably at least 90%, preferably at least 95%, preferably at least 97%, suitably at least 99% identity to SEQ ID No. 15.

Described herein, the enzyme may be a tripeptidyl peptidase comprising SEQ ID No. 16, a functional fragment thereof or a sequence having at least 70% identity to SEQ ID No. 16. Suitably the enzyme may have at least 80%, suitably at least 85%, preferably at least 90%, preferably at least 95%, preferably at least 97%, suitably at least 99% identity to SEQ ID No. 16.

Described herein, the enzyme may be a tripeptidyl peptidase comprising SEQ ID No. 17, a functional fragment thereof or a sequence having at least 70% identity to SEQ ID No. 17. Suitably the enzyme may have at least 80%, suitably at least 85%, preferably at least 90%, preferably at least 95%, preferably at least 97%, suitably at least 99% identity to SEQ ID No. 17.

Described herein, the enzyme may be a tripeptidyl peptidase comprising SEQ ID No. 18, a functional fragment thereof or a sequence having at least 70% identity to SEQ ID No. 18. Suitably the enzyme may have at least 80%, suitably at least 85%, preferably at least 90%, preferably at least 95%, preferably at least 97%, suitably at least 99% identity to SEQ ID No. 18.

Described herein, the enzyme may be a tripeptidyl peptidase comprising SEQ ID No. 19, a functional fragment thereof or a sequence having at least 70% identity to SEQ ID No. 19. Suitably the enzyme may have at least 80%, suitably at least 85%, preferably at least 90%, preferably at least 95%, preferably at least 97%, suitably at least 99% identity to SEQ ID No. 19.

Described herein, the enzyme may be a tripeptidyl peptidase comprising SEQ ID No. 20, a functional fragment thereof or a sequence having at least 70% identity to SEQ ID No. 20. Suitably the enzyme may have at least 80%, suitably at least 85%, preferably at least 90%, preferably at least 95%, preferably at least 97%, suitably at least 99% identity to SEQ ID No. 20.

Described herein, the enzyme may be a tripeptidyl peptidase comprising SEQ ID No. 21, a functional fragment thereof or a sequence having at least 70% identity to SEQ ID No. 21. Suitably the enzyme may have at least 80%, suitably at least 85%, preferably at least 90%, preferably at least 95%, preferably at least 97%, suitably at least 99% identity to SEQ ID No. 21.

Described herein, the enzyme may be a tripeptidyl peptidase comprising SEQ ID No. 22, a functional fragment thereof or a sequence having at least 70% identity to SEQ ID No. 22. Suitably the enzyme may have at least 80%, suitably at least 85%, preferably at least 90%, preferably at least 95%, preferably at least 97%, suitably at least 99% identity to SEQ ID No. 22.

Described herein, the enzyme may be a tripeptidyl peptidase comprising SEQ ID No. 23, a functional fragment thereof or a sequence having at least 70% identity to SEQ ID No. 23. Suitably the enzyme may have at least 80%, suitably at least 85%, preferably at least 90%, preferably at least 95%, preferably at least 97%, suitably at least 99% identity to SEQ ID No. 23.

Described herein, the enzyme may be a tripeptidyl peptidase comprising SEQ ID No. 24, a functional fragment thereof or a sequence having at least 70% identity to SEQ ID No. 24. Suitably the enzyme may have at least 80%, suitably at least 85%, preferably at least 90%, preferably at least 95%, preferably at least 97%, suitably at least 99% identity to SEQ ID No. 24.

Described herein, the enzyme may be a tripeptidyl peptidase comprising SEQ ID No. 25, a functional fragment thereof or a sequence having at least 70% identity to SEQ ID No. 25. Suitably the enzyme may have at least 80%, suitably at least 85%, preferably at least 90%, preferably at least 95%, preferably at least 97%, suitably at least 99% identity to SEQ ID No. 25.

Described herein, the enzyme may be a tripeptidyl peptidase comprising SEQ ID No. 26, a functional fragment thereof or a sequence having at least 70% identity to SEQ ID No. 26. Suitably the enzyme may have at least 80%, suitably at least 85%, preferably at least 90%, preferably at least 95%, preferably at least 97%, suitably at least 99% identity to SEQ ID No. 26.

Described herein, the enzyme may be a tripeptidyl peptidase comprising SEQ ID No. 27, a functional fragment thereof or a sequence having at least 70% identity to SEQ ID No. 27. Suitably the enzyme may have at least 80%, suitably at least 85%, preferably at least 90%, preferably at least 95%, preferably at least 97%, suitably at least 99% identity to SEQ ID No. 27.

Described herein, the enzyme may be a tripeptidyl peptidase comprising SEQ ID No. 28, a functional fragment thereof or a sequence having at least 70% identity to SEQ ID No. 28. Suitably the enzyme may have at least 80%, suitably at least 85%, preferably at least 90%, preferably at least 95%, preferably at least 97%, suitably at least 99% identity to SEQ ID No. 28.

In one embodiment the enzyme for use in the present invention may be a tripeptidyl peptidase comprising SEQ ID No. 29, a functional fragment thereof or a sequence having at least 80% identity to SEQ ID No. 29. Suitably the enzyme may have at least 85%, preferably at least 90%, preferably at least 95%, preferably at least 97%, suitably at least 99% identity to SEQ ID No. 29.

Described herein, the enzyme may be a tripeptidyl peptidase comprising SEQ ID No. 30, a functional fragment thereof or a sequence having at least 70% identity to SEQ ID No. 30. Suitably the enzyme may have at least 80%, suitably at least 85%, preferably at least 90%, preferably at least 95%, preferably at least 97%, suitably at least 99% identity to SEQ ID No. 30.

Described herein, the enzyme may be a tripeptidyl peptidase comprising SEQ ID No. 31, a functional fragment thereof or a sequence having at least 70% identity to SEQ ID No. 31. Suitably the enzyme may have at least 80%, suitably at least 85%, preferably at least 90%, preferably at least 95%, preferably at least 97%, suitably at least 99% identity to SEQ ID No. 31.

Described herein, the enzyme may be a tripeptidyl peptidase comprising SEQ ID No. 32, a functional fragment thereof or a sequence having at least 70% identity to SEQ ID No. 32. Suitably the enzyme may have at least 80%, suitably at least 85%, preferably at least 90%, preferably at least 95%, preferably at least 97%, suitably at least 99% identity to SEQ ID No. 32.

Described herein, the enzyme may be a tripeptidyl peptidase comprising SEQ ID No. 33, a functional fragment thereof or a sequence having at least 70% identity to SEQ ID No. 33. Suitably the enzyme may have at least 80%, suitably at least 85%, preferably at least 90%, preferably at least 95%, preferably at least 97%, suitably at least 99% identity to SEQ ID No. 33.

Described herein, the enzyme may be a tripeptidyl peptidase comprising SEQ ID No. 34, a functional fragment thereof or a sequence having at least 70% identity to SEQ ID No. 34. Suitably the enzyme may have at least 80%, suitably at least 85%, preferably at least 90%, preferably at least 95%, preferably at least 97%, suitably at least 99% identity to SEQ ID No. 34.

Described herein, the enzyme may be a tripeptidyl peptidase comprising SEQ ID No. 35, a functional fragment thereof or a sequence having at least 70% identity to SEQ ID No. 35. Suitably the enzyme may have at least 80%, suitably at least 85%, preferably at least 90%, preferably at least 95%, preferably at least 97%, suitably at least 99% identity to SEQ ID No. 35.

Described herein, the enzyme may be a tripeptidyl peptidase comprising SEQ ID No. 36, a functional fragment thereof or a sequence having at least 70% identity to SEQ ID No. 36. Suitably the enzyme may have at least 80%, suitably at least 85%, preferably at least 90%, preferably at least 95%, preferably at least 97%, suitably at least 99% identity to SEQ ID No. 36.

Described herein, the enzyme may be a tripeptidyl peptidase comprising SEQ ID No. 37, a functional fragment thereof or a sequence having at least 70% identity to SEQ ID No. 37. Suitably the enzyme may have at least 80%, suitably at least 85%, preferably at least 90%, preferably at least 95%, preferably at least 97%, suitably at least 99% identity to SEQ ID No. 37.

Described herein, the enzyme may be a tripeptidyl peptidase comprising SEQ ID No. 38, a functional fragment thereof or a sequence having at least 70% identity to SEQ ID No. 38. Suitably the enzyme may have at least 80%, suitably at least 85%, preferably at least 90%, preferably at least 95%, preferably at least 97%, suitably at least 99% identity to SEQ ID No. 38.

Described herein, the enzyme may be a tripeptidyl peptidase comprising SEQ ID No. 39, a functional fragment thereof or a sequence having at least 70% identity to SEQ ID No. 39. Suitably the enzyme may have at least 80%, suitably at least 85%, preferably at least 90%, preferably at least 95%, preferably at least 97%, suitably at least 99% identity to SEQ ID No. 39.

Described herein, the enzyme may be a tripeptidyl peptidase comprising SEQ ID No. 40, a functional fragment thereof or a sequence having at least 70% identity to SEQ ID No. 40. Suitably the enzyme may have at least 80%, suitably at least 85%, preferably at least 90%, preferably at least 95%, preferably at least 97%, suitably at least 99% identity to SEQ ID No. 40.

Described herein, the enzyme may be a tripeptidyl peptidase comprising SEQ ID No. 41, a functional fragment thereof or a sequence having at least 70% identity to SEQ ID No. 41. Suitably the enzyme may have at least 80%, suitably at least 85%, preferably at least 90%, preferably at least 95%, preferably at least 97%, suitably at least 99% identity to SEQ ID No. 41.

Described herein, the enzyme may be a tripeptidyl peptidase comprising SEQ ID No. 42, a functional fragment thereof or a sequence having at least 70% identity to SEQ ID No. 42. Suitably the enzyme may have at least 80%, suitably at least 85%, preferably at least 90%, preferably at least 95%, preferably at least 97%, suitably at least 99% identity to SEQ ID No. 42.

Described herein, the enzyme may be a tripeptidyl peptidase comprising SEQ ID No. 43, a functional fragment thereof or a sequence having at least 70% identity to SEQ ID No. 43. Suitably the enzyme may have at least 80%, suitably at least 85%, preferably at least 90%, preferably at least 95%, preferably at least 97%, suitably at least 99% identity to SEQ ID No. 43.

Described herein, the enzyme may be a tripeptidyl peptidase comprising SEQ ID No. 44, a functional fragment thereof or a sequence having at least 70% identity to SEQ ID No. 44. Suitably the enzyme may have at least 80%, suitably at least 85%, preferably at least 90%, preferably at least 95%, preferably at least 97%, suitably at least 99% identity to SEQ ID No. 44.

Described herein, the enzyme may be a tripeptidyl peptidase comprising SEQ ID No. 45, a functional fragment thereof or a sequence having at least 70% identity to SEQ ID No. 45. Suitably the enzyme may have at least 80%, suitably at least 85%, preferably at least 90%, preferably at least 95%, preferably at least 97%, suitably at least 99% identity to SEQ ID No. 45.

Described herein, the enzyme may be a tripeptidyl peptidase comprising SEQ ID No. 46, a functional fragment thereof or a sequence having at least 70% identity to SEQ ID No. 46. Suitably the enzyme may have at least 80%, suitably at least 85%, preferably at least 90%, preferably at least 95%, preferably at least 97%, suitably at least 99% identity to SEQ ID No. 46.

Described herein, the enzyme may be a tripeptidyl peptidase comprising SEQ ID No. 47, a functional fragment thereof or a sequence having at least 70% identity to SEQ ID No. 47. Suitably the enzyme may have at least 80%, suitably at least 85%, preferably at least 90%, preferably at least 95%, preferably at least 97%, suitably at least 99% identity to SEQ ID No. 47.

Described herein, the enzyme may be a tripeptidyl peptidase comprising SEQ ID No. 48, a functional fragment thereof or a sequence having at least 70% identity to SEQ ID No. 48. Suitably the enzyme may have at least 80%, suitably at least 85%, preferably at least 90%, preferably at least 95%, preferably at least 97%, suitably at least 99% identity to SEQ ID No. 48.

Described herein, the enzyme may be a tripeptidyl peptidase comprising SEQ ID No. 49, a functional fragment thereof or a sequence having at least 70% identity to SEQ ID No. 49. Suitably the enzyme may have at least 80%, suitably at least 85%, preferably at least 90%, preferably at least 95%, preferably at least 97%, suitably at least 99% identity to SEQ ID No. 49.

Described herein, the enzyme may be a tripeptidyl peptidase comprising SEQ ID No. 50, a functional fragment thereof or a sequence having at least 70% identity to SEQ ID No. 50. Suitably the enzyme may have at least 80%, suitably at least 85%, preferably at least 90%, preferably at least 95%, preferably at least 97%, suitably at least 99% identity to SEQ ID No. 50.

Described herein, the enzyme may be a tripeptidyl peptidase comprising SEQ ID No. 51, a functional fragment thereof or a sequence having at least 70% identity to SEQ ID No. 51. Suitably the enzyme may have at least 80%, suitably at least 85%, preferably at least 90%, preferably at least 95%, preferably at least 97%, suitably at least 99% identity to SEQ ID No. 51.

Described herein, the enzyme may be a tripeptidyl peptidase comprising SEQ ID No. 52, a functional fragment thereof or a sequence having at least 70% identity to SEQ ID No. 52. Suitably the enzyme may have at least 80%, suitably at least 85%, preferably at least 90%, preferably at least 95%, preferably at least 97%, suitably at least 99% identity to SEQ ID No. 52.

Described herein, the enzyme may be a tripeptidyl peptidase comprising SEQ ID No. 53, a functional fragment thereof or a sequence having at least 70% identity to SEQ ID No. 53. Suitably the enzyme may have at least 80%, suitably at least 85%, preferably at least 90%, preferably at least 95%, preferably at least 97%, suitably at least 99% identity to SEQ ID No. 53.

Described herein, the enzyme may be a tripeptidyl peptidase comprising SEQ ID No. 54, a functional fragment thereof or a sequence having at least 70% identity to SEQ ID No. 54. Suitably the enzyme may have at least 80%, suitably at least 85%, preferably at least 90%, preferably at least 95%, preferably at least 97%, suitably at least 99% identity to SEQ ID No. 54.

Described herein, the enzyme may be a tripeptidyl peptidase comprising SEQ ID No. 55, a functional fragment thereof or a sequence having at least 70% identity to SEQ ID No. 55. Suitably the enzyme may have at least 80%, suitably at least 85%, preferably at least 90%, preferably at least 95%, preferably at least 97%, suitably at least 99% identity to SEQ ID No. 55.

Described herein, the enzyme may be a tripeptidyl peptidase comprising SEQ ID No. 98, a functional fragment thereof or a sequence having at least 70% identity to SEQ ID No. 98. Suitably the enzyme may have at least 80%, suitably at least 85%, preferably at least 90%, preferably at least 95%, preferably at least 97%, suitably at least 99% identity to SEQ ID No. 98.

Described herein, the enzyme may be a tripeptidyl peptidase comprising SEQ ID No. 99, a functional fragment thereof or a sequence having at least 70% identity to SEQ ID No. 99. Suitably the enzyme may have at least 80%, suitably at least 85%, preferably at least 90%, preferably at least 95%, preferably at least 97%, suitably at least 99% identity to SEQ ID No. 99.

In one embodiment the enzyme for use in the present invention may be a tripeptidyl peptidase encoded by a nucleotide sequence comprising the sequence shown as SEQ ID No. 56 or a sequence having at least 80% identity thereto. Suitably at least 85%, preferably at least 90%, preferably at least 95%, preferably at least 97%, suitably at least 99% identity thereto.

Described herein, the enzyme may be a tripeptidyl peptidase encoded by a nucleotide sequence comprising the sequence shown as SEQ ID No. 57 or a sequence having at least 70% identity thereto. Suitably at least 80% identity, suitably at least 85%, preferably at least 90%, preferably at least 95%, preferably at least 97%, suitably at least 99% identity thereto.

Described herein, the enzyme may be a tripeptidyl peptidase encoded by a nucleotide sequence comprising the sequence shown as SEQ ID No. 58 or a sequence having at least 70% identity thereto. Suitably at least 80% identity, suitably at least 85%, preferably at least 90%, preferably at least 95%, preferably at least 97%, suitably at least 99% identity thereto.

Described herein, the enzyme may be a tripeptidyl peptidase encoded by a nucleotide sequence comprising the sequence shown as SEQ ID No. 59 or a sequence having at least 70% identity thereto. Suitably at least 80% identity, suitably at least 85%, preferably at least 90%, preferably at least 95%, preferably at least 97%, suitably at least 99% identity thereto.

Described herein, the enzyme may be a tripeptidyl peptidase encoded by a nucleotide sequence comprising the sequence shown as SEQ ID No. 60 or a sequence having at least 70% identity thereto. Suitably at least 80% identity, suitably at least 85%, preferably at least 90%, preferably at least 95%, preferably at least 97%, suitably at least 99% identity thereto.

Described herein, the enzyme may be a tripeptidyl peptidase encoded by a nucleotide sequence comprising the sequence shown as SEQ ID No. 61 or a sequence having at least 70% identity thereto. Suitably at least 80% identity, suitably at least 85%, preferably at least 90%, preferably at least 95%, preferably at least 97%, suitably at least 99% identity thereto.

Described herein, the enzyme may be a tripeptidyl peptidase encoded by a nucleotide sequence comprising the sequence shown as SEQ ID No. 62 or a sequence having at least 70% identity thereto. Suitably at least 80% identity, suitably at least 85%, preferably at least 90%, preferably at least 95%, preferably at least 97%, suitably at least 99% identity thereto.

Described herein, the enzyme may be a tripeptidyl peptidase encoded by a nucleotide sequence comprising the sequence shown as SEQ ID No. 63 or a sequence having at least 70% identity thereto. Suitably at least 80% identity, suitably at least 85%, preferably at least 90%, preferably at least 95%, preferably at least 97%, suitably at least 99% identity thereto.

Described herein, the enzyme may be a tripeptidyl peptidase encoded by a nucleotide sequence comprising the sequence shown as SEQ ID No. 64 or a sequence having at least 70% identity thereto. Suitably at least 80% identity, suitably at least 85%, preferably at least 90%, preferably at least 95%, preferably at least 97%, suitably at least 99% identity thereto.

Described herein, the enzyme may be a tripeptidyl peptidase encoded by a nucleotide sequence comprising the sequence shown as SEQ ID No. 65 or a sequence having at least 70% identity thereto. Suitably at least 80% identity, suitably at least 85%, preferably at least 90%, preferably at least 95%, preferably at least 97%, suitably at least 99% identity thereto.

Described herein, the enzyme may be a tripeptidyl peptidase encoded by a nucleotide sequence comprising the sequence shown as SEQ ID No. 66 or a sequence having at least 70% identity thereto. Suitably at least 80% identity, suitably at least 85%, preferably at least 90%, preferably at least 95%, preferably at least 97%, suitably at least 99% identity thereto.

Described herein, the enzyme may be a tripeptidyl peptidase encoded by a nucleotide sequence comprising the sequence shown as SEQ ID No. 67 or a sequence having at least 70% identity thereto. Suitably at least 80% identity, suitably at least 85%, preferably at least 90%, preferably at least 95%, preferably at least 97%, suitably at least 99% identity thereto.

Described herein, the enzyme may be a tripeptidyl peptidase encoded by a nucleotide sequence comprising the sequence shown as SEQ ID No. 68 or a sequence having at least 70% identity thereto. Suitably at least 80% identity, suitably at least 85%, preferably at least 90%, preferably at least 95%, preferably at least 97%, suitably at least 99% identity thereto.

Described herein, the enzyme may be a tripeptidyl peptidase encoded by a nucleotide sequence comprising the sequence shown as SEQ ID No. 69 or a sequence having at least 70% identity thereto. Suitably at least 80% identity, suitably at least 85%, preferably at least 90%, preferably at least 95%, preferably at least 97%, suitably at least 99% identity thereto.

Described herein, the enzyme may be a tripeptidyl peptidase encoded by a nucleotide sequence comprising the sequence shown as SEQ ID No. 70 or a sequence having at least 70% identity thereto. Suitably at least 80% identity, suitably at least 85%, preferably at least 90%, preferably at least 95%, preferably at least 97%, suitably at least 99% identity thereto.

Described herein, the enzyme may be a tripeptidyl peptidase encoded by a nucleotide sequence comprising the sequence shown as SEQ ID No. 71 or a sequence having at least 70% identity thereto. Suitably at least 80% identity, suitably at least 85%, preferably at least 90%, preferably at least 95%, preferably at least 97%, suitably at least 99% identity thereto.

Described herein, the enzyme may be a tripeptidyl peptidase encoded by a nucleotide sequence comprising the sequence shown as SEQ ID No. 72 or a sequence having at least 70% identity thereto. Suitably at least 80% identity, suitably at least 85%, preferably at least 90%, preferably at least 95%, preferably at least 97%, suitably at least 99% identity thereto.

Described herein, the enzyme may be a tripeptidyl peptidase encoded by a nucleotide sequence comprising the sequence shown as SEQ ID No. 73 or a sequence having at least 70% identity thereto. Suitably at least 80% identity, suitably at least 85%, preferably at least 90%, preferably at least 95%, preferably at least 97%, suitably at least 99% identity thereto.

Described herein, the enzyme may be a tripeptidyl peptidase encoded by a nucleotide sequence comprising the sequence shown as SEQ ID No. 74 or a sequence having at least 70% identity thereto. Suitably at least 80% identity, suitably at least 85%, preferably at least 90%, preferably at least 95%, preferably at least 97%, suitably at least 99% identity thereto.

Described herein, the enzyme may be a tripeptidyl peptidase encoded by a nucleotide sequence comprising the sequence shown as SEQ ID No. 75 or a sequence having at least 70% identity thereto. Suitably at least 80% identity, suitably at least 85%, preferably at least 90%, preferably at least 95%, preferably at least 97%, suitably at least 99% identity thereto.

Described herein, the enzyme may be a tripeptidyl peptidase encoded by a nucleotide sequence comprising the sequence shown as SEQ ID No. 76 or a sequence having at least 70% identity thereto. Suitably at least 80% identity, suitably at least 85%, preferably at least 90%, preferably at least 95%, preferably at least 97%, suitably at least 99% identity thereto.

Described herein, the enzyme may be a tripeptidyl peptidase encoded by a nucleotide sequence comprising the sequence shown as SEQ ID No. 77 or a sequence having at least 70% identity thereto. Suitably at least 80% identity, suitably at least 85%, preferably at least 90%, preferably at least 95%, preferably at least 97%, suitably at least 99% identity thereto.

Described herein, the enzyme may be a tripeptidyl peptidase encoded by a nucleotide sequence comprising the sequence shown as SEQ ID No. 78 or a sequence having at least 70% identity thereto. Suitably at least 80% identity, suitably at least 85%, preferably at least 90%, preferably at least 95%, preferably at least 97%, suitably at least 99% identity thereto.

Described herein, the enzyme may be a tripeptidyl peptidase encoded by a nucleotide sequence comprising the sequence shown as SEQ ID No. 79 or a sequence having at least 70% identity thereto. Suitably at least 80% identity, suitably at least 85%, preferably at least 90%, preferably at least 95%, preferably at least 97%, suitably at least 99% identity thereto.

Described herein, the enzyme may be a tripeptidyl peptidase encoded by a nucleotide sequence comprising the sequence shown as SEQ ID No. 80 or a sequence having at least 70% identity thereto. Suitably at least 80% identity, suitably at least 85%, preferably at least 90%, preferably at least 95%, preferably at least 97%, suitably at least 99% identity thereto.

Described herein, the enzyme may be a tripeptidyl peptidase encoded by a nucleotide sequence comprising the sequence shown as SEQ ID No. 81 or a sequence having at least 70% identity thereto. Suitably at least 80% identity, suitably at least 85%, preferably at least 90%, preferably at least 95%, preferably at least 97%, suitably at least 99% identity thereto.

Described herein, the enzyme may be a tripeptidyl peptidase encoded by a nucleotide sequence comprising the sequence shown as SEQ ID No. 82 or a sequence having at least 70% identity thereto. Suitably at least 80% identity, suitably at least 85%, preferably at least 90%, preferably at least 95%, preferably at least 97%, suitably at least 99% identity thereto.

Described herein, the enzyme may be a tripeptidyl peptidase encoded by a nucleotide sequence comprising the sequence shown as SEQ ID No. 83 or a sequence having at least 70% identity thereto. Suitably at least 80% identity, suitably at least 85%, preferably at least 90%, preferably at least 95%, preferably at least 97%, suitably at least 99% identity thereto.

Described herein, the enzyme may be a tripeptidyl peptidase encoded by a nucleotide sequence comprising the sequence shown as SEQ ID No. 84 or a sequence having at least 70% identity thereto. Suitably at least 80% identity, suitably at least 85%, preferably at least 90%, preferably at least 95%, preferably at least 97%, suitably at least 99% identity thereto.

Described herein, the enzyme may be a tripeptidyl peptidase encoded by a nucleotide sequence comprising the sequence shown as SEQ ID No. 85 or a sequence having at least 70% identity thereto. Suitably at least 80% identity, suitably at least 85%, preferably at least 90%, preferably at least 95%, preferably at least 97%, suitably at least 99% identity thereto.

Described herein, the enzyme may be a tripeptidyl peptidase encoded by a nucleotide sequence comprising the sequence shown as SEQ ID No. 86 or a sequence having at least 70% identity thereto. Suitably at least 80% identity, suitably at least 85%, preferably at least 90%, preferably at least 95%, preferably at least 97%, suitably at least 99% identity thereto.

Described herein, the enzyme may be a tripeptidyl peptidase encoded by a nucleotide sequence comprising the sequence shown as SEQ ID No. 87 or a sequence having at least 70% identity thereto. Suitably at least 80% identity, suitably at least 85%, preferably at least 90%, preferably at least 95%, preferably at least 97%, suitably at least 99% identity thereto.

Described herein, the enzyme may be a tripeptidyl peptidase encoded by a nucleotide sequence comprising the sequence shown as SEQ ID No. 88 or a sequence having at least 70% identity thereto. Suitably at least 80% identity, suitably at least 85%, preferably at least 90%, preferably at least 95%, preferably at least 97%, suitably at least 99% identity thereto.

Described herein, the enzyme may be a tripeptidyl peptidase encoded by a nucleotide sequence comprising the sequence shown as SEQ ID No. 89 or a sequence having at least 70% identity thereto. Suitably at least 80% identity, suitably at least 85%, preferably at least 90%, preferably at least 95%, preferably at least 97%, suitably at least 99% identity thereto.

Described herein, the enzyme may be a tripeptidyl peptidase encoded by a nucleotide sequence comprising the sequence shown as SEQ ID No. 90 or a sequence having at least 70% identity thereto. Suitably at least 80% identity, suitably at least 85%, preferably at least 90%, preferably at least 95%, preferably at least 97%, suitably at least 99% identity thereto.

Described herein, the enzyme may be a tripeptidyl peptidase encoded by a nucleotide sequence comprising the sequence shown as SEQ ID No. 91 or a sequence having at least 70% identity thereto. Suitably at least 80% identity, suitably at least 85%, preferably at least 90%, preferably at least 95%, preferably at least 97%, suitably at least 99% identity thereto.

Described herein, the enzyme may be a tripeptidyl peptidase encoded by a nucleotide sequence comprising the sequence shown as SEQ ID No. 92 or a sequence having at least 70% identity thereto. Suitably at least 80% identity, suitably at least 85%, preferably at least 90%, preferably at least 95%, preferably at least 97%, suitably at least 99% identity thereto.

Described herein, the enzyme may be a tripeptidyl peptidase encoded by a nucleotide sequence comprising the sequence shown as SEQ ID No. 93 or a sequence having at least 70% identity thereto. Suitably at least 80% identity, suitably at least 85%, preferably at least 90%, preferably at least 95%, preferably at least 97%, suitably at least 99% identity thereto.

Described herein, the enzyme may be a tripeptidyl peptidase encoded by a nucleotide sequence comprising the sequence shown as SEQ ID No. 94 or a sequence having at least 70% identity thereto. Suitably at least 80% identity, suitably at least 85%, preferably at least 90%, preferably at least 95%, preferably at least 97%, suitably at least 99% identity thereto.

Described herein, the enzyme may be a tripeptidyl peptidase encoded by a nucleotide sequence comprising the sequence shown as SEQ ID No. 95 or a sequence having at least 70% identity thereto. Suitably at least 80% identity, suitably at least 85%, preferably at least 90%, preferably at least 95%, preferably at least 97%, suitably at least 99% identity thereto.

Described herein, the enzyme may be a tripeptidyl peptidase encoded by a nucleotide sequence comprising the sequence shown as SEQ ID No. 96 or a sequence having at least 70% identity thereto. Suitably at least 80% identity, suitably at least 85%, preferably at least 90%, preferably at least 95%, preferably at least 97%, suitably at least 99% identity thereto.

Described herein, the enzyme may be a tripeptidyl peptidase encoded by a nucleotide sequence comprising the sequence shown as SEQ ID No. 97 or a sequence having at least 70% identity thereto. Suitably at least 80% identity, suitably at least 85%, preferably at least 90%, preferably at least 95%, preferably at least 97%, suitably at least 99% identity thereto.

## NUCLEOTIDE SEQUENCE

Disclosed herein is the use of nucleotide sequences encoding proteins having the specific properties as defined herein.

The term "nucleotide sequence" as used herein refers to an oligonucleotide sequence or polynucleotide sequence, and variant, homologues, fragments and derivatives thereof (such as portions thereof). The nucleotide sequence may be of genomic or synthetic or recombinant origin, which may be double-stranded or single-stranded whether representing the sense or anti-sense strand.

The term "nucleotide sequence" in relation to the present invention includes genomic DNA, cDNA, synthetic DNA, and RNA. Preferably it means DNA, more preferably cDNA sequence coding for the present invention.

Preferably the nucleotide sequence does not include the native nucleotide sequence when in its natural environment and when it is linked to its naturally associated sequence(s) that is/are also in its/their natural environment. For ease of reference, we shall call this preferred embodiment the "non-native nucleotide sequence". In this regard, the term "native nucleotide sequence" means an entire nucleotide sequence that is in its native environment and when operatively linked to an entire promoter with which it is naturally associated, which promoter is also in its native environment. However, the amino acid sequence can be isolated and/or purified post expression of a nucleotide sequence in its native organism. Preferably, however, the amino acid sequence may be expressed by a nucleotide sequence in its native organism but wherein the nucleotide sequence is not under the control of the promoter with which it is naturally associated within that organism.

Typically, the nucleotide sequence is prepared using recombinant DNA techniques (i.e. recombinant DNA). However, in an alternative embodiment of the invention, the nucleotide sequence could be synthesised, in whole or in part, using chemical methods well known in the art (see Caruthers MH et al., (1980) Nuc Acids Res Symp Ser 215-23 and Horn T et al., (1980) Nuc Acids Res Symp Ser 225-232).

## PREPARATION OF THE NUCLEOTIDE SEQUENCE

A nucleotide sequence encoding either a protein which has the specific properties as defined herein or a protein which is suitable for modification may be identified and/or isolated and/or purified from any cell or organism producing said protein. Various methods are well known within the art for the identification and/or isolation and/or purification of nucleotide sequences. By way of example, PCR amplification techniques to prepare more of a sequence may be used once a suitable sequence has been identified and/or isolated and/or purified.

By way of further example, a genomic DNA and/or cDNA library may be constructed using chromosomal DNA or messenger RNA from the organism producing the enzyme. If the amino acid sequence of the enzyme is known, labelled oligonucleotide probes may be synthesised and used to identify enzyme-encoding clones from the genomic library prepared from the organism. Alternatively, a labelled oligonucleotide probe containing sequences homologous to another known enzyme gene could be used to identify enzyme-encoding clones. In the latter case, hybridisation and washing conditions of lower stringency are used. Alternatively, enzyme-encoding clones could be identified by inserting fragments of genomic DNA into an expression vector, such as a plasmid, transforming enzyme-negative bacteria with the resulting genomic DNA library, and then plating the transformed bacteria onto agar plates containing a substrate for enzyme (i.e. maltose), thereby allowing clones expressing the enzyme to be identified.

In a yet further alternative, the nucleotide sequence encoding the enzyme may be prepared synthetically by established standard methods, e.g. the phosphoroamidite method described by Beucage S.L. et al, (1981) Tetrahedron Letters 22, p 1859-1869, or the method described by Matthes et al., (1984) EMBO J. 3, p 801-805. In the phosphoroamidite method, oligonucleotides are synthesised, e.g. in an automatic DNA synthesiser, purified, annealed, ligated and cloned in appropriate vectors.

The nucleotide sequence may be of mixed genomic and synthetic origin, mixed synthetic and cDNA origin, or mixed genomic and cDNA origin, prepared by ligating fragments of synthetic, genomic or cDNA origin (as appropriate) in accordance with standard techniques. Each ligated fragment corresponds to various parts of the entire nucleotide sequence. The DNA sequence may also be prepared by polymerase chain reaction (PCR) using specific primers, for instance as described in US 4,683,202 or in Saiki R K et al., (Science (1988) 239, pp 487-491).

## AMINO ACID SEQUENCES

Also disclosed herein is the use of amino acid sequences of enzymes having the specific properties as defined herein.

As used herein, the term "amino acid sequence" is synonymous with the term "polypeptide" and/or the term "protein". In some instances, the term "amino acid sequence" is synonymous with the term "peptide". In some instances, the term "amino acid sequence" is synonymous with the term "enzyme".

The amino acid sequence may be prepared/isolated from a suitable source, or it may be made synthetically or it may be prepared by use of recombinant DNA techniques.

The protein may be used in conjunction with other proteins, particularly enzymes. Thus the present disclosure also covers a combination of proteins wherein the combination comprises the protein/enzyme of the present disclosure and another protein/enzyme, which may be another protein/enzyme according to the present disclosure. This aspect is discussed in a later section. Preferably the amino acid sequence is not a native enzyme. In this regard, the term "native enzyme" means an entire enzyme that is in its native environment and when it has been expressed by its native nucleotide sequence.

## ISOLATED

In one aspect, preferably the amino acid sequence, or nucleic acid, or enzyme for use according to the present invention is in an isolated form. The term "isolated" means that the sequence or enzyme or nucleic acid is at least substantially free from at least one other component with which the sequence, enzyme or nucleic acid is naturally associated in nature and as found in nature. The sequence, enzyme or nucleic acid for use according to the present invention may be provided in a form that is substantially free of one or more contaminants with which the substance might otherwise be associated. Thus, for example it may be substantially free of one or more potentially contaminating polypeptides and/or nucleic acid molecules.

## PURIFIED

In one aspect, preferably the sequence, enzyme or nucleic acid for use according to the present invention is in a purified form. The term "purified" means that the given component is present at a high level. The component is desirably the predominant component present in a composition. Preferably, it is present at a level of at least about 80% said level being determined on a dry weight/dry weight basis with respect to the total composition under consideration. Suitably it may be present at a level of at least about 90%, or at least about 95, or at least about 98% said level being determined on a dry weight/dry weight basis with respect to the total composition under consideration.

## SEQUENCE IDENTITY OR SEQUENCE HOMOLOGY

The present disclosure also encompasses the use of sequences having a degree of sequence identity or sequence homology with amino acid sequence(s) of a polypeptide having the specific properties defined herein or of any nucleotide sequence encoding such a polypeptide (hereinafter referred to as a "homologous sequence(s)"). Here, the term "homologue" means an entity having a certain homology with the subject amino acid sequences and the subject nucleotide sequences. Here, the term "homology" can be equated with "identity".

The homologous amino acid sequence and/or nucleotide sequence should provide and/or encode a polypeptide which retains the functional activity and/or enhances the activity of the enzyme.

In the present context, a homologous sequence is taken to include an amino acid or a nucleotide sequence which may be at least 85 or 90% identical, preferably at least 95 or 98% identical to the subject sequence. Typically, the homologues will comprise the same active sites etc. as the subject amino acid sequence for instance. Although homology can also be considered in terms of similarity (i.e. amino acid residues having similar chemical properties/functions), in the context of the present invention it is preferred to express homology in terms of sequence identity.

In one embodiment, a homologous sequence is taken to include an amino acid sequence or nucleotide sequence which has one or several additions, deletions and/or substitutions compared with the subject sequence.

In one aspect the present disclosure relates to a protein whose amino acid sequence is represented herein or a protein derived from this (parent) protein by substitution, deletion or addition of one or several amino acids, such as 2, 3, 4, 5, 6, 7, 8, 9 amino acids, or more amino acids, such as 10 or more than 10 amino acids in the amino acid sequence of the parent protein and having the activity of the parent protein.

Suitably, the degree of identity with regard to an amino acid sequence is determined over at least 20 contiguous amino acids, preferably over at least 30 contiguous amino acids, preferably over at least 40 contiguous amino acids, preferably over at least 50 contiguous amino acids, preferably over at least 60 contiguous amino acids, preferably over at least 100 contiguous amino acids, preferably over at least 200 contiguous amino acids.

In one aspect the present disclosure relates to a nucleic acid sequence (or gene) encoding a protein whose amino acid sequence is represented herein or encoding a protein derived from this (parent) protein by substitution, deletion or addition of one or several amino acids, such as 2, 3, 4, 5, 6, 7, 8, 9 amino acids, or more amino acids, such as 10 or more than 10 amino acids in the amino acid sequence of the parent protein and having the activity of the parent protein.

In the present context, a homologous sequence is taken to include a nucleotide sequence which may be at least 85 or 90% identical, preferably at least 95 or 98% identical to a nucleotide sequence encoding a polypeptide of the present disclosure (the subject sequence). Typically, the homologues will comprise the same sequences that code for the active sites etc. as the subject sequence. Although homology can also be considered in terms of similarity (i.e. amino acid residues having similar chemical properties/functions), in the context of the present invention it is preferred to express homology in terms of sequence identity.

Homology comparisons can be conducted by eye, or more usually, with the aid of readily available sequence comparison programs. These commercially available computer programs can calculate % homology between two or more sequences.

% homology may be calculated over contiguous sequences, i.e. one sequence is aligned with the other sequence and each amino acid in one sequence is directly compared with the corresponding amino acid in the other sequence, one residue at a time. This is called an "ungapped" alignment. Typically, such ungapped alignments are performed only over a relatively short number of residues.

Although this is a very simple and consistent method, it fails to take into consideration that, for example, in an otherwise identical pair of sequences, one insertion or deletion will cause the following amino acid residues to be put out of alignment, thus potentially resulting in a large reduction in % homology when a global alignment is performed. Consequently, most sequence comparison methods are designed to produce optimal alignments that take into consideration possible insertions and deletions without penalising unduly the overall homology score. This is achieved by inserting "gaps" in the sequence alignment to try to maximise local homology.

However, these more complex methods assign "gap penalties" to each gap that occurs in the alignment so that, for the same number of identical amino acids, a sequence alignment with as few gaps as possible - reflecting higher relatedness between the two compared sequences - will achieve a higher score than one with many gaps. "Affine gap costs" are typically used that charge a relatively high cost for the existence of a gap and a smaller penalty for each subsequent residue in the gap. This is the most commonly used gap scoring system. High gap penalties will of course produce optimised alignments with fewer gaps. Most alignment programs allow the gap penalties to be modified. However, it is preferred to use the default values when using such software for sequence comparisons. Calculation of maximum % homology or % identity therefore firstly requires the production of an optimal alignment, taking into consideration gap penalties. A suitable computer program for carrying out such an alignment is the Vector NTI (Invitrogen Corp.). Examples of software that can perform sequence comparisons include, but are not limited to, the BLAST package (see Ausubel et al 1999 Short Protocols in Molecular Biology, 4th Ed - Chapter 18), BLAST 2 (see FEMS Microbiol Lett 1999 174(2): 247-50; FEMS Microbiol Lett 1999 177(1): 187-8 and tatiana@ncbi.nlm.nih.gov), FASTA (Altschul et al 1990 J. Mol. Biol. 403-410) and AlignX for example. At least BLAST, BLAST 2 and FASTA are available for offline and online searching (see Ausubel et al 1999, pages 7-58 to 7-60), such as for example in the GenomeQuest search tool (www.genomequest.com).

Although the final % homology can be measured in terms of identity, the alignment process itself is typically not based on an all-or-nothing pair comparison. Instead, a scaled similarity score matrix is generally used that assigns scores to each pairwise comparison based on chemical similarity or evolutionary distance. An example of such a matrix commonly used is the BLOSUM62 matrix - the default matrix for the BLAST suite of programs. Vector NTI programs generally use either the public default values or a custom symbol comparison table if supplied (see user manual for further details). For some applications, it is preferred to use the default values for the Vector NTI package.

Alternatively, percentage homologies may be calculated using the multiple alignment feature in Vector NTI (Invitrogen Corp.), based on an algorithm, analogous to CLUSTAL (Higgins DG & Sharp PM (1988), Gene 73(1), 237-244).

Once the software has produced an optimal alignment, it is possible to calculate % homology, preferably % sequence identity. The software typically does this as part of the sequence comparison and generates a numerical result.

Should Gap Penalties be used when determining sequence identity, then preferably the following parameters are used for pairwise alignment:

| FOR BLAST | |
|---|---|
| GAP OPEN | 9 |
| GAP EXTENSION | 2 |

| FOR CLUSTAL | DNA | PROTEIN |
|---|---|---|
| Weight Matrix | IUB | Gonnet 250 |
| GAP OPENING | 15 | 10 |
| GAP EXTEND | 6.66 | 0.1 |

In one embodiment, CLUSTAL may be used with the gap penalty and gap extension set as defined above.

Suitably, the degree of identity with regard to a nucleotide sequence is determined over at least 20 contiguous nucleotides, preferably over at least 30 contiguous nucleotides, preferably over at least 40 contiguous nucleotides, preferably over at least 50 contiguous nucleotides, preferably over at least 60 contiguous nucleotides, preferably over at least 100 contiguous nucleotides.

Suitably, the degree of identity with regard to a nucleotide sequence is determined over at least 100 contiguous nucleotides, preferably over at least 200 contiguous nucleotides, preferably over at least 300 contiguous nucleotides, preferably over at least 400 contiguous nucleotides, preferably over at least 500 contiguous nucleotides, preferably over at least 600 contiguous nucleotides, preferably over at least 700 contiguous nucleotides, preferably over at least 800 contiguous nucleotides.

Suitably, the degree of identity with regard to a nucleotide sequence may be determined over the whole sequence.

Suitably, the degree of identity with regard to a protein (amino acid) sequence is determined over at least 100 contiguous amino acids, preferably over at least 200 contiguous amino acids, preferably over at least 300 contiguous amino acids.

Suitably, the degree of identity with regard to an amino acid or protein sequence may be determined over the whole sequence taught herein.

In the present context, the term "query sequence" means a homologous sequence or a foreign sequence, which is aligned with a subject sequence in order to see if it falls within the scope of the present invention. Accordingly, such query sequence can for example be a prior art sequence or a third party sequence.

In one preferred embodiment, the sequences are aligned by a global alignment program and the sequence identity is calculated by identifying the number of exact matches identified by the program divided by the length of the subject sequence.

In one embodiment, the degree of sequence identity between a query sequence and a subject sequence is determined by 1) aligning the two sequences by any suitable alignment program using the default scoring matrix and default gap penalty, 2) identifying the number of exact matches, where an exact match is where the alignment program has identified an identical amino acid or nucleotide in the two aligned sequences on a given position in the alignment and 3) dividing the number of exact matches with the length of the subject sequence.

In yet a further preferred embodiment, the global alignment program is selected from the group consisting of CLUSTAL and BLAST (preferably BLAST) and the sequence identity is calculated by identifying the number of exact matches identified by the program divided by the length of the subject sequence.

The sequences may also have deletions, insertions or substitutions of amino acid residues which produce a silent change and result in a functionally equivalent substance. Deliberate amino acid substitutions may be made on the basis of similarity in polarity, charge, solubility, hydrophobicity, hydrophilicity, and/or the amphipathic nature of the residues as long as the secondary binding activity of the substance is retained. For example, negatively charged amino acids include aspartic acid and glutamic acid; positively charged amino acids include lysine and arginine; and amino acids with uncharged polar head groups having similar hydrophilicity values include leucine, isoleucine, valine, glycine, alanine, asparagine, glutamine, serine, threonine, phenylalanine, and tyrosine.

Conservative substitutions may be made, for example according to the Table below. Amino acids in the same block in the second column and preferably in the same line in the third column may be substituted for each other:

| ALIPHATIC | Non-polar | GAP |
| | | I L V |
| | Polar - uncharged | C S T M |
| | | N Q |
| | Polar - charged | D E |
| | | K R |
| AROMATIC | | H F W Y |

The present disclosure also encompasses homologous substitution (substitution and replacement are both used herein to mean the interchange of an existing amino acid residue, with an alternative residue) that may occur i.e. like-for-like substitution such as basic for basic, acidic for acidic, polar for polar etc. Non-homologous substitution may also occur i.e. from one class of residue to another or alternatively involving the inclusion of unnatural amino acids such as ornithine (hereinafter referred to as Z), diaminobutyric acid ornithine (hereinafter referred to as B), norleucine ornithine (hereinafter referred to as O), pyriylalanine, thienylalanine, naphthylalanine and phenylglycine.

Replacements may also be made by synthetic amino acids (e.g. unnatural amino acids) include; alpha* and alpha-disubstituted* amino acids, N-alkyl amino acids*, lactic acid*, halide derivatives of natural amino acids such as trifluorotyrosine*, p-Cl-phenylalanine*, p-Br-phenylalanine*, p-I-phenylalanine*, L-allyl-glycine*, β-alanine*, L-α-amino butyric acid*, L-γ-amino butyric acid*, L-α-amino isobutyric acid*, L-ε-amino caproic acid#, 7-amino heptanoic acid*, L-methionine sulfone#*, L-norleucine*, L-norvaline*, p-nitro-L-phenylalanine*, L-hydroxyproline#, L-thioproline*, methyl derivatives of phenylalanine (Phe) such as 4-methyl-Phe*, pentamethyl-Phe*, L-Phe (4-amino)#, L-Tyr (methyl)*, L-Phe (4-isopropyl)*, L-Tic (1,2,3,4-tetrahydroisoquinoline-3-carboxyl acid)*, L-diaminopropionic acid # and L-Phe (4-benzyl)*. The notation * has been utilised for the purpose of the discussion above (relating to homologous or non-homologous substitution), to indicate the hydrophobic nature of the derivative whereas # has been utilised to indicate the hydrophilic nature of the derivative, #* indicates amphipathic characteristics.

Variant amino acid sequences may include suitable spacer groups that may be inserted between any two amino acid residues of the sequence including alkyl groups such as methyl, ethyl or propyl groups in addition to amino acid spacers such as glycine or β-alanine residues. A further form of variation, involves the presence of one or more amino acid residues in peptoid form, will be well understood by those skilled in the art. For the avoidance of doubt, "the peptoid form" is used to refer to variant amino acid residues wherein the α-carbon substituent group is on the residue's nitrogen atom rather than the α-carbon. Processes for preparing peptides in the peptoid form are known in the art, for example Simon RJ et al., PNAS (1992) 89(20), 9367-9371 and Horwell DC, Trends Biotechnol. (1995) 13(4), 132-134.

The nucleotide sequences may include within them synthetic or modified nucleotides. A number of different types of modification to oligonucleotides are known in the art. These include methylphosphonate and phosphorothioate backbones and/or the addition of acridine or polylysine chains at the 3' and/or 5' ends of the molecule. It is to be understood that the nucleotide sequences described herein may be modified by any method available in the art. Such modifications may be carried out in order to enhance the *in vivo* activity or life span of nucleotide sequences for use in the present disclosure. The present disclosure also encompasses the use of nucleotide sequences that are complementary to the sequences presented herein, or any derivative, fragment or derivative thereof. If the sequence is complementary to a fragment thereof then that sequence can be used as a probe to identify similar coding sequences in other organisms etc.

Polynucleotides which are not 100% homologous to the sequences of the present disclosure can be obtained in a number of ways. Other variants of the sequences described herein may be obtained for example by probing DNA libraries made from a range of individuals, for example individuals from different populations. In addition, other homologues may be obtained and such homologues and fragments thereof in general will be capable of selectively hybridising to the sequences shown in the sequence listing herein. Such sequences may be obtained by probing cDNA libraries made from or genomic DNA libraries from other animal species, and probing such libraries with probes comprising all or part of any one of the sequences in the attached sequence listings under conditions of medium to high stringency. Similar considerations apply to obtaining species homologues and allelic variants of the polypeptide or nucleotide sequences of the disclosure.

Variants and strain/species homologues may also be obtained using degenerate PCR which will use primers designed to target sequences within the variants and homologues encoding conserved amino acid sequences within the sequences of the present disclosure. Conserved sequences can be predicted, for example, by aligning the amino acid sequences from several variants/homologues. Sequence alignments can be performed using computer software known in the art. For example the GCG Wisconsin PileUp program is widely used.

The primers used in degenerate PCR will contain one or more degenerate positions and will be used at stringency conditions lower than those used for cloning sequences with single sequence primers against known sequences.

Alternatively, such polynucleotides may be obtained by site directed mutagenesis of characterised sequences. This may be useful where for example silent codon sequence changes are required to optimise codon preferences for a particular host cell in which the polynucleotide sequences are being expressed. Other sequence changes may be desired in order to introduce restriction enzyme recognition sites, or to alter the property or function of the polypeptides encoded by the polynucleotides.

Polynucleotides (nucleotide sequences) for use according to the invention may be used to produce a primer, e.g. a PCR primer, a primer for an alternative amplification reaction, a probe e.g. labelled with a revealing label by conventional means using radioactive or nonradioactive labels, or the polynucleotides may be cloned into vectors. Such primers, probes and other fragments will be at least 15, preferably at least 20, for example at least 25, 30 or 40 nucleotides in length, and are also encompassed by the term polynucleotides as used herein.

Polynucleotides such as DNA polynucleotides and probes may be produced recombinantly, synthetically, or by any means available to those of skill in the art. They may also be cloned by standard techniques.

In general, primers will be produced by synthetic means, involving a stepwise manufacture of the desired nucleic acid sequence one nucleotide at a time. Techniques for accomplishing this using automated techniques are readily available in the art.

Longer polynucleotides will generally be produced using recombinant means, for example using a PCR (polymerase chain reaction) cloning techniques. The primers may be designed to contain suitable restriction enzyme recognition sites so that the amplified DNA can be cloned into a suitable cloning vector.

## HYBRIDISATION

The present disclosure also encompasses the use of sequences that are complementary to the nucleic acid sequences as described herein or sequences that are capable of hybridising either to the sequences as described herein or to sequences that are complementary thereto.

The term "hybridisation" as used herein shall include "the process by which a strand of nucleic acid joins with a complementary strand through base pairing" as well as the process of amplification as carried out in polymerase chain reaction (PCR) technologies.

The present disclosure also encompasses the use of nucleotide sequences that are capable of hybridising to the sequences that are complementary to the sequences presented herein, or any derivative, fragment or derivative thereof.

The term "variant" also encompasses sequences that are complementary to sequences that are capable of hybridising to the nucleotide sequences presented herein.

Preferably, the term "variant" encompasses sequences that are complementary to sequences that are capable of hybridising under medium stringency conditions (e.g. 50°C and 0.2xSSC {1xSSC = 0.15 M NaCl, 0.015 M Na$_3$citrate pH 7.0}) to the nucleotide sequences presented herein.

More preferably, the term "variant" encompasses sequences that are complementary to sequences that are capable of hybridising under high stringency conditions (e.g. 65°C and 0.1xSSC {1xSSC = 0.15 M NaCl, 0.015 M Na$_3$citrate pH 7.0}) to the nucleotide sequences presented herein.

The present disclosure also relates to the use of nucleotide sequences that can hybridise to the nucleotide sequences as described herein (including complementary sequences of those presented herein).

The present disclosure also relates to the use of nucleotide sequences that are complementary to sequences that can hybridise to the nucleotide sequences as described herein (including complementary sequences of those presented herein).

Also disclosed herein is the use of polynucleotide sequences that are capable of hybridising to the nucleotide sequences presented herein under conditions of intermediate to maximal stringency.

In a preferred aspect, the nucleotide sequences can hybridise to the nucleotide sequenceas described herein, or the complement thereof, under medium stringency conditions (e.g. 50°C and 0.2xSSC {1xSSC = 0.15 M NaCl, 0.015 M Na$_3$citrate pH 7.0}).

In a more preferred aspect, the nucleotide sequences can hybridise to the nucleotide sequence as described herein, or the complement thereof, under high stringent conditions (e.g. 65°C and 0.1xSSC {1xSSC = 0.15 M NaCl, 0.015 M Na$_3$citrate pH 7.0}).

Preferably hybridisation is analysed over the whole of the sequences taught herein.

## MOLECULAR EVOLUTION

As a non-limiting example, it is possible to produce numerous site directed or random mutations into a nucleotide sequence, either in vivo or in vitro, and to subsequently screen for improved functionality of the encoded polypeptide by various means.

In addition, mutations or natural variants of a polynucleotide sequence can be recombined with either the wildtype or other mutations or natural variants to produce new variants. Such new variants can also be screened for improved functionality of the encoded polypeptide. The production of new preferred variants can be achieved by various methods well established in the art, for example the Error Threshold Mutagenesis (WO 92/18645), oligonucleotide mediated random mutagenesis (US 5,723, 323), DNA shuffling (US 5,605,793), exo-mediated gene assembly WO00/58517. The application of these and similar random directed molecular evolution methods allows the identification and selection of variants of the enzymes disclosed herein which have preferred characteristics without any prior knowledge of protein structure or function, and allows the production of non-predictable but beneficial mutations or variants. There are numerous examples of the application of molecular evolution in the art for the optimisation or alteration of enzyme activity, such examples include, but are not limited to one or more of the following: optimised expression and/or activity in a host cell or in vitro, increased enzymatic activity, altered substrate and/or product specificity, increased or decreased enzymatic or structural stability, altered enzymatic activity/specificity in preferred environmental conditions, e.g. temperature, pH, substrate.

## SITE-DIRECTED MUTAGENESIS

Once a protein-encoding nucleotide sequence has been isolated, or a putative protein-encoding nucleotide sequence has been identified, it may be desirable to mutate the sequence in order to prepare a protein of the present disclosure.

Mutations may be introduced using synthetic oligonucleotides. These oligonucleotides contain nucleotide sequences flanking the desired mutation sites.

A suitable method is disclosed in Morinaga et al., (Biotechnology (1984) 2, p646-649). Another method of introducing mutations into enzyme-encoding nucleotide sequences is described in Nelson and Long (Analytical Biochemistry (1989), 180, p 147-151).

## RECOMBINANT

In one aspect the sequence for use in the present invention is a recombinant sequence - i.e. a sequence that has been prepared using recombinant DNA techniques.

These recombinant DNA techniques are within the capabilities of a person of ordinary skill in the art. Such techniques are explained in the literature, for example, J. Sambrook, E. F. Fritsch, and T. Maniatis, 1989, Molecular Cloning: A Laboratory Manual, Second Edition, Books 1-3, Cold Spring Harbor Laboratory Press.

## SYNTHETIC

In one aspect the sequence for use in the present invention is a synthetic sequence - i.e. a sequence that has been prepared by *in vitro* chemical or enzymatic synthesis. It includes, but is not limited to, sequences made with optimal codon usage for host organisms - such as the methylotrophic yeasts *Pichia* and *Hansenula.*

## EXPRESSION OF ENZYMES

The nucleotide sequence for use in the present invention may be incorporated into a recombinant replicable vector. The vector may be used to replicate and express the nucleotide sequence, in protein/enzyme form, in and/or from a compatible host cell. Expression may be controlled using control sequences e.g. regulatory sequences.

The protein produced by a host recombinant cell by expression of the nucleotide sequence may be secreted or may be contained intracellularly depending on the sequence and/or the vector used. The coding sequences may be designed with signal sequences which direct secretion of the substance coding sequences through a particular prokaryotic or eukaryotic cell membrane.

## EXPRESSION VECTOR

The term "expression vector" means a construct capable of *in vivo* or *in vitro* expression.

In one embodiment the proline tolerant tripeptidyl peptidase and/or endoprotease for use in the present invention may be encoded by a vector. In other words the vector may comprise a nucleotide sequence encoding the proline tolerant tripeptidyl peptidase.

Preferably, the expression vector is incorporated into the genome of a suitable host organism. The term "incorporated" preferably covers stable incorporation into the genome. The nucleotide sequence as described herein may be present in a vector in which the nucleotide sequence is operably linked to regulatory sequences capable of providing for the expression of the nucleotide sequence by a suitable host organism.

The vectors for use in the present invention may be transformed into a suitable host cell as described below to provide for expression of a polypeptide for use in the present invention.

The choice of vector e.g. a plasmid, cosmid, or phage vector will often depend on the host cell into which it is to be introduced.

The vectors for use in the present invention may contain one or more selectable marker genes- such as a gene, which confers antibiotic resistance e.g. ampicillin, kanamycin, chloramphenicol or tetracyclin resistance. Alternatively, the selection may be accomplished by co-transformation (as described in WO91/17243).

Vectors may be used *in vitro,* for example for the production of RNA or used to transfect, transform, transduce or infect a host cell.

Described herein is a method of making nucleotide sequences for use in the present invention by introducing a nucleotide sequence for use in the present invention into a replicable vector, introducing the vector into a compatible host cell, and growing the host cell under conditions which bring about replication of the vector.

The vector may further comprise a nucleotide sequence enabling the vector to replicate in the host cell in question. Examples of such sequences are the origins of replication of plasmids pUC19, pACYC177, pUB110, pE194, pAMB1 and pIJ702.

## CODON OPTIMISATION

The nucleotide sequence and/or vector encoding the proline tolerant tripeptidyl peptidase and/or the endoprotease may be codon optimised for expression in a particular host organism.

The nucleotide sequence and/or vector encoding the proline tolerant tripeptidyl peptidase and/or the endoprotease may be codon optimised for expression in a prokaryotic or eukaryotic cell. Suitably, the nucleotide sequence and/or vector encoding the proline tolerant tripeptidyl peptidase and/or the endoprotease may be codon optimised for expression in a fungal host organism (e.g. *Trichoderma,* preferably *Trichoderma reesei*).

Codon optimisation refers to a process of modifying a nucleic acid sequence for enhanced expression in a host cell of interest by replacing at least one codon (e.g. at least about more than 1, 2, 3, 4, 5, 10, 15, 20, 25, 50, 60, 70, 80 or 100 codons) of the native sequence with codons that are more frequently used in the genes of the host cell, whilst maintaining the native amino acid sequence. Various species exhibit particular bias for certain codons of a particular amino acid. Codon bias (differences in codon usage between organisms) often correlates with the efficiency of translation of messenger RNA (mRNA), which is in tum believed to be dependent on, amongst other things, the properties of the codons being translated and the availability of particular transfer RNA (tRNA) molecules. The predominance of selected tRNAs in a cell is generally a reflection of the codons used most frequently in peptide synthesis.

Accordingly, genes can be tailored for optimal gene expression in a given organism based on codon optimisation. A nucleotide sequence and/vector that has undergone this tailoring can be referred to therefore as a "codon optimised" nucleotide sequence and/or vector.

Codon usage tables are readily available, for example, at the "Codon Usage Database", and these tables can be adapted in a number of ways. See Nakamura, Y., et al. "Codon usage tabulated from the international DNA sequence databases: status for the year 2000" Nucl. Acids Res. 28:292 (2000). Computer algorithms for codon optimising a particular sequence for expression in a particular host cell are also available, such as Gene Forge (Aptagen; Jacobus,Pa.). In some embodiments, one or more codons (e.g. 1, 2, 3, 4, 5, 10, 15, 20, 25, 50, or more, or all codons) in a sequence encoding a proline tolerant tripeptidyl peptidase and/or endoprotease for use in the present invention correspond to the most frequently used codon for a particular amino acid.

In one embodiment the nucleotide sequence encoding the proline tolerant tripeptidyl peptidase may be a nucleotide sequence which has been codon optimised for expression in *Trichoderma reesei.*

Described herein, the codon optimised sequence may comprise a nucleotide sequence shown as SEQ ID No. 81, SEQ ID No. 82, SEQ ID No. 83, SEQ ID No. 84, SEQ ID No. 85, SEQ ID No. 86, SEQ ID No. 87, SEQ ID No. 88, SEQ ID No. 89, SEQ ID No. 90, SEQ ID No. 91, SEQ ID No. 92, SEQ ID No. 93, SEQ ID No. 94, SEQ ID No. 95, SEQ ID No. 97 or a nucleotide sequence having at least 70% identity thereto. Suitably a sequence having at least 80% thereto or at least 90% thereto.

Described herein, the codon optimised sequence may comprise a nucleotide sequence having at least 95% sequence identity to SEQ ID No. 81, SEQ ID No. 82, SEQ ID No. 83, SEQ ID No. 84, SEQ ID No. 85, SEQ ID No. 86, SEQ ID No. 87, SEQ ID No. 88, SEQ ID No. 89, SEQ ID No. 90, SEQ ID No. 91, SEQ ID No. 92, SEQ ID No. 93, SEQ ID No. 94, SEQ ID No. 95, more preferably at least 99% identity to SEQ ID No. 81, SEQ ID No. 82, SEQ ID No. 83, SEQ ID No. 84, SEQ ID No. 85, SEQ ID No. 86, SEQ ID No. 87, SEQ ID No. 88, SEQ ID No. 89, SEQ ID No. 90, SEQ ID No. 91, SEQ ID No. 92, SEQ ID No. 93, SEQ ID No. 94, SEQ ID No. 95 or SEQ ID No. 97.

Described herein, the proline tolerant tripeptidyl peptidase may be encoded by a nucleotide sequence which hybridises to SEQ ID No. 81, SEQ ID No. 82, SEQ ID No. 83, SEQ ID No. 84, SEQ ID No. 85, SEQ ID No. 86, SEQ ID No. 87, SEQ ID No. 88, SEQ ID No. 89, SEQ ID No. 90, SEQ ID No. 91, SEQ ID No. 92, SEQ ID No. 93, SEQ ID No. 94, SEQ ID No. 95 or SEQ ID No. 97 under medium stringency conditions. Suitably, a nucleotide sequence which hybridises to SEQ ID No. 81, SEQ ID No. 82, SEQ ID No. 83, SEQ ID No. 84, SEQ ID No. 85, SEQ ID No. 86, SEQ ID No. 87, SEQ ID No. 88, SEQ ID No. 89, SEQ ID No. 90, SEQ ID No. 91, SEQ ID No. 92, SEQ ID No. 93, SEQ ID No. 94, SEQ ID No. 95 or SEQ ID No. 97 under high stringency conditions.

Described herein, the proline tolerant tripeptidyl peptidase may be encoded by a nucleotide sequence which differs from SEQ ID No. 81, SEQ ID No. 82, SEQ ID No. 83, SEQ ID No. 84, SEQ ID No. 85, SEQ ID No. 86, SEQ ID No. 87, SEQ ID No. 88, SEQ ID No. 89, SEQ ID No. 90, SEQ ID No. 91, SEQ ID No. 92, SEQ ID No. 93, SEQ ID No. 94, SEQ ID No. 95 or SEQ ID No. 97 due to degeneracy of the genetic code.

Described herein isa vector (e.g. plasmid) comprising one or more of the sequences selected from the group consisting of: SEQ ID No. 81, SEQ ID No. 82, SEQ ID No. 83, SEQ ID No. 84, SEQ ID No. 85, SEQ ID No. 86, SEQ ID No. 87, SEQ ID No. 88, SEQ ID No. 89, SEQ ID No. 90, SEQ ID No. 91, SEQ ID No. 92, SEQ ID No. 93, SEQ ID No. 94, SEQ ID No. 95 or SEQ ID No. 97.

## REGULATORY SEQUENCES

In some applications, the nucleotide sequence for use in the present invention is operably linked to a regulatory sequence which is capable of providing for the expression of the nucleotide sequence, such as by the chosen host cell. By way of example, the present disclosure covers a vector comprising the nucleotide sequence for use in the present invention operably linked to such a regulatory sequence, i.e. the vector is an expression vector.

The term "operably linked" refers to a juxtaposition wherein the components described are in a relationship permitting them to function in their intended manner. A regulatory sequence "operably linked" to a coding sequence is ligated in such a way that expression of the coding sequence is achieved under condition compatible with the control sequences.

The term "regulatory sequences" includes promoters and enhancers and other expression regulation signals.

The term "promoter" is used in the normal sense of the art, e.g. an RNA polymerase binding site.

Enhanced expression of the nucleotide sequence encoding the enzyme for use in the present invention may also be achieved by the selection of heterologous regulatory regions, e.g. promoter, secretion leader and terminator regions.

Preferably, the nucleotide sequence for use in the present invention is operably linked to at least a promoter.

Other promoters may even be used to direct expression of the polypeptide for use in the present invention.

Examples of suitable promoters for directing the transcription of the nucleotide sequence in a bacterial, fungal or yeast host are well known in the art.

The promoter can additionally include features to ensure or to increase expression in a suitable host. For example, the features can be conserved regions such as a Pribnow Box or a TATA box.

## CONSTRUCTS

The term "construct" - which is synonymous with terms such as "conjugate", "cassette" and "hybrid" - includes a nucleotide sequence for use according to the present invention directly or indirectly attached to a promoter.

An example of an indirect attachment is the provision of a suitable spacer group such as an intron sequence, such as the Sh1-intron or the ADH intron, intermediate the promoter and the nucleotide sequence of the present invention. The same is true for the term "fused" in relation to the present invention which includes direct or indirect attachment. In some cases, the terms do not cover the natural combination of the nucleotide sequence coding for the protein ordinarily associated with the wild type gene promoter and when they are both in their natural environment.

The construct may even contain or express a marker, which allows for the selection of the genetic construct.

For some applications, preferably the construct of the present invention comprises at least the nucleotide sequence of the present invention operably linked to a promoter.

## HOST CELLS

The term "host cell" - in relation to the present invention includes any cell that comprises either the nucleotide sequence or an expression vector as described above and which is used in the recombinant production of a protein having the specific properties as defined herein.

Thus, described herein are host cells transformed or transfected with a nucleotide sequence that expresses the protein for use in the present invention. The cells will be chosen to be compatible with the said vector and may for example be prokaryotic (for example bacterial), fungal, yeast or plant cells.

Examples of suitable bacterial host organisms are gram positive or gram negative bacterial species.

Depending on the nature of the nucleotide sequence encoding the polypeptide for use in the present invention, and/or the desirability for further processing of the expressed protein, eukaryotic hosts such as yeasts or other fungi may be preferred. In general, yeast cells are preferred over fungal cells because they are easier to manipulate. However, some proteins are either poorly secreted from the yeast cell, or in some cases are not processed properly (e.g. hyperglycosylation in yeast). In these instances, a different fungal host organism should be selected.

The use of suitable host cells - such as yeast, fungal and plant host cells - may provide for post-translational modifications (e.g. myristoylation, glycosylation, truncation, lipidation and tyrosine, serine or threonine phosphorylation) as may be needed to confer optimal biological activity on recombinant expression products for use in the present invention. The host cell may be a protease deficient or protease minus strain. This may for example be the protease deficient strain *Aspergillus oryzae* JaL 125 having the alkaline protease gene named "alp" deleted. This strain is described in WO97/35956.

Suitably, the host cell may be a *Trichoderma* host cell, preferably a *Trichoderma reesei* host cell. In other embodiments, the host cell may be any member belonging to the genera *Escherichia, Bacillus, Thermomyces, Acremonium, Aspergillus, Penicillium, Mucor, Neurospora, Humicola* and the like.

## ORGANISM

The term "organism" in relation to the present invention includes any organism that could comprise the nucleotide sequence coding for the polypeptide according to the present invention and/or products obtained therefrom, and/or wherein a promoter can allow expression of the nucleotide sequence for use in the present invention when present in the organism.

Suitable organisms may include a prokaryote, fungus, yeast or a plant.

The term "transgenic organism" in relation to the present invention includes any organism that comprises the nucleotide sequence coding for the polypeptide according to the present invention and/or the products obtained therefrom, and/or wherein a promoter can allow expression of the nucleotide sequence for use in the present invention within the organism. Preferably the nucleotide sequence is incorporated in the genome of the organism.

The term "transgenic organism" does not cover native nucleotide coding sequences in their natural environment when they are under the control of their native promoter which is also in its natural environment.

Therefore, the transgenic organism described herein includes an organism comprising any one of, or combinations of, the nucleotide sequence coding for the polypeptide as described herein, constructs as described herein, vectors as described herein, plasmids as described herein, cells as described herein, tissues as described herein, or the products thereof.

For example the transgenic organism may also comprise the nucleotide sequence coding for the polypeptide as described herein under the control of a heterologous promoter.

## TRANSFORMATION OF HOST CELLS/ORGANISM

As indicated earlier, the host organism can be a prokaryotic or a eukaryotic organism. Examples of suitable prokaryotic hosts include *E. coli* and *Bacillus spp., including Bacillus subtilis* and *B. licheniformis.*

Teachings on the transformation of prokaryotic hosts are well documented in the art, for example see Sambrook et al (Molecular Cloning: A Laboratory Manual, 2nd edition, 1989, Cold Spring Harbor Laboratory Press). If a prokaryotic host is used then the nucleotide sequence may need to be suitably modified before transformation - such as by removal of introns.

Filamentous fungi cells may be transformed using various methods known in the art - such as a process involving protoplast formation and transformation of the protoplasts followed by regeneration of the cell wall in a manner known. The use of *Aspergillus* as a host microorganism is described in EP 0 238 023.

Another host organism can be a plant. A review of the general techniques used for transforming plants may be found in articles by Potrykus (Annu Rev Plant Physiol Plant Mol Biol [1991] 42:205-225) and Christou (Agro-Food-Industry Hi-Tech March/April 1994 17-27). Further teachings on plant transformation may be found in EP-A-0449375.

General teachings on the transformation of fungi, yeasts and plants are presented in following sections.

## TRANSFORMED FUNGUS

A host organism may be a fungus - such as a mould. Examples of suitable such hosts include any member belonging to the genera Thermomyces, Acremonium, Aspergillus, Penicillium, Mucor, Neurospora, Trichoderma and the like.

In one embodiment, the host organism may be a filamentous fungus.

Transforming filamentous fungi is discussed in US-A-5741665 which states that standard techniques for transformation of filamentous fungi and culturing the fungi are well known in the art. An extensive review of techniques as applied to *N. crassa* is found, for example in Davis and de Serres, Methods Enzymol (1971) 17A: 79-143.

Further teachings which may also be utilised in transforming filamentous fungi are reviewed in US-A-5674707.

In addition, gene expression in filamentous fungi is taught in in Punt et al. (2002) Trends Biotechnol 2002 May;20(5):200-6, Archer & Peberdy Crit Rev Biotechnol (1997) 17(4):273-306.

Described herein is the production of transgenic filamentous fungi according to the present invention prepared by use of these standard techniques.

Described herein, the host organism is a *Trichoderma* host organism, e.g. a *Trichoderma reesei* host organism.

Described herein, the host organism can be of the genus *Aspergillus,* such as *Aspergillus niger.*

A transgenic *Aspergillus* can also be prepared by following, for example, the teachings of Turner G. 1994 (Vectors for genetic manipulation. In: Martinelli S.D., Kinghorn J.R.(Editors) Aspergillus: 50 years on. Progress in industrial microbiology vol 29. Elsevier Amsterdam 1994. pp. 641-666).

## TRANSFORMED YEAST

Described herein, the transgenic organism can be a yeast.

A review of the principles of heterologous gene expression in yeast are provided in, for example, Methods Mol Biol (1995), 49:341-54, and Curr Opin Biotechnol (1997) Oct;8(5):554-60

In this regard, yeast - such as the species *Saccharomyces cerevisiae or Pichia pastoris* (see FEMS Microbiol Rev (2000 24(1):45-66), may be used as a vehicle for heterologous gene expression.

A review of the principles of heterologous gene expression in *Saccharomyces cerevisiae* and secretion of gene products is given by E Hinchcliffe E Kenny (1993, "Yeast as a vehicle for the expression of heterologous genes", Yeasts, Vol 5, Anthony H Rose and J Stuart Harrison, eds, 2nd edition, Academic Press Ltd.).

For the transformation of yeast, several transformation protocols have been developed. For example, a transgenic Saccharomyces can be prepared by following the teachings of Hinnen et al., (1978, Proceedings of the National Academy of Sciences of the USA 75, 1929); Beggs, J D (1978, Nature, London, 275, 104); and Ito, H et al (1983, J Bacteriology 153, 163-168).

The transformed yeast cells may be selected using various selective markers - such as auxotrophic markers dominant antibiotic resistance markers.

## CULTURING AND PRODUCTION

Host cells transformed with the nucleotide sequence as described herein may be cultured under conditions conducive to the production of the encoded polypeptide and which facilitate recovery of the polypeptide from the cells and/or culture medium.

The medium used to cultivate the cells may be any conventional medium suitable for growing the host cell in questions and obtaining expression of the polypeptide.

The protein produced by a recombinant cell may be displayed on the surface of the cell.

The protein may be secreted from the host cells and may conveniently be recovered from the culture medium using well-known procedures.

## SECRETION

Often, it is desirable for the protein to be secreted from the expression host into the culture medium from where the protein may be more easily recovered. Described herein, the secretion leader sequence may be selected on the basis of the desired expression host. Hybrid signal sequences may also be used with the context of the present invention.

Typical examples of heterologous secretion leader sequences are those originating from the fungal amyloglucosidase (AG) gene (*gla*A - both 18 and 24 amino acid versions e.g. from *Aspergillus*), the a-factor gene (yeasts e.g. *Saccharomyces, Kluyveromyces* and *Hansenula*) or the α-amylase gene (*Bacillus*).

By way of example, the secretion of heterologous proteins in *E. coli* is reviewed in Methods Enzymol (1990) 182:132-43.

## POST-TRANSCRIPTION AND POST-TRANSLATIONAL MODIFICATIONS

Suitably the proline tolerant tripeptidyl peptidase and/or the endoprotease for use in the present invention may be encoded by any one of the nucleotide sequences taught herein. Depending upon the host cell used post-transcriptional and/or post-translational modifications may be made. It is envisaged that the enzymes (e.g. the proline tolerant tripeptidyl peptidase and/or the endoprotease) for use in the present methods and/or uses encompasses enzymes (e.g. the proline tolerant tripeptidyl peptidase and/or the endoprotease) which have undergone post-transcriptional and/or post-translational modification.

One non-limiting example of a post-transcriptional and/or post-translational modifications is "clipping" or "cleavage" of a polypeptide (e.g. of the proline tolerant tripeptidyl peptidase and/or the endoprotease).

In some embodiments the polypeptide (e.g. the tripeptidyl peptidase for use in the present invention e.g. proline tolerant tripeptidyl peptidase and/or the endoprotease) may be clipped or cleaved. This may result in the conversion of the proline tolerant tripeptidyl peptidase and/or the endoprotease from an inactive or substantially inactive state to an active state (i.e. capable of performing the activity described herein).

The proline tolerant tripeptidyl peptidase may be a pro-peptide which undergoes further post-translational modification to a mature peptide, i.e. a polypeptide which has the proline tolerant tripeptidyl peptidase activity.

By way of example only SEQ ID No. 1 is the same as SEQ ID No. 29 except that SEQ ID No. 1 has undergone post-translational and/or post-transcriptional modification to remove some amino acids, more specifically 197 amino acids from the N-terminus. Therefore the polypeptide shown herein as SEQ ID No. 1 could be considered in some circumstances (i.e. in some host cells) as a pro-peptide - which is further processed to a mature peptide (SEQ ID No. 29) by post-translational and/or post-transcriptional modification. The precise modifications, e.g. cleavage site(s), in respect of the post-translational and/or post-transcriptional modification may vary slightly depending on host species. In some host species there may be no post translational and/or post-transcriptional modification, hence the pro-peptide would then be equivalent to the mature peptide (i.e. a polypeptide which has the tripeptidyl peptidase activity of the present invention). Without wishing to be bound by theory, the cleavage site(s) may be shifted by a few residues (e.g. 1, 2 or 3 residues) in either direction compared with the cleavage site shown by reference to SEQ ID No. 29 compared with SEQ ID No.1. In other words, rather than cleavage at position 197 (R) for example, the cleavage may be at position 196-A, 195-A, 194-A, 198Q, 199E, 200P for example. In addition or alternatively, the cleavage may result in the removal of about 197 amino acids, in some embodiments the cleavage may result in the removal of between 194 and 200 residues.

Other examples of post-transcriptional and/or post-translational modifications include but are not limited to myristoylation, glycosylation, truncation, lipidation and tyrosine, serine or threonine phosphorylation. The skilled person will appreciate that the type of post-transcriptional and/or post-translational modifications that may occur to a protein (e.g. the proline tolerant tripeptidyl peptidase and/or the endoprotease) may depend on the host organism in which the protein (e.g. the proline tolerant tripeptidyl peptidase and/or the endoprotease) is expressed.

## DETECTION

A variety of protocols for detecting and measuring the expression of the amino acid sequence are known in the art. Examples include enzyme-linked immunosorbent assay (ELISA), radioimmunoassay (RIA) and fluorescent activated cell sorting (FACS).

A wide variety of labels and conjugation techniques are known by those skilled in the art and can be used in various nucleic and amino acid assays.

A number of companies such as Pharmacia Biotech (Piscataway, NJ), Promega (Madison, WI), and US Biochemical Corp (Cleveland, OH) supply commercial kits and protocols for these procedures.

Suitable reporter molecules or labels include those radionuclides, enzymes, fluorescent, chemiluminescent, or chromogenic agents as well as substrates, cofactors, inhibitors, magnetic particles and the like. Patents teaching the use of such labels include US-A-3,817,837; US-A-3,850,752; US-A-3,939,350; US-A-3,996,345; US-A-4,277,437; US-A-4,275,149 and US-A-4,366,241.

Also, recombinant immunoglobulins may be produced as shown in US-A-4,816,567.

## FUSION PROTEINS

The amino acid sequence for use according to the present invention may be produced as a fusion protein, for example to aid in extraction and purification. Examples of fusion protein partners include glutathione-S-transferase (GST), 6xHis, GAL4 (DNA binding and/or transcriptional activation domains) and (β-galactosidase). It may also be convenient to include a proteolytic cleavage site between the fusion protein partner and the protein sequence of interest to allow removal of fusion protein sequences.

Preferably, the fusion protein will not hinder the activity of the protein sequence.

Gene fusion expression systems in *E. coli* have been reviewed in Curr Opin Biotechnol (1995) 6(5):501-6.

In another embodiment of the invention, the amino acid sequence may be ligated to a heterologous sequence to encode a fusion protein. For example, for screening of peptide libraries for agents capable of affecting the substance activity, it may be useful to encode a chimeric substance expressing a heterologous epitope that is recognised by a commercially available antibody.

## GENERAL RECOMBINANT DNA METHODOLOGY TECHNIQUES

The present invention employs, unless otherwise indicated, conventional techniques of chemistry, molecular biology, microbiology, recombinant DNA and immunology, which are within the capabilities of a person of ordinary skill in the art. Such techniques are explained in the literature. See, for example, J. Sambrook, E. F. Fritsch, and T. Maniatis, 1989, Molecular Cloning: A Laboratory Manual, Second Edition, Books 1-3, Cold Spring Harbor Laboratory Press; Ausubel, F. M. et al. (1995 and periodic supplements; Current Protocols in Molecular Biology, ch. 9, 13, and 16, John Wiley & Sons, New York, N.Y.); B. Roe, J. Crabtree, and A. Kahn, 1996, DNA Isolation and Sequencing: Essential Techniques, John Wiley & Sons; M. J. Gait (Editor), 1984, Oligonucleotide Synthesis: A Practical Approach, Irl Press; and, D. M. J. Lilley and J. E. Dahlberg, 1992, Methods of Enzymology: DNA Structure Part A: Synthesis and Physical Analysis of DNA Methods in Enzymology, Academic Press. Each of these general texts is herein incorporated by reference.

## DOSAGES

The proline tolerant tripeptidyl peptidase and/or the endoprotease for use in the methods and/or uses of the present invention may be dosed in any suitable amount.

In one embodiment the proline tolerant tripeptidyl peptidase may be dosed in an amount of about 0.01 mg -100 mg; 0.5 mg -100 mg; 1 mg -50 mg; 5 mg -100 mg; 5 mg - 20 mg, 10 mg -100 mg; 0.05 mg - 50 mg; or 0.10 mg -10 mg of enzyme per kg of feed additive composition. In certain embodiments the proline tolerant tripeptidyl peptidase may be dosed in an amount of about 0.01 g to 1000 g of enzyme per kg of feed additive composition, such as 0.1 g to 500 g, such as 0.5 g to 700 g, such as in an amount of about 0.01 g - 200 g, 0.01 g -100 g; 0.5 g -100 g; 1 g - 50 g; 5 g -100 g; 5 g - 20 g, 5 g - 15 g, 10 g -100 g; 0.05 g - 50 g; or 0.10 g -10 g of enzyme per kg of feed additive composition.

In one preferred embodiment, the proline tolerant tripeptidyl peptidase may be dosed in an amount of about 5 mg - 20 mg of enzyme per kg of feed additive composition,

The exact amount will depend on the particular type of composition employed and on the specific protease activity per mg of protein.

In another embodiment the proline tolerant tripeptidyl peptidase may be dosed in an amount of about 1 mg to about 1 kg of enzyme per kg of feed and/or feedstuff and/or premix. Suitably the proline tolerant tripeptidyl peptidase may be dosed at about 1 mg to about 250 g per kg of feed and/or feedstuff and/or premix. Preferably at about 1 mg to about 100 g (more preferably at about 1 mg to about 1 g) per kg of feed and/or feedstuff and/or premix.

The endoprotease may be dosed in an amount of less than about 6.0 g of enzyme per metric ton (MT) of feed.

Suitably, the endoprotease may be dosed in an amount of less than about 4.0 g of enzyme per MT, suitably less than about 2.0 g of enzyme per MT.

In another embodiment the endoprotease may be dosed at between about 0.5 g and about 5.0 g of enzyme per MT of feed. Suitably the endoprotease may be dosed at between about 0.5 g and about 3.0 g of enzyme per MT of feed. More suitably, the endoprotease may be dosed at about 1.0 g to about 2.0 g of enzyme per MT of feed.

In one embodiment the aminopeptidase may be dosed in an amount of between about 0.5 mg to about 2 g of enzyme per kg of protein substrate and/or feed additive composition. Suitably the aminopeptidase may be dosed in an amount of between about 1 mg to about 2 g of enzyme per kg of protein substrate and/or feed additive composition. More suitably in an amount of between about 5 mg to about 1.5 g of enzyme per kg of protein substrate and/or feed additive composition.

## ANIMAL

The term "animal", as used herein, means an animal that is to be or has been administered with a feed additive composition according to the present invention or a feedstuff comprising said feed additive composition according to the present invention.

Preferably, the animal is a mammal, a ruminant animal, monogastric animal, fish or crustacean including for example livestock or a domesticated animal (e.g. a pet).

In one embodiment the "animal" is livestock.

The term "livestock", as used herein refers to any farmed animal. Preferably, livestock is one or more of cows or bulls (including calves), pigs (including piglets, swine, growing pigs, sows), poultry (including broilers, chickens, egg layers and turkeys), birds, fish (including freshwater fish, such as salmon, cod, trout and carp, e.g. koi carp, and marine fish, such as sea bass), crustaceans (such as shrimps, mussels and scallops), horses (including race horses), sheep (including lambs).

In another embodiment the "animal" is a domesticated animal or pet or an animal maintained in a zoological environment.

The term "domesticated animal or pet or animal maintained in a zoological environment" as used herein refers to any relevant animal including canines (e.g. dogs), felines (e.g. cats), rodents (e.g. guinea pigs, rats, mice), birds, fish (including freshwater fish and marine fish), and horses.

In one embodiment the animal is a monogastric animal. In a preferred embodiment the monogastric animal may be poultry or pig (or a combination thereof).

In another embodiment the animal is a ruminant animal.

The term animal is not intended to refer to a human being.

## PACKAGING

In one embodiment the feed additive composition and/or premix and/or feed or feedstuff according to the present invention is packaged.

In one preferred embodiment the feed additive composition and/or premix and/or feed or feedstuff is packaged in a bag, such as a paper bag.

In an alternative embodiment the feed additive composition and/or premix and/or feed or feedstuff may be sealed in a container. Any suitable container may be used.

## FEED

The feed additive composition of the present invention may be used as - or in the preparation of - a feed.

The term "feed" is used synonymously herein with "feedstuff".

The feed may be in the form of a solution or as a solid - depending on the use and/or the mode of application and/or the mode of administration.

When used as - or in the preparation of - a feed - such as functional feed - the composition of the present invention may be used in conjunction with one or more of: a nutritionally acceptable carrier, a nutritionally acceptable diluent, a nutritionally acceptable excipient, a nutritionally acceptable adjuvant, a nutritionally active ingredient.

In a preferred embodiment the feed additive composition of the present invention is admixed with a feed component to form a feedstuff.

The term "feed component" as used herein means all or part of the feedstuff. Part of the feedstuff may mean one constituent of the feedstuff or more than one constituent of the feedstuff, e.g. 2 or 3 or 4. In one embodiment the term "feed component" encompasses a premix or premix constituents.

In one embodiment a feed additive composition comprising a proline tolerant tripeptidyl peptidase and one or more ingredients selected from the group consisting of: a wheat carrier, a polyol, a sugar, a salt and a preservative (optionally in combination with an endoprotease) may be admixed with at least one protein or portion thereof is an animal protein or a vegetable protein (e.g. selected from one or more of a gliadin, a beta-casein, a beta-lactoglobulin or an immunogenic fragment of a gliadin, a beta-casein, a beta-lactoglobulin, glycinin, beta-conglycinin, cruciferin, napin, collagen, whey protein, fish protein or meal, meat protein or meal including meat bone meal, feather protein or meal, egg protein, soy protein or grain protein), preferably comprised in corn, soybean meal, corn dried distillers grains with solubles (DDGS), wheat, wheat proteins including gluten, wheat by products, wheat bran, corn by products including corn gluten meal, barley, oat, rye, triticale, full fat soy, animal by-product meals, an alcohol-soluble protein (preferably a zein (e.g. a maize zein maize) and/or a kafirin (e.g. from sorghum)), a protein from oil seeds (preferably from soybean seed proteins, sun flower seed proteins, rapeseed proteins, canola (rape) seed proteins or combinations thereof) or a combination thereof.

Preferably the feed may be a fodder, or a premix thereof, a compound feed, or a premix thereof. In one embodiment the feed additive composition according to the present invention may be admixed with a compound feed, a compound feed component or to a premix of a compound feed or to a fodder, a fodder component, or a premix of a fodder.

The term fodder as used herein means any food which is provided to an animal (rather than the animal having to forage for it themselves). Fodder encompasses plants that have been cut.

The term fodder includes hay, straw, silage, compressed and pelleted feeds, oils and mixed rations, and also sprouted grains and legumes.

Fodder may be obtained from one or more of the plants selected from: alfalfa (Lucerne), barley, birdsfoot trefoil, brassicas, Chau moellier, kale, rapeseed (canola), rutabaga (swede), turnip, clover, alsike clover, red clover, subterranean clover, white clover, grass, false oat grass, fescue, Bermuda grass, brome, heath grass, meadow grasses (from naturally mixed grassland swards, orchard grass, rye grass, Timothy-grass, corn (maize), millet, oats, sorghum, soybeans, trees (pollard tree shoots for tree-hay), wheat, and legumes.

The term "compound feed" means a commercial feed in the form of a meal, a pellet, nuts, cake or a crumble. Compound feeds may be blended from various raw materials and additives. These blends are formulated according to the specific requirements of the target animal.

Compound feeds can be complete feeds that provide all the daily required nutrients, concentrates that provide a part of the ration (protein, energy) or supplements that only provide additional micronutrients, such as minerals and vitamins.

The main ingredients used in compound feed are the feed grains, which include corn, wheat, rye, maize, soybeans, sorghum, oats, and barley.

Suitably a premix as referred to herein may be a composition composed of microingredients such as vitamins, minerals, chemical preservatives, antibiotics, fermentation products, and other essential ingredients. Premixes are usually compositions suitable for blending into commercial rations.

Vitamins for use in the present invention may include vitamin A, vitamin D3, vitamin E, vitamin K3, vitamin B1, vitamin B2, vitamin B6, vitamin B12, Niacin, Pantothenic acid or mixtures thereof.

Any feedstuff of the present invention may comprise one or more feed materials selected from the group comprising a) cereals, such as small grains (e.g., wheat, barley, rye, oats and combinations thereof) and/or large grains such as maize or sorghum; b) by products from plants, such as Distillers Dried Grain Solubles (DDGS), wheat bran, wheat middlings, wheat shorts, rice bran, rice hulls, oat hulls, palm kernel, citrus pulp, corn fibre, corn germ meal, corn bran, Hominy feed, corn gluten feed, gluten meal, wheat shorts, wheat middlings or combinations thereof; c) protein obtained from sources such as soya, sunflower, peanut, lupin, peas, fava beans, cotton, canola (rapeseed), fish meal, dried plasma protein, meat and bone meal, potato protein, whey, copra, sesame; d) oils and fats obtained from vegetable and animal sources; e) minerals and vitamins.

A feedstuff of the present invention may contain at least 30%, at least 40%, at least 50% or at least 60% by weight corn and soybean meal or corn and full fat soy, or wheat meal or sunflower meal.

In addition or in the alternative, a feedstuff of the present invention may comprise at least one high fibre feed material and/or at least one by-product of the at least one high fibre feed material to provide a high fibre feedstuff. Examples of high fibre feed materials include: wheat, barley, rye, oats, by products from plants (e.g. cereals), such as Distillers Dried Grain Solubles (DDGS), wheat bran, wheat middlings, wheat shorts, rice bran, rice hulls, oat hulls, palm kernel, citrus pulp, corn fibre, corn germ meal, corn bran, Hominy feed, corn gluten feed, gluten meal, wheat shorts, wheat middlings or combinations thereof. Some protein sources may also be regarded as high fibre: protein obtained from sources such as sunflower, lupin, fava beans and cotton.

In the present invention the feed may be one or more of the following: a compound feed and premix, including pellets, nuts or (cattle) cake; a crop or crop residue: corn, soybeans, sorghum, oats, barley, corn stover, copra, straw, chaff, sugar beet waste; fish meal; freshly cut grass and other forage plants; meat and bone meal; molasses; oil cake and press cake; oligosaccharides; conserved forage plants: hay and silage; seaweed; seeds and grains, either whole or prepared by crushing, milling etc.; sprouted grains and legumes; yeast extract.

The term "feed" in the present invention also encompasses in some embodiments pet food. A pet food is plant or animal material intended for consumption by pets, such as dog food or cat food. Pet food, such as dog and cat food, may be either in a dry form, such as kibble for dogs, or wet canned form. Cat food may contain the amino acid taurine.

The term "feed" in the present invention also encompasses in some embodiments fish food. A fish food normally contains macro nutrients, trace elements and vitamins necessary to keep captive fish in good health. Fish food may be in the form of a flake, pellet or tablet. Pelleted forms, some of which sink rapidly, are often used for larger fish or bottom feeding species. Some fish foods also contain additives, such as beta carotene or sex hormones, to artificially enhance the colour of ornamental fish.

The term "feed" in the present invention also encompasses in some embodiment bird food. Bird food includes food that is used both in birdfeeders and to feed pet birds. Typically bird food comprises of a variety of seeds, but may also encompass suet (beef or mutton fat).

As used herein the term "contacting" refers to the indirect or direct application of the composition of the present invention to the product (e.g. the feed). Examples of the application methods which may be used, include, but are not limited to, treating the product in a material comprising the feed additive composition, direct application by mixing the feed additive composition with the product, spraying the feed additive composition onto the product surface or dipping the product into a preparation of the feed additive composition.

In one embodiment the feed additive composition of the present invention is preferably admixed with the product (e.g. feedstuff). Alternatively, the feed additive composition may be included in the emulsion or raw ingredients of a feedstuff.

For some applications, it is important that the composition is made available on or to the surface of a product to be affected/treated. This allows the composition to impart one or more of the following favourable characteristics: biophysical characteristic is selected from the group consisting of one or more of the following: performance of the animal, growth performance of an animal, feed conversion ratio (FCR), ability to digest a raw material (e.g. nutrient digestibility, including starch , fat, protein, fibre digestibility), nitrogen digestibility (e.g. ileal nitrogen digestibility) and digestible energy (e.g. ileal digestible energy) nitrogen retention, carcass yield, growth rate, weight gain, body weight, mass, feed efficiency, body fat percentage, body fat distribution, growth, egg size, egg weight, egg mass, egg laying rate, lean gain, bone ash %, bone ash mg, back fat %, milk output, milk fat %, reproductive outputs such as litter size, litter survivability, hatchability % and environmental impact, e.g. manure output and/or nitrogen excretion.

The feed additive compositions of the present invention may be applied to intersperse, coat and/or impregnate a product (e.g. feedstuff or raw ingredients of a feedstuff) with a controlled amount of enzyme(s).

Preferably, the feed additive composition of the present invention will be thermally stable to heat treatment up to about 70°C; up to about 85°C; or up to about 95°C. The heat treatment may be performed for up to about 1 minute; up to about 5 minutes; up to about 10 minutes; up to about 30 minutes; up to about 60 minutes. The term thermally stable means that at least about 75% of the enzyme components that were present/active in the additive before heating to the specified temperature are still present/active after it cools to room temperature. Preferably, at least about 80% of the enzyme components that were present and active in the additive before heating to the specified temperature are still present and active after it cools to room temperature.

In a particularly preferred embodiment the feed additive composition is homogenized to produce a powder.

In an alternative preferred embodiment, the feed additive composition is formulated to granules as described in WO2007/044968 (referred to as TPT granules) incorporated herein by reference.

In another preferred embodiment when the feed additive composition is formulated into granules the granules comprise a hydrated barrier salt coated over the protein core. The advantage of such salt coating is improved thermo-tolerance, improved storage stability and protection against other feed additives otherwise having adverse effect on the enzyme. Preferably, the salt used for the salt coating has a water activity greater than 0.25 or constant humidity greater than 60 % at 20°C.

Preferably, the salt coating comprises a $Na_2SO_4$.

The method of preparing a feed additive composition may also comprise the further step of pelleting the powder. The powder may be mixed with other components known in the art. The powder, or mixture comprising the powder, may be forced through a die and the resulting strands are cut into suitable pellets of variable length.

Optionally, the pelleting step may include a steam treatment, or conditioning stage, prior to formation of the pellets. The mixture comprising the powder may be placed in a conditioner, e.g. a mixer with steam injection. The mixture is heated in the conditioner up to a specified temperature, such as from 60-100°C, typical temperatures would be 70°C, 80°C, 85°C, 90°C or 95°C. The residence time can be variable from seconds to minutes and even hours. Such as 5 seconds, 10 seconds, 15 seconds, 30 seconds, 1 minutes 2 minutes., 5 minutes, 10 minutes, 15 minutes, 30 minutes and 1 hour.

It will be understood that the feed additive composition of the present invention is suitable for addition to any appropriate feed material.

As used herein, the term "feed material" refers to the basic feed material to be consumed by an animal. It will be further understood that this may comprise, for example, at least one or more unprocessed grains, and/or processed plant and/or animal material such as soybean meal or bone meal.

As used herein, the term "feedstuff" refers to a feed material to which one or more feed additive compositions have been added.

It will be understood by the skilled person that different animals require different feedstuffs, and even the same animal may require different feedstuffs, depending upon the purpose for which the animal is reared.

Preferably, the feedstuff may comprise feed materials comprising maize or corn, wheat, barley, triticale, rye, rice, tapioca, sorghum, and/ or any of the by-products, as well as protein rich components like soybean mean, rape seed meal, canola (rapeseed) meal, cotton seed meal, sunflower seed mean, animal-by-product meals and mixtures thereof. More preferably, the feedstuff may comprise animal fats and / or vegetable oils.

Optionally, the feedstuff may also contain additional minerals such as, for example, calcium and/or additional vitamins.

Preferably, the feedstuff is a corn soybean meal mix.

In another aspect there is provided a method of preparing a feedstuff comprising contacting a feed component with a feed additive composition or feed ingredient of the invention or a premix of the invention or at least one proline tolerant tripeptidyl peptidase predominantly having exopeptidase activity wherein said proline tolerant tripeptidyl peptidase is capable of cleaving tri-peptides from the N-terminus of peptides having:

(i)

(A) Proline at P1; and

(B) An amino acid selected from alanine, arginine, asparagine, aspartic acid, cysteine, glutamine, glutamic acid, glycine, histidine, isoleucine, leucine, lysine, methionine, phenylalanine, serine, threonine, tryptophan, tyrosine, valine or synthetic amino acids at P1; and/or

(ii)

(a') Proline at P1'; and

(b') An amino acid selected from alanine, arginine, asparagine, aspartic acid, cysteine, glutamine, glutamic acid, glycine, histidine, isoleucine, leucine, lysine, methionine, phenylalanine, serine, threonine, tryptophan, tyrosine, valine or synthetic amino acids at P1'

optionally in combination with at least one endoprotease preferably wherein the endoprotease and/or proline tolerant tripeptidyl peptidase are inactive (or substantially inactive) in the feedstuff prior to feeding the feedstuff to an animal, wherein the at least one proline tolerant tripeptidyl peptidase:

(a) comprises the amino acid sequence SEQ ID No. 29, SEQ ID No. 1, SEQ ID No. 2 or a functional fragment thereof that retains said peptidase activity;

(b) comprises an amino acid having at least 80% or at least 90% identity to SEQ ID No. 29, SEQ ID No. 1, SEQ ID No. 2 or a functional fragment thereof that retains said peptidase activity;

(c) is encoded by a nucleotide sequence comprising the sequence SEQ ID No. 56;

(d) is encoded by a nucleotide sequence comprising at least 80% or at least 90% sequence identity to SEQ ID No. 56;

(e) is encoded by a nucleotide sequence which hybridises to SEQ ID No. 56 under high stringency conditions; or

(f) is encoded by a nucleotide sequence which differs from SEQ ID No. 56 due to degeneracy of the genetic code.

There is also provided a feedstuff comprising a feed additive composition or feed ingredient of the invention or a premix of the invention.

Feedstuff is typically produced in feed mills in which raw materials are first ground to a suitable particle size and then mixed with appropriate additives. The feedstuff may then be produced as a mash or pellets; the later typically involves a method by which the temperature is raised to a target level and then the feed is passed through a die to produce pellets of a particular size. The pellets are allowed to cool. Subsequently liquid additives such as fat and enzyme may be added. Production of feedstuff may also involve an additional step that includes extrusion or expansion prior to pelleting - in particular by suitable techniques that may include at least the use of steam.

The feedstuff may be a feedstuff for a monogastric animal, such as poultry (for example, broiler, layer, broiler breeders, turkey, duck, geese, water fowl), swine (all age categories), a pet (for example dogs, cats) or fish, preferably the feedstuff is for poultry.

By way of example only a feedstuff for chickens, e.g. broiler chickens may be comprises of one or more of the ingredients listed in the table below, for example in the %ages given in the table below:

| Ingredients | Starter (%) | Finisher (%) |
|---|---|---|
| Maize | 46.2 | 46.7 |
| Wheat Middlings | 6.7 | 10.0 |
| Maize DDGS | 7.0 | 7.0 |
| Soyabean Meal 48%CP | 32.8 | 26.2 |
| An/Veg Fat blend | 3.0 | 5.8 |
| L-Lysine HCl | 0.3 | 0.3 |
| DL-methionine | 0.3 | 0.3 |
| L-threonine | 0.1 | 0.1 |
| Salt | 0.3 | 0.4 |
| Limestone | 1.1 | 1.1 |
| Dicalcium Phosphate | 1.2 | 1.2 |

(continued)

| Ingredients | Starter (%) | Finisher (%) |
|---|---|---|
| Poultry Vitamins and Micro-minerals | 0.3 | 0.3 |

By way of example only the diet specification for chickens, such as broiler chickens, may be as set out in the Table below:

| Diet specification | | |
|---|---|---|
| Crude Protein (%) | 23.00 | 20.40 |
| Metabolizable Energy Poultry (kcal/kg) | 2950 | 3100 |
| Calcium (%) | 0.85 | 0.85 |
| Available Phosphorus (%) | 0.38 | 0.38 |
| Sodium (%) | 0.18 | 0.19 |
| Dig. Lysine (%) | 1.21 | 1.07 |
| Dig. Methionine (%) | 0.62 | 0.57 |
| Dig. Methionine + Cysteine (%) | 0.86 | 0.78 |
| Dig. Threonine (%) | 0.76 | 0.68 |

By way of example only a feedstuff laying hens may be comprises of one or more of the ingredients listed in the table below, for example in the %ages given in the table below:

| Ingredient | Laying phase (%) |
|---|---|
| Maize | 10.0 |
| Wheat | 53.6 |
| Maize DDGS | 5.0 |
| Soybean Meal 48%CP | 14.9 |
| Wheat Middlings | 3.0 |
| Soybean Oil | 1.8 |
| L-Lysine HCl | 0.2 |
| DL-methionine | 0.2 |
| L-threonine | 0.1 |
| Salt | 0.3 |
| Dicalcium Phosphate | 1.6 |
| Limestone | 8.9 |
| Poultry Vitamins and Micro-minerals | 0.6 |

By way of example only the diet specification for laying hens may be as set out in the Table below:

| Diet specification | |
|---|---|
| Crude Protein (%) | 16.10 |
| Metabolizable Energy Poultry (kcal/kg) | 2700 |
| Lysine (%) | 0.85 |

(continued)

| Diet specification | |
|---|---|
| Methionine (%) | 0.42 |
| Methionine + Cysteine (%) | 0.71 |
| Threonine (%) | 0.60 |
| Calcium (%) | 3.85 |
| Available Phosphorus (%) | 0.42 |
| Sodium (%) | 0.16 |

By way of example only a feedstuff for turkeys may be comprises of one or more of the ingredients listed in the table below, for example in the %ages given in the table below:

| Ingredient | Phase 1 (%) | Phase 2 (%) | Phase 3 (%) | Phase 4 (%) |
|---|---|---|---|---|
| Wheat | 33.6 | 42.3 | 52.4 | 61.6 |
| Maize DDGS | 7.0 | 7.0 | 7.0 | 7.0 |
| Soyabean Meal 48%CP | 44.6 | 36.6 | 27.2 | 19.2 |
| Rapeseed Meal | 4.0 | 4.0 | 4.0 | 4.0 |
| Soyabean Oil | 4.4 | 4.2 | 3.9 | 3.6 |
| L-Lysine HCl | 0.5 | 0.5 | 0.4 | 0.4 |
| DL-methionine | 0.4 | 0.4 | 0.3 | 0.2 |
| L-threonine | 0.2 | 0.2 | 0.1 | 0.1 |
| Salt | 0.3 | 0.3 | 0.3 | 0.3 |
| Limestone | 1.0 | 1.1 | 1.1 | 1.0 |
| Dicalcium Phosphate | 3.5 | 3.0 | 2.7 | 2.0 |
| Poultry Vitamins and Micro-minerals | 0.4 | 0.4 | 0.4 | 0.4 |

By way of example only the diet specification for turkeys may be as set out in the Table below:

| Diet specification | | | | |
|---|---|---|---|---|
| Crude Protein (%) | 29.35 | 26.37 | 22.93 | 20.00 |
| Metabolizable Energy Poultry (kcal/kg) | 2.850 | 2.900 | 2.950 | 3.001 |
| Calcium (%) | 1.43 | 1.33 | 1.22 | 1.02 |
| Available Phosphorus (%) | 0.80 | 0.71 | 0.65 | 0.53 |
| Sodium (%) | 0.16 | 0.17 | 0.17 | 0.17 |
| Dig. Lysine (%) | 1.77 | 1.53 | 1.27 | 1.04 |
| Dig. Methionine (%) | 0.79 | 0.71 | 0.62 | 0.48 |
| Dig. Methionine + Cysteine (%) | 1.12 | 1.02 | 0.90 | 0.74 |
| Dig. Threonine (%) | 1.03 | 0.89 | 0.73 | 0.59 |

By way of example only a feedstuff for piglets may be comprises of one or more of the ingredients listed in the table below, for example in the %ages given in the table below:

| Ingredient | Phase 1 (%) | Phase 2 (%) |
|---|---|---|
| Maize | 20.0 | 7.0 |
| Wheat | 25.9 | 46.6 |
| Rye | 4.0 | 10.0 |
| Wheat middlings | 4.0 | 4.0 |
| Maize DDGS | 6.0 | 8.0 |
| Soyabean Meal 48% CP | 25.7 | 19.9 |
| Dried Whey | 10.0 | 0.0 |
| Soyabean Oil | 1.0 | 0.7 |
| L-Lysine HCl | 0.4 | 0.5 |
| DL-methionine | 0.2 | 0.2 |
| L-threonine | 0.1 | 0.2 |
| L-tryptophan | 0.03 | 0.04 |
| Limestone | 0.6 | 0.7 |
| Dicalcium Phosphate | 1.6 | 1.6 |
| Swine Vitamins and Micro-minerals | 0.2 | 0.2 |
| Salt | 0.2 | 0.4 |

By way of example only the diet specification for piglets may be as set out in the Table below:

| Diet specification | | |
|---|---|---|
| Crude Protein (%) | 21.50 | 20.00 |
| Swine Digestible Energy (kcal/kg) | 3380 | 3320 |
| Swine Net Energy (kcal/kg) | 2270 | 2230 |
| Calcium (%) | 0.80 | 0.75 |
| Digestible Phosphorus (%) | 0.40 | 0.35 |
| Sodium (%) | 0.20 | 0.20 |
| Dig. Lysine (%) | 1.23 | 1.14 |
| Dig. Methionine (%) | 0.49 | 0.44 |
| Dig. Methionine + Cysteine (%) | 0.74 | 0.68 |
| Dig. Threonine (%) | 0.80 | 0.74 |

By way of example only a feedstuff for grower/finisher pigs may be comprises of one or more of the ingredients listed in the table below, for example in the %ages given in the table below:

| Ingredient | Grower/ Finisher (%) |
|---|---|
| Maize | 27.5 |
| Soyabean Meal 48% CP | 15.4 |
| Maize DDGS | 20.0 |
| Wheat bran | 11.1 |
| Rice bran | 12.0 |

(continued)

| Ingredient | Grower/ Finisher (%) |
|---|---|
| Canola seed meal | 10.0 |
| Limestone | 1.6 |
| Dicalcium phosphate | 0.01 |
| Salt | 0.4 |
| Swine Vitamins and Micro-minerals | 0.3 |
| Lysine-HCl | 0.2 |
| Vegetable oil | 0.5 |

By way of example only the diet specification for grower/finisher pigs may be as set out in the Table below:

| Diet specification | |
|---|---|
| Crude Protein (%) | 22.60 |
| Swine Metabolizable Energy (kcal/kg) | 3030 |
| Calcium (%) | 0.75 |
| Available Phosphorus (%) | 0.29 |
| Digestible Lysine (%) | 1.01 |
| Dig. Methionine + Cysteine (%) | 0.73 |
| Digestible Threonine (%) | 0.66 |

## FORMS

The feed additive composition of the present invention and other components and/or the feedstuff comprising same may be used in any suitable form.

The feed additive composition of the present invention may be used in the form of solid or liquid preparations or alternatives thereof. Examples of solid preparations include powders, pastes, boluses, capsules, pellets, tablets, dusts, and granules which may be wettable, spray-dried or freeze-dried. Examples of liquid preparations include, but are not limited to, aqueous, organic or aqueous-organic solutions, suspensions and emulsions.

In some applications, feed additive composition of the present invention may be mixed with feed or administered in the drinking water.

Suitable examples of forms include one or more of: powders, pastes, boluses, pellets, tablets, pills, granules, capsules, ovules, solutions or suspensions, which may contain flavouring or colouring agents, for immediate-, delayed-, modified-, sustained-, pulsed- or controlled-release applications.

By way of example, if the composition of the present invention is used in a solid, e.g. pelleted form, it may also contain one or more of: excipients such as microcrystalline cellulose, lactose, sodium citrate, calcium carbonate, dibasic calcium phosphate and glycine; disintegrants such as starch (preferably corn, potato or tapioca starch), sodium starch glycollate, croscarmellose sodium and certain complex silicates; granulation binders such as polyvinylpyrrolidone, hydroxypropylmethylcellulose (HPMC), hydroxypropylcellulose (HPC), sucrose, gelatin and acacia; lubricating agents such as magnesium stearate, stearic acid, glyceryl behenate and talc may be included.

Examples of nutritionally acceptable carriers for use in preparing the forms include, for example, water, salt solutions, alcohol, silicone, waxes, petroleum jelly, vegetable oils, polyethylene glycols, propylene glycol, liposomes, sugars, gelatin, lactose, amylose, magnesium stearate, talc, surfactants, silicic acid, viscous paraffin, perfume oil, fatty acid monoglycerides and diglycerides, petroethral fatty acid esters, hydroxymethyl-cellulose, polyvinylpyrrolidone, and the like.

Preferred excipients for the forms include lactose, starch, a cellulose, milk sugar or high molecular weight polyethylene glycols.

For aqueous suspensions and/or elixirs, the composition of the present invention may be combined with various sweetening or flavouring agents, colouring matter or dyes, with emulsifying and/or suspending agents and with diluents such as water, propylene glycol and glycerin, and combinations thereof.

## COMBINATION WITH OTHER COMPONENTS

The feed additive composition, or feed ingredient, or feed or feedstuff or premix of the present invention may be used in combination with other components.

The combination of the present invention feed additive composition, or feed ingredient, or feed or feedstuff or premix of the present invention and another component which is suitable for animal consumption and is capable of providing a medical or physiological benefit to the consumer.

In one embodiment the "another component" may be one or more enzymes.

Suitable additional enzymes for use in the present invention may be one or more of the enzymes selected from the group consisting of: endoglucanases (E.C. 3.2.1.4); celliobiohydrolases (E.C. 3.2.1.91), β-glucosidases (E.C. 3.2.1.21), cellulases (E.C. 3.2.1.74), lichenases (E.C. 3.1.1.73), lipases (E.C. 3.1.1.3), lipid acyltransferases (generally classified as E.C. 2.3.1.x), phospholipases (E.C. 3.1.1.4, E.C. 3.1.1.32 or E.C. 3.1.1.5), phytases (e.g. 6-phytase (E.C. 3.1.3.26) or a 3-phytase (E.C. 3.1.3.8), alpha-amylases (E.C. 3.2.1.1), xylanases (E.C. 3.2.1.8, E.C. 3.2.1.32, E.C. 3.2.1.37, E.C. 3.1.1.72, E.C. 3.1.1.73), glucoamylases (E.C. 3.2.1.3), proteases (e.g. subtilisin (E.C. 3.4.21.62) or a bacillolysin (E.C. 3.4.24.28) or an alkaline serine protease (E.C. 3.4.21.x) or a keratinase (E.C. 3.4.x.x)) and/or mannanases (e.g. a β-mannanase (E.C. 3.2.1.78)).

Suitably the other component may be a phytase (e.g. a 6-phytase (E.C. 3.1.3.26) or a 3-phytase (E.C. 3.1.3.8)).

In one embodiment (particularly for feed applications) the other component may be one or more of the enzymes selected from the group consisting of xylanases (E.C. 3.2.1.8, E.C. 3.2.1.32, E.C. 3.2.1.37, E.C. 3.1.1.72, E.C. 3.1.1.73), an amylase (including α-amylases (E.C. 3.2.1.1), G4-forming amylases (E.C. 3.2.1.60), β-amylases (E.C. 3.2.1.2) and γ-amylases (E.C. 3.2.1.3); and/or a protease (e.g. subtilisin (E.C. 3.4.21.62) or a bacillolysin (E.C. 3.4.24.28) or an alkaline serine protease (E.C. 3.4.21.x) or a keratinase (E.C. 3.4.x.x)). In one embodiment (particularly for feed applications) the other component may be a combination of an amylase (e.g. α-amylases (E.C. 3.2.1.1)) and a protease (e.g. subtilisin (E.C. 3.4.21.62)).

In one embodiment (particularly for feed applications) the other component may be a β-glucanase, e.g. an endo-1,3(4)-β-glucanases (E.C. 3.2.1.6).

In one embodiment (particularly for feed applications) the other component may be a mannanases (e.g. a β-mannanase (E.C. 3.2.1.78)).

In one embodiment (particularly for feed applications) the other component may be a lipase (E.C. 3.1.1.3), a lipid acyltransferase (generally classified as E.C. 2.3.1.x), or a phospholipase (E.C. 3.1.1.4, E.C. 3.1.1.32 or E.C. 3.1.1.5), suitably a lipase (E.C. 3.1.1.3).

In one embodiment (particularly for feed applications) the other component may be a protease (e.g. subtilisin (E.C. 3.4.21.62) or a bacillolysin (E.C. 3.4.24.28) or an alkaline serine protease (E.C. 3.4.21.x) or a keratinase (E.C. 3.4.x.x)).

In another embodiment the other component may be a further protease. Suitably, the further protease may be selected from the group consisting of: an aminopeptidase, and a carboxypeptidase.

The term "aminopeptidase" as used in this context refers to an exopeptidase which is able to cleave single amino acids, di-amino acids or combinations thereof from the N-terminus of a protein and/or peptide substrate. Preferably, an aminopeptidase is able to cleave single amino acids only from the N-terminus of a protein and/or peptide substrate.

The aminopeptidase may be obtainable (e.g. obtained) from *Lactobacillus,* suitably obtainable from *Lactobacillus helveticus.*

In one embodiment the aminopeptidase may be an aminopeptidase N (e.g. PepN) (EC 3.4.11.2).

In one embodiment the aminopeptidase may comprise the sequence shown as:

MAVKRFYKTFHPEHYDLRINVNRKNKTINGTSTITGDVIENPVFINQKFM

TIDSVKVDGKNVDFDVIEKDEAIKIKTGVTGKAVIEIAYSAPLTDTMMGI

YPSYYELEGKKKQIIGTQFETTFARQAFPCVDEPEAKATFSLALKWDEQD

GEVALANMPEVEVDKDGYHHFEETVRMSSYLVAFAFGELQSKTTHTKDGV

LIGVYATKAHKPKELDFALDIAKRAIEFYEEFYQTKYPLPQSLQLALPDF

SAGAMENWGLVTYREAYLLLDPDNTSLEMKKLVATVITHELAHQWFGDLV

TMKWWDNLWLNESFANMMEYLSVDGLEPDWHIWEMFQTSEAASALNRDAT

DGVQPIQMEINDPADIDSVFDGAIVYAKGSRMLVMVRSLLGDDALRKGLK

YYFDHHKFGNATGDDLWDALSTATDLDIGKIMHSWLKQPGYPVVNAFVAE

DGHLKLTQKQFFIGEGEDKGRQWQIPLNANFDAPKIMSDKEIDLGNYKVL

REEAGHPLRLNVGNNSHFIVEYDKTLLDDILSDVNELDPIDKLQLLQDLR

LLAEGKQISYASIVPLLVKFADSKSSLVINALYTTAAKLRQFVEPESNEE

KNLKKLYDLLSKDQVARLGWEVKPGESDEDVQIRPYELSASLYAENADSI

KAAHQIFTENEDNLEALNADIRPYVLINEVKNFGNAELVDKLIKEYQRTA

DPSYKVDLRSAVTSTKDLAAIKAIVGDFENADVVKPQDLCDWYRGLLANH

YGQQAAWDWIREDWDWLDKTVGGDMEFAKFITVTAGVFHTPERLKEFKEF

FEPKINVPLLSREIKMDVKVIESKVNLIEAEKDAVNDAVAKAID

The term "carboxypeptidase" as used herein has its usual meaning in the art and refers to an exopeptidase that is capable of cleaving *n* amino acids from the C-terminus of a peptide and/or protein substrate. In one embodiment *n* may be at least 1, suitably *n* may be at least 2. In other embodiments *n* may be at least 3, suitably at least 4.

In other embodiments, the proline tolerant tripeptidyl peptidase (optionally in combination with an endoprotease) may be used with one or more further exopeptidase In one embodiment the proline tolerant tripeptidyl peptidase (optionally in combination with an endoprotease) is not combined with a proline-specific exopeptidase.

In a particularly preferred embodiment the proline tolerant tripeptidyl peptidase may not be combined with an enzyme having the following polypeptide sequence:

MRTAAASLTLAATCLFELASALMPRAPLIPAMKAKVALPSGNATFEQYIDHNNPGLG

TFPQRYWYNPEFWAGPGSPVLLFTPGESDAADYDGFLTNKTIVGRFAEEIGGAVILLE

HRYWGASSPYPELTTETLQYLTLEQSIADLVHFAKTVNLPFDEIHSSNADNAPWVMT

GGSYSGALAAWTASIAPGTFWAYHASSAPVQAIYDFWQYFVPVVEGMPKNCSKDL

NRVVEYIDHVYESGDIERQQEIKEMFGLGALKHFDDFAAAITNGPWLWQDMNFVSG

YSRFYKFCDAVENVTPGAKSVPGPEGVGLEKALQGYASWFNSTYLPGSCAEYKYW

TDKDAVDCYDSYETNSPIYTDKAVNNTSNKQWTWFLCNEPLFYWQDGAPKDEST

IVSRIVSAEYWQRQCHAYFPEVNGYTFGSANGKTAEDVNKWTKGWDLTNTTRLIW

ANGQFDPWRDASVSSKTRPGGPLQSTEQAPVHVIPGGFHCSDQWLVYGEANAGVQ

KVIDEEVAQIKAWVAEYPKYRKP

In one embodiment the additional component may be a stabiliser or an emulsifier or a binder or carrier or an excipient or a diluent or a disintegrant.

The term "stabiliser" as used here is defined as an ingredient or combination of ingredients that keeps a product (e.g. a feed product) from changing over time.

The term "emulsifier" as used herein refers to an ingredient (e.g. a feed ingredient) that prevents the separation of emulsions. Emulsions are two immiscible substances, one present in droplet form, contained within the other. Emulsions can consist of oil-in-water, where the droplet or dispersed phase is oil and the continuous phase is water; or water-in-oil, where the water becomes the dispersed phase and the continuous phase is oil. Foams, which are gas-in-liquid, and suspensions, which are solid-in-liquid, can also be stabilised through the use of emulsifiers.

As used herein the term "binder" refers to an ingredient (e.g. a feed ingredient) that binds the product together through a physical or chemical reaction. During "gelation" for instance, water is absorbed, providing a binding effect.

However, binders can absorb other liquids, such as oils, holding them within the product. In the context of the present invention binders would typically be used in solid or low-moisture products for instance baking products: pastries, doughnuts, bread and others. Examples of granulation binders include one or more of: polyvinylpyrrolidone, hydroxypropylmethylcellulose (HPMC), hydroxypropylcellulose (HPC), sucrose, maltose, gelatin and acacia.

"Carriers" mean materials suitable for administration of the enzyme and include any such material known in the art such as, for example, any liquid, gel, solvent, liquid diluent, solubilizer, or the like, which is non-toxic and which does not interact with any components of the composition in a deleterious manner.

In one embodiment, the present invention provides the use of a composition (e.g. a feed additive composition) comprising an enzyme of the present invention formulated with at least one physiologically acceptable carrier selected from at least one of maltodextrin, limestone (calcium carbonate), cyclodextrin, wheat or a wheat component, sucrose, starch, $Na_2SO_4$, Talc, polyvinyl alcohol (PVA), sorbitol, benzoate, sorbiate, glycerol, sucrose, propylene glycol, 1,3-propane diol, glucose, parabens, sodium chloride, citrate, acetate, phosphate, calcium, metabisulfite, formate and mixtures thereof.

In a preferred embodiment the present invention provides a composition (e.g. a feed additive composition) or the use thereof and methods of making the same comprising an enzyme of the present invention formulated with a compound selected from one or more of the group consisting of a salt, polyol including sorbitol and glycerol, wheat or a wheat component, sodium acetate, sodium acetate trihydrate, potassium sorbate Talc, polyvinyl alcohol (PVA), benzoate, sorbiate, 1,3-propane diol, glucose, parabens, sodium chloride, citrate, metabisulfite, formate or a combination thereof.

Examples of "excipients" include one or more of: microcrystalline cellulose and other celluloses, lactose, sodium citrate, calcium carbonate, dibasic calcium phosphate, glycine, starch, milk sugar and high molecular weight polyethylene glycols.

Examples of "disintegrants" include one or more of: starch (preferably corn, potato or tapioca starch), sodium starch glycollate, croscarmellose sodium and certain complex silicates. Examples of "diluents" include one or more of: water, ethanol, propylene glycol and glycerin, and combinations thereof.

The other components may be used simultaneously (e.g. when they are in admixture together or even when they are delivered by different routes) or sequentially (e.g. they may be delivered by different routes) to the feed additive of the present invention.

In one embodiment preferably the feed additive composition, or feed ingredient, or feed or feedstuff or premix according to the present invention does not comprise chromium or organic chromium.

In one embodiment preferably the feed additive composition, or feed ingredient, or feed or feedstuff or premix according to the present invention does not contain sorbic acid.

## BIOPHYSICAL CHARACTERISTIC

In one aspect there is provided a method for improving a biophysical characteristic of an animal or for improving protein digestibility of an animal which method comprises administering to an animal a feed additive composition, feedstuff or premix of the invention or at least one proline tolerant tripeptidyl peptidase predominantly having exopeptidase activity wherein said proline tolerant tripeptidyl peptidase is capable of cleaving tri-peptides from the N-terminus of peptides having:

(i)

(A) Proline at P1; and
(B) An amino acid selected from alanine, arginine, asparagine, aspartic acid, cysteine, glutamine, glutamic acid, glycine, histidine, isoleucine, leucine, lysine, methionine, phenylalanine, serine, threonine, tryptophan, tyrosine, valine or synthetic amino acids at P1; and/or

(ii)

(a') Proline at P1'; and
(b') An amino acid selected from alanine, arginine, asparagine, aspartic acid, cysteine, glutamine, glutamic acid, glycine, histidine, isoleucine, leucine, lysine, methionine, phenylalanine, serine, threonine, tryptophan, tyrosine, valine or synthetic amino acids at P1'; and

optionally at least one feed component and/or at least one mineral and/or at least one vitamin, preferably wherein the method comprises administering to an animal at least one endoprotease, wherein the at least one proline tolerant tripeptidyl peptidase:

(a) comprises the amino acid sequence SEQ ID No. 29, SEQ ID No. 1, SEQ ID No. 2 or a functional fragment thereof that retains said peptidase activity;

(b) comprises an amino acid having at least 80% or at least 90% identity to SEQ ID No. 29, SEQ ID No. 1, SEQ ID No. 2 or a functional fragment thereof that retains said peptidase activity;

(c) is encoded by a nucleotide sequence comprising the sequence SEQ ID No. 56;

(d) is encoded by a nucleotide sequence comprising at least 80% or at least 90% sequence identity to SEQ ID No. 56;

(e) is encoded by a nucleotide sequence which hybridises to SEQ ID No. 56 under high stringency conditions; or

(f) is encoded by a nucleotide sequence which differs from SEQ ID No. 56 due to degeneracy of the genetic code.

The term "administering" as used herein may mean feeding the animal the proline tolerant tripeptidyl peptidase or said feed additive composition either before, after or simultaneously with a feedstuff (e.g. the animals usual diet). Alternatively the term "administering" as used herein may mean feeding the animal with a feedstuff or premix comprising said feed additive composition.

Suitably the the at least one proline tolerant tripeptidyl peptidase may be capable of cleaving tri-peptides from the N-terminus of peptides having:

(i)

(A) Proline at P1; and
(B) An amino acid selected from alanine, arginine, asparagine, aspartic acid, cysteine, glutamine, glutamic acid, glycine, histidine, isoleucine, leucine, lysine, methionine, phenylalanine, serine, threonine, tryptophan, tyrosine, valine or synthetic amino acids at P1; and

(ii)

(a') Proline at P1'; and
(b') An amino acid selected from alanine, arginine, asparagine, aspartic acid, cysteine, glutamine, glutamic acid, glycine, histidine, isoleucine, leucine, lysine, methionine, phenylalanine, serine, threonine, tryptophan, tyrosine, valine or synthetic amino acids at P1'.

In another aspect there is provided the use of a feed additive composition or feed ingredient of the invention or a feed feedstuff or premix of the invention or at least one proline tolerant tripeptidyl peptidase predominantly having exopeptidase activity wherein said proline tolerant tripeptidyl peptidase is capable of cleaving tri-peptides from the N-terminus of peptides having:

(i)

(A) Proline at P1; and
(B) An amino acid selected from alanine, arginine, asparagine, aspartic acid, cysteine, glutamine, glutamic acid, glycine, histidine, isoleucine, leucine, lysine, methionine, phenylalanine, serine, threonine, tryptophan, tyrosine, valine or synthetic amino acids at P1; and/or

(ii)

(a') Proline at P1'; and
(b') An amino acid selected from alanine, arginine, asparagine, aspartic acid, cysteine, glutamine, glutamic acid, glycine, histidine, isoleucine, leucine, lysine, methionine, phenylalanine, serine, threonine, tryptophan, tyrosine, valine or synthetic amino acids at P1'

for improving protein digestibility in an animal or for improving a biophysical characteristic of an animal, preferably wherein at least one endoprotease is used in combination, wherein the at least one proline tolerant tripeptidyl peptidase:

(a) comprises the amino acid sequence SEQ ID No. 29, SEQ ID No. 1, SEQ ID No. 2 or a functional fragment thereof that retains said peptidase activity;

(b) comprises an amino acid having at least 80% or at least 90% identity to SEQ ID No. 29, SEQ ID No. 1, SEQ ID No. 2 or a functional fragment thereof that retains said peptidase activity;

(c) is encoded by a nucleotide sequence comprising the sequence SEQ ID No. 56;

(d) is encoded by a nucleotide sequence comprising at least 80% or at least 90% sequence identity to SEQ ID No. 56;

(e) is encoded by a nucleotide sequence which hybridises to SEQ ID No. 56 under high stringency conditions; or

(f) is encoded by a nucleotide sequence which differs from SEQ ID No. 56 due to degeneracy of the genetic code.

Suitably the method and/or use may further comprising administering to an animal at least one feed component, at least one mineral, at least one vitamin or combinations thereof. Alternatively or additionally the method and/or use may further comprise administering to an animal at least one endoprotease.

As used herein, "biophysical characteristic" means any biophysical property of an animal which improves its health and/or performance and/or output.

By way of example, the biophysical characteristic may be one or more selected from the group consisting of one or more of the following: performance of the animal, growth performance of an animal, feed conversion ratio (FCR), ability to digest a raw material (e.g. nutrient digestibility, including starch , fat, protein, fibre digestibility), nitrogen digestibility (e.g. ileal nitrogen digestibility) and digestible energy (e.g. ileal digestible energy), nitrogen retention, carcass yield, growth rate, weight gain, body weight, mass, feed efficiency, body fat percentage, body fat distribution, growth, egg size, egg weight, egg mass, egg laying rate, lean gain, bone ash %, bone ash mg, back fat %, milk output, milk fat %, reproductive outputs such as litter size, litter survivability, hatchability % and environmental impact, e.g. manure output and/or nitrogen excretion.

Suitably the biophysical characteristic may be one or more selected from the group consisting of: feed conversion ratio, nitrogen digestibility (e.g. ileal nitrogen digestibility) and digestible energy (e.g. ileal digestible energy).

In a preferred embodiment the biophysical characteristic may be the ability to digest a protein.

In one embodiment the biophysical characteristic of the animal means the performance of the animal.

Suitably, administering to an animal a feed additive composition and/or feed and/or feedstuff and/or feed ingredient and/or premix of the invention may not substantially increase the incidence of necrotic enteritis in the animal when compared to an animal not fed with the feed additive composition and/or feed and/or feedstuff and/or feed ingredient and/or premix of the invention.

The term "substantially increase the incidence of necrotic enteritis" as used herein means that the incidence is not increased by more than about 20%, suitably not increased by more than about 10%. Preferably it is meant that the incidence of necrotic enteritis is not increased by more than about 5%, more preferably more than about 1%.

## PERFORMANCE

As used herein, "performance of the animal" may be determined by the feed efficiency and/or weight gain of the animal and/or by the feed conversion ratio and/or by the digestibility of a nutrient in a feed (e.g. amino acid digestibility) and/or digestible energy or metabolizable energy in a feed and/or by nitrogen retention.

Preferably "performance of the animal" is determined by feed efficiency and/or weight gain of the animal and/or by the feed conversion ratio.

By "improved performance of the animal" it is meant that there is increased feed efficiency, and/or increased weight gain and/or reduced feed conversion ratio and/or improved digestibility of nutrients or energy in a feed and/or by improved nitrogen retention in the subject resulting from the use of feed additive composition of the present invention compared with feeding the animal a feed composition which does not contain the proline tolerant tripeptidyl peptidase in accordance with the present invention.

Preferably, by "improved animal performance" it is meant that there is increased feed efficiency and/or increased weight gain and/or reduced feed conversion ratio.

As used herein, the term "feed efficiency" refers to the amount of weight gain in an animal that occurs when the animal is fed ad-libitum or a specified amount of food during a period of time.

By "increased feed efficiency" it is meant that the use of a feed additive composition according the present invention in feed results in an increased weight gain per unit of feed intake compared with an animal fed with the feed composition which does not contain the proline tolerant tripeptidyl peptidase in accordance with the present invention.

## FEED CONVERSION RATIO (FCR)

As used herein, the term "feed conversion ratio" refers to the amount of feed fed to an animal to increase the weight of the animal by a specified amount.

An improved feed conversion ratio means a lower feed conversion ratio.

By "lower feed conversion ratio" or "improved feed conversion ratio" it is meant that the use of the proline tolerant tripeptidyl peptidase or a feed additive composition in accordance with the present invention in feed results in a lower amount of feed being required to be fed to an animal to increase the weight of the animal by a specified amount compared to the amount of feed required to increase the weight of the animal by the same amount without said proline tolerant tripeptidyl peptidase or without said feed additive composition in accordance with the present invention.

## NUTRIENT DIGESTIBILITY

Nutrient digestibility as used herein means the fraction of a nutrient that disappears from the gastro-intestinal tract or a specified segment of the gastrointestinal tract, e.g. the small intestine. Nutrient digestibility may be measured as the difference between what is administered to the subject and what comes out in the faeces of the subject, or between what is administered to the subject and what remains in the digesta on a specified segment of the gastro intestinal tract, e.g. the ileum.

Nutrient digestibility as used herein may be measured by the difference between the intake of a nutrient and the excreted nutrient by means of the total collection of excreta during a period of time; or with the use of an inert marker that is not absorbed by the animal, and allows the researcher calculating the amount of nutrient that disappeared in the entire gastro-intestinal tract or a segment of the gastro-intestinal tract. Such an inert marker may be titanium dioxide, chromic oxide or acid insoluble ash. Digestibility may be expressed as a percentage of the nutrient in the feed, or as mass units of digestible nutrient per mass units of nutrient in the feed.

Nutrient digestibility as used herein encompasses starch digestibility, fat digestibility, protein digestibility, and amino acid digestibility.

Suitably use of a proline tolerant tripeptidyl peptidase according to the methods and/or uses or any of the aspects of the present invention (optionally in combination with at least one endoprotease) increases protein and/or amino acid digestibility in an animal fed with the feed additive composition and/or feed ingredient and/or feed and/or feedstuff and/or premix of the invention.

Energy digestibility as used herein means the gross energy of the feed consumed minus the gross energy of the faeces or the gross energy of the feed consumed minus the gross energy of the remaining digesta on a specified segment of the gastro-intestinal tract of the animal, e.g. the ileum. Metabolizable energy as used herein refers to apparent metabolizable energy and means the gross energy of the feed consumed minus the gross energy contained in the faeces, urine, and gaseous products of digestion. Energy digestibility and metabolizable energy may be measured as the difference between the intake of gross energy and the gross energy excreted in the faeces or the digesta present in specified segment of the gastro-intestinal tract using the same methods to measure the digestibility of nutrients, with appropriate corrections for nitrogen excretion to calculate metabolizable energy of feed.

## NITROGEN RETENTION

Nitrogen retention as used herein means as subject's ability to retain nitrogen from the diet as body mass. A negative nitrogen balance occurs when the excretion of nitrogen exceeds the daily intake and is often seen when the muscle is being lost. A positive nitrogen balance is often associated with muscle growth, particularly in growing animals.

Nitrogen retention may be measured as the difference between the intake of nitrogen and the excreted nitrogen by means of the total collection of excreta and urine during a period of time. It is understood that excreted nitrogen includes undigested protein from the feed, endogenous proteinaceous secretions, microbial protein, and urinary nitrogen.

## CARCASS YIELD AND MEAT YIELD

The term carcass yield as used herein means the amount of carcass as a proportion of the live body weight, after a commercial or experimental process of slaughter. The term carcass means the body of an animal that has been slaughtered for food, with the head, entrails, part of the limbs, and feathers or skin removed. The term meat yield as used herein means the amount of edible meat as a proportion of the live body weight, or the amount of a specified meat cut as a proportion of the live body weight.

## WEIGHT GAIN

The present invention further provides a method of increasing weight gain in a subject, e.g. poultry or swine, comprising feeding said subject a feedstuff comprising a feed additive composition according to the present invention.

An "increased weight gain" refers to an animal having increased body weight on being fed feed comprising a proline tolerant tripeptidyl peptidase or feed additive composition according to the present invention compared with an animal being fed a feed not comprising said proline tolerant tripeptidyl peptidase or said feed additive composition according to the present invention.

## ADVANTAGES

The inventors have shown for the first time that such a proline tolerant tripeptidyl peptidase is highly advantageous for use in feed and feedstuffs and confers advantages to an animal fed the proline tolerant tripeptidyl peptidase or a feed and/or feedstuff and/or feed additive composition comprising the same.

Advantageously, a proline tolerant tripeptidyl peptidase taught for use in the present invention is capable of acting on a wide range of peptide and/or protein substrates and due to having such a broad substrate-specificity is not readily inhibited from cleaving substrates enriched in certain amino acids (e.g. proline). The use of such a proline tolerant tripeptidyl peptidase therefore may efficiently and/or rapidly breakdown protein substrates (e.g. present in feed and/or feedstuffs). This confers the further advantage of efficiently and/or rapidly digesting a protein substrate *in situ* in an animal fed with such a protein substrate (e.g. as present in a feed or feedstuff) allowing rapid and/or efficient uptake of digested peptides by the animal.

Also disclosed are proline tolerant tripeptidyl peptidase that, in addition to having the activities described above, may be tolerant of proline at position P2, P2', P3 and P3'. This is advantageous as it allows the efficient cleavage of peptide and/or protein substrates having stretches of proline and allows cleavage of a wide range of peptide and/or protein substrates.

Also disclosed are thermostable proline tolerant tripeptidyl peptidases which are less prone to being denatured and/or will therefore retain activity for a longer period of time in e.g. an animal when compared to a non-thermostable variant.

The proline tolerant tripeptidyl peptidases herein may be active at an acid pH. Advantageously, a proline tolerant tripeptidyl peptidase having activity at an acidic pH can be active in the upper gastrointestinal tract of an animal (e.g. in the gizzard, proventriculus or stomach) and/or can digest a peptide and/or protein substrate in combination with endogenous proteases (e.g. pepsin) that are present in the gastrointestinal tract of the animal.

Many current feeding practices involve administering an alkaline protease active at a high pH (e.g. pH 8) to animals. Alkaline proteases are therefore only active lower down (e.g. later) in the gastrointestinal tract of an animal where the gastrointestinal tract becomes more alkaline, such as in the later part of the small intestine and the large intestine and caecum. Without wishing to be bound by theory, it is believed that producing oligopeptides in the later parts of the gastrointestinal tract increases populations of microbes which utilise the oligopeptides which in turn can lead to enteric disease challenges and/or reduced nutrients available for uptake by the animal. Additionally, later in the gastrointestinal tract (i.e. lower down) the mucosa is less well-protected than in the upper portions (e.g. the gizzard, proventriculus or stomach) and so is more easily damaged leading to inflammation. Advantageously, the use of a proline tolerant tripeptidyl peptidase having activity at an acid pH alleviates this problem as it is capable of digesting its substrate in the upper gastrointestinal tract thereby not substantially increasing populations of microbes and/or increasing the amount of nutrient (e.g. amino acids/peptides) available for uptake by an animal and/or reducing inflammation. Advantageously, the use of an endoprotease in combination with a proline tolerant tripeptidyl peptidase can increase the efficiency of substrate cleavage. Without wishing to be bound by theory, it is believed that an endoprotease is able to cleave a peptide and/or protein substrate at multiple regions away from the C or N-terminus, thereby producing more N-terminal ends for the proline tolerant tripeptidyl peptidase to use as a substrate, thereby advantageously increasing reaction efficiency and/or reducing reaction times.

The use of an acid endoprotease and a proline tolerant tripeptidyl peptidase having activity at an acid pH is highly advantageous as the two enzymes can co-operate to digest a peptide and/or protein substrate in the upper gastrointestinal tract (e.g. gizzard, proventriculus or stomach) of an animal and can be active in combination with other endogenous proteases (e.g. pepsin) present in the animal.

Advantageously feeding a proline tolerant tripeptidyl peptidase to an animal results in increased body weight gain and/or a reduction in feed conversion ratio and/or increased nitrogen digestibility (e.g. ileal nitrogen digestibility) and/or increased energy digestion (e.g. ileal energy digestion).

In one embodiment the compositions and proline tolerant tripeptidyl peptidase for use in the present invention has been shown to be less harmful to cells (e.g. of the GI tract) compared with conventional proteases used in feed applications. Without wishing to be bound by theory, as ATP plays a central role in cellular metabolism, it is present in all metabolically active cells, and its intracellular level is regulated precisely in healthy cells. ATP has been used as a tool for the functional integrity of living cells since all cells require ATP to remain alive and carry out their specialized function. Most ATP is found within living cells and links catabolic and anabolic processes. Cell injury or oxygen / substrate depletion results in a rapid decrease in cytoplasmic ATP. The decrease in ATP indicates often either an injury in mitochondria, that produce the ATP, or an increase in level of ATPases that degrade ATP. ATPases are located for instance in transporters that couple transportation of a specific molecules to the transportation. However, peptide transportation in the intestine usually is mediated by sodium cotransport, or the sodium binds to the transporter along the peptide. After binding, the sodium ion then moves down its electrochemical gradient to the interior of the cell and pulls the amino acid or peptide along with it. Of course, after the peptide transportation, the sodium would be transported against the concentration gradient back outside the cells by utilising the ATP energy, but normally this decrease in ATP is restored quite rapidly (Normal, healthy cells produce ATP as fast as they use it). Thus ATP content can be used as an indicator of healthy cells. The present inventors have surprisingly found that commercially available proteases uses in the feed industry have a negative effect on living cells. In contrast with the proline tolerant tripeptidyl peptidases for use in the present invention such a negative effect was not observed.

Likewise a decrease in tight junction integrity was observed with commercially available proteases uses in the feed industry, which again was not the case with the proline tolerant tripeptidyl peptidases for use in the present invention.

## ADDITIONAL DEFINITIONS

Unless defined otherwise, all technical and scientific terms used herein have the same meaning as commonly understood by one of ordinary skill in the art to which this disclosure belongs. Singleton, et al., DICTIONARY OF MICROBIOLOGY AND MOLECULAR BIOLOGY, 20 ED., John Wiley and Sons, New York (1994), and Hale & Marham, THE HARPER COLLINS DICTIONARY OF BIOLOGY, Harper Perennial, NY (1991) provide one of skill with a general dictionary of many of the terms used in this disclosure.

This disclosure is not limited by the exemplary methods and materials disclosed herein, and any methods and materials similar or equivalent to those described herein can be used in the practice or testing of embodiments of this disclosure. Numeric ranges are inclusive of the numbers defining the range. Unless otherwise indicated, any nucleic acid sequences are written left to right in 5' to 3' orientation; amino acid sequences are written left to right in amino to carboxy orientation, respectively.

The headings provided herein are not limitations of the various aspects or embodiments of this disclosure which can be had by reference to the specification as a whole. Accordingly, the terms defined immediately below are more fully defined by reference to the specification as a whole.

Amino acids are referred to herein using the name of the amino acid, the three letter abbreviation or the single letter abbreviation.

The term "protein", as used herein, includes proteins, polypeptides, and peptides.

As used herein, the term "amino acid sequence" is synonymous with the term "polypeptide" and/or the term "protein". In some instances, the term "amino acid sequence" is synonymous with the term "peptide". In some instances, the term "amino acid sequence" is synonymous with the term "enzyme".

The terms "protein" and "polypeptide" are used interchangeably herein. In the present disclosure and claims, the conventional one-letter and three-letter codes for amino acid residues may be used. The 3-letter code for amino acids as defined in conformity with the IUPACIUB Joint Commission on Biochemical Nomenclature (JCBN). It is also understood that a polypeptide may be coded for by more than one nucleotide sequence due to the degeneracy of the genetic code.

Other definitions of terms may appear throughout the specification. Before the exemplary embodiments are described in more detail, it is to understand that this disclosure is not limited to particular embodiments described, as such may, of course, vary. It is also to be understood that the terminology used herein is for the purpose of describing particular embodiments only, and is not intended to be limiting, since the scope of the present disclosure will be limited only by the appended claims.

Where a range of values is provided, it is understood that each intervening value, to the tenth of the unit of the lower limit unless the context clearly dictates otherwise, between the upper and lower limits of that range is also specifically disclosed. Each smaller range between any stated value or intervening value in a stated range and any other stated or intervening value in that stated range is encompassed within this disclosure. The upper and lower limits of these smaller ranges may independently be included or excluded in the range, and each range where either, neither or both limits are included in the smaller ranges is also encompassed within this disclosure, subject to any specifically excluded limit in the stated range. Where the stated range includes one or both of the limits, ranges excluding either or both of those included limits are also included in this disclosure.

It must be noted that as used herein and in the appended claims, the singular forms "a", "an", and "the" include plural referents unless the context clearly dictates otherwise. Thus, for example, reference to "a proline tolerant tripeptidyl peptidase", "an endoprotease" or "an enzyme" includes a plurality of such candidate agents and reference to "the feed", "the feedstuff", "the premix" or "the feed additive composition" includes reference to one or more feeds, feedstuffs, premixes and equivalents thereof known to those skilled in the art, and so forth.

The publications discussed herein are provided solely for their disclosure prior to the filing date of the present application. Nothing herein is to be construed as an admission that such publications constitute prior art to the claims appended hereto.

The invention will now be described, by way of example only, with reference to the following Figures and Examples.

## EXAMPLES

## EXAMPLE 1

**Cloning and expression of proline tolerant tripeptidyl peptidases in Trichoderma reesei.**

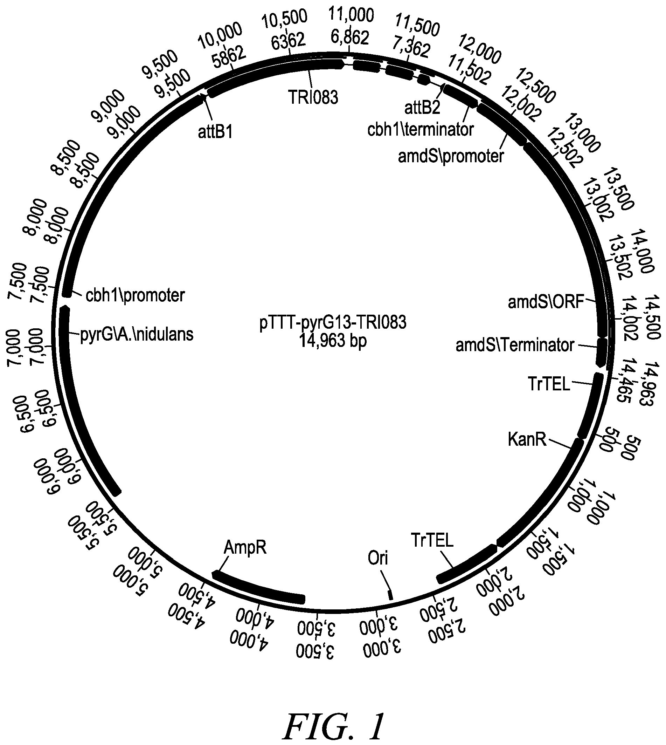

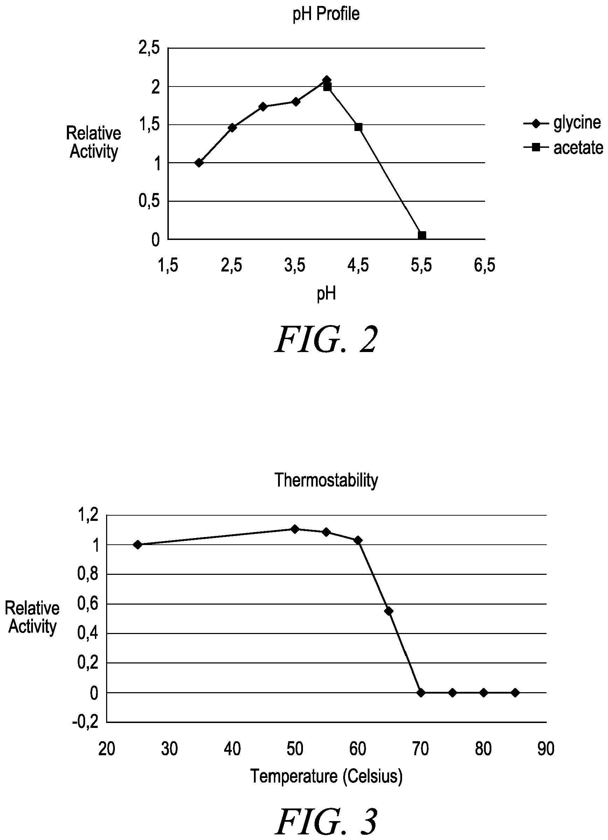

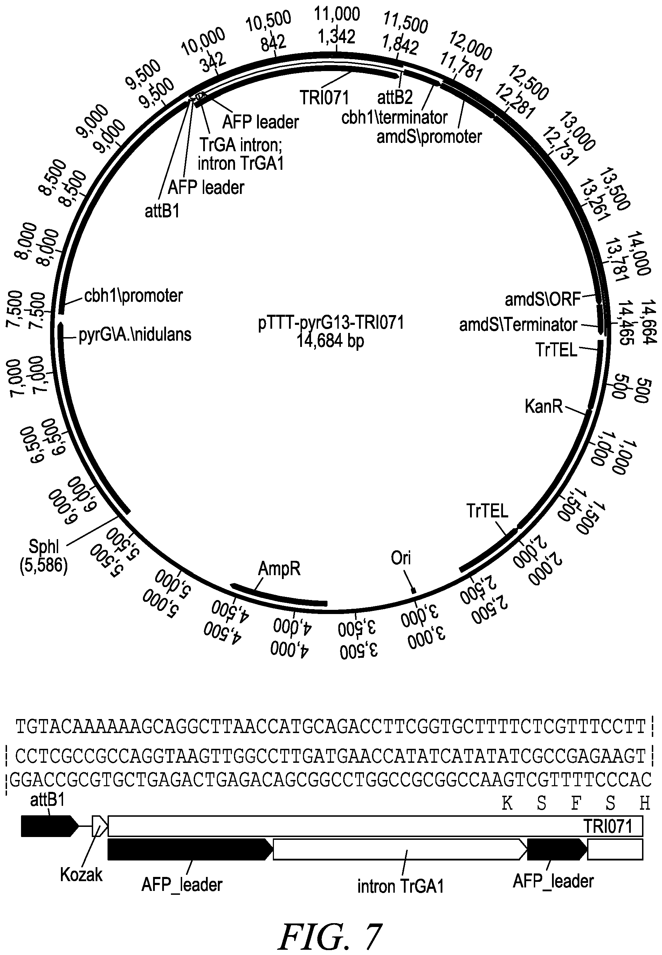

Synthetic genes encoding proline tolerant tripeptidyl peptidases were generated using preferred codons for expression in *Trichoderma reesei* except for TRI079 (SEQ ID No. 57) and TRI083 (SEQ ID No. 56) that were generated as genomic sequences. The predicted secretion signal sequences (SignalP 4.0: Discriminating signal peptides from transmembrane regions. Thomas Nordahl Petersen, Søren Brunak, Gunnar von Heijne & Henrik Nielsen. Nature Methods, 8:785-786, 2011) were replaced (except for TRI079 and TRI083) by the secretion signal sequence from the *Trichoderma reesei* acidic fungal protease (AFP) and an intron from a *Trichoderma reesei* glucoamylase gene (TrGA1) (see Figure 7 lower panel). Synthetic genes were introduced into the destination vector pTTT-pyrG13 (as described in US8592194 B2) using LR Clonase™ enzyme mix (Life Technologies) resulting in the construction of expression vectors pTTT-pyrG13 for the proline tolerant tripeptidyl peptidases herein. Expression vectors encoding SEQ ID No's 1, 2 and 29 are shown in Figure 1 and encoding SEQ ID No's 12 and 39 are shown in Figure 7. Expression vectors encoding SEQ ID No's 96 or 97 (TRI045) are shown in Figure 2.

5-10 μg of the expression vectors were transformed individually into a suitable Trichoderma reesei strain using PEG mediated protoplast transformation essentially as described previously (US8592194 B2). Germinating spores were harvested by centrifugation, washed and treated with 45 mg/ml of lysing enzyme solution (*Trichoderma harzianum* , Sigma L1412) to lyse the fungal cell walls. Further preparation of protoplasts was performed by a standard method, as described by Penttilä et al. [Gene 61(1987) 155-164] the contents of which are incorporated herein by reference.

Spores were harvested using a solution of 0.85% NaCl, 0.015% Tween 80. Spore suspensions were used to inoculate liquid cultures Cultures were grown for 7 days at 28°C and 80% humidity with shaking at 180 rpm. Culture supernatants were harvested by vacuum filtration and used to measure expression and enzyme performance.

## Purification and Characterization

## A. Purification of proline tolerant tripeptidyl peptidase

Desalting of samples was performed on PD10 column (GE Life Sciences, USA) equilibrated with 20 mM Na-acetate, pH 4.5 (buffer A). For ion exchange chromatography on Source S15 HR25/5 (GE Life Sciences, USA) the column was equilibrated with buffer A. The desalted sample (7 ml) was applied to the column at a flow rate of 6 ml/min and the column was washed with buffer A. The bound proteins were eluted with a linier gradient of 0-0.35 M NaCl in 20 mM Na-acetate, pH 4.5 (35 min). During the entire run 10 ml fractions were collected. The collected samples were assayed for tripeptidyl amino-activity as described below. Protein concentration was calculated based on the absorbance measure at 280 nm and the theoretical absorbance of the protein calculated using the ExPASy ProtParam tool (http://web.expasy.org/cgi-bin/protparam/protparam).

## B. Determination of proline tolerant tripeptidyl peptidase and endopeptidase activity

The chromogenic peptide H-Ala-Ala-Ala-pNA (MW = 387.82; Bachem, Switzerland) was used to determine the activity of proline tolerant tripeptidyl peptidase in the samples produced as described above. The assay was conducted as follows, 10 μL of the chromogenic peptide solution (10 mM dissolved in dimethly sulfoxide; DMSO) were added to 130 μl Na-acetate (20 mM, adjusted to pH 4.0 with acetic acid) in a microtiter plate and heated for 5 minutes at 40°C. 10 μL of appropriately diluted enzyme was added and the absorption was measured in an MTP reader (Versa max, Molecular Devices, Denmark) at 405 nm. One katal of proteolytic activity was defined as the amount of enzyme required to release 1 mole of p-nitroaniline per second.

Azoscasein assay for endoprotease activity.

A modified version of the endoprotease assay described by Iversen and Jorgensen, 1995 is used. An enzyme sample of 50 μl is added to 250 μl of azocasein (0.25% w/v; from Sigma) in 4 times diluted McIlvaine buffer, pH 5 and incubated for 15 min at 40°C with shaking (800 rpm). The reaction is terminated by adding 50 μl of 2 M TCA and centrifugation for 5 min at 20,000 g. To a 195 μl sample of the supernatant 65 μl of 1 M NaOH is added and absorbance at 450 nm is measured. One unit of endoprotease activity is defined as the amount which yields an increase in absorbance of 0.1 in 15 min at 40°C.

The proline tolerant tripeptidyl peptidase samples produced as described in Example 1 were found to be essentially free of endopeptidase side-activity. Upon purification as described in Example 2A, substantially no endopeptidase side activity was detected.

**C. pH profile**

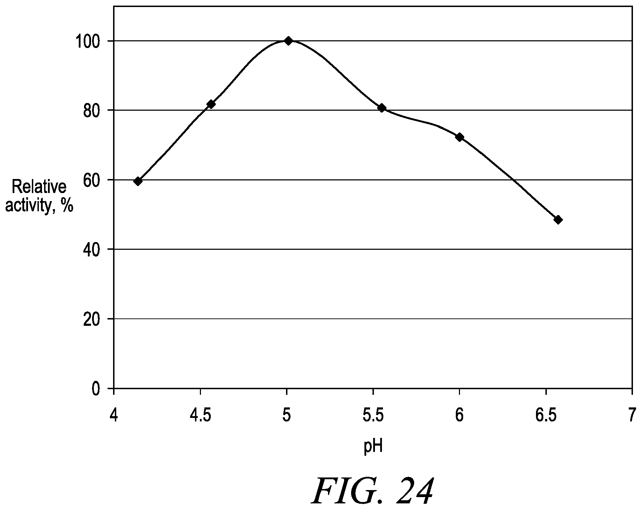

The proline tolerant tripeptidyl peptidase assay described with H-Ala-Ala-Ala-pNA substrate above with modification of using the buffers 20 mM Na-glycine (pH 2.0, 2.5, 3.0, 3.5 and 4.0) or 20 mM Na-acetate buffer (pH 4.0, 4.5 and 5.5) was used to determine the pH profile of proline tolerant tripeptidyl peptidase TRI083 (Figure 2) and TRI045 (Figure 24). Optimum pH of TRI083 and TRI045 was observed to be 4.0.

**D. Temperature profile**

The proline tolerant tripeptidyl peptidase assay described above was used at temperatures 25, 50, 55, 60, 65, 70, 75, 80 and 85°C. The optimum temperature of proline tolerant tripeptidyl peptidase TRI083 was found to be 50° C, whereas no activity was found at 70°C and higher temperatures (Figure 3).

**Protein hydrolysis using a proline tolerant tripeptidyl peptidase in combination with an endoprotease (Alphalase® AFP)**

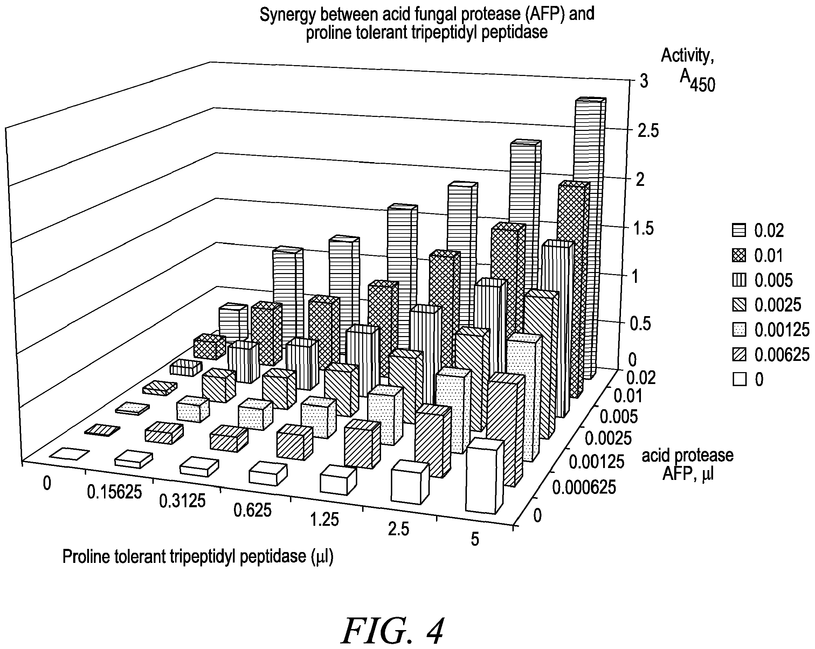

The enzymes: Alphalase® AFP and the proline tolerant tripeptidyl peptidase TRI083 expressed as described in Example 1.
Assay buffer: 50 mM NaOAc, pH 4.0, 3% dimethylhemoglobin, 37 °C, 1h incubation (100 μl reaction mixture per MTP well).
Stop/colour reagent: 0.05% trinitrobenzenesulfonic acid in 125 mM Na borate pH 8.6 (200 μl per well).
The plate was read at 450 nm using a Versa max microplate reader (Molecular Devices) after about 20 min incubation with stop/colour reagent. The results of the assay (Figure 4) show a synergistic effect when an endoprotease (Alphalase® AFP) is used in combination with the proline tolerant tripeptidyl peptidase.

**Hydrolysis of 33-mer gliadin peptide**

The tripeptidyl amino-peptidase was examined for its ability to hydrolyse a synthetic substrate from alpha gliadin (alpha-2-gliadin) by LC-MS and label-free quantification. It was found to cleave the substrate into tri-peptides irrespective of high proline content in the substrate.

*Experimental set-up*

The 33-mer of gliadin (alpha-2-gliadin) (aa56-88) H-LQLQPFPQPQLPYPQPQLPYPQPQLPYPQPQPF-OH (Zedira GmbH; D-64293 Darmstadt, Germany) $C_{190}H_{273}N_{43}O_{47}$ (MW=3911.46) (1mg/ml; 0.26 mM) was incubated at 24 C in the presence of proline tolerant tripeptidyl peptidase (0.01mg/ml) in a total volume of 1000 ul buffer (pH=4.5) (the ratio substrate/enzyme 100:1, w/w). Aliquots (100 ul) of enzyme reaction were stopped with 20 ul 5% trifluoroacetic acid (TFA) after 0, 1, 3, 5, 10, 15 and 30 minutes, respectively. The samples were then transferred to new vials and analyzed on the LTQ Orbitrap mass spectrometer.

*Data Acquisition, label free quantification and MS/MS data analysis*

Nano LC-MS/MS analyses were performed using an Easy LC system (Thermo Scientific, Odense, DK) interfaced to a LTQ Orbitrap Classic hybrid mass spectrometer (Thermo Scientific, Bremen, Germany). Samples were loaded onto a custom-made 2 cm trap column (100 μm i.d., 375 μm o.d., packed with Reprosil C18, 5 μm reversed phase particles (Dr. Maisch GmbH, Ammerbuch-Entringen, Germany)) connected to a 10 cm analytical column (75 μm i.d., 375 μm o.d., packed with Reprosil C18, 3 μm reversed phase particles (Dr. Maisch GmbH, Ammerbuch-Entringen, Germany)) with a steel needle. Separation was performed at a flow rate of 300nL/min using a 10 min gradient of 0-34% Solvent B (H2O/CH3CN/TFE/HCOOH (100/800//100/1 v/v/v/v)) into the nanoelectrospray ion source (Thermo Scientific, Odense, DK). The LTQ Orbitrap Classic instrument was operated in a data-dependent MS/MS mode. The peptide masses were measured by the Orbitrap (MS scans were obtained with a resolution of 60 000 at m/z 400), and up to 2 of the most intense peptide m/z were selected and subjected to fragmentation using CID in the linear ion trap (LTQ). Dynamic exclusion was enabled with a list size of 500 masses, duration of 40 s, and an exclusion mass width of ± 10 ppm relative to masses on the list.
The RAW files were accessed with the open source program Skyline 1.4.0.4421 (available from MacCoss Lab Software, University of Washington, Department of Genome Sciences, 3720 15th Ave NE Seattle, Washington, US) which can use the MS1 intensities to build chromatograms. The precursor isotopic import filter was set to a count of three, (M, M+1, and M+2) at a resolution of 60,000 and the most intense charge state was used. Peptide sequences of the two substrates as well as their cleavage products were typed into Skyline and intensities were calculated in each sample.

The LC-MS/MS data was manually annotated using GPMAW to calculate theoretical values of fragmentation.

The triple charged mass of the intact alpha-2-gliadin peptide was isolated and followed over time. The intact peptide was not detectable after 3 min and was very fast hydrolyzed (Table 1). Full hydrolysis of the 33-mer alpha-2-gliadin peptide would give the following tri-peptides: LQL' QPF' PQP' QLP' YPQ' PQL' PYP' QPQ' LPY' PQP' QPF. The intermediate product YPQPQLPYPQPQLPYPQPQPF resulting from cleaving off the four tri-peptides LQL, QPF, PQP and QLP from the alpha-2-gliadin substrate was found to accumulate and then to decrease (Table 1).

**Table 1: Relative MS peak intensities of alpha-2-gliadin and derived peptides**

|  | 0 min | 1 min | 3min | 5 min | 10 min | 15 min | 30 min |
|---|---|---|---|---|---|---|---|
| Alpha-2 gliadin | 100 | 10 | 0 | 0 | 0 | 0 | 0 |
| YPQPQLPYPQPQLPYPQPQPF | 79 | 100 | 52 | 23 | 23 | 23 | 20 |
| LQL | 3 | 91 | 88 | 94 | 94 | 100 | 100 |
| QPF | 0 | 4 | 55 | 7 | 129 | 26 | 100 |
| PQL | 8 | 21 | 30 | 32 | 42 | 58 | 100 |
| PYP | 4 | 7 | 12 | 15 | 28 | 48 | 101 |
| LPY | 5 | 21 | 46 | 62 | 90 | 100 | 94 |

The accumulation of most of the expected tri-peptides was detected. The underlined tri-peptides were found and confirmed based their MS/MS fragmentation: LQL' QPF' PQP' QLP' YPQ' PQL' PYP' QPQ' LPY' PQP' QPF, whereas the QPF tri-peptides were found only based on their mass.

In conclusion, proline tolerant tripeptidyl peptidase was found to cleave the substrate alpha-2-gliadin consecutively into tri-peptides irrespective of a high proline content. During the hydrolysis proline was present in P3,P2,P1,P1',P2'and P3' positions, respectively.

This is in contrast to that previously found tripeptidyl amino-peptidase do not cleave proline in P1 or P1' positions (US7972808B2, US5821104, Reichard et al. 2006, Applied and Environmental Microbiology 72, 1739-1748).

## Cleavage of AAPPA peptide

Proline tolerant tripeptidyl peptidase was examined for its ability to hydrolyse a synthetic substrate AAPPA by LC-MS and label free quantification. The peptide H-AAPPA-NH2 (MW=424.49, from Schafer-N, Copenhagen) was dissolved in 20 mM MES buffer, pH=4.0 (1mg/ml). 1000 ul of the H-AAPPA-NH2 solution was incubated with 200 ul proline tolerant tripeptidyl peptidase (TRI083) solution (40ug/ml) (substrate/enzyme 100:0.8) at room temperature. At seven time points (0, 5, 15, 60, 180, 720 and 1440 min) 100 ul samples were withdrawn, diluted with 50ul 5% TFA, heat inactivated (10 min at 80°C) and kept at -20°C until LC-MS analysis.

Nano LC-MS/MS analyses were performed using an Easy LC system (Thermo Scientific, Odense, DK) interfaced to a LTQ Orbitrap Classic hybrid mass spectrometer (Thermo Scientific, Bremen, Germany). Samples were loaded onto a custom-made 2 cm trap column (100 $\mu$m i.d., 375 $\mu$m o.d., packed with Reprosil C18, 5 $\mu$m reversed phase particles (Dr. Maisch GmbH, Ammerbuch-Entringen, Germany)) connected to a 10 cm analytical column (75 $\mu$m i.d., 375 $\mu$m o.d., packed with Reprosil C18, 3 $\mu$m reversed phase particles (Dr. Maisch GmbH, Ammerbuch-Entringen, Germany)) with a steel needle. Separation was performed at a flow rate of 300nL/min using a 10 min gradient of 0-34% Solvent B

(H2O/CH3CN/TFE/HCOOH (100/800//100/1 v/v/v/v)) into the nanoelectrospray ion source (Thermo Scientific, Odense, DK). The LTQ Orbitrap Classic instrument was operated in a data-dependent MS/MS mode. The peptide masses were measured by the Orbitrap (MS scans were obtained with a resolution of 60 000 at m/z 400), and up to 2 of the most intense peptide m/z were selected and subjected to fragmentation using CID in the linear ion trap (LTQ). Dynamic exclusion was enabled with a list size of 500 masses, duration of 40 s, and an exclusion mass width of $\pm$10 ppm relative to masses on the list.

The RAW files were accessed with the open source program Skyline 1.4.0.4421 which can use the MS1 intensities to build chromatograms. The precursor isotopic import filter was set to a count of three, (M, M+1, and M+2) at a resolution of 60,000 and the most intense charge state was used. Peptide sequences of the substrate as well as a cleavage product was typed into Skyline and intensities were calculated in each sample.

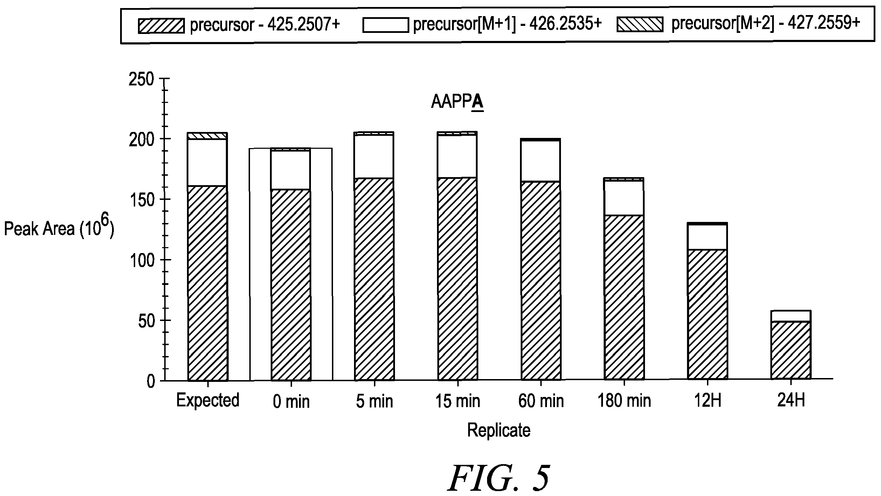

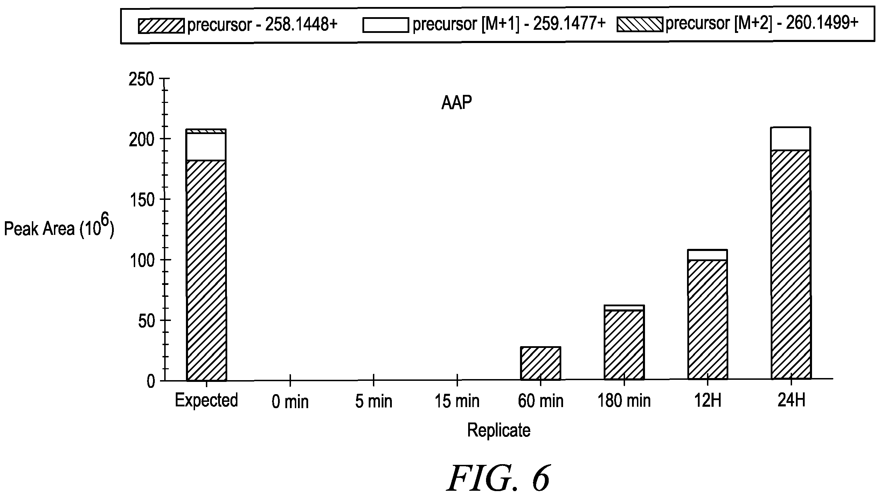

The analysis showed that proline tolerant tripeptidyl peptidase (TRI083) over time is able to degrade the peptide AAPPA (Figure 5) and generate the product AAP (Figure 6), indicating that the PP peptide bond in AAPPA is hydrolysed by proline tolerant tripeptidyl peptidase (TRI083).

## EXAMPLE 2

### Materials and Methods

### 1. Enzyme treatment of cornsoy feed.

Feed flour was sifted to a particle size less than 212$\mu$m and suspended in water to 10% (w/w) slurry and pH adjusted to pH3.5. Then, 138$\mu$L 10 % this slurry was added to each well in 96 MTP well-plate using a Beckman Coulter Biomek NXp laboratory automation workstaton. Wide bore tips were used. Then 20 $\mu$l of enzyme solution containing proteases to be tested in 20mM acetate pH 3.5 was added, after that 10$\mu$L (1.14U/$\mu$L) pepsin dissolved in water was added. The plate was incubated at 40°C for 45minutes in iEMS incubator/shaker at 1150 rpm. Then 34$\mu$L pancreatin 1.126mg/mL in 1M Na-bicarbonate was added and the plate was incubated at 40°C for 60minutes in iEMS at 1150 rpm. Afterwards, the plate was centrifuged at 5°C, 4000 rpm for 15 min, 10$\mu$L supernatant was transferred to new plates (corning plate #3641 nonbinding) containing 190$\mu$L water in each well to a 20x dilution. The obtained plates (master plates) were stored in the freezer -20°C.

### 2. Degree of Hydrolysis measurements.

The method of analysis of degree of hydrolysis (DH) of soluble protein is based on the reaction of primary amino groups with o-phthaldialdehyde (OPA - assay). Reference: P.M. Nielsen, D. Petersen and C. Dambmann. Improved Method for Determining Food Protein Degree of Hydrolysis. Journal of Food Science. 66 (2001) 642-646.

For OPA assay the following procedure was carried on. 10-25 $\mu$l feed sample treated by enzyme from master plate was transferred to the new plate, then 175 $\mu$l of OPA reagent containing sodium borate, dodecyl sulfate and dithiothreitol, were added to the plate. The end point measurements of optical density at 340 nm were performed right after 2 min and 5 second mixing.

**The effect of Alphalase® AFP (an acid protease) on cornsoy feed in the presence of pepsin and pancreatin. *In vitro* studies.**

The composition of cornsoy feed is presented in the Table below (Interactions of phytate and *myo*-inositol phosphate esters (IP$_{1-5}$) including IP$_5$ isomers with dietary protein and iron and inhibition of pepsin. S. Yu, A. Cowieson, C. Gilbert, P. Plumstead and S. Dalsgaard J. Anim. Sci. 90 (2012) 1824-1832. Supplementary Information).

| Ingredient | Amount, % |
|---|---|
| Corn | 60.01 |
| Soybean meal | 31.52 |
| Soy oil | 4.00 |
| Salt | 0.40 |
| DL-Methionine | 0.20 |
| Limestone | 1.16 |
| Dicalcium Phosphate | 1.46 |
| Vitamin and mineral mixture | 1.25 |

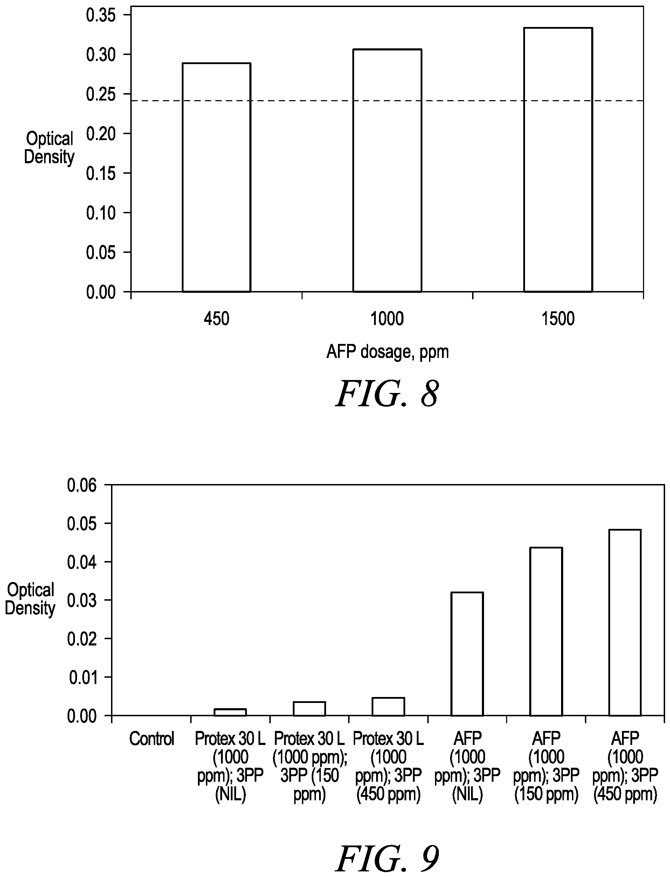

Cornsoy feed were treated with Alphalase® AFP (NSP24, available at Genencor Division, Food Enzymes) (herein referred to as "AFP") at different concentrations (450, 1000 and 1500 ppm in relation to the cornsoy feed) in the presence of pepsin and pancreatin (Figure 8). The results are presented below; the level of improvement of cornsoy feed DH is 4.5, 6.3 and 9.0 %, respectively.

## EXAMPLE 3

**The effect of a proline tolerant tripeptidyl peptidase on cornsoy feed in the presence of pepsin and pancreatin and in the presence and in the absence of** Alphalase® **AFP. *In vitro* studies.**

In these studies Alphalase® AFP and proline tolerant tripeptidyl peptidase were used only at the dosages of 1000 and 450 ppm, respectively. The experiment was carried out as per Example 2. The results are presented in Table 2. In the control experiment only pepsin and pancreatin were used. Improvement of hydrolysis is the ratio between the treatment and control.

Table 2. The effect of proline tolerant tripeptidyl peptidase and Alphalase® AFP on hydrolysis of cornsoy feed.

|  | Control | 3PP | AFP | AFP+3PP |
|---|---|---|---|---|
| **Degree of hydrolysis, %** | 24.5±2.0 | 25.5±1.6 | 28.6±3.6 | 32.3±2.6 |
| **Improvement of hydrolysis, %.** | 100±8.2 | 104±6.5 | 117±14.7 | 132±10.7 |

As can be seen from Table 2 proline tolerant tripeptidyl peptidase on its own does not give sufficient benefit in cornsoy protein hydrolysis. The performance of Alphalase® AFP is similar to the results presented in the Example 2. However, the combination of proline tolerant tripeptidyl peptidase and Alphalase® AFP gives the maximal results and can be associated with the synergetic action of endo- and exo- proteases.

## EXAMPLE 4a

**Validation of dose response of proline tolerant tripeptidyl peptidase (sometimes referred to herein as 3PP) in combination with endo-proteases on cornsoy feed hydrolysis in the absence of pepsin and pancreatin.**

The aim of the work was to identify the origin of the enzymes performance and truly monitor possible additive or synergetic performance of endo- and exo-proteases. For this reason, the experiments were performed in the absence of pepsin and pancreatin and the results are summarized in Figure 9. Otherwise the experiment was carried out as per Example 2.
As can be seen from Figure 9, the degree of hydrolysis when proline tolerant tripeptidyl peptidase is used with endoproteases is very pronounced. It is clear that this phenomenon is synergetic, since the dose response depends on the level of proline tolerant tripeptidyl peptidase and the nature of endoprotease. In the case of Alphalase® the effect is much more pronounced.

## EXAMPLE 4b

**Validation of dose response of 3PP in combination with different dosages of AFP endo-proteases on cornsoy feed hydrolysis in the presence of pepsin and pancreatin.**

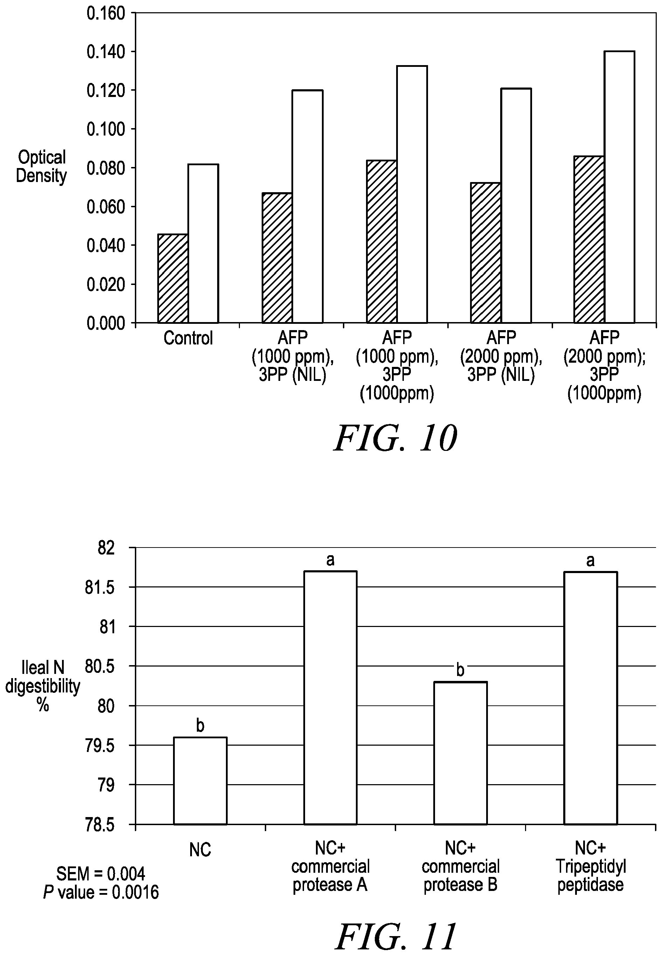

To estimate the level of synergism between AFP and 3PP, the experiment carried out in accordance with Example 2 with different dosages of the enzymes and in the presence of pepsin and pancreatin were performed.
As can be seen from Figure 10 the level of degree of hydrolysis increases with the inclusion of AFP at both 1000 and 2000ppm. Addition of 3PP further increases the hydrolysis. This is the case for a reaction time of 100 and 200min.

## EXAMPLE 5

**The stability of proline tolerant tripeptidyl peptidases in the presence of pepsin**

*Material and Methods*

Pepsin solution: Swine pepsin from Sigma (P7000, 674 Sigma units/mg) was used in this example and was prepared in MilliQ water at 10000 Sigma unit/ml. The tripeptidyl peptidase pepsin pre-incubation mixture contained: 2.5μl tripeptidyl peptidase sample, 95μl GAT buffer (50mM glycine-50mM acetic acid-50mM tris, pH3.5), 5μl pepsin in milliQ water (10000 Sigma unit/ml) (final pepsin unit concentration: 500 Sigma unit/ml reaction mixture) in a half bottom area 96 well Corning MTP. For control, the pre-incubation mixture contained 5μl water instead of 5μl pepsin. The mixture

was incubated at 40°C for 60min and then kept on ice. For residual activity, the assay mixture contained the pre-incubation mixture 5μl, 0.1 M acetic acid-sodium acetate (pH4.0) 85μl, 5μl substrate AAF-pNA (2.5mg/ml)(H-Ala-Ala-Phe-pNA . BACHEM, L-1095). The 410nm reading using a microplate reader (Power Wave X from Bio-Tek Instruments Inc.) was followed every 0.5min at 30°C. N=2.

*Results*

Table 3 shows that under the assay conditions and pre-incubation at 40°C for 60min, the residual activity was found to be over 80% for all the tripeptidyl peptidases in the presence of 500 units pepsin/ml pre-incubation mixture.

Table 3: Activity recovery for tripeptidyl peptidases

| Samples | TRI043 | TRI050 | TRI053 | TRI071 | TRI071 | TRI071 | TRI071 |
|---|---|---|---|---|---|---|---|
| No pepsin | 0.379 | 0.919 | 0.525 | 1.219 | 1.105 | 0.936 | 1.214 |
| With pepsin | 0.529 | 0.880 | 0.588 | 1.118 | 1.127 | 0.924 | 1.091 |
| Activity recovery (%) | 140 | 96 | 112 | 112 | 102 | 99 | 90 |

## EXAMPLE 6

### Analysis of proline tolerant tripeptidyl peptidases (3PP) for their low pH stability at pH 2.5 40°C 60min

As a requirement for utilization of the enzymes in feed for monogastric animals, the enzymes should be active at lower pH. For this reason the number of tripeptidyl peptidases has been tested with the reaction on synthetic substrate AAF-pNA, at pH 2.5.

The tripeptide substrate AAF-pNA was prepared at 2.5mg/ml in DMSO. Tripeptidyl peptidases were used as broth. The reaction mixture containing 2.5μl enzyme and 90μL GAT buffer (pH 2.5) was prepared in 96 well incubation plates.

The incubation plate was mixed and incubated at 40°C for 60min. Then 50μl 0.2M Mes-NaOH pH6.0 was added to each well, mixed at 600rpm for 2min. After that, 5μl of the incubation mixture were taken to 96 well assay plate already filled with 85μl 0.1 M acetic acid buffer, pH4.0 and 5μl 2.5mg/ml AAF-pNA solution.

The reaction mixture was stirred at 600rpm for 2min and read directly in a microplate reader at 410nm at 30°C every 0.5min for 15min.

To measure initial enzyme activity, similar procedure was performed except the step of enzyme incubation at 40°C for 60 min.

The results of measuring initial and final activity with activity recovery are presented in Table 4.

**Table 4. Initial, final activity and its recovery for tripeptidyl peptidases.**

| Protein name | OD410nm (Initial) | OD410nm (Final) | Activity recovery (%) |
|---|---|---|---|
| TRI050.3 (SEQ ID No. 7/SEQ ID No. 34) | 0.603 | 0.501 | 83 |
| TRI053.1 (SEQ ID No. 10/SEQ ID No. 37) | 0.345 | 0.356 | 103 |
| 29.9 Induction control | | | |
| Morph 1.1 Background | | | |
| TRI050 (SEQ ID No. 7/SEQ ID No. 34) | 0.895 | 0.814 | 91 |
| TRI071 (pool1) 2% glu/soph (SEQ ID No. 12/SEQ ID No. 39) | 0.757 | 0.589 | 78 |
| TRI071 (pool1) 2% glu/soph (SEQ ID No. 12/SEQ ID No. 39) | 0.815 | 0.672 | 82 |
| TRI071 (pool1) 4% glu/soph (SEQ ID No. 12/SEQ ID No. 39) | 0.466 | 0.453 | 97 |
| TRI071 (pool1) 4% glu/soph (SEQ ID No. 12/SEQ ID No. 39) | 0.67 | 0.6 | 90 |

From Table 4 it can been seen that the tripeptidyl peptidases listed above are considerably stable at pH2.5 and 40°C for 60min since over 70% activities were retained. From these results it can be concluded that the proline tolerant tripeptidyl peptidases listed above are stale at low pH, for example in the upper digest tract of monogastrics.

**Conclusions**

This work demonstrated the activity of the enzymes under conditions mimicking the monogastric digestion system. It is shown that the tripeptidyl peptidases listed above are stable at low pH and in the presence of pepsin and thus are ideal for feed applications.

**EXAMPLE 7**

**Proline tolerant tripeptidyl peptidase in animal feed**

A total of 288 one day old Ross 308 male broiler chicks were purchased from a commercial hatchery. At study initiation, 8 birds were randomly allocated to battery cages according to respective treatments by blocks. Only healthy birds were selected for the experiment, and no birds were replaced throughout the course of the study. The study consisted of the following treatments (Table 5):

**Table 5 - Experimental design**

| Dietary treatment | Protease inclusion | Phytase level (FTU/kg) |
|---|---|---|
| **1. Negative control (NC)** | - | 500 |
| **2. NC + commercial protease product A** (trypsin family protease 75000 PROT/g activity) | 0.2g/ kg | 500 |
| **3. NC + commercial protease product B** (subtilisin family protease 2750-3500 GSU/g activity) | 4000U/ kg | 500 |
| **4. NC** + **tripeptidyl peptidase** | 0.01 g/ kg | 500 |
| Commercial protease product A is a trypsin family protease and Commercial protease product B is a subtilisin family protease, neither of which are proline tolerant tripeptidyl peptidases in accordance with the present invention. | | |

Bird weights were recorded at study initiation (d0), day 14, and at study termination (d21). The cage is the experimental unit. Diets were fed in mash form and were formulated to meet or exceed NRC standards, except for Ca and AvP (Table 5). All feed was mixed using a Davis S-20 mixer, the mixer was flushed between each treatment to prevent cross contamination between rations. Samples were collected from each treatment diet from the beginning, middle and end of each batch and were minced together for analysis of enzyme activity in feed.

All birds were fed a corn soy base ration until day 14; from day 14 the treatment rations were fed. Phytase was added to all treatment rations. At the feed change, feeders were removed from the cages, weighed back, emptied, and refilled with the appropriate treatment diet. On the final day of the study (d21), feed and birds were weighed, to determine feed intake (FI) and body weight gain (BWG) for the experimental period. Pens were checked daily for mortality. When a bird was culled or found dead, the date and removal weight (kg) were recorded. A gross necropsy was performed on all dead or culled birds to determine the possible cause of death. Feed conversion ratio (FCR) corrected for mortality was determined.

On d21, all birds per cage were euthanised by intracardial injection of sodium pentobarbitone and contents of the lower ileum were expressed by gentle flushing with distilled water. Digesta from birds within a cage were pooled, resulting in eight samples per dietary treatment. The digesta samples were frozen immediately after collection, lyophilised and processed. Diets and digesta samples were analysed for the marker, nitrogen (N) and gross energy to enable calculation of digestibility co-efficients.

**Table 6 - Diet formulations**

| Ingredient % | 0-14 days | 14-21 days |
|---|---|---|
| Maize | 48.78 | 57.09 |
| Soybean Meal, 48%CP | 40.06 | 34.7 |

(continued)

| Ingredient % | 0-14 days | 14-21 days |
|---|---|---|
| Canola meal | 4 | 4 |
| Soybean Oil | 3 | 1.35 |
| L-Lysine HCl | 0.13 | 0.07 |
| DL-methionine | 0.28 | 0.22 |
| L-threonine | 0.03 | 0 in |
| Salt | 0.33 | 0.33 |
| Limestone | 1 | 0.98 |
| Dicalcium Phosphate | 2.09 | 0.97 |
| Poultry Vits/TE's | 0.3 | 0.3 |
| *Calculated Analyses* | | |
| PROTEIN % | 24.98 | 22.97 |
| MEP MJ/KG | 12.4 | 12.34 |
| CALCIUM % | 1.05 | 0.76 |
| AV PHOS % | 0.5 | 0.3 |
| ALYS % | 1.27 | 1.1 20 |
| AM+C % | 0.94 | 0.84 |
| ATHRE % | 0.83 | 0.73 |
| ATRYP % | 0.26 | 0.23 |

**Statistical Analysis**

Data were analyzed using ANOVA, and means separation conducted to test differences between the different enzymes and enzyme dosages. Cage was used as the experimental unit.

**Results**

**Table 7 - Performance and digestibility results**

| | BWG (g) | FI (g) | FCR (kg/kg) | Ileal N Digestibility % | Ileal digestible energy (MJ/kg) |
|---|---|---|---|---|---|
| NC | 386.3[b] | 576.3[a] | 1.496[a] | 79.6[b] | 12.45[b] |
| NC + commercial protease A (trypsin family protease 75000 PROT/g activity) | 414.1[ab] | 582[a] | 1.429[b] | 81.7[a] | 12.98[a] |
| NC + commercial protease B (subtilisin family protease 2750-3500 GSU/g activity) | 386.2[b] | 572.2[a] | 1.486[a] | 80.3[b] | 12.89[a] |
| NC + tripeptidyl peptidase | 420.8[a] | 597.7[a] | 1.422[b] | 81.7[a] | 13.12[a] |
| SEM | 11.1 | 9.4 | 0.018 | 0.4 | 0.112 |
| *Effect tests* | | | | | |

(continued)

| Effect tests | | | | | |
|---|---|---|---|---|---|
| Treatment | 0.0627 | 0.2563 | 0.0092 | 0.0016 | 0.0011 |
| Each value represents the mean of 9 replicates (8 birds per replicate). [a,b]Means in a column not sharing a common superscript are different (P < 0.05). Commercial protease A is a trypsin family protease and Commercial protease B is a subtilisin family protease, neither of which are proline tolerant tripeptidyl peptidases in accordance with the present invention. | | | | | |

Supplementation of the proline tolerant tripeptidyl peptidase resulted in a significant reduction in FCR compared to the negative control and commercial protease B and a numerical reduction in comparison to commercial protease A.

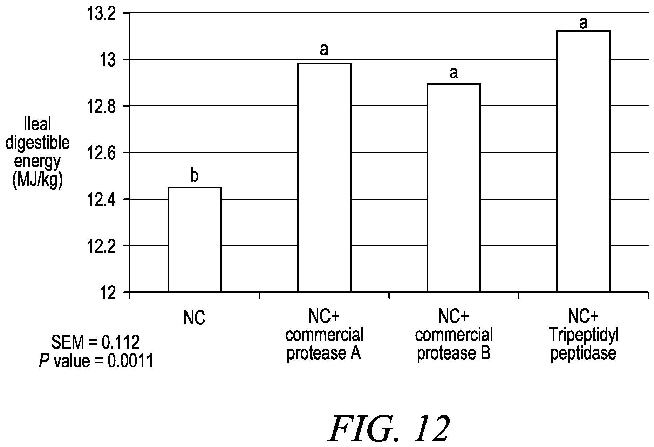

Proline tolerant tripeptidyl peptidase supplementation significantly increased BWG compared to the NC, this was not the case for either commercial protease A or commercial protease B. Proline tolerant tripeptidyl peptidase significantly increased ileal N digestibility % compared to the control and commercial protease B (Figure 11). Proline tolerant tripeptidyl peptidase also significantly increased the energy digested to a level significantly greater than the negative control and numerically greater than the two commercial proteases (Figure 12).

**Conclusions**

In conclusion, supplementation of proline tolerant tripeptidyl peptidase resulted in significantly better bird performance than the NC and commercial protease B in terms of FCR and BWG. There was a numerical increase in BWG and reduction in FCR when proline tolerant tripeptidyl peptidase was supplemented compared to commercial protease A.

The improvements in bird performance ware driven by improved energy and protein (N) digestibility compared to the commercial proteases.

**EXAMPLE 8**

**Combination of acid fungal protease (Alphalase® AFP) and proline tolerant tripeptidyl peptidase (TRI083) in Animal Feed**

A total of 432 one day old Ross 308 male broiler chicks were purchased form a commercial hatchery. At study initiation, 8 birds were randomly allocated to battery cages according to respective treatments by blocks. Only healthy birds were selected for the experiment, and no birds were replaced throughout the course of the study. The study consisted of the following treatments (Table 8):

**Table 8 - Experimental design**

| Dietary treatment | Protease inclusion | Phytase level (FTU/kg) |
|---|---|---|
| 1. Negative control (NC) | - | 500 |
| 2. NC + commercial protease product A | 0.2g/ kg | 500 |
| 3. NC + commercial protease product B | 4000U/ kg | 500 |
| 4. NC + acid fungal protease (AFP) | 0.0015g/ kg of AFP | 500 |
| 5. NC + AFP + 0.0035g/ kg tripeptidyl peptidase | 0.0015g/ kg of AFP 0.0035g/ kg tripeptidyl peptidase | 500 |
| 6. NC + AFP + 0.007g/ MT tripeptidyl peptidase | 0.0015g/ kg of AFP 0.007g/ kg of tripeptidyl peptidase | 500 |
| Commercial protease product A is a trypsin family protease and Commercial protease product B is a subtilisin family protease, neither of which are proline tolerant tripeptidyl peptidases in accordance with the present invention. | | |

Bird weights were recorded at study initiation (d0), day 14, and at study termination (d21). The cage is the experimental unit. Diets were fed in mash form and were formulated to meet or exceed NRC standards, except for Ca and AvP (Table 9.2). All feed was mixed using a Davis S-20 mixer, the mixer was flushed between each treatment to prevent cross contamination between rations. Samples were collected from each treatment diet from the beginning, middle and end of each batch and were minced together for analysis of enzyme activity in feed.

**Table 7 - Diet formulations**

| Ingredient % | 0-14 days | 14-21 days |
|---|---|---|
| **Maize** | 48.78 | 57.09 |
| **Soybean Meal, 48%CP** | 40.06 | 34.7 |
| **Canola meal** | 4 | 4 |
| **Soybean Oil** | 3 | 1.35 |
| **L-Lysine HCl** | 0.13 | 0.07 |
| **DL-methionine** | 0.28 | 0.22 |
| **L-threonine** | 0.03 | 0 |
| **Salt** | 0.33 | 0.33 |
| **Limestone** | 1 | 0.98 |
| **Dicalcium Phosphate** | 2.09 | 0.97 |
| **Poultry Vits/TE's** | 0.3 | 0.3 |
| *Calculated Analyses* | | |
| **PROTEIN %** | 24.98 | 22.97 |
| **MEP MJ/KG** | 12.4 | 12.34 |
| **CALCIUM %** | 1.05 | 0.76 |
| **AV PHOS %** | 0.5 | 0.3 |
| **ALYS %** | 1.27 | 1.1 |
| **AM+C %** | 0.94 | 0.84 |
| **ATHRE %** | 0.83 | 0.73 |
| **ATRYP %** | 0.26 | 0.23 |

All birds were fed a corn soy base ration until day 14; from day 14 the treatment rations were fed. Enzyme doses used in the study were selected for being most commercially relevant. Phytase was added to all treatment rations. At the feed change, feeders were removed from the cages, weighed back, emptied, and refilled with the appropriate treatment diet. On the final day of the study (d21), feed and birds were weighed, to determine feed intake (FI) and body weight gain (BWG) for the experimental period. Pens were checked daily for mortality. When a bird was culled or found dead, the date and removal weight (kg) were recorded. A gross necropsy was performed on all dead or culled birds to determine the possible cause of death. Feed conversion ratio (FCR) corrected for mortality was determined.

On d21, all birds per cage were euthanised by intracardial injection of sodium pentobarbitone and contents of the lower ileum were expressed by gentle flushing with distilled water. Digesta from birds within a cage were pooled, resulting in eight samples per dietary treatment. The digesta samples were frozen immediately after collection, lyophilised and processed. Diets and digesta samples were analysed for the marker, nitrogen (N) and gross energy to enable calculation of digestibility co-efficients.

*Statistical Analysis*

Data were analyzed using ANOVA, and means separation conducted to test differences between the different enzymes and enzyme dosages. Cage was used as the experimental unit.

*Results*

**Table 8 - Performance and digestibility results**

| | BWG (g) | FI (g) | FCR (kg/kg) | Ileal N Destibility % | Ileal digestible energy (MJ/kg) |
|---|---|---|---|---|---|
| NC | 386.3 | 576.3 | 1.496a | 79.6c | 12.45b |
| NC + commercial protease A | 414.1 | 582.0 | 1.429c | 81.7a | 12.98a |
| NC + commercial protease B | 386.2 | 572.2 | 1.486ab | 80.3bc | 12.89a |
| NC + acid fungal protease (AFP) | 391.2 | 575.8 | 1.475abc | 79.8bc | 12.67ab |
| NC + AFP + 0.0035g/ kg tripeptidyl peptidase | 418.1 | 595.9 | 1.428c | 80.9ab | 12.78a |
| NC + AFP + 0.007g/ MT tripeptidyl peptidase | 404.6 | 583.3 | 1.443bc | 81.5a | 12.92a |
| SEM | 10.5 | 8.9 | 0.017 | 0.406 | 0.11 |
| *Effect tests* | | | | | |
| Treatment | 0.1258 | 0.4891 | 0.021 | 0.0012 | 0.0144 |
| Each value represents the mean of 9 replicates (8 birds per replicate). <br> ab Means in a column not sharing a common superscript are different (P < 0.05). | | | | | |

From Table 8 it can be seen that there was a significant effect of treatment on FCR, there was no significant difference between birds fed the NC diet and the AFP alone, even though there was a numerical reduction in FCR. However, diets supplemented with a combination of AFP and proline tolerant tripeptidyl peptidase significantly reduced FCR to a level lower than the control and commercial protease B.

There was a numerical increase in ileal N digestibility (%) when the NC diet was supplemented with AFP alone. There was a stepwise improvement in ileal N digestibility (%) as tripeptidyl peptidase dose increased on top of the AFP, with the combination of AFP and 0.007g/MT tripeptidyl peptidase having significantly higher ileal N digestibility (%) than the NC, commercial protease B and the AFP alone.

Similarly there was a stepwise numerical increase in ileal energy digestibility as tripeptidyl peptidase was added in increasing doses on top of the AFP, in all cases the combination of AFP and tripeptidyl peptidase was significantly better than the negative control.

*Conclusions*

In conclusion, there is a beneficial effect of the combination of AFP and proline tolerant tripeptidyl peptidase on improving bird performance. This is driven by an increase in the ileal digestibility of N and energy in birds fed the combination rather than just the AFP enzyme alone. The combination of AFP and proline tolerant tripeptidyl peptidase conferred significantly higher levels of N and energy digestibility than some commercial proteases.

## EXAMPLE 9

**MATERIALS AND METHODS**

**Evaluation of the protein content in the proteases**

In order to add equivalent amounts of proteases into the tests, the protein contents of the proteases were measured using Pierce™ BCA protein assay kit. Enzymes stock solution was prepared, whereas rest of the enzymes were in the liquid form. Proteases were diluted 1:10, 1:100 and 1:1000 with MQ water, after which the protein content was measured according to the instructions in the kit.

**In vitro digestion of the feed with proteases**

*In vitro* digestions were performed by simulation of the upper gastrointestinal tract (UGIT) with soy-corn based feed to produce test material for HT-29 MTX E12 cell culture experiments. 5.0 g of pelletized chicken feed was crushed by a mortar and hydrated with 15 ml of MQ water. Proteases were added to the digestions according to Table 1, pH adjusted to 5.5, and the digestions were incubated at 39°C water bath for 20 min by mixing the bottles at five minutes intervals. The conditions of proventriculus and gizzard were simulated by adding 2.5 ml of 1.5 M HCl and 2.5 ml of pepsin solution (1.8 mg/ml in MQ-water) to the digestions, adjusting pH to 2.5, and incubating the bottles at 39°C water bath for 40 min with mixing at 5 min intervals. The condition of small intestine was simulated by adding 2.5 ml of $NaHCO_3$ containing 18.5 mg pancreatin, adjusting the pH to 6.3-6.5, and incubating the bottles at 39°C water bath for 60 min by mixing at 5 min intervals. Digestions were centrifuged at 30 000 x g for 30 min and the supernatants were collected to new tubes. Digestive and protease enzymes were inactivated by incubating the supernatants in boiling water for 4 minutes. The digestions were cooled on ice and stored in freezer (-20°C) for cell culture experiments.

The protein amount was adjusted so that for each protein the final concentration was about 1 $\mu$g/ml solution added to the cells.

**Table 9: Amount of protease in the digestions**

| Treatment # | Protease product | Dose (microgram/g feed) |
|---|---|---|
| 1 | Control | 0 |
| 2 | Commercial Protease A | 10 |
| 3 | Commercial Protease B | 1000 |
| 4 | TRIO83 | 10 |
| 5 | TRIO83 + Alphalase® AFP | 10+10 |

**Cell culture**

HT-29 MTX E12 cells (HPA Cultures, Salisbury, England) were maintained in DMEM (high glucose) supplemented with 10 % FBS, 1× MEM non-essential amino acids, 1× sodium pyruvate, 1× antibiotic-antimycotic at 37 °C, in 8% $CO_2$ atmosphere. All the media and supplements were purchased from Life Technologies.

For studies investigating the effects of proteases on tight junctions and inflammation markers on differentiated cells, the HT-29 MTX E12 cells were seeded at a density of 5.7 × 10$^4$ cells/well on cell culture inserts coated with rat tail collagen type I in complete cultivation medium and differentiated for 14 days. On the 4$^{th}$ day of differentiation, the cells were moved to asymmetric serum-conditions using the serum-free cultivation medium on the apical side and the complete medium on the basal side of the insert. This condition was used until the end of the differentiation by changing the media at three days intervals. The test solutions were applied on the apical chamber of the cells.

**ATP measurements**

HT-29 MTXE12 cells were seeded at a density of 2500 cells/well on white 96-well microplates with complete cultivation medium overnight, and then the serum-free medium was changed to the cells for yet another overnight incubation. On the third day of cultivation, the cells were treated with test materials and the ATP measurement was performed. As a test material, proteases were diluted as such 0.01, 0.1, 0.25, 0.5, 1, 2.5, and 5 $\mu$g/ml (based on their protein amount) in SFM. For combination of proline tolerant tripeptidyl peptidase (TRI083) and Alphalase® AFP, the amount of both enzymes was added the equal amount. When proteases in feed were used the feed supernatant was diluted 1:1 in SFM and equal amount of proteases were added as was in the in vitro digestion (table 3). The ATP content of the cells was measured using ATPLite™ monitoring system (Perkin Elmer) following the manufacturer's instructions after 15 min or 1h from starting the experiment.

**FITC-Dextran permeability assay**

The effect of proteases on macromolecular permeability of epithelial layer was studied by monitoring the FITC dextran flux through the differentiated HT-29 MTX E12 cell layer. Differentiated cells were washed with HBSS medium and equilibrated in HBSS at +37°C, with for 30 minutes. HBSS medium was removed and 0.5 ml of test solutions were added to the cells. Test solutions were prepared by diluting the in vitro digested feed 1:1 with HBSS and adding two amounts of proteases into the samples (1 and 10 $\mu$g/ml). The FITC-Dextran (mol wt 10 000) was included in the test solutions as 1 mg/ml. The HBSS in the apical chambers of the cells were replaced with the test solutions, and the cells were placed in the incubator for 1 h. The cells were shaken with orbital shaker (30 rpm) during this time to minimize the effect of the unstirred water layer. After 1 h 200 $\mu$l samples were drawn from the basolateral chamber, and 200 $\mu$l of fresh HBSS was added into the basolateral side. This was continued for additional 3 hours. The final sample was drawn after 24 hours had gone from the beginning of the study. After that the fluorescence of the samples was measured, and the Papp calculated using the following formula:

$$P_{app} = \frac{V\,(cm^3)}{A\,(cm^2)} \times \frac{Ci\,(100\ \mu M)}{T \times Cf}$$

## TEER measurements

The integrity of HT-29 MTXE12 cell monolayer was studied by measurement of Transepithelial Electrical Resistance (TEER) using Epithelial Voltohmmeter. TEER was measured before and after the treatment, the resistance of an empty filter without cells was subtracted from the measured values, multiplied with the surface area of the filter, and the results were expressed as $\Omega \times cm^2$. A percentual change in TEER after treatment was calculated by subtracting the TEER measured before treatment from the TEER measured after treatment, dividing this value with the TEER measured before treatment and multiplying with 100.

## RESULTS

### Example 9.1

**Effect of proteases on ATP content in the cells treated by proteases**

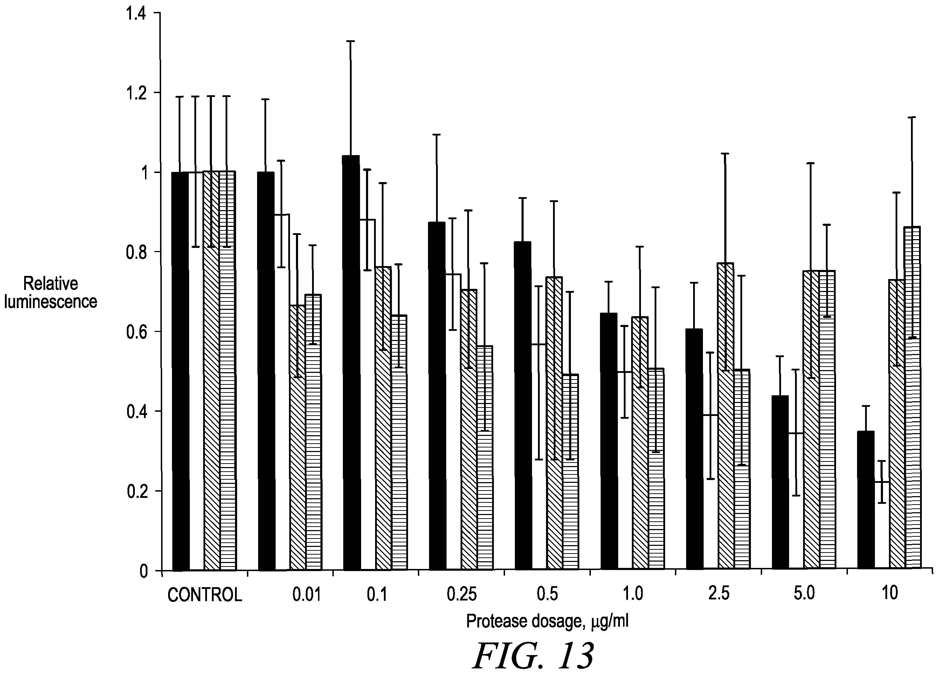

ATP deficiency is a marker for cytotoxicity but it also describes the overall situation in the energy reservoir of the cells. ATP content was measured from HT-29 MTXE12cells after treatment with proteases mixed directly with cell cultivation medium by ATPLiteTM system. Significant dose-dependent changes were observed after one hour treatment with Commercial Protease 1 and Commercial Protease 2 (Figure 13). That would lead to the assumption that Commercial Protease 1 and Commercial Protease 2 have a negative effect on living cells.

### Example 9.2

**Effect of in vitro digested feed with proteases on cellular ATP content**

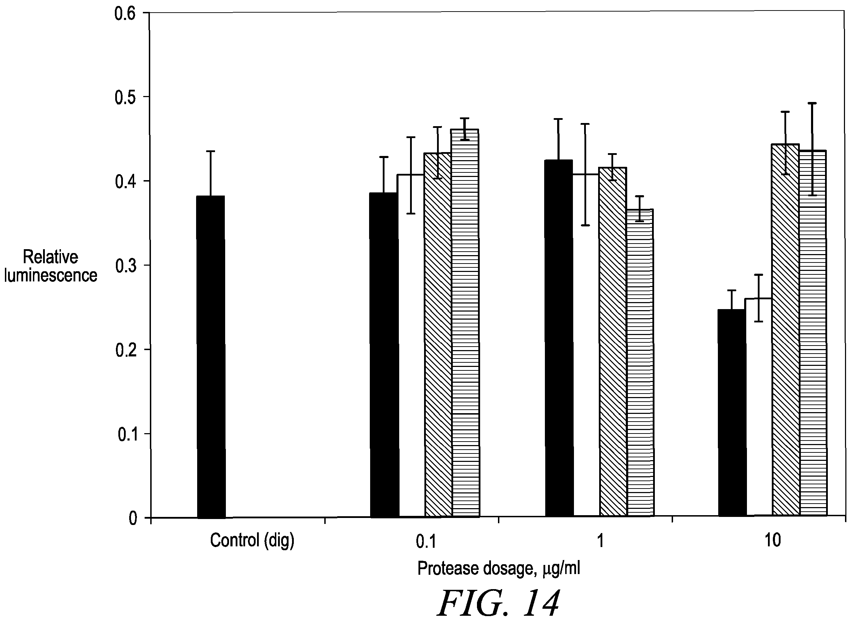

ATP content was also measured from HT-29 MTXE12 cells after one-hour treatment using in vitro digested feed containing proteases. The highest amount of Commercial Protease 1 and Commercial Protease 2 are showing significant reduction in the amount of ATP compared to control in vitro digested feed, and thus leading to the possible cytotoxicity of cells. That would confirm the negative effect of high dosages of Commercial Protease 1 and Commercial Protease 2 on living cells (see Figure 14).

### Example 9.3

**Permeability assessment**

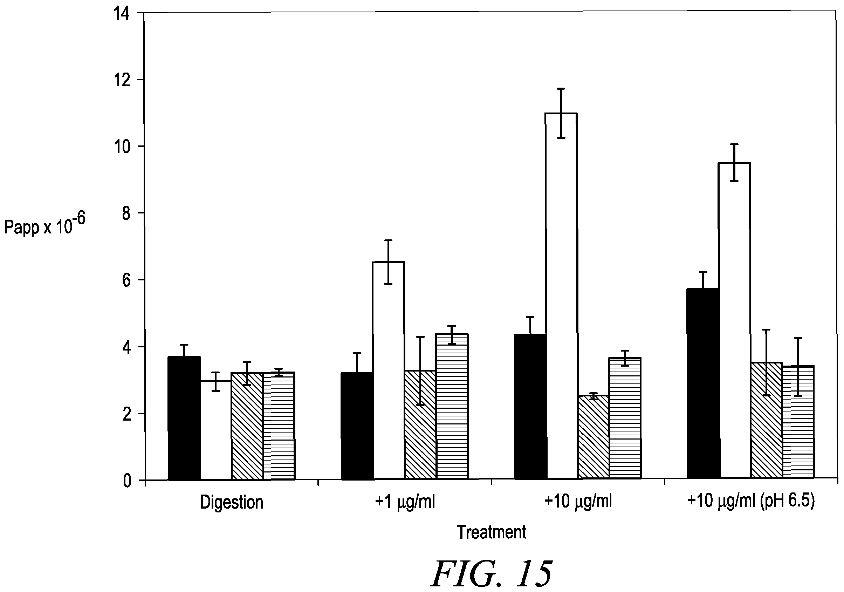

The effect of the in vitro digested feed with proteases on macromolecular permeability was studied using FITC-Dextran permeability assay. For the after the heat inactivation of the digested feed 1 and 10 $\mu$g/ml of protease was added. In some of the assays medium with pH 6.5 was used in order to evaluate the effect of lower pH. For the FITC-Dextran permeability, the tracer was mixed with the in vitro digested feed and applied on the apical compartment. After the four hours the permeability was calculated (Figure 15).

As can be seen from the figure that both Commercial Protease 1 and Commercial Protease 2 significantly increase the macromolecular permeability of cells, thus it has a negative effect on those cells. Neither TRI083 alone nor in combination with Alphalase® AFP induced such response.

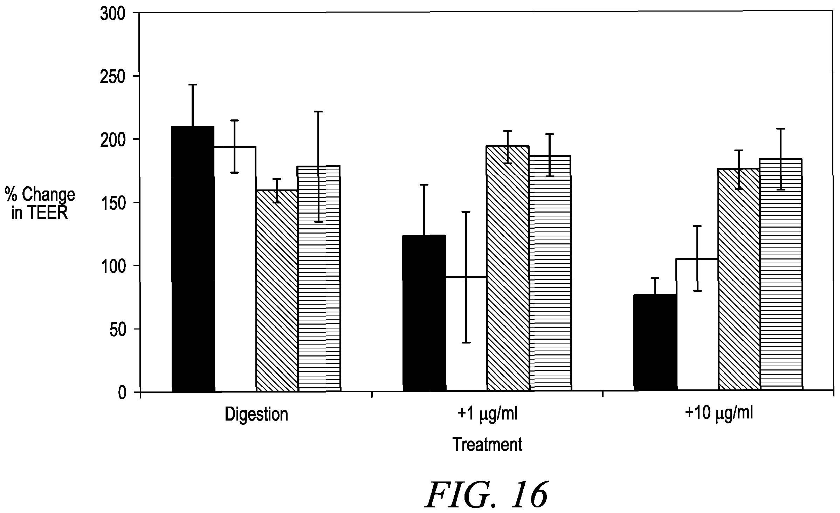

A similar phenomenon was observed during the TEER measurement. A decrease in tight junction integrity was observed both for Commercial Protease 1 and Commercial Protease 2 (Figure 16)which would also lead to the conclusion of negative effect of Commercial Protease 1 and Commercial protease 2 on living cells, while TRI083 alone or in combination with Alphalase® AFP does not contribute to any negative effects for the cells.

## EXAMPLE 10

**Supplementation of tripeptidyl peptidase (TRIO83) in animal feed improves nutrient digestibility**

A total of 288 one day old Ross 308 male broiler chicks were purchased from a commercial hatchery. At study initiation, 8 birds were randomly allocated to battery cages according to respective treatments by blocks. Only healthy birds were selected for the experiment, and no birds were replaced throughout the course of the study. The study consisted of the following treatments (Table 10.1):

**Table 10.1 - Experimental design**

| Dietary treatment | Protease inclusion | Phytase level (FTU/kg) |
|---|---|---|
| **1. Negative control (NC)** | - | 500 |
| **2. NC + commercial product A** | 0.2g/ kg | 500 |
| **3. NC + commercial product B** | 4000U/ kg | 500 |
| **4. NC + tripeptidyl peptidase (TRIO83)** | 0.01 g/ kg of TRIO83 | 500 |

Bird weights were recorded at study initiation (d0) on day 14, and at study termination (d21). The cage is the experimental unit. Diets were fed in mash form and were formulated to meet or exceed NRC standards, except for Ca and AvP (Table 10.2). All feed was mixed using a Davis S-20 mixer, the mixer was flushed between each treatment to prevent cross contamination between rations. Samples were collected from each treatment diet from the beginning, middle and end of each batch and were minced together for analysis of enzyme activity in feed.

**Table 10.2 - Diet formulations**

| Ingredient % | 0-14 days | 14-21 days |
|---|---|---|
| **Maize** | 48.48 | 56.78 |
| **Soybean Meal 48%CP** | 40.06 | 34.7 |
| **Canola meal** | 4 | 4 |
| **Soybean Oil** | 3 | 1.35 |
| **L-Lysine HCl** | 0.13 | 0.07 |
| **DL-methionine** | 0.28 | 0.22 |
| **L-threonine** | 0.03 | 0 |
| **Salt** | 0.33 | 0.33 |
| **Limestone** | 1 | 0.98 |
| **Dicalcium Phosphate** | 2.09 | 0.97 |
| **Poultry Vits/TE's** | 0.3 | 0.3 |
| *Calculated Analyses* | | |
| **PROTEIN %** | 24.98 | 22.97 |
| **MEP MJ/KG** | 12.4 | 12.34 |
| **CALCIUM %** | 1.05 | 0.76 |
| **AV PHOS %** | 0.5 | 0.3 |
| **ALYS %** | 1.27 | 1.1 |
| **AM+C %** | 0.94 | 0.84 |
| **ATHRE %** | 0.83 | 0.73 |
| **ATRYP %** | 0.26 | 0.23 |

All birds were fed a corn soy base ration until day 14; from day 14 the treatment rations were fed. Enzyme doses used in the study were selected for being most commercially relevant. Phytase was added to all treatment rations. At the feed change, feeders were removed from the cages, weighed back, emptied, and refilled with the appropriate treatment diet. On the final day of the study (d21), feed and birds were weighed, to determine feed intake (FI) and body weight gain (BWG) for the experimental period. Pens were checked daily for mortality. When a bird was culled or found dead, the date and removal weight (kg) were recorded. A gross necropsy was performed on all dead or culled birds to determine the possible cause of death. Feed conversion ratio (FCR) corrected for mortality was determined.

On d 21, all birds per cage were euthanised by intracardial injection of sodium pentobarbitone and contents of the lower ileum were expressed by gentle flushing with distilled water. Digesta from birds within a cage were pooled, resulting in eight samples per dietary treatment. The digesta samples were frozen immediately after collection, lyophilised and processed. Diets and digesta samples were analysed for the marker, nitrogen (N) and gross energy to enable calculation of digestibility co-efficients.

*Statistical Analysis*

Data were analyzed using ANOVA, and means separation conducted to test differences between the different enzymes and enzyme dosages. Cage was used as the experimental unit.

*Results*

**Table 10.3 - Performance and digestibility results**

| | BWG (g) | FI (g) | FCR (kg/kg) | Ileal N Digestibility % | Ileal digestible energy (MJ/kg) |
|---|---|---|---|---|---|
| NC | 386.3[y] | 576.3 | 1.496[a] | 79.6[b] | 12.45[b] |
| NC + commercial protease A | 414.1[xy] | 582.0 | 1.429[b] | 81.7[a] | 12.98[a] |
| NC + commercial protease B | 386.2[y] | 572.2 | 1.486[a] | 80.3[b] | 12.89[a] |
| NC + tripeptidyl peptidase (TRIO83) | 420.8[X] | 597.7 | 1.422[b] | 81.7[a] | 13.12[a] |
| SEM | 11.1 | 9.4 | 0.018 | 0.4 | 0.112 |
| *Effect tests* | | | | | |
| Treatment | 0.0627 | 0.2563 | 0.0092 | 0.0016 | 0.0011 |

Each value represents the mean of 9 replicates (8 birds per replicate).
[ab] Means in a column not sharing a common superscript are different ($P < 0.05$).
[xy] Means in a column not sharing a common superscript are different ($P < 0.1$)

There was a significant effect of treatment on FCR, birds fed the tripeptidyl peptidase (TRIO83) has significantly lower FCR than the NC diet and commercial protease A.

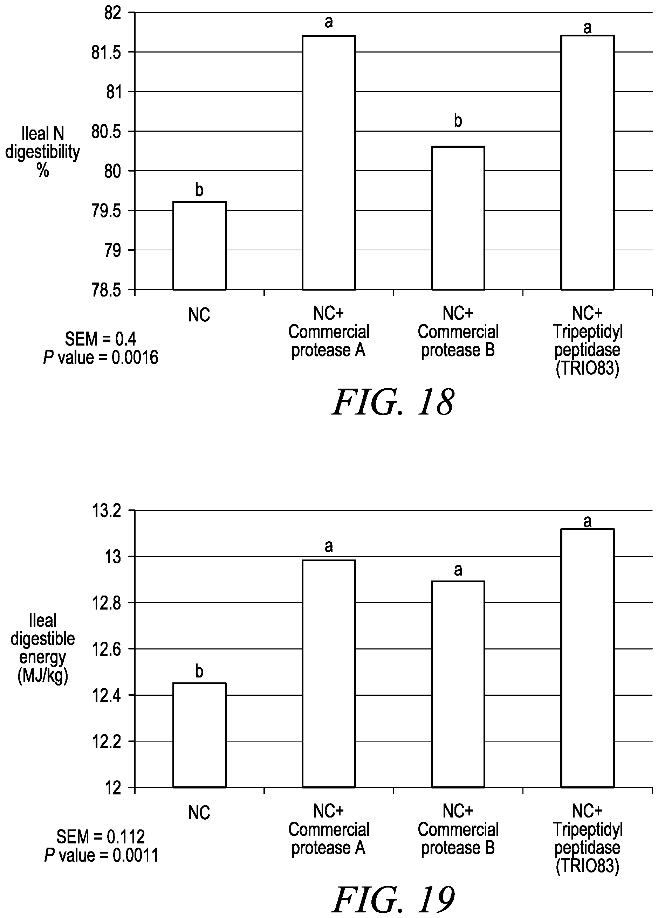

There was a significant increase in ileal N digestibility (%) when the NC diet was supplemented with tripeptidyl peptidase (TRIO83). The increase in N digestibility % was comparable between tripeptidyl peptidase (TRIO83) and commerical protease A, both conferred significantly higher N digestibility than commercial protease B (Figure 18). Similarly, there was a significant increase in ileal digestible energy versus the negative control when each of the protease enzymes was supplemented; ileal energy digestibility was numerically highest when tripeptidyl peptidase (TRIO83) was supplemented (Figure 19).

*Conclusions*

In conclusion, there is a beneficial effect of tripeptidyl peptidase (TRIO83) on improving bird performance through reducing FCR. This is driven by an increase in the ileal digestibility of N and energy in birds fed tripeptidyl peptidase (TRIO83).

Tripeptidyl peptidase (TRIO83) conferred significantly higher levels of N and energy digestibility than some commercial proteases.

## EXAMPLE 11

**Supplementation of tripeptidyl peptidase** (TRIO83) **in animal feed improves broiler performance**

A total of 1600 one day old Ross 708 male broiler chicks were purchased from a commercial hatchery. At study initiation, 40 birds were randomly allocated to floor pens according to respective treatments by blocks. Only healthy birds were selected for the experiment, and no birds were replaced throughout the course of the study. The study consisted of the following treatments (Table 11.1):

**Table 11.1 - Experimental design**

| Dietary treatment | Protease inclusion | Phytase level (FTU/kg) |
|---|---|---|
| **1. Positive control (PC)** | | 500 |
| **2. Negative control (NC)** | - | 500 |
| **3. NC + commercial protease A** | 0.2g/ kg | 500 |
| **4. NC + commercial protease B** | 4000U/ kg | 500 |
| **5. NC + tripeptidyl peptidase (TRIO83)** | 0.01 g/ kg of TRIO83 | 500 |

Bird weights were recorded at study initiation (d0) and at diet changes on d10, d25 and at study termination (d42). The pen is the experimental unit. All diets were fed in mash form. The PC diets were formulated to meet or exceed NRC standards and the NC diets were down-specified by 50% of the undigested fraction of the amino acids in the PC diets (Table 11.2). All feed was mixed using a Davis S-20 mixer, the mixer was flushed between each treatment to prevent cross contamination between rations. Samples were collected from each treatment diet from the beginning, middle and end of each batch and were minced together for analysis of enzyme activity in feed.

**Table 11.2 - Diet formulations**

| | Starter | | Grower | | Finisher | |
|---|---|---|---|---|---|---|
| | PC | NC | PC | NC | PC | NC |
| Maize | 59.75 | 61.22 | 64.91 | 70.35 | 69.14 | 74.34 |
| SBM 48% CP | 30.99 | 27.82 | 25.31 | 21.70 | 21.23 | 17.95 |
| Rapeseed Meal | 4.00 | 4.00 | 4.00 | 4.00 | 4.00 | 4.00 |
| Wheat Bran | 0.00 | 2.78 | 0.00 | 0.00 | 0.00 | 0.00 |
| Soybean Oil | 1.03 | 0.00 | 2.15 | 0.24 | 2.26 | 0.33 |
| L-Lysine HCl | 0.310 | 0.314 | 0.280 | 0.304 | 0.247 | 0.272 |
| DL-methionine | 0.327 | 0.300 | 0.280 | 0.257 | 0.237 | 0.210 |
| L-threonine | 0.097 | 0.079 | 0.085 | 0.075 | 0.066 | 0.051 |
| Sodium Bicarbonate | 0.17 | 0.17 | 0.00 | 0.17 | 0.00 | 0.00 |
| Salt | 0.23 | 0.22 | 0.34 | 0.22 | 0.35 | 0.35 |
| Limestone Dicalcium | 1.26 | 1.27 | 1.05 | 1.06 | 1.03 | 1.04 |
| Phosphate | 1.29 | 1.27 | 1.04 | 1.06 | 0.90 | 0.91 |
| Poultry Vits/TE's | 0.30 | 0.30 | 0.30 | 0.30 | 0.30 | 0.30 |
| Enzymes | 0.25 | 0.25 | 0.25 | 0.25 | 0.25 | 0.25 |
| Calculated Nutrients | | | | | | |
| [VOLUME] | 100.00 | 100.00 | 100.00 | 100.00 | 100.00 | 100.00 |
| DRY_MAT | 87.94 | 87.80 | 87.94 | 87.69 | 87.88 | 87.62 |
| PROTEIN % | 21.34 | 20.32 | 18.94 | 17.63 | 17.24 | 16.06 |
| MEP MJ/KG | 12.52 | 12.18 | 13.07 | 12.74 | 13.27 | 12.93 |
| MEP Kcal/KG | 2992 | 2911 | 3124 | 3045 | 3172 | 3090 |
| TLYSINE % | 1.40 | 1.33 | 1.22 | 1.14 | 1.08 | 1.01 |
| ALYS % | 1.27 | 1.20 | 1.10 | 1.03 | 0.97 | 0.91 |
| METH % | 0.67 | 0.63 | 0.60 | 0.56 | 0.53 | 0.49 |

(continued)

| Calculated Nutrients | | | | | | |
|---|---|---|---|---|---|---|
| AMETH % | 0.64 | 0.60 | 0.57 | 0.53 | 0.51 | 0.47 |
| M+C % | 1.04 | 0.99 | 0.93 | 0.88 | 0.85 | 0.79 |
| AM+C % | 0.94 | 0.89 | 0.84 | 0.79 | 0.76 | 0.71 |
| THREO % | 0.96 | 0.89 | 0.84 | 0.78 | 0.76 | 0.69 |
| ATHRE % | 0.83 | 0.77 | 0.73 | 0.67 | 0.65 | 0.59 |
| TRYPTO % | 0.28 | 0.26 | 0.24 | 0.22 | 0.22 | 0.20 |
| ATRYP % | 0.27 | 0.25 | 0.23 | 0.21 | 0.20 | 0.18 |
| ARGINI % | 1.51 | 1.42 | 1.31 | 1.20 | 1.17 | 1.07 |
| AARG % | 1.31 | 1.21 | 1.14 | 1.04 | 1.02 | 0.93 |
| ISOLEUC % | 1.04 | 0.97 | 0.91 | 0.83 | 0.81 | 0.74 |
| AISO % | 0.95 | 0.87 | 0.82 | 0.75 | 0.73 | 0.67 |
| VAL % | 1.17 | 1.09 | 1.03 | 0.96 | 0.94 | 0.88 |
| AVAL % | 1.04 | 0.97 | 0.92 | 0.85 | 0.83 | 0.77 |
| CALCIUM % | 0.92 | 0.92 | 0.77 | 0.77 | 0.72 | 0.72 |
| TPHOS % | 0.63 | 0.64 | 0.56 | 0.55 | 0.52 | 0.51 |
| AV PHOS % | 0.35 | 0.35 | 0.30 | 0.30 | 0.27 | 0.27 |
| NA % | 0.16 | 0.16 | 0.16 | 0.16 | 0.16 | 0.16 |
| K % | 0.82 | 0.80 | 0.73 | 0.68 | 0.67 | 0.62 |
| CL % | 0.23 | 0.23 | 0.30 | 0.23 | 0.29 | 0.30 |

All birds were fed treatment rations throughout the study. Enzyme doses used in the study were selected for being most commercially relevant. Phytase was added to all treatment rations at 500 FTU/kg. At the feed change, feeders were removed from the cages, weighed back, emptied, and refilled with the appropriate treatment diet. On the final day of the study (d42), feed and birds were weighed, to determine feed intake (FI) and body weight gain (BWG) for the experimental period. Pens were checked daily for mortality. When a bird was culled or found dead, the date and removal weight (kg) were recorded. A gross necropsy was performed on all dead or culled birds to determine the possible cause of death. Feed conversion ratio (FCR) corrected for mortality was determined.

*Statistical Analysis*

Data were analyzed using ANOVA, and means separation conducted to test differences between the different enzymes and enzyme dosages. Pen was used as the experimental unit.

*Results*

**Table 11.3 - Performance results**

| | BWG (g/bird) | FI (kg/pen) | FCR (kg/kg) |
|---|---|---|---|
| PC | 2235[a] | 159.37 | 1.870[b] |
| NC | 2135[c] | 158.75 | 1.957[a] |
| NC + commercial protease A | 2145[c] | 157.70 | 1.940[a] |
| NC + commercial protease B | 2160[bc] | 159.52 | 1.949[a] |
| NC + tripeptidyl peptidase (TRIO83) | 2215[ab] | 158.82 | 1.888[b] |
| SEM | 23.4 | 2.4 | 0.018 |
| *Effect tests* | | | |

(continued)

| Effect tests | | | |
|---|---|---|---|
| Treatment | 0.01 | 0.986 | 0.004 |
| Each value represents the mean of 9 replicates (8 birds per replicate). ab Means in a column not sharing a common superscript are different ($P < 0.05$). | | | |

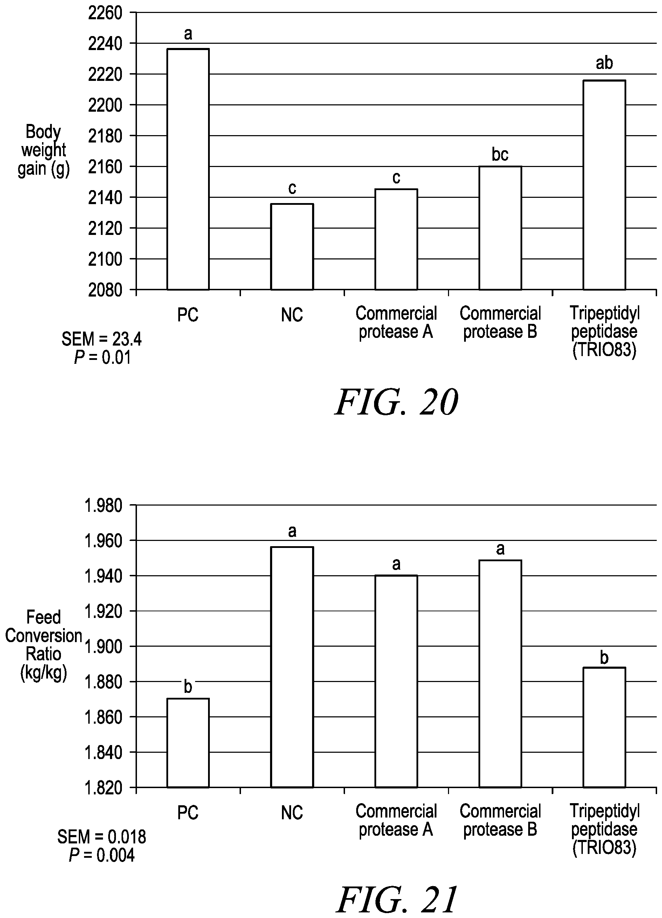

There was a significant effect of treatment on BWG; birds fed the NC diet had significantly lower bodyweights than birds fed the PC diet. Enzyme treatment with commercial proteases A + B numerically increased bodyweight but not to a level significantly different to the NC birds. Supplementation with tripeptidyl peptidase (TRIO83) significantly increased bodyweight gain of birds compared to the NC to a level not significantly different to the PC birds (Figure 20).

Similarly, FCR in NC birds was significantly higher than the PC birds. There was no significant effect of commercial protease A + B on FCR compared to the NC. Tripeptidyl peptidase (TRIO83) significantly reduced FCR compared to the NC birds to a level not significantly different to the PC birds (Figure 21).

*Conclusions*

In conclusion, there is a beneficial effect of tripeptidyl peptidase (TRIO83) on improving bird performance through reducing FCR and increasing BWG in nutrient deficient diets. Tripeptidyl peptidase (TRIO83) increased BWG and reduced FCR to greater levels than commercial proteases A + B.

## EXAMPLE 12

**Stability of TRI045 (SEQ ID No. 99) tripeptidyl peptidase in the presence of pepsin**

*Material and methods*

Pepsin solution: Swine pepsin from Sigma (P7000, 674 Sigma units/mg, www.sigma.com) was used in this example and prepared in MilliQ water at 10000 Sigma unit/ml at 10000 Sigma unit/mL.

Pre-incubation mixture contained: 2.5μl sedolisin sample, 95μl GAT buffer (50mM glycine-50mM acetic acid-50mM Tris, pH3.5), 5μl pepsin in milliQ water (10000 Sigma unit/ml) (final pepsin unit concentration: 500 Sigma unit/ml reaction mixture) in a half bottom area 96 well Corning MTP. For control, the pre-incubation mixture contained 5μl water instead of 5μl pepsin. The mixture was incubated at 40°C for 60min and then kept on ice. For assaying the residual activity, the assay mixture contained the pre-incubation mixture 5μl, 0.1M acetic acid-sodium acetate (pH4.0) 85μl, 5μl H-Ala-Ala-Phe-pNA (2.5mg/ml) from BACHEM.com (L-1095.0250). The 410nm reading was followed in a microplate reader every 0.5min at 30°C. N=2.

*Results*

Under the assay conditions and pre-incubation at 40°C for 60min, the residual activity was found to be over 90% for TRI045 in the presence of 500 units pepsin/mL pre-incubation mixture.

## EXAMPLE 13

**TRI045 (SEQ ID No. 99) as feed enzyme to promote feed protein hydrolysis**

Animals produce and secrete proteases into their digestive tracts for feed digestion. These proteases include endoproteases of pepsin, trypsin and chymotrypsin and the exopeptidases of Carboxypeptidase A and B etc. However, animals do not produce aminopeptidases as digestive enzymes or at least not in appreciable amount. As shown in this example, the addition of the tripeptidyl peptidase TRI045 (sedolisin) promotes protein digestion in an *in vitro* system for corn soy based feed. This example shows that TRI045 had high activity between pH 4 and 6.5

*Material and Methods*

The tripeptide substrate H-Ala-Ala-Phe-pNA (AAF-pNA from BACHEM.com, L-1095.0250) for sedolisin was prepared at 2.5mg/ml in DMSO. The reaction was prepared by mixing 8.5μl AAF-pNA, 7 μl DMSO, 32μl water and 50

μl buffer with pH4.14-6.57, and 0.8 μl or 1.5ul TRI045. The reaction was started at 30°C by the addition of the enzyme TRI045. The initial reaction rate was recorded using a microplate reader at every 0.5min interval at 410nm in 96 well plate.

*Results*

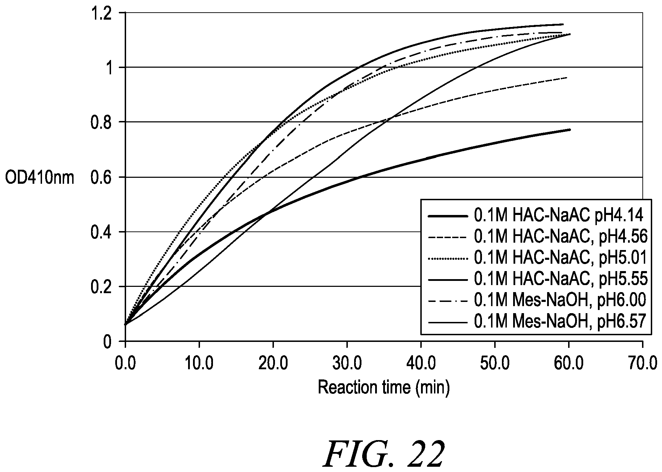

Table 12 shows that TRI045 had optimal activity around pH5.0, but still had around 50% activity at around pH4 and pH6.5, which is ideal for gastric feed digestion. Figure 22 furthers shows that the final reaction degree reached at pH5, pH5.5 and pH6 are very similar. The high pH optimum is ideal for animal feed as in such situation pancreatin would have time to make more oligopeptide substrates for TRI045.

Table 12. Effect of pH on the TRI045 activity using H-Ala-Ala-Phe-pNA as substrate (values are the average one test with 0.8μl TRI045 (n=2) and one test with 1.5μl TRI045 dose (n=1).

| Buffer | 0.1M HAC-NaAC | 0.1M HAC-NaAC | 0.1M HAC-NaAC | 0.1M HAC-NaAC | 0.1M Mes-NaOH | 0.1M Mes-NaOH |
| --- | --- | --- | --- | --- | --- | --- |
| pH | 4.1 | 4.6 | 5.0 | 5.6 | 6.0 | 6.6 |
| Relative activity with pH 5.01 as 100% | 59.4 | 81.7 | 100.0 | 80.7 | 72.2 | 48.5 |

## EXAMPLE 14

**Effect of TRI045 (SEQ ID No. 99) plus pepsin and pancreatin on hydrolysis of corn soy feed substrate.**

The *in vitro* reaction system contained 140μl 10% (w/v) corn soy feed slurry (14mg corn soy feed), 10μl TRI045 in 50m M MES-NaOH pH 6.0, 10μl swine pepsin (1,14U/μL in water). The reaction was incubated with shaking at 40°C for 45min and subsequently 34μl swine pancreatin (0.4636mg/mL in 1M Na-bicarbonate) were added and further incubated for additional 60min. After incubation, the 96-well plate was centrifuged and supernatants were used for residual TRI045 activity assay and for OPA andBCA assays (see below).

The degree of Hydrolysis measurements of soluble protein is based on the reaction of primary amino groups with o-phthaldialdehyde (OPA - assay). Reference: P.M. Nielsen, D. Petersen and C. Dambmann. Improved Method for Determining Food Protein Degree of Hydrolysis. Journal of Food Science. 66 (2001) 642-646.

For OPA assay the following procedure was carried out. 10-25 μl feed sample treated by enzyme from master plate was transferred to the new plate, then 175 μl of OPA reagent containing sodium borate, dodecyl sulfate and dithiothreitol, were added to the plate. The end point measurements of optical density at 340 nm were performed right after 2 min and 5 second mixing.

To quantify the protein concentrations of each protease samples, the Pierce BCA Protein Assay Reagent Kit (Thermo Scientific, cat no. 23228) was used. The TRI045 sample was not purified before quantification. The Pierce BCA Protein Assay Kit is a detergent-compatible formulation based on bicinchoninic acid (BCA) for colorimetric detection and quantification of total proteins. This method combines the well-known reduction of $Cu^{2+}$ to $Cu^{1+}$ by protein in an alkaline medium with the highly sensitive and selective colorimetric detection of the cuprous cat ion ($Cu^{+1}$) using a unique reagent containing bicinchoinic acid. The purple-coloured reaction product of this assay is formed by the chelation of two molecules of BCA with one cuprous ion. This water soluble complex exhibits a strong absorbance at 562nm that is nearly linear with increasing protein concentration over a broad working range (20-2000μg/mL). The macromolecular structure of protein, the number of peptide bonds and the presence of four particular amino acids Cysteine, Cystine, Trytophan and Tyrosine are reported to be responsible for colour formation with BCA.

For OPA (total amino group released) and BCA (total protein in the soluble fraction) determinations the supernatants were diluted 20 times and 10ul was used.

In the system containing TRI045 at 1000ppm, in the presence of swine pepsin and pancreatin, the release of free amino groups from corn soy feed (as a measure of protein hydrolysis (OPA value)) increased by 9% and the protein solubility increased by 5%.

In order to test the stability of TRI045 under the *in vitro* assay conditions described above (incubation in the presence of pepsin at pH3 for 45min at 40°C and subsequently in the presence of pancreatin for 60min at 40°C). The residual activity of TRI045 was subsequently assayed. The reaction mixture contained 50μl buffer 0.1M HAC-NaAC (pH5.0), 10μl the supernatant, and 5μl AAF-pNA (5mg/ml in DMSO). The reaction rate was followed at 410nm and 30°C every 30 seconds using a microplate reader. As a control, a commercial protease at the same concentration of 1000ppm was used.

**Conclusion for Examples 12-14**

The reaction rate for the control (pepsin and pancreatin only) was 4.3mOD/min, for the commercial protease it was 11.0 mOD/min, and for TRI045 it was 19.3 mOD/min. After the subtraction of the control (4.3mOD/min), the residual activity of TRI045 on AAF-pNA substrate was 2.2 times higher than for the commercial protease. These results indicate that TRI045 is stable to pepsin and pancreatin at 40°C for at least 100 minutes.

In conclusion, this example demonstrates that TRI045 is stable to pepsin and pancreatin when incubated for 105min at 40°C in the presence of corn soy feed at a pH range of 3 to 7. It has also the additional effect of increasing protein solubilization and protein hydrolysis in the presence of pepsin and pancreatin when corn soy feed was used as the substrate under conditions mimicking the monogastric digestion system (BEDFORD, M.R., & CLASSEN, H.L. (1993) "An in vitro assay for prediction of broiler intestinal viscosity and growth when fed rye-based diets in the presence of exogenous enzymes". Poultry Science, 72: 137-143)., *i.e.,* the reaction was carried out at 40°C at pH3.0-3.3 in the presence of pepsin for 45min and then pH raised to pH6.5-7.0 and addition of pancreatin for additional 60min incubation.

**Claims**

1. A method of preparing a feed additive composition comprising:

    admixing at least one proline tolerant tripeptidyl peptidase predominantly having exopeptidase activity wherein said proline tolerant tripeptidyl peptidase is capable of cleaving tri-peptides from the N-terminus of peptides having

    (i)

        (A) Proline at P1; and
        (B) An amino acid selected from alanine, arginine, asparagine, aspartic acid, cysteine, glutamine, glutamic acid, glycine, histidine, isoleucine, leucine, lysine, methionine, phenylalanine, serine, threonine, tryptophan, tyrosine, valine or synthetic amino acids at P1; and/or

    (ii)

        (a') Proline at P1'; and
        (b') An amino acid selected from alanine, arginine, asparagine, aspartic acid, cysteine, glutamine, glutamic acid, glycine, histidine, isoleucine, leucine, lysine, methionine, phenylalanine, serine, threonine, tryptophan, tyrosine, valine or synthetic amino acids or amines at P1'; and

    one or more ingredients selected from the group consisting of: a salt, polyol including sorbitol and glycerol, wheat or a wheat component, sodium acetate, sodium acetate trihydrate, potassium sorbate Talc, polyvinyl alcohol (PVA), benzoate, sorbiate, 1,3-propane diol, glucose, parabens, sodium chloride, citrate, metabisulfite, formate or a combination thereof and

    optionally packaging,
    wherein the at least one proline tolerant tripeptidyl peptidase:

        (a) comprises the amino acid sequence SEQ ID No. 29, SEQ ID No. 1, SEQ ID No. 2 or a functional fragment thereof that retains said peptidase activity;
        (b) comprises an amino acid having at least 80% or at least 90% identity to SEQ ID No. 29, SEQ ID No. 1, SEQ ID No. 2 or a functional fragment thereof that retains said peptidase activity;
        (c) is encoded by a nucleotide sequence comprising the sequence SEQ ID No. 56;
        (d) is encoded by a nucleotide sequence comprising at least 80% or at least 90% sequence identity to SEQ ID No. 56;
        (e) is encoded by a nucleotide sequence which hybridises to SEQ ID No. 56 under high stringency conditions; or
        (f) is encoded by a nucleotide sequence which differs from SEQ ID No. 56 due to degeneracy of the genetic code.

2. A method according to claim 1, wherein the feed additive composition further comprises at least one endoprotease, preferably wherein the at least one endoprotease is an acid endoprotease.

**3.** A method according to any one of the preceding claims, wherein:

the at least one proline tolerant tripeptidyl peptidase is further capable of cleaving tri-peptides from the N-terminus of a peptide having Proline at P1 and P1'; and/or

the endoprotease and proline tolerant tripeptidyl peptidase are admixed with a composition comprising at least one protein or at least a portion of a protein, wherein the composition, the endoprotease and proline tolerant tripeptidyl peptidase or combinations thereof are in a dry or substantially dry state when admixed; and/or

the at least one proline tolerant tripeptidyl peptidase or the at least one endoprotease, or a combination thereof is encapsulated or otherwise inactivated prior to admixing with said composition; and/or

the at least one protein or portion thereof is an animal protein or a vegetable protein (e.g. selected from one or more of a gliadin, a beta-casein, a beta-lactoglobulin or an immunogenic fragment of a gliadin, a beta-casein, a beta-lactoglobulin, glycinin, beta-conglycinin, cruciferin, napin, hordeins, keratins, feather or hair meals, collagen, whey protein, fish protein, fish meals, meat protein, egg protein, soy protein or grain protein), preferably comprised in corn, soybean meal, corn dried distillers grains with solubles (cDDGS), wheat, wheat proteins including gluten, wheat by products, wheat bran, wheat dried distillers grains with solubles (wDDGS), corn by products including corn gluten meal, barley, oat, rye, triticale, full fat soy, animal by-product meals, an alcohol-soluble protein (preferably a zein (e.g. a maize zein maize) and/or a kafirin (e.g. from sorghum)), a protein from oil seeds (preferably from soybean seed proteins, sun flower seed proteins, rapeseed proteins, canola seed proteins or combinations thereof) or a combination thereof; and/or

the at least one proline tolerant tripeptidyl peptidase is obtainable from *Trichoderma,* preferably *Trichoderma reesei.*

**4.** A feed additive composition or a feed ingredient comprising at least one proline tolerant tripeptidyl peptidase predominantly having exopeptidase activity wherein said proline tolerant tripeptidyl peptidase is capable of cleaving tri-peptides from the N-terminus of peptides having:

(i)

(A) Proline at P1; and
(B) An amino acid selected from alanine, arginine, asparagine, aspartic acid, cysteine, glutamine, glutamic acid, glycine, histidine, isoleucine, leucine, lysine, methionine, phenylalanine, serine, threonine, tryptophan, tyrosine, valine or synthetic amino acids at P1; and/or

(ii)

(a') Proline at P1'; and
(b') An amino acid selected from alanine, arginine, asparagine, aspartic acid, cysteine, glutamine, glutamic acid, glycine, histidine, isoleucine, leucine, lysine, methionine, phenylalanine, serine, threonine, tryptophan, tyrosine, valine or synthetic amino acids or amines at P1'; and

one or more ingredients selected from the group consisting of: a salt, polyol including sorbitol and glycerol, wheat or a wheat component, sodium acetate, sodium acetate trihydrate, potassium sorbate Talc, polyvinyl alcohol (PVA), benzoate, sorbiate, 1,3-propane diol, glucose, parabens, sodium chloride, citrate, metabisulfite, formate or a combination thereof, wherein the at least one proline tolerant tripeptidyl peptidase:

(a) comprises the amino acid sequence SEQ ID No. 29, SEQ ID No. 1, SEQ ID No. 2 or a functional fragment thereof that retains said peptidase activity;
(b) comprises an amino acid having at least 80% or at least 90% identity to SEQ ID No. 29, SEQ ID No. 1, SEQ ID No. 2 or a functional fragment thereof that retains said peptidase activity;
(c) is encoded by a nucleotide sequence comprising the sequence SEQ ID No. 56;
(d) is encoded by a nucleotide sequence comprising at least 80% or at least 90% sequence identity to SEQ ID No. 56;
(e) is encoded by a nucleotide sequence which hybridises to SEQ ID No. 56 under high stringency conditions; or
(f) is encoded by a nucleotide sequence which differs from SEQ ID No. 56 due to degeneracy of the genetic code, preferably wherein the endoprotease and/or proline tolerant tripeptidyl peptidase are inactive (or substantially inactive) in the feed additive composition prior to feeding the feed additive composition to an animal.

**5.** A feed additive composition or feed ingredient according to claim 4, wherein said feed additive composition or feed ingredient further comprises at least one endoprotease.

6. A kit comprising at least one proline tolerant tripeptidyl peptidase predominantly having exopeptidase activity wherein said proline tolerant tripeptidyl peptidase is capable of cleaving tri-peptides from the N-terminus of peptides having:

(i)

(A) Proline at P1; and
(B) An amino acid selected from alanine, arginine, asparagine, aspartic acid, cysteine, glutamine, glutamic acid, glycine, histidine, isoleucine, leucine, lysine, methionine, phenylalanine, serine, threonine, tryptophan, tyrosine, valine or synthetic amino acids at P1; and/or

(ii)

(a') Proline at P1'; and
(b') An amino acid selected from alanine, arginine, asparagine, aspartic acid, cysteine, glutamine, glutamic acid, glycine, histidine, isoleucine, leucine, lysine, methionine, phenylalanine, serine, threonine, tryptophan, tyrosine, valine or synthetic amino acids or amines at P1'; and instructions for administering same to an animal,

wherein the at least one proline tolerant tripeptidyl peptidase:

(a) comprises the amino acid sequence SEQ ID No. 29, SEQ ID No. 1, SEQ ID No. 2 or a functional fragment thereof that retains said peptidase activity;
(b) comprises an amino acid having at least 80% or at least 90% identity to SEQ ID No. 29, SEQ ID No. 1, SEQ ID No. 2 or a functional fragment thereof that retains said peptidase activity;
(c) is encoded by a nucleotide sequence comprising the sequence SEQ ID No. 56;
(d) is encoded by a nucleotide sequence comprising at least 80% or at least 90% sequence identity to SEQ ID No. 56;
(e) is encoded by a nucleotide sequence which hybridises to SEQ ID No. 56 under high stringency conditions; or
(f) is encoded by a nucleotide sequence which differs from SEQ ID No. 56 due to degeneracy of the genetic code.

7. A kit according to claim 6 wherein the kit further comprises at least one endoprotease.

8. A premix comprising a feed additive composition or feed ingredient according claim 4 or claim 5 and at least one mineral and/or at least one vitamin, preferably wherein the endoprotease and/or proline tolerant tripeptidyl peptidase are inactive (or substantially inactive) in the premix prior to feeding the premix to an animal.

9. A method of preparing a feedstuff comprising contacting a feed component with a feed additive composition or feed ingredient according claim 4 or claim 5 or a premix according to claim 8 or at least one proline tolerant tripeptidyl peptidase predominantly having exopeptidase activity wherein said proline tolerant tripeptidyl peptidase is capable of cleaving tri-peptides from the N-terminus of peptides having:

(i)

(A) Proline at P1; and
(B) An amino acid selected from alanine, arginine, asparagine, aspartic acid, cysteine, glutamine, glutamic acid, glycine, histidine, isoleucine, leucine, lysine, methionine, phenylalanine, serine, threonine, tryptophan, tyrosine, valine or synthetic amino acids at P1; and/or

(ii)

(a') Proline at P1'; and
(b') An amino acid selected from alanine, arginine, asparagine, aspartic acid, cysteine, glutamine, glutamic acid, glycine, histidine, isoleucine, leucine, lysine, methionine, phenylalanine, serine, threonine, tryptophan, tyrosine, valine or synthetic amino acids or amines at P1'

optionally in combination with at least one endoprotease preferably wherein the endoprotease and/or proline tolerant tripeptidyl peptidase are inactive (or substantially inactive) in the feedstuff prior to feeding the feedstuff to an animal, wherein the at least one proline tolerant tripeptidyl peptidase:

> (a) comprises the amino acid sequence SEQ ID No. 29, SEQ ID No. 1, SEQ ID No. 2 or a functional fragment thereof that retains said peptidase activity;
> (b) comprises an amino acid having at least 80% or at least 90% identity to SEQ ID No. 29, SEQ ID No. 1, SEQ ID No. 2 or a functional fragment thereof that retains said peptidase activity;
> (c) is encoded by a nucleotide sequence comprising the sequence SEQ ID No. 56;
> (d) is encoded by a nucleotide sequence comprising at least 80% or at least 90% sequence identity to SEQ ID No. 56;
> (e) is encoded by a nucleotide sequence which hybridises to SEQ ID No. 56 under high stringency conditions; or
> (f) is encoded by a nucleotide sequence which differs from SEQ ID No. 56 due to degeneracy of the genetic code.

10. A feedstuff comprising a feed additive composition or feed ingredient according to claim 4 or claim 5, or a premix according to claim 8, or comprising at least one proline tolerant tripeptidyl peptidase predominantly having exopeptidase activity wherein said proline tolerant tripeptidyl peptidase is capable of cleaving tri-peptides from the N-terminus of peptides having

> (i)
>
> > (A) Proline at P1; and
> > (B) An amino acid selected from alanine, arginine, asparagine, aspartic acid, cysteine, glutamine, glutamic acid, glycine, histidine, isoleucine, leucine, lysine, methionine, phenylalanine, serine, threonine, tryptophan, tyrosine, valine or synthetic amino acids at P1; and/or
>
> (ii)
>
> > (a') Proline at P1'; and
> > (b') An amino acid selected from alanine, arginine, asparagine, aspartic acid, cysteine, glutamine, glutamic acid, glycine, histidine, isoleucine, leucine, lysine, methionine, phenylalanine, serine, threonine, tryptophan, tyrosine, valine or synthetic amino acids or amines at P1' preferably wherein the endoprotease and/or proline tolerant tripeptidyl peptidase are inactive (or substantially inactive) in the in the feedstuff prior to feeding the feedstuff to an animal, wherein the at least one proline tolerant tripeptidyl peptidase:
> >
> > > (a) comprises the amino acid sequence SEQ ID No. 29, SEQ ID No. 1, SEQ ID No. 2 or a functional fragment thereof that retains said peptidase activity;
> > > (b) comprises an amino acid having at least 80% or at least 90% identity to SEQ ID No. 29, SEQ ID No. 1, SEQ ID No. 2 or a functional fragment thereof that retains said peptidase activity;
> > > (c) is encoded by a nucleotide sequence comprising the sequence SEQ ID No. 56;
> > > (d) is encoded by a nucleotide sequence comprising at least 80% or at least 90% sequence identity to SEQ ID No. 56;
> > > (e) is encoded by a nucleotide sequence which hybridises to SEQ ID No. 56 under high stringency conditions; or
> > > (f) is encoded by a nucleotide sequence which differs from SEQ ID No. 56 due to degeneracy of the genetic code.

11. A non-therapeutic method for improving a biophysical characteristic of an animal or for improving protein digestibility of an animal which method comprises administering to an animal a feed additive composition according to claim 4 or claim 5, or a feedstuff according to claim 10, or a premix according to claim 8, or at least one proline tolerant tripeptidyl peptidase predominantly having exopeptidase activity wherein said proline tolerant tripeptidyl peptidase is capable of cleaving tri-peptides from the N-terminus of peptides having:

> (i)
>
> > (A) Proline at P1; and

(B) An amino acid selected from alanine, arginine, asparagine, aspartic acid, cysteine, glutamine, glutamic acid, glycine, histidine, isoleucine, leucine, lysine, methionine, phenylalanine, serine, threonine, tryptophan, tyrosine, valine or synthetic amino acids at P1; and/or

(ii)

(a') Proline at P1'; and
(b') An amino acid selected from alanine, arginine, asparagine, aspartic acid, cysteine, glutamine, glutamic acid, glycine, histidine, isoleucine, leucine, lysine, methionine, phenylalanine, serine, threonine, tryptophan, tyrosine, valine or synthetic amino acids or amines at P1'; and

optionally at least one feed component and/or at least one mineral and/or at least one vitamin, preferably wherein the method comprises administering to an animal at least one endoprotease, wherein the at least one proline tolerant tripeptidyl peptidase:

(a) comprises the amino acid sequence SEQ ID No. 29, SEQ ID No. 1, SEQ ID No. 2 or a functional fragment thereof that retains said peptidase activity;
(b) comprises an amino acid having at least 80% or at least 90% identity to SEQ ID No. 29, SEQ ID No. 1, SEQ ID No. 2 or a functional fragment thereof that retains said peptidase activity;
(c) is encoded by a nucleotide sequence comprising the sequence SEQ ID No. 56;
(d) is encoded by a nucleotide sequence comprising at least 80% or at least 90% sequence identity to SEQ ID No. 56;
(e) is encoded by a nucleotide sequence which hybridises to SEQ ID No. 56 under high stringency conditions; or
(f) is encoded by a nucleotide sequence which differs from SEQ ID No. 56 due to degeneracy of the genetic code.

12. Use of a feed additive composition or feed ingredient according to claim 4 or claim 5, or a feed or feedstuff according to claim 10, or a premix according to claim 8, or at least one proline tolerant tripeptidyl peptidase predominantly having exopeptidase activity wherein said proline tolerant tripeptidyl peptidase is capable of cleaving tri-peptides from the N-terminus of peptides having:

(i)

(A) Proline at P1; and
(B) An amino acid selected from alanine, arginine, asparagine, aspartic acid, cysteine, glutamine, glutamic acid, glycine, histidine, isoleucine, leucine, lysine, methionine, phenylalanine, serine, threonine, tryptophan, tyrosine, valine or synthetic amino acids at P1; and/or

(ii)

(a') Proline at P1'; and
(b') An amino acid selected from alanine, arginine, asparagine, aspartic acid, cysteine, glutamine, glutamic acid, glycine, histidine, isoleucine, leucine, lysine, methionine, phenylalanine, serine, threonine, tryptophan, tyrosine, valine or synthetic amino acids or amines at P1'

for improving protein digestibility in an animal or for non-therapeutically improving a biophysical characteristic of an animal, preferably wherein at least one endoprotease is used in combination, wherein the at least one proline tolerant tripeptidyl peptidase:

(a) comprises the amino acid sequence SEQ ID No. 29, SEQ ID No. 1, SEQ ID No. 2 or a functional fragment thereof that retains said peptidase activity;
(b) comprises an amino acid having at least 80% or at least 90% identity to SEQ ID No. 29, SEQ ID No. 1, SEQ ID No. 2 or a functional fragment thereof that retains said peptidase activity;
(c) is encoded by a nucleotide sequence comprising the sequence SEQ ID No. 56;
(d) is encoded by a nucleotide sequence comprising at least 80% or at least 90% sequence identity to SEQ ID No. 56;
(e) is encoded by a nucleotide sequence which hybridises to SEQ ID No. 56 under high stringency conditions;

or

(f) is encoded by a nucleotide sequence which differs from SEQ ID No. 56 due to degeneracy of the genetic code.

**13.** A method according to claim 11 or a use according to claim 12, wherein the biophysical characteristic is selected from the group consisting of one or more of the following: performance of the animal, growth performance of an animal, feed conversion ratio (FCR), ability to digest a raw material (e.g. nutrient digestibility, including starch, fat, protein, fibre digestibility), nitrogen digestibility (e.g. ileal nitrogen digestibility) and digestible energy (e.g. ileal digestible energy), nitrogen retention, carcass yield, growth rate, weight gain, body weight, mass, feed efficiency, body fat percentage, body fat distribution, growth, egg size, egg weight, egg mass, egg laying rate, lean gain, bone ash %, bone ash mg, back fat %, milk output, milk fat %, reproductive outputs such as litter size, litter survivability, hatchability % and environmental impact, e.g. manure output and/or nitrogen excretion, preferably wherein the biophysical characteristic is the ability to digest protein.

**14.** A method according to any one of claims 1-3 or 11 or 12 or a use according to claim 12 or 13 wherein: the at least one endoprotease, proline tolerant tripeptidyl peptidase or combination thereof is admixed with the at least one protein or portion thereof immediately prior to feeding the feed additive composition to an animal; and/or
wherein the at least one endoprotease, proline tolerant tripeptidyl peptidase or combination thereof is activated by feeding the at least one endoprotease, tripeptidyl peptidase or combination thereof to an animal; and/or
the endoprotease and proline tolerant tripeptidyl peptidase is functional, or primarily functional, in the gastrointestinal tract of the animal; and/or
the at least one endoprotease and at least one proline tolerant tripeptidyl peptidase are active in the duodenum and parts of the gastrointestinal tract of the animal preceding the duodenum; and/or
when fed to an animal the feed additive composition does not substantially increase the incidence of necrotic enteritis in said animal when compared to an animal not fed said feed additive composition.

**Patentansprüche**

**1.** Verfahren zum Herstellen einer Futtermittelzusatz-Zusammensetzung, umfassend:

Zumischen von mindestens einer Prolin-toleranten Tripeptidylpeptidase, die überwiegend Exopeptidase-Aktivität aufweist, wobei die Prolin-tolerante Tripeptidylpeptidase in der Lage ist, Tripeptide vom N-Terminus von Peptiden abzuspalten, die aufweisen:

(i)

(A) Prolin an P1; und
(B) eine Aminosäure, die ausgewählt ist aus Alanin, Arginin, Asparagin, Asparaginsäure, Cystein, Glutamin, Glutaminsäure, Glycin, Histidin, Isoleucin, Leucin, Lysin, Methionin, Phenylalanin, Serin, Threonin, Tryptophan, Tyrosin, Valin oder synthetischen Aminosäuren an P1; und/oder

(ii)

(a') Prolin an P1'; und
(b') eine Aminosäure, die ausgewählt ist aus Alanin, Arginin, Asparagin, Asparaginsäure, Cystein, Glutamin, Glutaminsäure, Glycin, Histidin, Isoleucin, Leucin, Lysin, Methionin, Phenylalanin, Serin, Threonin, Tryptophan, Tyrosin, Valin oder synthetischen Aminosäuren oder Aminen an P1'; und

einen oder mehrere Bestandteile, die ausgewählt sind aus der Gruppe bestehend aus: einem Salz, einem Polyol, einschließend Sorbit und Glycerin, Weizen oder einer Weizenkomponente, Natriumacetat, Natriumacetat-trihydrat, Kaliumsorbat Talkum, Polyvinylalkohol (PVA), Benzoat, Sorbat, 1,3-Propandiol, Glucose, Parabenen, Natriumchlorid, Citrat, Metabisulfit, Formiat oder einer Kombination davon; und
gegebenenfalls Verpacken,
wobei die mindestens eine Prolin-tolerante Tripeptidylpeptidase:

(a) die Aminosäuresequenz SEQ ID No. 29, SEQ ID No. 1, SEQ ID No. 2, oder ein funktionelles Fragment davon umfasst, das die Peptidase-Aktivität bewahrt;

(b) eine Aminosäure umfasst, die mindestens 80% oder mindestens 90% Identität zu SEQ ID No. 29, SEQ ID No. 1, SEQ ID No. 2, oder einem funktionellen Fragment davon aufweist, das die Peptidase-Aktivität bewahrt;

(c) codiert wird durch eine Nucleotidsequenz, die die Sequenz ID No. 56 umfasst;

(d) codiert wird durch eine Nucleotidsequenz, die mindestens 80% oder mindestens 90% Identität zu SEQ ID No. 56 umfasst;

(e) codiert wird durch eine Nucleotidsequenz, die unter Bedingungen hoher Stringenz hybridisiert wird zu SEQ ID NO: 56; oder

(f) codiert wird durch eine Nucleotidsequenz, die sich von SEQ ID NO: 56 aufgrund einer Degeneration des genetischen Codes unterscheidet.

2. Verfahren nach Anspruch 1, wobei die Futtermittelzusatz-Zusammensetzung ferner mindestens eine Endoprotease umfasst, bevorzugt wobei die mindestens eine Endoprotease eine saure Endoprotease ist.

3. Verfahren nach einem der vorhergehenden Ansprüche, wobei:

die mindestens eine Prolin-tolerante Tripeptidylpeptidase in der Lage ist, Tripeptide von dem N-Terminus eines Peptids abzuspalten, das an P1 und P1' Prolin aufweist; und/oder

die Endoprotease und Prolin-tolerante Tripeptidylpeptidase mit einer Zusammensetzung abgemischt werden, die mindestens ein Protein oder mindestens einen Teil eines Proteins umfasst, wobei sich, wenn sie abgemischt werden, die Zusammensetzung, die Endoprotease und Prolin-tolerante Tripeptidylpeptidase oder Kombinationen davon in einem trockenen Zustand oder weitgehend trockenen Zustand befinden; und/oder

die mindestens eine Prolin-tolerante Tripeptidylpeptidase oder die mindestens eine Endoprotease oder eine Kombination davon verkapselt werden oder auf andere Weise vor dem Abmischen mit der Zusammensetzung inaktiviert werden; und/oder

das mindestens eine Protein oder ein Teil davon ein tierisches Protein oder pflanzliches Protein ist (das z.B. ausgewählt ist aus einem oder mehreren der folgenden: einem Gliadin, einem Beta-Casein, einem Beta-Lactoglobulin oder einem immunogenen Fragment eines Gliadins, einem Beta-Casein, einem Beta-Lactoglobulin, Glycinin, Beta-Conglycinin, Cruciferin, Napin, Hordeinen, Keratinen, Feder- oder Haarmehlen, Kollagen, Molkenprotein, Fischprotein, Fischmehlen, Fleischprotein, Eiprotein, Sojaprotein oder Getreideprotein), die bevorzugt enthalten sind in Mais, Sojabohnenmehl, maistrockener Getreideschlempe mit löslichen Stoffen (cDDGS), Weizen, Weizenproteinen, einschließend Gluten, Weizennebenprodukten, Weizenkleie, weizentrockener Getreideschlempe mit löslichen Stoffen (wDDGS); Maisnebenprodukten, einschließend Maisglutenmehl, Gerste, Hafer, Roggen, Triticale, Vollfettsoja, tierischen Nebenproduktmehlen, einem alkohollöslichen Protein (vorzugsweise ein Zein (z.B. ein Mais-Zein-Mais) und/oder einem Kafirin (z.B. aus Sorghum)), einem Protein aus Ölsaaten (bevorzugt aus Sojabohnensamen-Protein, Sonnenblumensamenproteine, Rapssamenproteine, Rapsproteine oder Kombinationen davon), oder einer Kombination davon; und/oder

die mindestens eine Prolin-tolerante Tripeptidylpeptidase kann von *Trichoderma,* bevorzugt *Trichoderma reesei,* erhalten werden.

4. Futtermittelzusatz-Zusammensetzung oder ein Futtermittelbestandteil, umfassend mindestens eine Prolin-tolerante Tripeptidylpeptidase, die überwiegend Exopeptidase-Aktivität aufweist, wobei die Prolin-tolerante Tripeptidylpeptidase in der Lage ist, von dem N-Terminus von Peptiden Tripeptide abzuspalten, die aufweisen:

(i)

(A) Prolin an P1; und
(B) eine Aminosäure, die ausgewählt ist aus Alanin, Arginin, Asparagin, Asparaginsäure, Cystein, Glutamin, Glutaminsäure, Glycin, Histidin, Isoleucin, Leucin, Lysin, Methionin, Phenylalanin, Serin, Threonin, Tryptophan, Tyrosin, Valin oder synthetischen Aminosäuren an P1; und/oder

(ii)

(a') Prolin an P1'; und
(b') eine Aminosäure, die ausgewählt ist aus Alanin, Arginin, Asparagin, Asparaginsäure, Cystein, Glutamin, Glutaminsäure, Glycin, Histidin, Isoleucin, Leucin, Lysin, Methionin, Phenylalanin, Serin, Threonin, Tryptophan, Tyrosin, Valin oder synthetischen Aminosäuren oder Aminen an P1'; und

einen oder mehrere Bestandteile, die ausgewählt sind aus der Gruppe bestehend aus: einem Salz, einem Polyol, einschließlich Sorbit und Glycerin, Weizen oder einer Weizenkomponente, Natriumacetat, Natriumacetat-trihydrat, Kaliumsorbat Talkum, Polyvinylalkohol (PVA), Benzoat, Sorbat, 1,3-Propandiol, Glucose, Parabenen, Natriumchlorid, Citrat, Metabisulfit, Formiat oder eine Kombination davon, wobei die mindestens eine Prolin-tolerante Tripeptidylpeptidase:

(a) die Aminosäuresequenz SEQ ID No. 29, SEQ ID No. 1, SEQ ID No. 2, oder ein funktionelles Fragment davon umfasst, das die Peptidase-Aktivität bewahrt;
(b) eine Aminosäure umfasst, die mindestens 80% oder mindestens 90% Identität zu SEQ ID No. 29, SEQ ID No. 1, SEQ ID No. 2, oder einem funktionellen Fragment davon aufweist, das die Peptidase-Aktivität bewahrt;
(c) codiert wird durch eine Nucleotidsequenz, die die Sequenz ID No. 56 umfasst;
(d) codiert wird durch eine Nucleotidsequenz, die mindestens 80% oder mindestens 90% Identität zu SEQ ID No. 56 umfasst;
(e) codiert wird durch eine Nucleotidsequenz, die unter Bedingungen hoher Stringenz hybridisiert wird zu SEQ ID NO: 56; oder
(f) codiert wird durch eine Nucleotidsequenz, die sich von SEQ ID NO: 56 aufgrund einer Degeneration des genetischen Codes unterscheidet, bevorzugt wobei die Endoprotease und/oder Prolin-tolerante Tripeptidylpeptidase in der Futtermittelzusatz-Zusammensetzung inaktiv sind (oder im Wesentlichen inaktiv sind), bevor die Futtermittelzusatz-Zusammensetzung an ein Tier gefüttert wird.

5. Futtermittelzusatz-Zusammensetzung oder Futtermittelbestandteil nach Anspruch 4, wobei die Futtermittelzusatz-Zusammensetzung oder der Futtermittelbestandteil ferner mindestens eine Endoprotease umfassen.

6. Kit umfassend mindestens eine Prolin-tolerante Tripeptidylpeptidase, die überwiegend eine Exopeptidase-Aktivität aufweist, wobei die Prolin-tolerante Tripeptidylpeptidase in der Lage ist, Tripeptide von dem N-Terminus von Peptiden abzuspalten, die aufweisen:

(i)

(A) Prolin bei P1; und
(B) eine Aminosäure, die ausgewählt ist aus Alanin, Arginin, Asparagin, Asparaginsäure, Cystein, Glutamin, Glutaminsäure, Glycin, Histidin, Isoleucin, Leucin, Lysin, Methionin, Phenylalanin, Serin, Threonin, Tryptophan, Tyrosin, Valin oder synthetischen Aminosäuren an P1; und/oder

(ii)

(a') Prolin an P1'; und
(b') eine Aminosäure, die ausgewählt ist aus Alanin, Arginin, Asparagin, Asparaginsäure, Cystein, Glutamin, Glutaminsäure, Glycin, Histidin, Isoleucin, Leucin, Lysin, Methionin, Phenylalanin, Serin, Threonin, Tryptophan, Tyrosin, Valin oder synthetischen Aminosäuren oder Aminen an P1' sowie Anweisungen zum Verabreichen derselben an ein Tier,

wobei die mindestens eine Prolin-tolerante Tripeptidylpeptidase:

(a) die Aminosäuresequenz SEQ ID No. 29, SEQ ID No. 1, SEQ ID No. 2, oder ein funktionelles Fragment davon umfasst, das die Peptidase-Aktivität bewahrt;
(b) eine Aminosäure umfasst, die mindestens 80% oder mindestens 90% Identität zu SEQ ID No. 29, SEQ ID No. 1, SEQ ID No. 2, oder einem funktionellen Fragment davon aufweist, das die Peptidase-Aktivität bewahrt;
(c) codiert wird durch eine Nucleotidsequenz, die die Sequenz ID No: 56 umfasst;
(d) codiert wird durch eine Nucleotidsequenz, die mindestens 80% oder mindestens 90% Identität zu SEQ ID No. 56 umfasst;
(e) codiert wird durch eine Nucleotidsequenz, die unter Bedingungen hoher Stringenz hybridisiert wird zu SEQ ID NO: 56; oder
(f) codiert wird durch eine Nucleotidsequenz, die sich von SEQ ID NO: 56 aufgrund einer Degeneration des genetischen Codes unterscheidet.

**7.** Kit nach Anspruch 6, wobei das Kit ferner mindestens eine Endoprotease umfasst.

**8.** Vormischfutter, umfassend eine Futtermittelzusatz-Zusammensetzung oder einen Futtermittelbestandteil nach Anspruch 4, oder Anspruch 5 und mindestens ein Mineral und/oder mindestens ein Vitamin, bevorzugt wobei die Endoprotease und/oder Prolin-tolerante Tripeptidylpeptidase in dem Vormischfutter inaktiv sind (oder im Wesentlichen inaktiv sind), bevor das Vormischfutter an ein Tier gefüttert wird.

**9.** Verfahren zum Herstellen eines Futtermittels, umfassend Inkontaktbringen einer Futtermittelkomponente mit einer Futtermittelzusatz-Zusammensetzung oder einem Futtermittelbestandteil nach Anspruch 4 oder Anspruch 5 oder einem Vormischfutter nach Anspruch 8 oder mindestens einer Prolin-toleranten Tripeptidylpeptidase, die überwiegend Exopeptidase-Aktivität aufweist, wobei die Prolin-tolerante Tripeptidylpeptidase in der Lage ist, Tripeptide von dem N-Terminus von Peptiden abzuspalten, die aufweisen:

    (i)

        (A) Prolin an P1; und
        (B) eine Aminosäure, die ausgewählt ist aus Alanin, Arginin, Asparagin, Asparaginsäure, Cystein, Glutamin, Glutaminsäure, Glycin, Histidin, Isoleucin, Leucin, Lysin, Methionin, Phenylalanin, Serin, Threonin, Tryptophan, Tyrosin, Valin oder synthetischen Aminosäuren an P1; und/oder

    (ii)

        (a') Prolin an P1'; und
        (b') eine Aminosäure, die ausgewählt ist aus Alanin, Arginin, Asparagin, Asparaginsäure, Cystein, Glutamin, Glutaminsäure, Glycin, Histidin, Isoleucin, Leucin, Lysin, Methionin, Phenylalanin, Serin, Threonin, Tryptophan, Tyrosin, Valin oder synthetischen Aminosäuren oder Aminen an P1', wahlweise in Kombination mit mindestens einer Endoprotease, bevorzugt wobei die Endoprotease und/oder Prolin-tolerante Tripeptidylpeptidase in dem Futtermittel vor dem Verfüttern an ein Tier inaktiv sind (oder weitgehend inaktiv sind), wobei die mindestens eine Prolin-tolerante Tripeptidylpeptidase:

        (a) die Aminosäuresequenz SEQ ID No. 29, SEQ ID No. 1, SEQ ID No. 2, oder ein funktionelles Fragment davon umfasst, das die Peptidase-Aktivität bewahrt;
        (b) eine Aminosäure umfasst, die mindestens 80% oder mindestens 90% Identität zu SEQ ID No. 29, SEQ ID No. 1, SEQ ID No. 2, oder einem funktionellen Fragment davon aufweist, das die Peptidase-Aktivität bewahrt;
        (c) codiert wird durch eine Nucleotidsequenz, die die Sequenz ID No: 56 umfasst;
        (d) codiert wird durch eine Nucleotidsequenz, die mindestens 80% oder mindestens 90% Identität zu SEQ ID No. 56 umfasst;
        (e) codiert wird durch eine Nucleotidsequenz, die unter Bedingungen hoher Stringenz hybridisiert wird zu SEQ ID NO: 56; oder
        (f) codiert wird durch eine Nucleotidsequenz, die sich von SEQ ID NO: 56 aufgrund einer Degeneration des genetischen Codes unterscheidet.

**10.** Futtermittel, umfassend eine Futtermittelzusatz-Zusammensetzung oder einen Futtermittelbestandteil nach Anspruch 4 oder Anspruch 5 oder ein Vormischfutter nach Anspruch 8 oder umfassend mindestens eine Prolin-tolerante Tripeptidylpeptidase , die überwiegend Exopeptidase-Aktivität aufweist, wobei die Prolin-tolerante Tripeptidylpeptidase in der Lage ist, Tripeptide von dem N-Terminus von Peptiden abzuspalten, die aufweisen:

    (i)

        (A) Prolin an P1; und
        (B) eine Aminosäure, die ausgewählt ist aus Alanin, Arginin, Asparagin, Asparaginsäure, Cystein, Glutamin, Glutaminsäure, Glycin, Histidin, Isoleucin, Leucin, Lysin, Methionin, Phenylalanin, Serin, Threonin, Tryptophan, Tyrosin, Valin oder synthetischen Aminosäuren an P1; und/oder

    (ii)

        (a') Prolin an P1'; und

(b') eine Aminosäure, die ausgewählt ist aus Alanin, Arginin, Asparagin, Asparaginsäure, Cystein, Glutamin, Glutaminsäure, Glycin, Histidin, Isoleucin, Leucin, Lysin, Methionin, Phenylalanin, Serin, Threonin, Tryptophan, Tyrosin, Valin oder synthetischen Aminosäuren oder Aminen an P1', bevorzugt wobei die Endoprotease und/oder Prolin-tolerante Tripeptidylpeptidase in dem Futtermittel vor dem Verfüttern des Futtermittels an ein Tier inaktiv sind (oder weitgehend inaktiv sind), wobei die mindestens eine Prolin-tolerante Tripeptidylpeptidase:

(a) die Aminosäuresequenz SEQ ID No. 29, SEQ ID No. 1, SEQ ID No. 2, oder ein funktionelles Fragment davon umfasst, das die Peptidase-Aktivität bewahrt;

(b) eine Aminosäure umfasst, die mindestens 80% oder mindestens 90% Identität zu SEQ ID No. 29, SEQ ID No. 1, SEQ ID No. 2, oder einem funktionellen Fragment davon aufweist, das die Peptidase-Aktivität bewahrt;

(c) codiert wird durch eine Nucleotidsequenz, die die Sequenz ID No: 56 umfasst;

(d) codiert wird durch eine Nucleotidsequenz, die mindestens 80% oder mindestens 90% Identität zu SEQ ID No. 56 umfasst;

(e) codiert wird durch eine Nucleotidsequenz, die unter Bedingungen hoher Stringenz hybridisiert wird zu SEQ ID NO: 56; oder

(f) codiert wird durch eine Nucleotidsequenz, die sich von SEQ ID NO: 56 aufgrund einer Degeneration des genetischen Codes unterscheidet.

11. Nichttherapeutisches Verfahren zum Verbessern eines biophysikalischen Merkmals eines Tieres oder zum Verbessern der Proteinverdaulichkeit eines Tieres, wobei das Verfahren Verabreichen an ein Tier einer Futtermittelzusatz-Zusammensetzung nach Anspruch 4 oder Anspruch 5 oder eines Futtermittels nach Anspruch 10 oder eines Vormischfutters nach Anspruch 8 oder mindestens einer Prolin-toleranten Tripeptidylpeptidase umfasst, die überwiegend Exopeptidase-Aktivität aufweist, wobei die Prolin-tolerante Tripeptidylpeptidase in der Lage ist, Tripeptide von dem N-Terminus von Peptiden abzuspalten, die aufweisen:

(i)

(A) Prolin an P1; und

(B) eine Aminosäure, die ausgewählt ist aus Alanin, Arginin, Asparagin, Asparaginsäure, Cystein, Glutamin, Glutaminsäure, Glycin, Histidin, Isoleucin, Leucin, Lysin, Methionin, Phenylalanin, Serin, Threonin, Tryptophan, Tyrosin, Valin oder synthetischen Aminosäuren an P1; und/oder

(ii)

(a') Prolin an P1'; und

(b') eine Aminosäure, die ausgewählt ist aus Alanin, Arginin, Asparagin, Asparaginsäure, Cystein, Glutamin, Glutaminsäure, Glycin, Histidin, Isoleucin, Leucin, Lysin, Methionin, Phenylalanin, Serin, Threonin, Tryptophan, Tyrosin, Valin oder synthetischen Aminosäuren oder Aminen an P1', wahlweise mindestens eine Futtermittelkomponente und/oder mindestens ein Mineral und/oder mindestens ein Vitamin, wobei das Verfahren das Verabreichen an ein Tier mindestens einer Endoprotease umfasst, wobei die mindestens eine Prolin-tolerante Tripeptidylpeptidase:

(a) die Aminosäuresequenz SEQ ID No. 29, SEQ ID No. 1, SEQ ID No. 2, oder ein funktionelles Fragment davon umfasst, das die Peptidase-Aktivität bewahrt;

(b) eine Aminosäure umfasst, die mindestens 80% oder mindestens 90% Identität zu SEQ ID No. 29, SEQ ID No. 1, SEQ ID No. 2, oder einem funktionellen Fragment davon aufweist, das die Peptidase-Aktivität bewahrt;

(c) codiert wird durch eine Nucleotidsequenz, die die Sequenz ID No: 56 umfasst;

(d) codiert wird durch eine Nucleotidsequenz, die mindestens 80% oder mindestens 90% Identität zu SEQ ID No. 56 umfasst;

(e) codiert wird durch eine Nucleotidsequenz, die unter Bedingungen hoher Stringenz hybridisiert wird zu SEQ ID NO: 56; oder

(f) codiert wird durch eine Nucleotidsequenz, die sich von SEQ ID NO: 56 aufgrund einer Degeneration des genetischen Codes unterscheidet.

12. Verwendung einer Futtermittelzusatz-Zusammensetzung oder eines Futtermittelbestandteils nach Anspruch 4 oder Anspruch 5 oder eines Futters oder Futtermittels nach Anspruch 10 oder eines Vormischfutters nach Anspruch 8 oder mindestens einer Prolin-toleranten Tripeptidylpeptidase, die überwiegend Exopeptidase-Aktivität aufweist, wobei die Prolin-tolerante Tripeptidylpeptidase in der Lage ist, Tripeptide von dem N-Terminus von Peptiden abzuspalten, die aufweisen:

(i)

(A) Prolin an P1; und
(B) eine Aminosäure, die ausgewählt ist aus Alanin, Arginin, Asparagin, Asparaginsäure, Cystein, Glutamin, Glutaminsäure, Glycin, Histidin, Isoleucin, Leucin, Lysin, Methionin, Phenylalanin, Serin, Threonin, Tryptophan, Tyrosin, Valin oder synthetischen Aminosäuren an P1; und/oder

(ii)

(a') Prolin an P1'; und
(b') eine Aminosäure, die ausgewählt ist aus Alanin, Arginin, Asparagin, Asparaginsäure, Cystein, Glutamin, Glutaminsäure, Glycin, Histidin, Isoleucin, Leucin, Lysin, Methionin, Phenylalanin, Serin, Threonin, Tryptophan, Tyrosin, Valin oder synthetischen Aminosäuren oder Aminen an P1'

zum Verbessern der Proteinverdaulichkeit in einem Tier oder zum nichttherapeutischen Verbessern eines biophysikalischen Merkmals eines Tiers, wobei mindestens eine Endoprotease in Kombination verwendet wird, wobei die mindestens eine Prolin-tolerante Tripeptidylpeptidase:

(a) die Aminosäuresequenz SEQ ID No. 29, SEQ ID No. 1, SEQ ID No. 2, oder ein funktionelles Fragment davon umfasst, das die Peptidase-Aktivität bewahrt;
(b) eine Aminosäure umfasst, die mindestens 80% oder mindestens 90% Identität zu SEQ ID No. 29, SEQ ID No. 1, SEQ ID No. 2, oder einem funktionellen Fragment davon aufweist, das die Peptidase-Aktivität bewahrt;
(c) codiert wird durch eine Nucleotidsequenz, die die Sequenz ID No: 56 umfasst;
(d) codiert wird durch eine Nucleotidsequenz, die mindestens 80% oder mindestens 90% Identität zu SEQ ID No. 56 umfasst;
(e) codiert wird durch eine Nucleotidsequenz, die unter Bedingungen hoher Stringenz hybridisiert wird zu SEQ ID NO: 56; oder
(f) codiert wird durch eine Nucleotidsequenz, die sich von SEQ ID NO: 56 aufgrund einer Degeneration des genetischen Codes unterscheidet.

13. Verfahren nach Anspruch 11 oder Verwendung nach Anspruch 12, wobei das biophysikalische Merkmal ausgewählt ist aus der Gruppe bestehend aus einem oder mehrere der folgenden: Leistung des Tieres, Wachstumsleistung eines Tiers, Futterquotient (FCR), Fähigkeit, ein Rohmaterial zu verdauen (z.B. Nährstoffverdaulichkeit, einschließlich Stärke-, Fett-, Protein-, Faserverdaulichkeit), Stickstoffverdaulichkeit (z. B. ileale Stickstoffverdaulichkeit) und verdauliche Energie (z.B. ileal verdauliche Energie), Stickstoffretention, Schlachtkörperausbeute, Wachstumsrate, Gewichtszunahme, Körpergewicht, Masse, Futtereffizienz, Körperfettanteil, Körperfettverteilung, Wachstum, Eigröße, Eigewicht, Eimasse, Eiablagerate, Magerzuwachs, Knochenasche in %, Knochenasche in mg, Rückenfett in %, Milchleistung, Milchfett in %, Reproduktionsleistung, wie beispielsweise Wurfgröße, wurfbasierte Überlebensfähigkeit, Schlupffähigkeit in % und Umweltauswirkungen, z.B. Gülleanfall und/oder Stickstoffausscheidung, wobei das biophysikalische Merkmal bevorzugt die Fähigkeit ist, Protein zu verdauen.

14. Verfahren nach einem der Ansprüche 1 bis 3 oder 11 oder 12 oder eine Verwendung nach Anspruch 12 oder 13, wobei: die mindestens eine Endoprotease, Prolin-tolerante Tripeptidylpeptidase oder Kombinationen davon mit dem mindestens einen Protein oder Teil davon unmittelbar vor dem Füttern an ein Tier abgemischt wird; und/oder wobei die mindestens eine Endoprotease, Prolin-tolerante Tripeptidylpeptidase oder Kombinationen davon aktiviert werden, indem die mindestens eine Endoprotease, Prolin-tolerante Tripeptidylpeptidase oder Kombinationen davon an ein Tier gefüttert werden; und/oder die Endoprotease und Prolin-tolerante Tripeptidylpeptidase in dem gastrointestinalen Trakt des Tieres funktionsfähig oder primär funktionsfähig sind; und/oder die mindestens eine Endoprotease und die mindestens eine Prolin-tolerante Tripeptidylpeptidase in dem Duodenum und Teilen des gastrointestinalen Trakts des Tieres aktiv sind, die dem Duodenum vorgelagert sind; und/oder, bei Fütterung an ein Tier die Futtermittelzusatz-Zusammensetzung die Inzidenz von nekrotischer Enteritis in dem Tier im Vergleich zu einem Tier nicht wesentlich erhöht, das nicht mit dem Futtermittelzusatz-Zusammensetzung gefüttert wird.

**Revendications**

1. Procédé de préparation d'une composition d'additifs alimentaires, comprenant:

ajouter et mélanger au moins une tripeptidyl peptidase tolérante à la proline ayant de manière prédominante une activité d'exopeptidase, où ladite tripeptidyl peptidase tolérante à la proline est capable de cliver des tripeptides de la terminaison N de peptides ayant

(i)

(A) une proline à P1; et
(B) un acide aminé sélectionné parmi alanine, arginine, asparagine, acide aspartique, cystéine, glutamine, acide glutamique, glycine, histidine, isoleucine, leucine, lysine, méthionine, phénylalanine, sérine, thréonine, tryptophane, tyrosine, valine ou bien des acides aminés synthétiques à P1; et/ou

(ii)

(a') une proline à P1'; et
(b') un acide aminé sélectionné parmi alanine, arginine, asparagine, acide aspartique, cystéine, glutamine, acide glutamique, glycine, histidine, isoleucine, leucine, lysine, méthionine, phénylalanine, sérine, thréonine, tryptophane, tyrosine, valine ou bien des acides aminés synthétiques ou des amines à P1'; et

un ou plusieurs ingrédients sélectionnés parmi le groupe consistant en: un sel, polyol y compris sorbitol et glycérol, blé ou composant de blé, acétate de sodium, acétate de sodium trihydraté, sorbate de potassium talc, alcool polyvinylique (PVA), benzoate, sorbiate, 1,3-propane diol, glucose, parabens, chlorure de sodium, citrate, métabisulfite, formiate ou une combinaison de ceux-ci et
optionnellement un emballage,
où la au moins une tripeptidyl peptidase tolérante à la proline:

(a) comprend la séquence d'acides aminés SEQ ID NO: 29, SEQ ID NO: 1, SEQ ID NO: 2 ou un fragment fonctionnel de celle-ci qui conserve ladite activité de peptidase;
(b) comprend un acide aminé ayant au moins 80 % ou au moins 90 % d'identité avec la SEQ ID NO: 29, SEQ ID NO: 1, SEQ ID NO: 2 ou un fragment fonctionnel de celui-ci qui conserve ladite activité de peptidase;
(c) est codée par une séquence de nucléotides comprenant la séquence SEQ ID NO: 56;
(d) est codée par une séquence de nucléotides comprenant au moins 80 % ou au moins 90 % d'identité de séquence avec la SEQ ID NO: 56;
(e) est codée par une séquence de nucléotides qui s'hybride à la SEQ ID NO: 56 dans des conditions de stringence élevée; ou bien
(f) est codée par une séquence de nucléotides qui diffère de la SEQ ID NO: 56 du fait de la dégénérescence du code génétique.

2. Procédé selon la revendication 1, dans lequel la composition d'additifs alimentaires comprend en outre au moins une endoprotéase, de préférence dans lequel la au moins une endoprotéase est une endoprotéase acide.

3. Procédé selon l'une quelconque des revendications précédentes, dans lequel:

la au moins une tripeptidyl peptidase tolérante à la proline est en outre capable de cliver des tripeptides de la terminaison N d'un peptide ayant une proline à P1 et P1'; et/ou
l'endoprotéase et la tripeptidyl protéase tolérante à la proline sont ajoutées à et mélangées avec une composition comprenant au moins une protéine ou au moins une partie d'une protéine, où la composition, l'endoprotéase et la tripeptidyl protéase tolérante à la proline ou des combinaisons de celles-ci sont dans un état sec ou sensiblement sec lorsque ajoutées et mélangées; et/ou
la au moins une tripeptidyl protéase tolérante à la proline ou la au moins une endoprotéase, ou une combinaison de celles-ci, est encapsulée ou inactivée autrement préalablement à l'ajout à et mélange avec ladite composition; et/ou

la au moins une protéine ou partie de celle-ci est une protéine animale ou une protéine végétale (sélectionnée par ex. parmi une ou plusieurs d'entre une gliadine, une bêta-caséine, une bêta-lactoglobuline ou un fragment immunogène d'une gliadine, d'une bêta-caséine, d'une bêta-lactoglobuline, glycine, bêta-conglycine, cruciférine, napine, hordéines, kératines, farines de plume ou poils, collagène, protéine de lactosérum, protéine de poisson, farines de poisson, protéine de viande, protéine d'œuf, protéine de soja ou protéine de graine), présente de préférence dans du maïs, farine de soja, drêche sèche de maïs de distillerie avec solubles (cDDGS), blé, protéines de blé gluten compris, sous-produits de blé, son de blé, drêche sèche de blé de distillerie avec solubles (wDDGS), sous-produits de maïs y compris farine de gluten de maïs, orge, avoine, seigle, triticale, soja entier, farines de sous-produits animaux, une protéine soluble dans l'alcool (de préférence une zéine (par exemple une zéine de maïs) et/ou une kafirine (par ex. du sorgho)), une protéine de graines oléagineuses (de préférence de protéines de graines de soja, de protéines de graines de tournesol, de protéines de graines de colza, de protéines de graines de canola ou des combinaisons de celles-ci) ou une combinaison de celles-ci; et/ou

la au moins une tripeptidyl peptidase tolérante à la proline peut être obtenue de *Trichoderma*, de préférence de *Trichoderma reesei.*

4. Composition d'additifs alimentaires ou ingrédient alimentaire pour animaux comprenant au moins une tripeptidyl peptidase tolérante à la proline ayant de manière prédominante une activité d'exopeptidase où ladite tripeptidyl peptidase tolérante à la proline est capable de cliver des tripeptides de la terminaison N de peptides ayant:

   (i)

   (A) une proline à P1; et
   (B) un acide aminé sélectionné parmi alanine, arginine, asparagine, acide aspartique, cystéine, glutamine, acide glutamique, glycine, histidine, isoleucine, leucine, lysine, méthionine, phénylalanine, sérine, thréonine, tryptophane, tyrosine, valine ou bien des acides aminés synthétiques à P1; et/ou

   (ii)

   (a') une proline à P1'; et
   (b') un acide aminé sélectionné parmi alanine, arginine, asparagine, acide aspartique, cystéine, glutamine, acide glutamique, glycine, histidine, isoleucine, leucine, lysine, méthionine, phénylalanine, sérine, thréonine, tryptophane, tyrosine, valine ou bien des acides aminés synthétiques ou des amines à P1'; et

   un ou plusieurs ingrédients sélectionnés parmi le groupe consistant en: un sel, polyol y compris sorbitol et glycérol, blé ou composant de blé, acétate de sodium, acétate de sodium trihydraté, sorbate de potassium talc, alcool polyvinylique (PVA), benzoate, sorbiate, 1,3-propane diol, glucose, parabens, chlorure de sodium, citrate, métabisulfite, formiate ou une combinaison de ceux-ci, où la au moins une tripeptidyl peptidase tolérante à la proline:

   (a) comprend la séquence d'acides aminés SEQ ID NO: 29, SEQ ID NO: 1, SEQ ID NO: 2 ou un fragment fonctionnel de celle-ci qui conserve ladite activité de peptidase;
   (b) comprend un acide aminé ayant au moins 80 % ou au moins 90 % d'identité avec la SEQ ID NO: 29, SEQ ID NO: 1, SEQ ID NO: 2 ou un fragment fonctionnel de celui-ci qui conserve ladite activité de peptidase;
   (c) est codée par une séquence de nucléotides comprenant la séquence SEQ ID NO: 56;
   (d) est codée par une séquence de nucléotides comprenant au moins 80 % ou au moins 90 % d'identité de séquence avec la SEQ ID NO: 56;
   (e) est codée par une séquence de nucléotides qui s'hybride à la SEQ ID NO: 56 dans des conditions de stringence élevée; ou bien
   (f) est codée par une séquence de nucléotides qui diffère de la SEQ ID NO: 56 du fait de la dégénérescence du code génétique, de préférence où l'endoprotéase et/ou la tripeptidyl protéase tolérante à la proline sont inactives (ou sensiblement inactives) dans la composition d'additifs alimentaires avant d'administrer la composition d'additifs alimentaires à un animal.

5. Composition d'additifs alimentaires ou ingrédient alimentaire pour animaux selon la revendication 4, où ladite composition d'additifs alimentaires ou ingrédient alimentaire pour animaux comprend en outre au moins une endoprotéase.

6. Kit comprenant au moins une tripeptidyl protéase tolérante à la proline ayant de manière prédominante une activité d'exopeptidase où ladite tripeptidyl protéase tolérante à la proline est capable de cliver des tripeptides de la terminaison N de peptides ayant:

(i)

(A) une proline à P1; et
(B) un acide aminé sélectionné parmi alanine, arginine, asparagine, acide aspartique, cystéine, glutamine, acide glutamique, glycine, histidine, isoleucine, leucine, lysine, méthionine, phénylalanine, sérine, thréonine, tryptophane, tyrosine, valine ou bien des acides aminés synthétiques à P1; et/ou

(ii)

(a') une proline à P1'; et
(b') un acide aminé sélectionné parmi alanine, arginine, asparagine, acide aspartique, cystéine, glutamine, acide glutamique, glycine, histidine, isoleucine, leucine, lysine, méthionine, phénylalanine, sérine, thréonine, tryptophane, tyrosine, valine ou bien des acides aminés synthétiques ou des amines à P1';

et des instructions pour son administration à un animal,
où la au moins une tripeptidyl protéase tolérante à la proline:

(a) comprend la séquence d'acides aminés SEQ ID NO: 29, SEQ ID NO: 1, SEQ ID NO: 2 ou un fragment fonctionnel de celle-ci qui conserve ladite activité de peptidase;
(b) comprend un acide aminé ayant au moins 80 % ou au moins 90 % d'identité avec la SEQ ID NO: 29, SEQ ID NO: 1, SEQ ID NO: 2 ou un fragment fonctionnel de celui-ci qui conserve ladite activité de peptidase;
(c) est codée par une séquence de nucléotides comprenant la séquence SEQ ID NO: 56;
(d) est codée par une séquence de nucléotides comprenant au moins 80 % ou au moins 90 % d'identité de séquence avec la SEQ ID NO: 56;
(e) est codée par une séquence de nucléotides qui s'hybride à la SEQ ID NO: 56 dans des conditions de stringence élevée; ou bien
(f) est codée par une séquence de nucléotides qui diffère de la SEQ ID NO: 56 du fait de la dégénérescence du code génétique.

7. Kit selon la revendication 6, où le kit comprend en outre au moins une endoprotéase.

8. Prémélange comprenant une composition d'additifs alimentaires ou ingrédient alimentaire pour animaux selon la revendication 4 ou la revendication 5 et au moins un minéral et/ou au moins une vitamine, de préférence où l'endoprotéase et/ou la tripeptidyl protéase tolérante à la proline sont inactives (ou sensiblement inactives) dans le prémélange préalablement à l'administration du prémélange à un animal.

9. Procédé de préparation d'un aliment pour animaux comprenant mettre un composant alimentaire pour animaux en contact avec une composition d'additifs alimentaires ou ingrédient alimentaire pour animaux selon la revendication 4 ou la revendication 5 ou un prémélange selon la revendication 8 ou au moins une tripeptidyl protéase tolérante à la proline ayant de manière prédominante une activité d'exopeptidase, où ladite tripeptidyl protéase tolérante à la proline est capable de cliver des tripeptides de la terminaison N de peptides ayant:

(i)

(A) une proline à P1; et
(B) un acide aminé sélectionné parmi alanine, arginine, asparagine, acide aspartique, cystéine, glutamine, acide glutamique, glycine, histidine, isoleucine, leucine, lysine, méthionine, phénylalanine, sérine, thréonine, tryptophane, tyrosine, valine ou bien des acides aminés synthétiques à P1; et/ou

(ii)

(a') une proline à P1'; et
(b') un acide aminé sélectionné parmi alanine, arginine, asparagine, acide aspartique, cystéine, glutamine, acide glutamique, glycine, histidine, isoleucine, leucine, lysine, méthionine, phénylalanine, sérine, thréonine, tryptophane, tyrosine, valine ou bien des acides aminés synthétiques ou des amines à P1' optionnellement en combinaison avec au moins une endoprotéase, de préférence où l'endoprotéase et/ou la tripeptidyl protéase tolérante à la proline sont inactives (ou sensiblement inactives) dans l'aliment pour animaux préalablement à l'administration de l'aliment pour animaux à un animal, où la au moins une tripeptidyl protéase tolérante à la proline:

(a) comprend la séquence d'acides aminés SEQ ID NO: 29, SEQ ID NO: 1, SEQ ID NO: 2 ou un fragment fonctionnel de celle-ci qui conserve ladite activité de peptidase;
(b) comprend un acide aminé ayant au moins 80 % ou au moins 90 % d'identité avec la SEQ ID NO: 29, SEQ ID NO: 1, SEQ ID NO: 2 ou un fragment fonctionnel de celui-ci qui conserve ladite activité de peptidase;
(c) est codée par une séquence de nucléotides comprenant la séquence SEQ ID NO: 56;
(d) est codée par une séquence de nucléotides comprenant au moins 80 % ou au moins 90 % d'identité de séquence avec la SEQ ID NO: 56;
(e) est codée par une séquence de nucléotides qui s'hybride à la SEQ ID NO: 56 dans des conditions de stringence élevée; ou bien
(f) est codée par une séquence de nucléotides qui diffère de la SEQ ID NO: 56 du fait de la dégénérescence du code génétique.

10. Aliment pour animaux comprenant une composition d'additifs alimentaires ou ingrédient alimentaire pour animaux selon la revendication 4 ou la revendication 5, ou prémélange selon la revendication 8, ou comprenant au moins une tripeptidyl protéase tolérante à la proline ayant de manière prédominante une activité d'exopeptidase, où ladite tripeptidyl protéase tolérante à la proline est capable de cliver des tripeptides de la terminaison N de peptides ayant

(i)

(A) une proline à P1; et
(B) un acide aminé sélectionné parmi alanine, arginine, asparagine, acide aspartique, cystéine, glutamine, acide glutamique, glycine, histidine, isoleucine, leucine, lysine, méthionine, phénylalanine, sérine, thréonine, tryptophane, tyrosine, valine ou bien des acides aminés synthétiques à P1; et/ou

(ii)

(a') une proline à P1'; et
(b') un acide aminé sélectionné parmi alanine, arginine, asparagine, acide aspartique, cystéine, glutamine, acide glutamique, glycine, histidine, isoleucine, leucine, lysine, méthionine, phénylalanine, sérine, thréonine, tryptophane, tyrosine, valine ou bien des acides aminés synthétiques ou des amines à P1', de préférence où l'endoprotéase et/ou la tripeptidyl protéase tolérante à la proline sont inactives (ou sensiblement inactives) dans l'aliment pour animaux préalablement à l'administration de l'aliment pour animaux à un animal, où la au moins une tripeptidyl protéase tolérante à la proline:

(a) comprend la séquence d'acides aminés SEQ ID NO: 29, SEQ ID NO: 1, SEQ ID NO: 2 ou un fragment fonctionnel de celle-ci qui conserve ladite activité de peptidase;
(b) comprend un acide aminé ayant au moins 80 % ou au moins 90 % d'identité avec la SEQ ID NO: 29, SEQ ID NO: 1, SEQ ID NO: 2 ou un fragment fonctionnel de celui-ci qui conserve ladite activité de peptidase;
(c) est codée par une séquence de nucléotides comprenant la séquence SEQ ID NO: 56;
(d) est codée par une séquence de nucléotides comprenant au moins 80 % ou au moins 90 % d'identité de séquence avec la SEQ ID NO: 56;
(e) est codée par une séquence de nucléotides qui s'hybride à la SEQ ID NO: 56 dans des conditions de stringence élevée; ou bien
(f) est codée par une séquence de nucléotides qui diffère de la SEQ ID NO: 56 du fait de la dégénérescence du code génétique.

11. Procédé non thérapeutique destiné à améliorer une caractéristique biophysique d'un animal ou à améliorer la digestibilité de protéine d'un animal, lequel procédé comprend le fait d'administrer à un animal une composition d'additifs alimentaires selon la revendication 4 ou la revendication 5, ou un aliment pour animaux selon la revendication 10, ou un prémélange selon la revendication 8, ou bien au moins une tripeptidyl protéase tolérante à la proline ayant de manière prédominante une activité d'exopeptidase, où ladite tripeptidyl protéase tolérante à la proline est capable de cliver des tripeptides de la terminaison N de peptides ayant:

(i)

(A) une proline à P1; et
(B) un acide aminé sélectionné parmi alanine, arginine, asparagine, acide aspartique, cystéine, glutamine, acide glutamique, glycine, histidine, isoleucine, leucine, lysine, méthionine, phénylalanine, sérine, thréonine, tryptophane, tyrosine, valine ou bien des acides aminés synthétiques à P1; et/ou

(ii)

(a') une proline à P1'; et
(b') un acide aminé sélectionné parmi alanine, arginine, asparagine, acide aspartique, cystéine, glutamine, acide glutamique, glycine, histidine, isoleucine, leucine, lysine, méthionine, phénylalanine, sérine, thréonine, tryptophane, tyrosine, valine ou bien des acides aminés synthétiques ou des amines à P1';

et optionnellement au moins un composant alimentaire pour animaux et/ou au moins un minéral et/ou au moins une vitamine, de préférence où le procédé comprend le fait d'administrer à un animal au moins une endoprotéase, où la au moins une tripeptidyl protéase tolérante à la proline:

(a) comprend la séquence d'acides aminés SEQ ID NO: 29, SEQ ID NO: 1, SEQ ID NO: 2 ou un fragment fonctionnel de celle-ci qui conserve ladite activité de peptidase;
(b) comprend un acide aminé ayant au moins 80 % ou au moins 90 % d'identité avec la SEQ ID NO: 29, SEQ ID NO: 1, SEQ ID NO: 2 ou un fragment fonctionnel de celui-ci qui conserve ladite activité de peptidase;
(c) est codée par une séquence de nucléotides comprenant la séquence SEQ ID NO: 56;
(d) est codée par une séquence de nucléotides comprenant au moins 80 % ou au moins 90 % d'identité de séquence avec la SEQ ID NO: 56;
(e) est codée par une séquence de nucléotides qui s'hybride à la SEQ ID NO: 56 dans des conditions de stringence élevée; ou bien
(f) est codée par une séquence de nucléotides qui diffère de la SEQ ID NO: 56 du fait de la dégénérescence du code génétique.

12. Utilisation d'une composition d'additifs alimentaires ou d'un ingrédient alimentaire pour animaux selon la revendication 4 ou la revendication 5, ou d'une nourriture pour animaux ou aliment pour animaux selon la revendication 10, ou d'un prémélange selon la revendication 8, ou d'au moins une tripeptidyl protéase tolérante à la proline ayant de manière prédominante une activité d'exopeptidase, où ladite tripeptidyl protéase tolérante à la proline est capable de cliver des tripeptides de la terminaison N de peptides ayant:

(i)

(A) une proline à P1; et
(B) un acide aminé sélectionné parmi alanine, arginine, asparagine, acide aspartique, cystéine, glutamine, acide glutamique, glycine, histidine, isoleucine, leucine, lysine, méthionine, phénylalanine, sérine, thréonine, tryptophane, tyrosine, valine ou bien des acides aminés synthétiques à P1; et/ou

(ii)

(a') une proline à P1'; et
(b') un acide aminé sélectionné parmi alanine, arginine, asparagine, acide aspartique, cystéine, glutamine, acide glutamique, glycine, histidine, isoleucine, leucine, lysine, méthionine, phénylalanine, sérine, thréonine, tryptophane, tyrosine, valine ou bien des acides aminés synthétiques ou des amines à P1'

pour améliorer la digestibilité de protéine chez un animal ou bien pour améliorer de manière non thérapeutique une caractéristique biophysique d'un animal, de préférence où la au moins une endoprotéase est utilisée en combinaison, où la au moins une tripeptidyl protéase tolérante à la proline:

(a) comprend la séquence d'acides aminés SEQ ID NO: 29, SEQ ID NO: 1, SEQ ID NO: 2 ou un fragment fonctionnel de celle-ci qui conserve ladite activité de peptidase;

(b) comprend un acide aminé ayant au moins 80 % ou au moins 90 % d'identité avec la SEQ ID NO: 29, SEQ ID NO: 1, SEQ ID NO: 2 ou un fragment fonctionnel de celui-ci qui conserve ladite activité de peptidase;

(c) est codée par une séquence de nucléotides comprenant la séquence SEQ ID NO: 56;

(d) est codée par une séquence de nucléotides comprenant au moins 80 % ou au moins 90 % d'identité de séquence avec la SEQ ID NO: 56;

(e) est codée par une séquence de nucléotides qui s'hybride à la SEQ ID NO: 56 dans des conditions de stringence élevée; ou bien

(f) est codée par une séquence de nucléotides qui diffère de la SEQ ID NO: 56 du fait de la dégénérescence du code génétique.

13. Procédé selon la revendication 11 ou utilisation selon la revendication 12, où la caractéristique biophysique est sélectionnée parmi le groupe consistant en un ou plusieurs des éléments suivants: performance de l'animal, performance de croissance d'un animal, taux de conversion alimentaire (FCR), aptitude à digérer une matière brute (par ex. digestibilité de nutriments, y compris digestibilité d'amidon, de graisse, de protéine, de fibre), digestibilité d'azote (par ex. digestibilité iléale d'azote) et énergie digestible (par ex. énergie iléale digestible), rétention d'azote, rendement de carcasse, taux de croissance, gain de poids, poids corporel, masse, efficacité alimentaire, pourcentage de graisse corporelle, distribution de graisse corporelle, croissance, taille d'oeuf, poids d'oeuf, masse d'oeuf, taux de ponte d'oeufs, gain en masse maigre, pourcentage de cendre d'os, mg de cendre d'os, pourcentage de gras dorsal, rendement en lait, pourcentage de matières grasses du lait, rendements reproductifs tels que taille de portée, capacité de survie des portées, pourcentage d'éclosabilité et impact sur l'environnement, par ex. production de fumier et/ou excrétion d'azote, de préférence où la caractéristique biophysique est l'aptitude à digérer les protéines.

14. Procédé selon l'une quelconque des revendications 1 à 3 ou 11 ou 12 ou utilisation selon la revendication 12 ou 13, dans lequel/laquelle: la au moins une endoprotéase, tripeptidyl protéase tolérante à la proline ou combinaison de celles-ci est ajoutée à et mélangée avec la au moins une protéine ou partie de celle-ci immédiatement avant d'administrer la composition d'additifs alimentaires à un animal; et/ou

dans lequel/laquelle la au moins une endoprotéase, tripeptidyl protéase tolérante à la proline ou combinaison de celles-ci est activée en administrant la au moins une endoprotéase, tripeptidyl protéase tolérante à la proline ou combinaison de celles-ci à un animal; et/ou

l'endoprotéase et la tripeptidyl protéase tolérante à la proline sont fonctionnelles, ou essentiellement fonctionnelles dans les voies digestives de l'animal; et/ou

la au moins une endoprotéase et au moins une tripeptidyl protéase tolérante à la proline sont actives dans le duodénum et dans des parties des voies digestives de l'animal qui précèdent le duodénum; et/ou

lorsqu'administrée à un animal la composition d'additifs alimentaires n'augmente pas substantiellement l'incidence d'entérite nécrotique chez ledit animal, lorsque comparé à un animal auquel ladite composition d'additifs alimentaires n'est pas administrée.

*FIG. 1*

pH Profile

FIG. 2

Thermostability

FIG. 3

Synergy between acid fungal protease (AFP) and proline tolerant tripeptidyl peptidase

*FIG. 4*

FIG. 5

*FIG. 6*

FIG. 7

FIG. 8

FIG. 9

*FIG. 10*

SEM = 0.004
*P* value = 0.0016

*FIG. 11*

**FIG. 12**

*FIG. 13*

FIG. 14

FIG. 15

EP 3 209 774 B1

*FIG. 16*

## A. Prepro sequences

CLUSTAL O(1.2.1) multiple sequence alignment

```
SEQIDNO8    ------------------------------------------------------------
SEQIDNO9    ------------------------------------------------------------
SEQIDNO7    ------------------------------------------------------------
SEQIDNO12   ------------------------------------------------------------
SEQIDNO14   ------------------------------------------------------------
SEQIDNO5    ------------------------------------------------------------
SEQIDNO1    ----------------------------------------------------MAKLST---L
SEQIDNO2    ------------------------------------------------------------
SEQIDNO13   ------------------------------------------------------------
SEQIDNO11   ------------------------------------------------------------
SEQIDNO10   ------------------------------------------------------------
SEQIDNO27   -------------------------------------------------------MLSS---T
SEQIDNO28   -------------------------------------------------------MLSS---T
SEQIDNO15   ------------------------------------------------------------
SEQIDNO16   ------------------------------------------------------------
SEQIDNO4    ------------------------------------------------------------
SEQIDNO20   MAATGRFTAFWNVASVPALIGILPLAGSHLRAVLCPVCIWRHSKAVCAPDTLQAMRAFTR
SEQIDNO24   ------------------------------------------------------MHVPLL-N
SEQIDNO23   ------------------------------------------------------MIASLF-N
SEQIDNO25   ------------------------------------------------------MIASLF-S
SEQIDNO26   ------------------------------------------------------MIASLF-N
SEQIDNO21   -------------------------------------------------------MLSFV-R
SEQIDNO22   ------------------------------------------------------MLPSLV-N
SEQIDNO3    ------------------------------------------------------------
SEQIDNO19   -----------------------------------------------------MLSSFLSQ
SEQIDNO6    ------------------------------------------------------------
SEQIDNO17   ------------------------------------------------------------
SEQIDNO18   ------------------------------------------------------------


SEQIDNO8    -----------------------AVVIRAAVLPDAVKLMGKA---MPDDIISLQFSLKQQNIDQ
SEQIDNO9    -----------------------AVVIRAAPLPESVKLVRKA---AAEDGINLQLSLKRQNMDQ
SEQIDNO7    -----------------------KSYSHHAEAPKGWKVDDTARVASTGKQQVFSIALTMQNVDQ
SEQIDNO12   -----------------------KSFSHHAEAPQGWQVQKTAKVASN-TQHVFSLALTMQNVDQ
SEQIDNO14   -----------------------AVLVESLKQVPNGWNAVSTP---DPSTSIVLQIALAQQNIDE
SEQIDNO5    ------------------NAAVLLDSLDKVPVGWQAASAP---APSSKITLQVALTQQNIDQ
SEQIDNO1    RLASLLSLVS-VQVS-ASVHLLESLEKLPHGWKAAETP---SPSSQIVLQVALTQQNIDQ
SEQIDNO2    -----------------SVHLLESLEKLPHGWKAAETP---SPSSQIVLQVALTQQNIDQ
SEQIDNO13   ------------------SVLVESLEKLPHGWKAASAP---SPSSQITLQVALTQQNIDQ
SEQIDNO11   ------------A-AALVGHESLAALPVGWDKVSTP---AAGTNIQLSVALALQNIEQ
SEQIDNO10   -------------------VVHEKLAAVPSGWHHLEDA---GSDHQISLSIALARKNLDQ
SEQIDNO27   LYAGL--LCS-LAAP-ALGVVHEKLSAVPSGWTLVEDA---SESDTTTLSIALARQNLDQ
SEQIDNO28   LYAGW--LLS-LAAP-ALCVVQEKLSAVPSGWTLIEDA---SESDTITLSIALARQNLDQ
SEQIDNO15   ---------SPLARRWDDFAEKHAWVEVPRGWEMVSEA---PSDHTFDLRIGVKSSGMEQ
SEQIDNO16   ------------------RVFDSLPHPPRGWSYSHAA---ESTEPLTLRIALRQQNAAA
SEQIDNO4    ------------------EPFEKLFSTPEGWKMQGLA---TNEQIVKLQIALQQGDVAG
SEQIDNO20   VTAISLAGFSCFAA--AAAAAFESLRAVPDGWIYESTP---DPNQPLRLRIALKQHNVAG
SEQIDNO24   QGALSLAVVSLLAST-VSAEVFDKLVAVPEGWRFSRTP---SGDQPIRLQVALTQGDVEG
SEQIDNO23   RRALTLALLSLFASS-ATADVFESLSAVPQGWRYSRTP---SANQPLKLQIALAQGDVAG
```

*FIG. 17*

```
SEQIDNO25    RGALSLAVLSLLASS-AAADVFESLSAVPQGWRYSRRP---RADQPLKLQIALTQGDTAG
SEQIDNO26    RGALSLAVLSLLASS-ASADVFESLSAVPQGWRYSRRP---RADQPLKLQIALAQGDTAG
SEQIDNO21    RGALSLALVSLLTSS-VAAEVFEKLHVVPEGWRYASTP---NPKQPIRLQIALQQHDVTG
SEQIDNO22    NGALSLAVLSLLTSS-VAGEVFEKLSAVPKGWHFSHAA---QADAPINLKIALKQHDVEG
SEQIDNO3     ------------------EAFEKLSAVPKGWHYSSTP----KGNTEVCLKIALAQKDAAG
SEQIDNO19    GAAVSLALLSLLPSP-VAAEIFEKLSGVPNGWRYANNP---HGNEVIRLQIALQQHDVAG
SEQIDNO6     ----------KPTPG-ASHKVIEHLDFVPEGWQMVGAA---DPAAIIDFWLAIERENPEK
SEQIDNO17    -----------------SDVVLESLREVPQGWKRLRDA---DPEQSIKLRIALEQPNLDL
SEQIDNO18    ----------STTSH-VEGEVVERLHGVPEGWSQVGAP---NPDQKLRFRIAVRSADSEL
                                                   *  .            :  ..:

SEQIDNO8     LETRLRAVSDPSSPEYGQYMSES-EVNEFFKPRDDSFAEVIDWVAASGFQDIHLT--PQA
SEQIDNO9     LEKFLRAVSDPFSPKYGQYMSDA-EVHEIFRPTEDSFDQVIDWLTKSGFGNLHIT--PQA
SEQIDNO7     LESKLLDLSSPDSKNYGQWMSQK-DVTTAFYPSKEAVSSVTKWLKSKGVK--HYN--VNG
SEQIDNO12    LESKLLDLSSPDSANYGNWLSHD-ELTSTFSPSKEAVASVTKWLKSKGIK--HYK--VNG
SEQIDNO14    LEWRLAAVSTPNSGNYGKYLDIG-EIEGIFAPSNASYKAVASWLQSHGVK--NFV--KQA
SEQIDNO5     LESKLAAVSTPNSSNYGKYLDVD-EINQIFAPSSASTAAVESWLKSYGV---DYK--VQG
SEQIDNO1     LESRLAAVSTPTSSTYGKYLDVD-EINSIFAPSDASSSAVESWLQSHGVT--SYT--KQG
SEQIDNO2     LESRLAAVSTPTSSTYGKYLDVD-EINSIFAPSDASSSAVESWLQSHGVT--SYT--KQG
SEQIDNO13    LESRLAAVSTPNSKTYGNYLDLD-EINEIFAPSDASSAAVESWLHSHGVT--KYT--KQG
SEQIDNO11    LEDHLKSVSTPGSASYGQYLDSD-GIAAQYGPSDASVEAVTNWLKEAGVT--DIY--NNG
SEQIDNO10    LESKLKDLSTPGESQYGQWLDQE-EVDTLFPV--ASDKAVISWLRSANIT--HIA--RQG
SEQIDNO27    LESKLTTLATPGNAEYGKWLDQS-DIESLFPT--ASDDAVIQWLKDAGVT--QVS--RQG
SEQIDNO28    LESKLTTLATPGNPEYGKWLDQS-DIESLFPT--ASDDAVLQWLKAAGIT--QVS--RQG
SEQIDNO15    LIENLMQTSDPTHSRYGQHLSKE-ELHDFVQPHPDSTGAVEAWLEDFGISDDFIDRTGSG
SEQIDNO16    LEQVVLQVSNPRHANYGQHLTRD-ELRSYTAPTPRAVRSVTSWLVDNGVD--DYT--VEH
SEQIDNO4     FEQHVIDISTPSHPSSYGSHEEMKRMIQPSSETVASVSAWLKAAGIN--DAE--IDS
SEQIDNO20    FEQALLDMSTPDHPSSYGQHFGSYHEMKQLLLPTEEASSSVRDWLSAAGV---EFE--QDA
SEQIDNO24    FEKAVLDMSTPDHPNYGKHFKSHEEVKRMLQPAGESVEAIHQWLEKAGIT--HIQ--QDA
SEQIDNO23    FEAAVIDMSTPDHPSYGNHFNTHEEMKRMLQPSAESVDSIRNWLESAGIS--KIE--QDA
SEQIDNO25    FEEAVMEMSTPDHPSYGHHFTTHEEMKRMLQPSAESAESIRDWLEGAGIT--RIE--QDA
SEQIDNO26    FEEAVMDMSTPDHPSYGNHFHTHEEMKRMLQPSAESADSIRDWLESAGIN--RIE--QDA
SEQIDNO21    FEQSLLEMSTPDHPNYGKHFRTHDEMKRMLLPNENAVHAVREWLQDAGIS--DIE--EDA
SEQIDNO22    FEQALLDMSTPGHENYGKHFHEHDEMKRMLLPSDSAVDAVQTWLTSAGIT--DYD--LDA
SEQIDNO3     FEKTVLEMSDPDHPSYGQHFTTHDEMKRMLLPRDDTVDAVRQWLENGGVT--DFT--QDA
SEQIDNO19    FEQAVMDMSTPGHADYGKHFRTHDEMKRMLLPSDTAVDSVRDWLESAGVH--NIQ--VDA
SEQIDNO6     LYDTIYDVSTPGRAQYGKHLKRE-ELDDLLRPRAETSESIINWLTNGGVNPQHIR--DEG
SEQIDNO17    FEQTLYDISSPDHPKYGQHLKSH-ELRDIMAPREESTAAVIAWLQDAGLSGSQIE--DDS
SEQIDNO18    FERTLMEVSSPSHPRYGQHLKRH-ELKDLIKPRAKSTSNILNWLQESGIEARDIQ--NDG
              :      :   :  *     **          :            :   :    *:      .           .

SEQIDNO8     AAINLAATVETADQLLGANFSWFDVDG-----TRKLRTLEYTIPDRLADHVDLISPTTYF
SEQIDNO9     AAINVATTVETADQLFGANFSWFDVDG-----TPKLRTGEYTIPDRLVEHVDLVSPTTYF
SEQIDNO7     GFIDFALDVKGANALLDSDYQYYTKEG-----QTKLRTLSYSIPDDVAEHVQFVDPSTNF
SEQIDNO12    AFIDFAADVEKANTLLGGDYQYYTKDG-----QTKLRTLSYSIPDDVAGHVQFVDPSTNF
SEQIDNO14    GSIWFYTTVSTANKMLSTDFKHYSDPV----GIEKLRTLQYSIPEELVGHVDLISPTTYF
SEQIDNO5     SSIWFQTDVSTANKMLSTNFHTYTDSV----GAKKVRTLQYSVPETLADHIDLISPTTYF
SEQIDNO1     SSIWFQTNISTANAMLSTNFHTYSDLT----GAKKVRTLKYSIPESLIGHVDLISPTTYF
SEQIDNO2     SSIWFQTNISTANAMLSTNFHTYSDLT----GAKKVRTLKYSIPESLIGHVDLISPTTYF
SEQIDNO13    SSIWFQTEVSTANAMLSTNFHTYSDAA----GVKKLRTLQYSIPESLVGHVDLISPTTYF
SEQIDNO11    QSIHFATSVSKANSLLGADFNYYSDGS-----ATKLRTLAYSVPSDLKEAIDLVSPTTYF
SEQIDNO10    SLVNFATTVDKVNKLLNTTFAYYQRGS-----SQRLRTTEYSIPDDLVDSIDLISPTTFF
SEQIDNO27    SLVNFATTVGTANKLFDTKFSYYRNGA-----SQKLRTTQYSIPDSLTESIDLIAPTVFF
SEQIDNO28    SLVNFATTVGTANKLFDTKFSYYRNGA-----SQKLRTTQYSIPDHLTESIDLIAPTVFF
SEQIDNO15    NWVTVRVSVAQAERMLGTKYNVYRHSE---SGESVVRTMSYSLPSELHSHIDVVAPTTYF
SEQIDNO16    DWVTLRTTVGAADRLLGADFAWYAGPG----ETLQLRTLSYGVDDSVAPHVDLVQPTTRF
SEQIDNO4     DWVTFKTTVGVANKMLDTKFAWYVSEE--AKPRKVLRTLEYSVPDDVAEHINLIQPTTRF
SEQIDNO20    DWINFRTTVDQANALLDADFLWYTTTGSTGNPTRILRTLSYSVPSELAGYVNMIQPTTRF
SEQIDNO24    DWMTFYTTVEKANNLLDANFQYYLNEN--KQVERLRTLEYSVPDELVSHINLVTPTTRF
SEQIDNO23    DWMTFYTTVKTANELLAANFQFYINGV---KKIERLRTLKYSVPDALVSHINMIQPTTRF
SEQIDNO25    DWMTFYTTVETANELLAANFQFYVSNV---RHIERLRTLKYSVPKALVPHINMIQPTTRF
SEQIDNO26    DWMTFYTTVETANELLAANFQFYANSA---HIERLRTLQYSVPEALMPHINMIQPTTRF
SEQIDNO21    DWVRFHTTVDQANDLLDANFLWYAHKS--HRNTARLRTLEYSIPDSIAPQVNVIQPTTRF
SEQIDNO22    DWINLRTTVEHANALLDTQFGWYENEV---RHITRLRTLQYSIPETVAAHINMVQPTTRF
SEQIDNO3     DWINFCTTVDTANKLLNAQFKWYVSDV---KHIRRLRTLQYDVPESVTPHINTIQPTTRF
SEQIDNO19    DWVKFHTTVNKANALLDADFKWYVSEA---KHIRRLRTLQYSIPDALVSHINMIQPTTRF
```

## FIG. 17 Continued

```
SEQIDNO6     DWVRFSTNVKTAETLMNTRFNVFKDNL---NSVSKIRTLEYSVPVAISAHVQMIQPTTLF
SEQIDNO17    DWINIQTTVAQANDMLNTTFGLFAQEG---TEVNRIRALAYSVPEEIVPHVKMIAPIIRF
SEQIDNO18    EWISFYAPVKRAEQMMSTTFKTYQNEA--RANIKKIRSLDYSVPKHIRDDIDIIQPTTRF
              :  .    :  .: ::    :  :          :*:  *  :    :  :.  :  *    *

SEQIDNO8     GRARLDG------------PRETPTRLDKRQRDPVADKA-----------YFHLKWDRG
SEQIDNO9     GRMRPPPRGDGVNDWITENSPEQPAPLNKRDTKTESDQA-----------RDHPSWDSR
SEQIDNO7     GGTLAFAPVT---------------HPSRTLTE--R-----------KNKPTKSTV
SEQIDNO12    GGTVAFNPVP---------------HPSRTLQE--R-----------KVSPSKSTV
SEQIDNO14    GNNHPATART----------------------------------------------
SEQIDNO5     GTSKAMRALK---------------IQNAASAVS---------------PLAARQE
SEQIDNO1     GTTKAMRKLK---------------SSGVSPAAD---------------ALAARQE
SEQIDNO2     GTTKAMRKLK---------------SSGVSPAAD---------------ALAARQE
SEQIDNO13    GTSNAMRALR---------------SKSVASVAQ---------------SVAARQE
SEQIDNO11    GKTTASRSIQ---------------AYKNKRASTTS-----------KSGSSSVQV
SEQIDNO10    GKEKTSAGLT---------------QRSQKVDNHV---------------AKRSN
SEQIDNO27    GKEQDSAL-P---------------PHAVKLPALP---------------RRAAT
SEQIDNO28    GKEQNSAL-S---------------SHAVKLPALP---------------RRAAT
SEQIDNO15    GTMKSMRVTS---------------FLQPEIEPVDP-----------SAKPSAAP
SEQIDNO16    GGPVGQASHI---------------FKQDDFDEQQLKTLSVGFQVMADLPANGPGSI
SEQIDNO4     AAIRQNHEVA---------------HEIVG-----------------LQFAALANN
SEQIDNO20    GGTHANRATV---------------RAKPIFLETNR------Q-----LINAISSGS
SEQIDNO24    GQLHAEGVTL---------------HGKSKD-VDEQ------F-----RQAA--TSP
SEQIDNO23    GQLRAQRAIL---------------HTEVKD-NDEA------F-----RSNA--MSA
SEQIDNO25    GQLRAHRGIL---------------HGQVKE-SDEA------F-----RSNA--VSA
SEQIDNO26    GQLRVQGAIL---------------HTQVKE-TDEA------F-----RSNA--VST
SEQIDNO21    GQIRANRATH---------------SSKPKGGLDEL------A-----ISQAATADD
SEQIDNO22    GQIRPDRATF---------------HAHHT--SDAR------I-----LSALAAASN
SEQIDNO3     GKISPKKAVT---------------HSKPSQ-LDVT------A-----LAAAVVAKN
SEQIDNO19    GQIQPNRATM---------------RSKPKH-ADET------F-----LTAATLAQN
SEQIDNO6     GRQKPQNSLI---------------LNPLTKDLE-------------SMSVEEFA
SEQIDNO17    GQLRPQMSHI---------------FSHEKVEETPS------IGTIKAAAIPSVDLN
SEQIDNO18    GQIQPERSQV---------------FSQEEVPFSA-------------------LVV
             .

SEQIDNO8     TSNC-D----------LVITPPCLEAAYNYKNYMPDPNSGSRVSFTSFLEQAAQQSDLTK
SEQIDNO9     TPDC-A----------TIITPPCLETAYNYKGYIPDPKSGSRVSFTSFLEQAAQQADLTK
SEQIDNO7     DASC-Q----------TSITPSCLKQMYNIGDYTPKVESGSTIGFSSFLGESAIYSDVFL
SEQIDNO12    DASC-Q----------TSITPSCLKQMYNIGDYTPDAKSGSEIGFSSFLGQAAIYSDVFK
SEQIDNO14    ------PNMKAINVTYQIFHPDCLKTKYGVDGYAPSPRCGSRIGFGSFLNETASYSDLAQ
SEQIDNO5     PSSC-KGTIEFENRTFNVFQPDCLRTEYSVNGYKPSAKSGSRIGFGSFLNQSASSSDLAL
SEQIDNO1     PSSC-KGTLVFEGETFNVFQPDCLRTEYSVDGYTPSVKSGSRIGFGSFLNESASFADQAL
SEQIDNO2     PSSC-KGTLVFEGETFNVFQPDCLRTEYSVDGYTPSVKSGSRIGFGSFLNESASFADQAL
SEQIDNO13    PSSC-KGTLVFEGRTFNVFQPDCLRTEYNVNGYTPSAKSGSRIGFGSFLNQSASFSDLAL
SEQIDNO11    SASC-Q----------TSITPACLKQMYNVGNYTPSVAHGSRVGFGSFLNQSAIFDDLFT
SEQIDNO10    SSSC-A----------DTITLSCLKEMYNFGNYTPSASSGSKLGFASFLNESASYSDLAK
SEQIDNO27    NSSC-A----------NLITPDCLVEMYNLGDYKPDASSGSRVGFGSFLNQSANYADLAA
SEQIDNO28    NSSC-A----------NLITPDCLVEMYNLGDYKPDASSGSRVGFGSFLNESANYADLAA
SEQIDNO15    ASCLST----------TVITPDCLRDLYNTADYVPSATSRNAIGIAGYLDRSNR-ADLQT
SEQIDNO16    KAACNE----------SGVTPLCLRTLYRVNYKP--ATTGNLVAFASFLEQYARYSDQQA
SEQIDNO4     TVNC-D----------ATITPQCLKTLYKI-DYKADPKSGSKVAFASYLEQYARYNDLAL
SEQIDNO20    LEHC-E----------KAITPSCLADLYNTEGYKASNRSGSKVAFASFLEEYARYDDLAE
SEQIDNO24    SSDC-N----------SAITPQCLKDLYKVGDYKASASNGNKVAFTSYLEQYARYSDLAL
SEQIDNO23    NPDC-N----------SIITPQCLKDLYSIGDYEADPTNGNKVAFASYLEEYARYSDLAL
SEQIDNO25    QPDC-N----------SIITPQCLKDIYNIGDYQANDTNGNKVGFASYLEEYARYSDLAL
SEQIDNO26    SPDC-N----------SIITPQCLKNMYNVGDYQADDDNGNKVGFASYLEEYARYSDLEL
SEQIDNO21    DSIC-D----------QITTPHCLRKLYNVNGYKADPASGSKIGFASFLEEYARYSDLVL
SEQIDNO22    STSC-D----------SVITPKCLKDLYKVGDYEADPDSGSQVAFASYLEEYARYADMVK
SEQIDNO3     ISHC-D----------SIITPTCLKELYNIGDYQADANSGSKIAFASYLEEYARYADLEN
SEQIDNO19    TSHC-D----------SIITPHCLKQLYNIGDYQADPKSGSKVGFASYLEEYARYADLER
SEQIDNO6     ASQC-R----------SLVTTACLRELYGLGDRVTQARDDNRIGVSGFLEEYAQYRDLEL
SEQIDNO17    VTAC-N----------ASITPECLRALYNVGDYEADPSKKSLFGVCGYLEQYAKHDQLAK
SEQIDNO18    NATC-N----------KKITPDCLANLYNFKDYDASDA-NVTIGVSGFLEQYARFDDLKQ
                              **     *              ... .:*  .        :
```

## FIG. 17 Continued

```
SEQIDNO8     FLSLTGLDRLRPPSSKPASFDTVLINGGETHQGTPP-NKTSEANLDVQWLAAVIKARLPI
SEQIDNO9     FLSLTRLEGFRTPASKKKTFKTVLINGGESHEGVHKKSKTSEANLDVQWLAAVTQTKLPI
SEQIDNO7     FEEKFGIP--------TQNFTTVLINNGTD-DQNTAHKNFGEADLDAENIVGIA-HPLPF
SEQIDNO12    FEELFGIP--------KQNYTTLLINNGTD-DQNTAHGNFGEANLDAENIVGIA-HPLPF
SEQIDNO14    FEKYFDLP--------NQNLSTLLINGAID-VQPPSNKNDSEANMDVQTILTFV-QPLPI
SEQIDNO5     FEKHFGFA--------SQGFSVLLINGGS-NPQPPTDANDGEANLDAQNIVSFV-QPLPI
SEQIDNO1     FEKHFNIP--------SQNFSVVLINGGTDLPQPPSDANDGEANLDAQTILTIA-HPLPI
SEQIDNO2     FEKHFNIP--------SQNFSVVLINGGTDLPQPPSDANDGEANLDAQTILTIA-HPLPI
SEQIDNO13    FEKHFGFS--------SQNFSVVLINGGTDLPQPPSDDNDGEANLDVQNILTIA-HPLPI
SEQIDNO11    YEKVNDIP--------SQNFTKVIIANASN-SQDASDGNYGEANLDVQNIVGIS-HPLPV
SEQIDNO10    FERLFNLP--------SQNFSVLLINGGVN-DQNQSTASLTEADLDVELLVGVG-HPLPV
SEQIDNO27    YEQLFNIP--------PQNFSVLLINGGAN-DQNWATASLGEANLDVELIVAVS-HALPV
SEQIDNO28    YEQLFNIP--------PQNFSVLLINRGVN-DQNWATASLGEANLDVELIVAVS-HPLPV
SEQIDNO15    FFRR-F-----RPDAVGFNYTTVQLNGGGD-DQNDP-G--VEANLDIQYAAGIAF-PTPA
SEQIDNO16    FTQRVL-----GPGVPLQNFSVTTVNGGAN-DQQSK-LDSGEANLDLQYVMAMSH-PIPI
SEQIDNO4     FEKA-F-----LPEAVGQNFSVVQFSGGLN-DQNTT-QDSGEANLDLQYIVGVSA-PLPV
SEQIDNO20    FEET-Y-----APYAIGQNFSVISINGGLN-DQDST-ADSGEANLDLQYIIGVSS-PLPV
SEQIDNO24    FEQN-I-----APYAQGQNFTVIQYNGGLN-DQSSP-ADSSEANLDLQYIIGTSS-PVPV
SEQIDNO23    FEKN-I-----APFAKGQNFSVVQYNGGGN-DQQSS-SGSSEANLDLQYIVGVSS-PVPV
SEQIDNO25    FEKN-I-----APSAKGQNFSVTRYNGGLN-DQSSS-GSSSEANLDLQYIVGVSS-PVPV
SEQIDNO26    FEKN-V-----APFAKGQNFSVIQYNGGLN-DQHSS-ASSSEANLDLQYIVGVSS-PVPV
SEQIDNO21    FEEN-L-----APFAEGENFTVVMYNGGKN-DQNSK-SDSGEANLDLQYIVGMSA-GAPV
SEQIDNO22    FQNS-L-----APYAKGQNFSVVLYNGGVN-DQSSS-ADSGEANLDLQTIMGLSA-PLPI
SEQIDNO3     FENY-L-----APWAKGQNFSVTTFNGGLN-DQNSS-SDSGEANLDLQYILGVSA-PLPV
SEQIDNO19    FEQH-L-----APNAIGQNFSVVQFNGGLN-DQLSL-SDSGEANLDLQYILGVSA-PVPV
SEQIDNO6     FLSR-F-----EPSAKGFNFSEGLIAGGKN-TQGGP-GSSTEANLDMQYVVGLSH-KAKV
SEQIDNO17    FEQT-Y-----APYAIGADFSVVTINGGGD-NQTST-IDDGEANLDMQYAVSMAY-KTPI
SEQIDNO18    FIST-F-----QPKAAGSTFQVTSVNAGPF-DQNST-ASSVEANLDIQYTTGLVAPDIET
             :                     .              **::*  :

SEQIDNO8     TQWITGGRPPFVPNLRLRHEKDNTNEPYLEFFEYLVRLPARDLPQVISNSYAEDEQTVPE
SEQIDNO9     TQWITGGRPPFVPNLRIPTPEANTNEPYLEFLEYLFRLPDKDLPQVISNSYAEDEQSVPE
SEQIDNO7     TQYITGGSPPFLPNIDQPTAADNQNEPYVPFFRYLLSQ--KEVPAVVSTSYGDEEDSVPR
SEQIDNO12    KQYITGGSPPFVPNIDQPTEKDNQNEPYVPFFRYLLGQ--KDLPAVISTSYGDEEDSVPR
SEQIDNO14    TEFVVAGIPPYIPDAALPIGDPVQNEPWLEYFEFLMSRTNAELPQVIANSYGDEEQTVPQ
SEQIDNO5     TEFIAGGTAPYFPDPVEPAGTPDENEPYLEYYEYLLSKSNKELPQVITNSYGDEEQTVPQ
SEQIDNO1     TEFITAGSPPYFPDPVEPAGTPNENEPYLQYYEFLLSKSNAEIPQVITNSYGDEEQTVPR
SEQIDNO2     TEFITAGSPPYFPDPVEPAGTPNENEPYLQYYEFLLSKSNAEIPQVITNSYGDEEQTVPR
SEQIDNO13    TEFITAGSPPYFPDPVEPAGTPDENEPYLQYFEYLLSKPNRDLPQVITNSYGDEEQTVPQ
SEQIDNO11    TEFLTGGSPPFVASLDTPT---NQNEPYIPYYEYLLSQKNEDLPQVISNSYGDDEQSVPY
SEQIDNO10    TEFITSGEPPFIPDPDEPSAADNENEPYLQYYEYLLSKPNSALPQVISNSYGDDEQTVPE
SEQIDNO27    VEFITGGSPPFVPNVDEPTAADNQNEPYLQYYEYLLSKPNSHLPQVISNSYGDDEQTVPE
SEQIDNO28    VEFITGALPPVLRVLAL-------------------------QTQLPSSSGDFQLTVPE
SEQIDNO15    TYWSTGGSPPFIPDTQTPT---NTNEPYLDWINFVLG--QDEIPQVISTSYGDDEQTVPE
SEQIDNO16    LEYSTGGRGPLVPTLDQPNANNSSNEPYLEFLTYLLAQPDSAIPQTLSVSYGEEEQSVPR
SEQIDNO4     TEFSTGGRGPWVADLDQPDEADSANEPYLEFLQGVLKLPQSELPQVISTSYGENEQSVPK
SEQIDNO20    TEFTTGGRGKLIPDLSSPDPNDNTNEPFLDFLEAVLKLDQKDLPQVISTSYGEDEQTIPE
SEQIDNO24    TEFSTGGRGPLVPDLDQPDINDNNNEPYLDFLQNVIKMSDKDLPQVISTSYGEDEQSVPA
SEQIDNO23    TEFSTGGRGELVPDLDQPNPNDNNNEPYLEFLQNVLKHKKDLPQVISTSYGEDEQSVPE
SEQIDNO25    TEFSVGGRGELVPDLDQPDPNDNNNEPYLEFLQNVLKLDKKDLPQVISTSYGEDEQSIPE
SEQIDNO26    TEFSVGGRGELVPDLDQPDPNDNNNEPYLEFLQNVLKMEQQDLPQVISTSYGENEQSVPE
SEQIDNO21    TEFSTAGRAPVIPDLDQPDPSAGTNEPYLEFLQNVLHMDQEHLPQVISTSYGENEQTIPE
SEQIDNO22    TEYITGGRGKLIPDLSQPNPNDNSNEPYLEFLQNILKLDQDELPQVISTSYGEDEQTIPR
SEQIDNO3     TEFSTGGRGPLVPDLTQPDPNSNSNEPYLEFFQNVLKLDQKDLPQVISTSYGENEQEIPE
SEQIDNO19    TEYSTGGRGELVPDLSSPDPNDNSNEPYLDFLQGILKLDNSDLPQVISTSYGEDEQTIPV
SEQIDNO6     TYYSTAGRGPLIPDLSQPSQASNNNEPYLEQLRYLVKLPKNQLPSVLTTSYGDTEQSLPA
SEQIDNO17    TYYSTGGRGPLVPDLDQPDPNDVSNEPYLDFVSYLLKLPDSKLPQTITTSYGEDEQSVPR
SEQIDNO18    RYFTVPGRGILIPDLDQPTESDNANEPYLDYFTYLNNLEDEELPDVLTTSYGESEQSVPA
             :   .   .      .                         :  *  .: :   :*
```

## FIG. 17 Continued

```
SEQIDNO8    AYARRVCNLIGIMGLRGVTVLTASGDSGVG-APCRANDGSDRLEFSPQFPTSCPY IATVG
SEQIDNO9    AYARRVCGLLGIMGLRGVTVLTASGDSGVG-APCRANDGSGREEFSPQFPSSCPY ITTVG
SEQIDNO7    EYATMTCNLIGLLGLRGISVIFSSGDIGVG-AGCLGPDH-KTVEFNAIFPATCPY LTSVG
SEQIDNO12   EYATLTCNMIGLLGLRGISVIFSSGDIGVG-SGCLAPDY-KTVEFNAIFPATCPY LTSVG
SEQIDNO14   AYAVRVCNQIGLLGLRGISVIASSGDTGVG-MSCMASNS-TTPQFNPMFPASCPY ITTVG
SEQIDNO5    AYAVRVCNLIGLMGLRGISILESSGDEGVG-ASCLATNSTTTPQFNPIFPATCPY VTSVG
SEQIDNO1    SYAVRVCNLIGLLGLRGISVLHSSGDEGVG-ASCVATNS-TTPQFNPIFPATCPY VTSVG
SEQIDNO2    SYAVRVCNLIGLLGLRGISVLHSSGDEGVG-ASCVATNS-TTPQFNPIFPATCPY VTSVG
SEQIDNO13   AYAVRVCNLIGLMGLRGISILESSGDEGVG-ASCVATNS-TTPQFNPIFPATCPY VTSVG
SEQIDNO11   KYAIRACNLIGLTGLRGISVLESSGDLGVG-AGCRSNDGKNKTQFDPIFPATCPY VTSVG
SEQIDNO10   YYAKRVCNLIGLVGLRGISVLESSGDEGIG-SGCRTTDGTNSTQFNPIFPATCPY VTAVG
SEQIDNO27   YYARRVCNLIGLMGLRGITVLESSGDTGIG-SACMSNDGTNTPQFTPTFPGTCPF ITAVG
SEQIDNO28   YYARRVCNLIGLMGLRGITVLESSGDTGIG-SACMSNDGTNKPQFTPTFPGTCPF ITAVG
SEQIDNO15   DYATSVCNLFAQLGSRGVTVFFSSGDFGVGGGDCLTNDGSNQVLFQPAFPASCPF VTAVG
SEQIDNO16   DYAIKVCNMFMQLGARGVSVMFSSGDSGPG-NDCVRA-SDNATFFGSTFPAGCPY VTSVG
SEQIDNO4    SYALSVCNLFAQLGSRGVSVIFSSGDSGPG-SACQSNDGKNTTKFQPQYPAACPF VTSVG
SEQIDNO20   PYARSVCNLYAQLGSRGVSVLFSSGDSGVG-AACQTNDGKNTTHFPPQFPASCPW VTAVG
SEQIDNO24   SYARSVCNLIAQLGGRGVSVIFSSGDSGVG-SACQTNDGKNTTRFPAQFPAACPW VTSVG
SEQIDNO23   KYARAVCNLYSQLGSRGVSVIFSSGDSGVG-AACQTNDGRNATHFPPQFPAACPW VTSVG
SEQIDNO25   KYARSVCNLYSQLGSRGVSVIFSSGDSGVG-SACLTNDGRNATRFPPQFPAACPW VTSVG
SEQIDNO26   KYARTVCNLFSQLGSRGVSVIFASGDSGVG-AACQTNDGRNATRFPAQFPAACPW VTSVG
SEQIDNO21   KYARTVCNMYAQLGSRGVSVIFSSGDSGVG-SACMTNDGTNRTHFPPQFPASCPW VTSVG
SEQIDNO22   GYAESVCNMLAQLGSRGVSVVFSSGDSGVG-AACQTNDGRNQTHFNPQFPASCPW VTSVG
SEQIDNO3    KYARTVCNLIAQLGSRGVSVLFSSGDSGVG-EGCMTNDGTNRTHFPPQFPAACPW VTSVG
SEQIDNO19   PYARTVCNLYAQLGSRGVSVIFSSGDSGVG-AACLTNDGTNRTHFPPQFPASCPW VTSVG
SEQIDNO6    SYTKATCDLFAQLGTMGVSVIFSSGDTGPG-SSCQTNDGKNATRFNPIYPASCPF VTSIG
SEQIDNO17   SYVEKVCTMFGALGARGVSVIFSSGDTGVG-SACQTNDGKNTTRFLPIFPAACPY VTSVG
SEQIDNO18   EYAKKVCNLIGQLGARGVSVIFSSGDTGPG-SACQTNDGKNTTRFLPIFPASCPY VTSVG
            *.   .*        *  *:::. :*** * *      *           *  :* **::*::*


SEQIDNO8    GTEGWDPEVAWEASSGGFSHYFLRPWYQANAVEKYLDEELDPATRAYYDGNGFVQFAGRA
SEQIDNO9    GTQAWDPEVAWKGSSGGFSNYFPRPWYQVAAVEKYLEEQLDPAAREYYEENGFVRFAGRA
SEQIDNO7    GTVDVTPEIAWEGSSGGFSKYFPRPSYQDKAVKTYMK-TVSKQTKKYYG--PYTNWEGRG
SEQIDNO12   GTVDVTPEIAWEGSSGGFSKYFPRPSYQDKAIKKYMK-TVSKETKKYYG--PYTNWEGRG
SEQIDNO14   GTQHLDNEIAWELSSGGFSNYFTRPWYQEDAAKTYLERHVSTETKAYYE--RYANFLGRG
SEQIDNO5    GTVSFNPEVAWDGSSGGFSYYFSRPWYQEAAVGTYLNKYVSEETKEYYK--SYVDFSGRG
SEQIDNO1    GTVSFNPEVAWAGSSGGFSYYFSRPWYQQEAVGTYLEKYVSAETKKYYG--PYVDFSGRG
SEQIDNO2    GTVSFNPEVAWAGSSGGFSYYFSRPWYQQEAVGTYLEKYVSAETKKYYG--PYVDFSGRG
SEQIDNO13   GTVNFNPEVAWDGSSGGFSYYFSRPWYQEEAVGNYLEKHVSAETKKYYG--PYVDFSGRG
SEQIDNO11   GTQSVTPEIAWVASSGGFSNYFPRTWYQEPAIQTYLG-LLDDETKTYYS--QYTNFEGRG
SEQIDNO10   GTMSYAPEIAWEASSGGFSNYFERAWFQKEAVQNYLANHITNETKQYYS--QFANFSGRG
SEQIDNO27   GTQSYAPEVAWDASSGGFSNYFSRPWYQYFAVENYLNNHITKDTKKYYS--QYTNFKGRG
SEQIDNO28   GTQSYAPEVAWDGSSGGFSNYFSRPWYQSFAVDNYLNNHITKDTKKYYS--QYTNFKGRG
SEQIDNO15   GTVRLDPEIAVSFSGGGFSRYFSRPSYQNQTVAQFVS-NLGNTFNGLY------NKNGRA
SEQIDNO16   STVGFEPERAVSFSSGGFSIYHARPDYQNEVVPKYIESIKASGYEKFF------DGNGRG
SEQIDNO4    STRY-LNETATGFSSGGFSDYWKRPSYQDDAVKAYFH-HLGEKFKPYF------NRHGRG
SEQIDNO20   GTNGTAPESGVYFSSGGFSDYWARPAYQNAAVESYLR-KLGSTQAYF------NRSGRA
SEQIDNO24   ATTGISPERGVFFSSGGFSDLWSRPSWQSHAVKAYLH-KLGKRQDGLF------NREGRA
SEQIDNO23   ATTHTAPERAVYFSSGGFSDLWDRPTWQEDAVSEYLE-NLGDRWSGLF------NPKGRA
SEQIDNO25   ATTHTAPEQAVYFSSGGFSDLWARPKWQEEAVSEYLE-ILGNRWSGLF------NPKGRA
SEQIDNO26   ATTHTAPEKAVYFSSGGFSDLWDRPKWQEDAVSDYLD-TLGDRWSGLF------NPKGRA
SEQIDNO21   ATEKMAPEQATYFSSGGFSDLFPRPKYQDAAVSSYLQ-TLGSRYQGLY------NGSNRA
SEQIDNO22   ATTKTNPEQAVYFSSGGFSDFWKRPKYQDEAVAAYLD-TLGDKFAGLF------NKGGRA
SEQIDNO3    ATFKTTPERGTYFSSGGFSDYWPRPEWQDEAVSSYLE-TIGDTFKGLY------NSSGRA
SEQIDNO19   ATSKTSPEQAVSFSSGGFSDLWPRPSYQQAAVQTYLTQHLGNKFSGLF------NASGRA
SEQIDNO6    GTVGTGPERAVSFSSGGFSDRFPRPQYQDNAVKDYLK-ILGNQWSGLF------DPNGRA
SEQIDNO17   GTRYVDPEVAVSFSSGGFSDIFPTPLYQKGAVSGYLK-ILGDRWKGLY------NPHGRG
SEQIDNO18   GTVGVEPEKAVSFSSGGFSDLWPRPAYQEKAVSEYLE-KLGDRWNGLY------NPQGRG
            .*    *  .  *.****     :*   .  :.         :         *.
```

## FIG. 17 Continued

```
SEQIDNO8    YPDLSAHSSSPRYAYIDKLAPGLTGGTSASCPVVAGIVGLLNDARLRRGLPTMGFINPWL
SEQIDNO9    FPDLSAHSSSPKYAYVDKRVPGLTGGTSASCPVVAGIVGLLNDARLRRGLPTMGFINPWL
SEQIDNO7    FPDVAGHSVSPNYEVIYAGKQSASGGTSAAAPVWAAIVGLLNDARFRAGKPSLGWLNPLV
SEQIDNO12   FPDVAGHSVAPDYEVIYNGKQARSGGTSAAAPVWAAIVGLLNDARFKAGKKSLGWLNPLI
SEQIDNO14   FPDVAALSLNPDYPVIIGGELGPNGGTSAAAPVVASIIALLNDARLCLGKPALGFLNPLI
SEQIDNO5    FPDVAAHSVSPDYPVFQGGELTPSGGTSAASPIVASVIALLNDARLRAGKPALGFLNPLI
SEQIDNO1    FPDVAAHSVSPDYPVFQGGELTPSGGTSAASPVVAAIVALLNDARLREGKPTLGFLNPLI
SEQIDNO2    FPDVAAHSVSPDYPVFQGGELTPSGGTSAASPVVAAIVALLNDARLREGKPTLGFLNPLI
SEQIDNO13   FPDVAAHSVSPDYPVFQGGQLTPSGGTSAASPVVASIIALLNDARLREGKPTLGFLNPLI
SEQIDNO11   FPDVSAHSLTPDYQVVGGGYLQPSGGTSAASPVFAGIIALLNDARLAAGKPTLGFLNPFF
SEQIDNO10   FPDVSAHSFEPSYEVIFYGARYGSGGTSAACPLFSALVGMLNDARLRAGKSTLGFLNPLL
SEQIDNO27   FPDVSAHSLTPDYEVVLTGKHYKSGGTSAACPVFAGIVGLLNDARLRAGKSTLGFLNPLL
SEQIDNO28   FPDVSAHSLTPYYEVVLTGKHYKSGGTSAASPVFAGIVGLLNDARLRAGKSTLGFLNPLL
SEQIDNO15   YPDLAAQGN--GFQVVIDGIVRSVGGTSASSPTVAGIFALLNDFKLSRGQSTLGFINPLI
SEQIDNO16   IPDVAAQGA--RFVVIDKGRVSLISGTSASSPAFAGMVALVNAARKSKDMPALGFLNPML
SEQIDNO4    FPDVATQGY--GFRVYDQGKLKGLQGTSASAPAFAGVIGLLNDARLKAKKPTLGFLNPLL
SEQIDNO20   FPDVAAQAQ--NFAVVDKGRVGLFDGTSCSSPVFAGIVALLNDVRLKAGLPVLGFLNPWL
SEQIDNO24   FPDVSAQGE--NYAIYAKGRLGKVDGTSCSAPAFAGLVSLLNDARIKAGKSSLGFLNPWL
SEQIDNO23   FPDVAAQGE--NYAIYDKGSLISVDGTSCSAPAFAGVIALLNDARIKANRPPMGFLNPWL
SEQIDNO25   FPDVTAQGR--NYAIYDKGSLTSVDGTSCSAPAFAGVVALLNDARLKVNKPPMGFLNPWL
SEQIDNO26   FPDVSAQGQ--NYAIYDKGSLTSVDGTSCSAPAFAGVIALLNDARLKANKPPMGFLNPWL
SEQIDNO21   FPDVSAQGT--NFAVYDKGRLGQFDGTSCSAPAFSGIIALLNDVRLQNNKPVLGFLNPWL
SEQIDNO22   FPDVAAQGM--NYAIYDKGTLGRLDGTSCSAPAFSAIISLLNDARLREGKPTMGFLNPWL
SEQIDNO3    FPDVAAQGM--NFAVYDKGTLGEFDGTSASAPAFSAVIALLNDARLRAGKPTLGFLNPWL
SEQIDNO19   FPDVAAQGV--NYAVYDKGMLGQFDGTSCSAPTFSGVIALLNDARLRAGLPVMGFLNPFL
SEQIDNO6    FPDIAAQGS--NYAVYDKGRMTGVSGTSASAPAMAAIIAQLNDFRLAKGSPVLGFLNPWI
SEQIDNO17   FPDVSGQSV--RYHVFDYGKDVMYSGTSASAPMFAALVSLLNNARLAKKLPPMGFLNPWL
SEQIDNO18   FPDVAAQGQ--GFQVFDKGRLISVGGTSASAPVFASVVALLNNARKAAGMSSLGFLNPWI
            **::   .      :          ***.:.*  :.:.. :*   :        :*::** .


SEQIDNO8    YTRGFE--ALQDVTGGRASGCQGIDLQRGTRVP-GAGIIPWASWNATPGWDPATGLGLPD
SEQIDNO9    YAKGYQ--ALEDVTGGAAVGCQGIDIQTGKRVP-GAGIIPGASWNATPDWDPATGLGLPN
SEQIDNO7    YKYGPK--VLTDITGGYAIGCDGNNTQSGKPEPAGSGIVPGARWNATAGWDPVTGYGTPD
SEQIDNO12   YKHGPK--VLTDITGGYAIGCDGNNTQSGKPEPAGSGLVPGARWNATAGWDPTTGYGTPN
SEQIDNO14   YQYADK-GGFTDITSGQSWGCAGNTTQTGPPPP-GAGVIPGAHWNATKGWDPVTGFGTPN
SEQIDNO5    YGYAYK--GFTDITSGQAVGCNGNNTQTGGPLP-GAGVIPGAFWNATKGWDPTTGFGVPN
SEQIDNO1    YLHASK--GFTDITSGQSEGCNGNNTQTGSPLP-GAGFIAGAHWNATKGWDPTTGFGVPN
SEQIDNO2    YLHASK--GFTDITSGQSEGCNGNNTQTGSPLP-GAGFIAGAHWNATKGWDPTTGFGVPN
SEQIDNO13   YQYAYK--GFTDITSGQSDGCNGNNTQTDAPLP-GAGVVLGAHWNATKGWDPTTGFGVPN
SEQIDNO11   YLYGYK--GLNDITGGQSVGCNGINGQTGAPVP-GGGIVPGAAWNSTTGWDPATGLGTPD
SEQIDNO10   YSKGYK--ALTDVTAGQSIGCNGIDPQSDEAVA-GAGIIPWAHWNATVGWDPVTGLGLPD
SEQIDNO27   YSILAE--GFTDITAGSSIGCNGINPQTGKPVP-GGGIIPYAHWNATAGWDPVTGLGVPD
SEQIDNO28   YSILAE--GFTDITAGSSIGCNGINPQTGKPVP-GGGIIPYAHWNATAGWDPVTGLGVPD
SEQIDNO15   YSSATS--GFNDIRAGTNPGCGTRG-----------------FTAGTGWDPVTGLGTPD
SEQIDNO16   YQNAA---AMTDIVNGAGIGCRKQR----------TEFPNGARFNATAGWDPVTGLGTPL
SEQIDNO4    YSNS---DALNDIVLGGSKGCDGHARFNGPP--NGSPVIPYAGWNATAGWDPVTGLGTPN
SEQIDNO20   YQDGLN--GLNDIVDGGSTGCDGNNRFNGSP--NGSPVIPYAGWNATEGWDPVTGLGTPD
SEQIDNO24   YSHP-D--ALNDITVGGSTGCDGNARFGGRP--NGSPVVPYASWNATEGWDPVTGLGTPN
SEQIDNO23   YSEGRS--GLNDIVNGGSTGCDGHGRFSGPT--NGGTSIPGASWNATKGWDPVSGLGSPN
SEQIDNO25   YSTGRA--GLKDIVDGGSTGCDGKSRFGGAN--NGGPSIPGASWNATKGWDPVSGLGSPN
SEQIDNO26   YSTGRD--GLNDIVHGGSTGCDGNARFGGPG--NGSPRVPGASWNATKGWDPVSGLGSPN
SEQIDNO21   YGAGSK--GLNDVVHGGSTGCDGQERFAGKA--NGSPVVPYASWNATQGWDPVTGLGTPD
SEQIDNO22   YGEGRE--ALNDVVVGGSKGCDGRDRFGGKP--NGSPVVPFASWNATQGWDPVTGLGTPN
SEQIDNO3    YKTGRQ--GLQDITLGASIGCTGRARFGGAP--DGGPVVPYASWNATQGWDPVTGLGTPD
SEQIDNO19   YGVGSESGALNDIVNGGSLGCDGRNRFGGTP--NGSPVVPFASWNATTGWDPVSGLGTPD
SEQIDNO6    YSKGFS--GFTDIVDGGSRGCTGYDIYSGLK----AKKVPYASWNATKGWDPVTGFGTPN
SEQIDNO17   YTVGFN--GLTDIVHGGSTGCTGTDVYSGLP----TPFVPYASWNATVGWDPVTGLGTPL
SEQIDNO18   YEQGYK--GLTDIVAGGSTGCTGRSIYSGLP----APLVPYASWNATEGWDPVTGYGTPD
            *      :  *:  *    **                    :.:   ***.:* * *
```

## FIG. 17 Continued

```
SEQIDNO8       FWAMRGLALGRGT--------------
SEQIDNO9       FWAMRELALED---------------
SEQIDNO7       FGKLKDLVLSF---------------
SEQIDNO12      FQKLKDLVLSL---------------
SEQIDNO14      FKKLLSLALSV---------------
SEQIDNO5       FKKLLELVRY----------------
SEQIDNO1       LKKLLALVRF----------------
SEQIDNO2       LKKLLALVRF----------------
SEQIDNO13      FKKLLELIRYI---------------
SEQIDNO11      FQKLKELVLSF---------------
SEQIDNO10      FEKLRQLVLSL---------------
SEQIDNO27      FMKLKELVLSL---------------
SEQIDNO28      FMKLKELVLSL---------------
SEQIDNO15      FLRLQGLI------------------
SEQIDNO16      FDKLLAVGAPGVPNA-----------
SEQIDNO4       FPKLLKAAVPSRYRA-----------
SEQIDNO20      FAKLKALVLDA---------------
SEQIDNO24      FQKLLKSAVKQK--------------
SEQIDNO23      FAAMRKLANAE---------------
SEQIDNO25      FATMRKLANAE---------------
SEQIDNO26      FATMRKLANGE---------------
SEQIDNO21      FGKLKDLALSA---------------
SEQIDNO22      FAKMLELAP-----------------
SEQIDNO3       FAELKKLALGN---------------
SEQIDNO19      FAKLRGVALGEAKAYGN---------
SEQIDNO6       FQALTKVLP-----------------
SEQIDNO17      FDKLLNLSTPNFHL------PHIGGH-
SEQIDNO18      FKQLLTLATAPKSGERRVRRGGLGGQA
                :   :
```

# FIG. 17 Continued

B. Mature sequences
CLUSTAL O(1.2.1) multiple sequence alignment

```
SEQIDNO35       --------------CDLVITPPCLEAAYNYKNYMPDPNSGSRVSFTSFLEQAAQQSDLT
SEQIDNO36       --------------CATIITPPCLETAYNYKGYIPDPKSGSRVSFTSFLEQAAQQADLT
SEQIDNO41       ---------------QIFHPDCLKTKYGVDGYAPSPRCGSRIGFGSFLNETASYSDLA
SEQIDNO32       ---------------VFQPDCLRTEYSVNGYKPSAKSGSRIGFGSFLNQSASSSDLA
SEQIDNO29       QEPSSCKGTLVFEGETFNVFQPDCLRTEYSVDGYTPSVKSGSRIGFGSFLNESASFADQA
SEQIDNO40       ---------------VFQPDCLRTEYNVNGYTPSAKSGSRIGFGSFLNQSASFSDLA
SEQIDNO34       --------------CQTSITPSCLKQMYNIGDYTPKVESGSTIGFSSFLGESAIYSDVF
SEQIDNO39       --------------CQTSITPSCLKQMYNIGDYTPDAKSGSEIGFSSFLGQAAIYSDVF
SEQIDNO38       --------------CQTSITPACLKQMYNVGNYTPSVAHGSRVGFGSFLNQSAIFDDLF
SEQIDNO37       --------------CADTITLSCLKEMYNFGNYTPSASSGSKLGFASFLNESASYSDLA
SEQIDNO54       --------------CANLITPDCLVEMYNLGDYKPDASSGSRVGFGSFLNQSANYADLA
SEQIDNO55       --------------CANLITPDCLVEMYNLGDYKPDASSGSRVGFGSFLNESANYADLA
SEQIDNO42       ---------------TVITPDCLRDLYNTADYVPSATSRNAIGIAGYLDRSNR-ADLQ
SEQIDNO43       ----------------GVTPLCLRTLYRVNYKP--ATTGNLVAFASFLEQYARYSDQQ
SEQIDNO31       --------------CDATITPQCLKTLYKI-DYKADPKSGSKVAFASYLEQYARYNDLA
SEQIDNO51       --------------CNSAITPQCLKDLYKVGDYKASASNGNKVAFTSYLEQYARYSDLA
SEQIDNO53       --------------CNSIITPQCLKNMYNVGDYQADDDNGNKVGFASYLEEYARYSDLE
SEQIDNO50       --------------CNSIITPQCLKDLYSIGDYEADPTNGNKVAFASYLEEYARYSDLA
SEQIDNO52       --------------CNSIITPQCLKDIYNIGDYQANDTNGNKVGFASYLEEYARYSDLA
SEQIDNO47       --------------CEKAITPSCLADLYNTEGYKASNRSGSKVAFASFLEEYARYDDLA
SEQIDNO48       --------------CDQITTPHCLRKLYNVNGYKADPASGSKIGFASFLEEYARYSDLV
SEQIDNO49       --------------CDSVITPKCLKDLYKVGDYEADPDSGSQVAFASYLEEYARYADMV
SEQIDNO30       --------------CDSIITPTCLKELYNIGDYQADANSGSKIAFASYLEEYARYADLE
SEQIDNO46       --------------CDSIITPHCLKQLYNIGDYQADPKSGSKVGFASYLEEYARYADLE
SEQIDNO33       --------------CRSLVTTACLRELYGLGDRVTQARDDNRIGVSGFLEEYAQYRDLE
SEQIDNO44       --------------CNASITPECLRALYNVGDYEADPSKKSLFGVCGYLEQYAKHDQLA
SEQIDNO45       --------------CNKKITPDCLANLYNFKDYDASDA-NVTIGVSGFLEQYARFDDLK
                                **    *             ...  .:*  .      :

SEQIDNO35       KFLSLTGLDRLRPPSSK[ASFDT]VLINGGETHQGTPP-NKT[SEANLD]VQWLAAVIKARLP
SEQIDNO36       KFLSLTRLEGFRTPASKK[KTFKT]VLINGGESHEGVHKKSKT[SEANLD]VQWLAAVTQTKLP
SEQIDNO41       QFEKYFDLP--------N[QNLST]LLINGAID-VQPPSNKND[SEANMD]VQTILTFV-QPLP
SEQIDNO32       LFEKHFGFA--------S[QGFSV]ELINGGS-NPQPPTDAND[GEANLD]AQNIVSFV-QPLP
SEQIDNO29       LFEKHFNIP--------S[QNFSV]VLINGGTDLPQPPSDAND[GEANLD]AQTILTIA-HPLP
SEQIDNO40       LFEKHFGFS--------S[QNFSV]VLINGGTDLPQPPSDDND[GEANLD]VQNILTIA-HPLP
SEQIDNO34       LFEEKFGIP--------T[QNFTT]VLINNGTD-DQNTAHKNF[GEADLD]AENIVGIA-HPLP
SEQIDNO39       KFEELFGIP--------K[QNYTT]ILINNGTD-DQNTAHGNF[GEANLD]AENIVGIA-HPLP
SEQIDNO38       TYEKVNDIP--------S[QNFTK]VIIANASN-SQDASDGNY[GEANLD]VQNIVGIS-HPLP
SEQIDNO37       KFERLFNLP--------S[QNFSV]ELINGGVN-DQNQSTASL[TEADLD]VELLVGVG-HPLP
SEQIDNO54       AYEQLFNIP--------P[QNFSV]ELINGGAN-DQNWATASL[GEANLD]VELIVAVS-HALP
SEQIDNO55       AYEQLFNIP--------P[QNFSV]ELINRGVN-DQNWATASL[GEANLD]VELIVAVS-HPLP
SEQIDNO42       TFFRR-F-----RPDAVG[FNYTT]VQLNGGGD-DQND-PG--[VEANLD]IQYAAGIAF-PTP
SEQIDNO43       AFTQRVL-----GPGVPL[QNFSV]ETVNGGAN-DQQS-KLDS[GEANLD]LQYVMAMSH-PIP
SEQIDNO31       LFEKA-F-----LPEAVG[QNFSV]QFSGGLN-DQNT-TQDS[GEANLD]LQYIVGVSA-PLP
SEQIDNO51       LFEQN-I-----APYAQG[QNFTV]IQYNGGLN-DQSS-PADS[SEANLD]LQYIIGTSS-PVP
SEQIDNO53       LFEKN-V-----APFAKG[QNFSV]IQYNGGLN-DQHS-SASS[SEANLD]LQYIVGVSS-PVP
SEQIDNO50       LFEKN-I-----APFAKG[QNFSV]VQYNGGGN-DQQS-SSGS[SEANLD]LQYIVGVSS-PVP
SEQIDNO52       LFEKN-I-----APSAKG[QNFSV]TRYNGGLN-DQSS-SGGS[SEANLD]LQYIVGVSS-PVP
SEQIDNO47       EFEET-Y-----APYAIG[QNFSV]ISINGGLN-DQDS-TADS[GEANLD]LQYIIGVSS-PLP
SEQIDNO48       LFEEN-L-----APFAEG[ENFTV]VMYNGGKN-DQNS-KSDS[GEANLD]LQYIVGMSA-GAP
SEQIDNO49       KFQNS-L-----APYAKG[QNFSV]VLYNGGVN-DQSS-SADS[GEANLD]LQTIMGLSA-PLP
SEQIDNO30       NFENY-L-----APWAKG[QNFSV]TTFNGGLN-DQNS-SSDS[GEANLD]LQYILGVSA-PLP
SEQIDNO46       RFEQH-L-----APNAIG[QNFSV]VQFNGGLN-DQLS-LSDS[GEANLD]LQYILGVSA-PVP
SEQIDNO33       LFLSR-F-----EPSAKG[FNFSE]GLIAGGKN-TQGG-PGSS[TEANLD]MQYVVGLSH-KAK
SEQIDNO44       KFEQT-Y-----APYAIG[ADFSV]TINGGGD-NQTS-TIDD[GEANLD]MQYAVSMAY-KTP
SEQIDNO45       QFIST-F-----QPKAAG[STFQV]TSVNAGPF-DQNS-TASS[VEANLD]IQYTTGLVAPDIE
                :                                    .        **::*  :
```

FIG. 17 Continued

```
SEQIDNO35    ITQWITGGRPPFVPNLRLRHEKDNTNEPYLEFFEYLVRLPARDLPQVISNSYAEDEQTVP
SEQIDNO36    ITQWITGGRPPFVPNLRIPTPEANTNEPYLEFLEYLFRLPDKDLPQVISNSYAEDEQSVP
SEQIDNO41    ITEFVVAGIPPYIPDAALPIGDPVQNEPWLEYFEFLMSRTNAELPQVIANSYGDEEQTVP
SEQIDNO32    ITEFIAGGTAPYFPDPVEPAGTPDENEPYLEYYEYLLSKSNKELPQVITNSYGDEEQTVP
SEQIDNO29    ITEFITAGSPPYFPDPVEPAGTPNENEPYLQYYEFLLSKSNAEIPQVITNSYGDEEQTVP
SEQIDNO40    ITEFITAGSPPYFPDPVEPAGTPDENEPYLQYFEYLLSKPNRDLPQVITNSYGDEEQTVP
SEQIDNO34    FTQYITGGSPPFLPNIDQPTAADNQNEPYVPFFRYLLSQK--EVPAVVSTSYGDEEDSVP
SEQIDNO39    FKQYITGGSPPFVPNIDQPTEKDNQNEPYVPFFRYLLGQK--DLPAVISTSYGDEEDSVP
SEQIDNO38    VTEFLTGGSPPFVASLDTPT---NQNEPYIPYYEYLLSQKNEDLPQVISNSYGDDEQSVP
SEQIDNO37    VTEFITSGEPPFIPDPDEPSAADNENEPYLQYYEYLLSKPNSALPQVISNSYGDDEQTVP
SEQIDNO54    VVEFITGGSPPFVPNVDEPTAADNQNEPYLQYYEYLLSKPNSHLPQVISNSYGDDEQTVP
SEQIDNO55    VVEFITGALPPVLRVLAL------------------------QTQLPSSSGDFQLTVP
SEQIDNO42    ATYWSTGGSPPFIPDTQTPT---NTNEPYLDWINFVL--GQDEIPQVISTSYGDDEQTVP
SEQIDNO43    ILEYSTGGRGPLVPTLDQPNANNSSNEPYLEFLTYLLAQPDSAIPQTLSVSYGEEEQSVP
SEQIDNO31    VTEFSTGGRGPWVADLDQPDEADSANEPYLEFLQGVLKLPQSELPQVISTSYGENEQSVP
SEQIDNO51    VTEFSTGGRGPLVPDLDQPDINDNNNEPYLDFLQNVIKMSDKDLPQVISTSYGEDEQSVP
SEQIDNO53    VTEFSVGGRGELVPDLDQPDPNDNNNEPYLEFLQNVLKMEQQDLPQVISTSYGENEQSVP
SEQIDNO50    VTEFSTGGRGELVPDLDQPNPNDNNNEPYLEFLQNVLKLHKKDLPQVISTSYGEDEQSVP
SEQIDNO52    VTEFSVGGRGELVPDLDQPDPNDNNNEPYLEFLQNVLKLDKKDLPQVISTSYGEDEQSIP
SEQIDNO47    VTEFTTGGRGKLIPDLSSPDPNDNTNEPFLDFLEAVLKLDQKDLPQVISTSYGEDEQTIP
SEQIDNO48    VTEFSTAGRAPVIPDLDQPDPSAGTNEPYLEFLQNVLHMDQEHLPQVISTSYGENEQTIP
SEQIDNO49    ITEYITGGRGKLIPDLSQPNPNDNSNEPYLEFLQNILKLDQDELPQVISTSYGEDEQTIP
SEQIDNO30    VTEFSTGGRGPLVPDLTQPDPNSNSNEPYLEFFQNVLKLDQKDLPQVISTSYGENEQEIP
SEQIDNO46    VTEYSTGGRGELVPDLSSPDPNDNSNEPYLDFLQGILKLDNSDLPQVISTSYGEDEQTIP
SEQIDNO33    VTYYSTAGRGPLIPDLSQPSQASNNNEPYLEQLRYLVKLPKNQLPSVLTTSYGDTEQSLP
SEQIDNO44    ITYYSTGGRGPLVPDLDQPDPNDVSNEPYLDFVSYLLKLPDSKLPQTITTSYGEDEQSVP
SEQIDNO45    TRYFTVPGRGILIPDLDQPTESDNANEPYLDYFTYLNNLEDEELPDVLTTSYGESEQSVP
             :  .  .      .                                 :   * .: :   :*
```

```
SEQIDNO35    EAYARRVCNLIGIMGLRGVTVLTASGDSGVG-APCRANDGSDRLEFSPQFPTSCPY|ITAV|
SEQIDNO36    EAYARRVCGLLGIMGLRGVTVLTASGDSGVG-APCRANDGSGREEFSPQFPSSCPY|ITTV|
SEQIDNO41    QAYAVRVCNQIGLLGLRGISVIASSGDTGVG-MSCMASNS-TTPQFNPMFPASCPY|ITTV|
SEQIDNO32    QAYAVRVCNLIGLMGLRGISILESSGDEGVG-ASCLATNSTTTPQFNPIFPATCPY|VTSV|
SEQIDNO29    RSYAVRVCNLIGLLGLRGISVLHSSGDEGVG-ASCVATNS-TTPQFNPIFPATCPY|VTSV|
SEQIDNO40    QAYAVRVCNLIGLMGLRGISILESSGDEGVG-ASCVATNS-TTPQFNPIFPATCPY|VTSV|
SEQIDNO34    REYATMTCNLIGLLGLRGISVIFSSGDIGVG-AGCLGPDHK-TVEFNAIFPATCPY|LTSV|
SEQIDNO39    REYATLTCNMIGLLGLRGISVIFSSGDIGVG-SGCLAPDYK-TVEFNAIFPATCPY|LTSV|
SEQIDNO38    YKYAIRACNLIGLTGLRGISVLESSGDLGVG-AGCRSNDGKNKTQFDPIFPATCPY|VTSV|
SEQIDNO37    EYYAKRVCNLIGLVGLRGISVLESSGDEGIG-SGCRTTDGTNSTQFNPIFPATCPY|VTAV|
SEQIDNO54    EYYARRVCNLIGLMGLRGITVLESSGDTGIG-SACMSNDGTNTPQFTPTFPGTCPF|ITAV|
SEQIDNO55    EYYARRVCNLIGLMGLRGITVLESSGDTGIG-SACMSNDGTNKPQFTPTFPGTCPF|ITAV|
SEQIDNO42    EDYATSVCNLFAQLGSRGVTVFFSSGDFGVGGGDCLTNDGSNQVLFQPAFPASCPF|VTAV|
SEQIDNO43    RDYAIKVCNMFMQLGARGVSVMFSSGDSGPG-NDCVRA-SDNATFFGSTFPAGCPY|VTSV|
SEQIDNO31    KSYALSVCNLFAQLGSRGVSVIFSSGDSGPG-SACQSNDGKNTTKFQPQYPAACPF|VTSV|
SEQIDNO51    ASYARSVCNLIAQLGGRGVSVIFSSGDSGVG-SACQTNDGKNTTRFPAQFPAACPW|VTSV|
SEQIDNO53    EKYARTVCNLFSQLGSRGVSVIFASGDSGVG-AACQTNDGRNATRFPAQFPAACPW|VTSV|
SEQIDNO50    EKYARAVCNLYSQLGSRGVSVIFSSGDSGVG-AACQTNDGRNATHFPPQFPAACPW|VTSV|
SEQIDNO52    EKYARVSQLGSRGVSVIFSSGDSGVG-SACLTNDGRNATRFPPQFPAACPW|VTSV|
SEQIDNO47    EPYARSVCNLYAQLGSRGVSVLFSSGDSGVG-AACQTNDGKNTTHFPPQFPASCPW|VTAV|
SEQIDNO48    EKYARTVCNMYAQLGSRGVSVIFSSGDSGVG-SACMTNDGTNRTHFPPQFPASCPW|VTSV|
SEQIDNO49    RGYAESVCNMLAQLGSRGVSVVFSSGDSGVG-AACQTNDGRNQTHFNPQFPASCPW|VTSV|
SEQIDNO30    EKYARTVCNLIAQLGSRGVSVLFSSGDSGVG-EGCMTNDGTNRTHFPPQFPAACPW|VTSV|
SEQIDNO46    VPYARTVCNLYAQLGSRGVSVIFSSGDSGVG-AACLTNDGTNRTHFPPQFPASCPW|VTSV|
SEQIDNO33    ASYTKATCDLFAQLGTMGVSVIFSSGDTGPG-SSCQTNDGKNATRFNPIYPASCPF|VTSI|
SEQIDNO44    RSYVEKVCTMFGALGARGVSVIFSSGDTGVG-SACQTNDGKNTTRFLPIFPAACPY|VTSV|
SEQIDNO45    AEYAKKVCNLIGQLGARGVSVIFSSGDTGPG-SACQTNDGKNTTRFLPIFPASCPY|VTSV|
             *.  .*      *  *:::. :*** * *   *            *   :*  **:.:*::
```

# FIG. 17 Continued

```
SEQIDNO35  GGTEGWDPEVAWEASSGGFSHYFLRPWYQANAVEKYLDEELDPATRAYYDGNGFVQFAGR
SEQIDNO36  GGTQAWDPEVAWKGSSGGFSNYFPRPWYQVAAVEKYLEEQLDPAAREYYEENGFVRFAGR
SEQIDNO41  GGTQHLDNEIAWELSSGGFSNYFTRPWYQEDAAKTYLERHVSTETKAYYE--RYANFLGR
SEQIDNO32  GGTVSFNPEVAWDGSSGGFSYYFSRPWYQEAAVGTYLNKYVSEETKEYYK--SYVDFSGR
SEQIDNO29  GGTVSFNPEVAWAGSSGGFSYYFSRPWYQQEAVGTYLEKYVSAETKKYYG--PYVDFSGR
SEQIDNO40  GGTVNFNPEVAWDGSSGGFSYYFSRPWYQEEAVGNYLEKHVSAETKKYYG--PYVDFSGR
SEQIDNO34  GGTVDVTPEIAWEGSSGGFSKYFPRPSYQDKAVKTYMK-TVSKQTKKYYG--PYTNWEGR
SEQIDNO39  GGTVDVTPEIAWEGSSGGFSKYFPRPSYQDKAIKKYMK-TVSKETKKYYG--PYTNWEGR
SEQIDNO38  GGTQSVTPEIAWVASSGGFSNYFPRTWYQEPAIQTYLG-LLDDETKTYYS--QYTNFEGR
SEQIDNO37  GGTMSYAPEIAWEASSGGFSNYFERAWFQKEAVQNYLANHITNETKQYYS--QFANFSGR
SEQIDNO54  GGTQSYAPEVAWDASSGGFSNYFSRPWYQYFAVENYLNNHITKDTKKYYS--QYTNFKGR
SEQIDNO55  GGTQSYAPEVAWDGSSGGFSNYFSRPWYQSFAVDNYLNNHITKDTKKYYS--QYTNFKGR
SEQIDNO42  GGTVRLDPEIAVSFSGGGFSRYFSRPSYQNQTVAQFVS-NLGNTFNGLY------NKNGR
SEQIDNO43  GSTVGFEPERAVSFSSGGFSIYHARPDYQNEVVPKYIESIKASGYEKFF------DGNGR
SEQIDNO31  GSTRY-LNETATGFSSGGFSDYWKRPSYQDDAVKAYFH-HLGEKFKPYF------NRHGR
SEQIDNO51  GATTGISPERGVFFSSGGFSDLWSRPSWQSHAVKAYLH-KLGKRQDGLF------NREGR
SEQIDNO53  GATTHTAPEKAVYFSSGGFSDLWDRPKWQEDAVSDYLD-TLGDRWSGLF------NPKGR
SEQIDNO50  GATTHTAPERAVYFSSGGFSDLWDRPTWQEDAVSEYLE-NLGDRWSGLF------NPKGR
SEQIDNO52  GATTHTAPEQAVYFSSGGFSDLWARPKWQEEAVSEYLE-ILGNRWSGLF------NPKGR
SEQIDNO47  GGTNGTAPESGVYFSSGGFSDYWARPAYQNAAVESYLR-KLGSTQAYF------NRSGR
SEQIDNO48  GATEKMAPEQATYFSSGGFSDLFPRPKYQDAAVSSYLQ-TLGSRYQGLY------NGSNR
SEQIDNO49  GATTKTNPEQAVYFSSGGFSDFWKRPKYQDEAVAAYLD-TLGDKFAGLF------NKGGR
SEQIDNO30  GATFKTTPERGTYFSSGGFSDYWPRPEWQDEAVSSYLE-TIGDTFKGLY------NSSGR
SEQIDNO46  GATSKTSPEQAVSFSSGGFSDLWPRPSYQQAAVQTYLTQHLGNKFSGLF------NASGR
SEQIDNO33  GGTVGTGPERAVSFSSGGFSDRFPRPQYQDNAVKDYLK-ILGNQWSGLF------DPNGR
SEQIDNO44  GGTRYVDPEVAVSFSSGGFSDIFPTPLYQKGAVSGYLK-ILGDRWKGLY------NPHGR
SEQIDNO45  GGTVGVEPEKAVSFSSGGFSDLWPRPAYQEKAVSEYLE-KLGDRWNGLY------NPQGR
           *.*     *  .   *.****       :*   .   :.          :        *

SEQIDNO35  AYPDLSAHSSSPRYAYIDKLAPGLTGGTSASCPVVAGIVGLLNDARLRRGLPTMGFINPW
SEQIDNO36  AFPDLSAHSSSPKYAYVDKRVPGLTGGTSASCPVVAGIVGLLNDARLRRGLPTMGFINPW
SEQIDNO41  GFPDVAALSLNPDYPVIIGGELGPNGGTSAAAPVVASIIALLNDARLCLGKPALGFLNPL
SEQIDNO32  GFPDVAAHSVSPDYPVFQGGELTPSGGTSAASPIVASVIALLNDARLRAGKPALGFLNPL
SEQIDNO29  GFPDVAAHSVSPDYPVFQGGELTPSGGTSAASPVVAAIVALLNDARLREGKPTLGFLNPL
SEQIDNO40  GFPDVAAHSVSPDYPVFQGGQLTPSGGTSAASPVVASIIALLNDARLREGKPTLGFLNPL
SEQIDNO34  GFPDVAGHSVSPNYEVIYAGKQSASGGTSAAAPVWAAIVGLLNDARFRAGKPSLGWLNPL
SEQIDNO39  GFPDVAGHSVAPDYEVIYNGKQARSGGTSAAAPVWAAIVGLLNDARFKAGKKSLGWLNPL
SEQIDNO38  GFPDVSAHSLTPDYQVVGGGYLQPSGGTSAASPVFAGIIALLNDARLAAGKPTLGFLNPF
SEQIDNO37  GFPDVSAHSFEPSYEVIFYGARYGSGGTSAACPLFSALVGMLNDARLRAGKSTLGFLNPL
SEQIDNO54  GFPDVSAHSLTPDYEVVLTGKHYKSGGTSAACPVFAGIVGLLNDARLRAGKSTLGFLNPL
SEQIDNO55  GFPDVSAHSLTPYYEVVLTGKHYKSGGTSAASPVFAGIVGLLNDARLRAGKSTLGFLNPL
SEQIDNO42  AYPDLAAQGN--GFQVVIDGIVRSVGGTSASSPTVAGIFALLNDFKLSRGQSTLGFINPL
SEQIDNO43  GIPDVAAQGA--RFVVIDKGRVSLISGTSASSPAFAGMVALVNAARKSKDMPALGFLNPM
SEQIDNO31  GFPDVATQGY--GFRVYDQGKLKGLQGTSASAPAFAGVIGLLNDARLKAKKPTLGFLNPL
SEQIDNO51  AFPDVSAQGE--NYAIYAKGRLGKVDGTSCSAPAFAGLVSLLNDARIKAGKSSLGFLNPW
SEQIDNO53  AFPDVSAQGQ--NYAIYDKGSLTSVDGTSCSAPAFAGVIALLNDARLKANKPPMGFLNPW
SEQIDNO50  AFPDVAAQGE--NYAIYDKGSLISVDGTSCSAPAFAGVIALLNDARIKANRPPMGFLNPW
SEQIDNO52  AFPDVTAQGR--NYAIYDKGSLTSVDGTSCSAPAFAGVVALLNDARLKVNKPPMGFLNPW
SEQIDNO47  AFPDVAAQAQ--NFAVVDKGRVGLFDGTSCSSPVFAGIVALLNDVRLKAGLPVLGFLNPW
SEQIDNO48  AFPDVSAQGT--NFAVYDKGRLGQFDGTSCSAPAFSGIIALLNDVRLQNNKPVLGFLNPW
SEQIDNO49  AFPDVAAQGM--NYAIYDKGTLGRLDGTSCSAPAFSAIISLLNDARLREGKPTMGFLNPW
SEQIDNO30  AFPDVAAQGM--NFAVYDKGTLGEFDGTSASAPAFSAVIALLNDARLRAGLPVMGFLNPW
SEQIDNO46  AFPDVAAQGV--NYAVYDKGMLGQFDGTSCSAPTFSGVIALLNDARLRAGLPVMGFLNPF
SEQIDNO33  AFPDIAAQGS--NYAVYDKGRMTGVSGTSASAPAMAAIIAQLNDFRLAKGSPVLGFLNPW
SEQIDNO44  GFPDVSGQSV--RYHVFDYGKDVMYSGTSASAPMFAALVSLLNNARLAKKLPPMGFLNPW
SEQIDNO45  GFPDVAAQGQ--GFQVFDKGRLISVGGTSASAPVFASVVALLNNARKAAGMSSLGFLNPW
           . **::  .    :      .     ***.:.*  :.:..:*  :       :*::**
```

## FIG. 17 Continued

```
SEQIDNO35    LYTRGF--E-ALQDVTGGRASGCQGIDLQRGTRVP-GAGIIPWASWNATPGWDPATGLGL
SEQIDNO36    LYAKGY--Q-ALEDVTGGAAVGCQGIDIQTGKRVP-GAGIIPGASWNATPDWDPATGLGL
SEQIDNO41    IYQYAD--KGGFTDITSGQSWGCAGNTTQTGPPPP-GAGVIPGAHWNATKGWDPVTGFGT
SEQIDNO32    IYGYAY--K-GFTDITSGQAVGCNGNNTQTGGPLP-GAGVIPGAFWNATKGWDPTTGFGV
SEQIDNO29    IYLHAS--K-GFTDITSGQSEGCNGNNTQTGSPLP-GAGFIAGAHWNATKGWDPTTGFGV
SEQIDNO40    IYQYAY--K-GFTDITSGQSDGCNGNNTQTDAPLP-GAGVVLGAHWNATKGWDPTTGFGV
SEQIDNO34    VYKYGP--K-VLTDITGGYAIGCDGNNTQSGKPEPAGSGIVPGARWNATAGWDPVTGYGT
SEQIDNO39    IYKHGP--K-VLTDITGGYAIGCDGNNTQSGKPEPAGSGLVPGARWNATAGWDPTTGYGT
SEQIDNO38    FYLYGY--K-GLNDITGGQSVGCNGINGQTGAPVP-GGGIVPGAAWNSTTGWDPATGLGT
SEQIDNO37    LYSKGY--K-ALTDVTAGQSIGCNGIDPQSDEAVA-GAGIIPWAHWNATVGWDPVTGLGL
SEQIDNO54    LYSILA--E-GFTDITAGSSIGCNGINPQTGKPVP-GGGIIPYAHWNATAGWDPVTGLGV
SEQIDNO55    LYSILA--E-GFTDITAGSSIGCNGINPQTGKPVP-GGGIIPYAHWNATAGWDPVTGLGV
SEQIDNO42    IYSSAT--S-GFNDIRAGTNPGCGTRG------------------FTAGTGWDPVTGLGT
SEQIDNO43    LYQNA---A-AMTDIVNGAGIGCRKQR----------TEFPNGARFNATAGWDPVTGLGT
SEQIDNO31    LYSNS---D-ALNDIVLGGSKGCDGHARFNGPP--NGSPVIPYAGWNATAGWDPVTGLGT
SEQIDNO51    LYSHP---D-ALNDITVGGSTGCDGNARFGGRP--NGSPVVPYASWNATEGWDPVTGLGT
SEQIDNO53    LYSTGR--D-GLNDIVHGGSTGCDGNARFGGPG--NGSPRVPGASWNATKGWDPVSGLGS
SEQIDNO50    LYSEGR--S-GLNDIVNGGSTGCDGHGRFSGPT--NGGTSIPGASWNATKGWDPVSGLGS
SEQIDNO52    LYSTGR--A-GLKDIVDGGSTGCDGKSRFGGAN--NGGPSIPGASWNATKGWDPVSGLGS
SEQIDNO47    LYQDGL--N-GLNDIVDGGSTGCDGNNRFNGSP--NGSPVIPYAGWNATEGWDPVTGLGT
SEQIDNO48    LYGAGS--K-GLNDVVHGGSTGCDGQERFAGKA--NGSPVVPYASWNATQGWDPVTGLGT
SEQIDNO49    LYGEGR--E-ALNDVVVGGSKGCDGRDRFGGKP--NGSPVVPFASWNATQGWDPVTGLGT
SEQIDNO30    LYKTGR--Q-GLQDITLGASIGCTGRARFGGAP--DGGPVVPYASWNATQGWDPVTGLGT
SEQIDNO46    LYGVGSESG-ALNDIVNGGSLGCDGRNRFGGTP--NGSPVVPFASWNATTGWDPVSGLGT
SEQIDNO33    IYSKGF--S-GFTDIVDGGSRGCTGYDIYSGLK----AKKVPYASWNATKGWDPVTGFGT
SEQIDNO44    LYTVGF--N-GLTDIVHGGSTGCTGTDVYSGLP----TPFVPYASWNATVGWDPVTGLGT
SEQIDNO45    IYEQGY--K-GLTDIVAGGSTGCTGRSIYSGLP----APLVPYASWNATEGWDPVTGYGT
             .*        :  *:    *    **                     :.:    ***.:* *

SEQIDNO35    PDFWAMRGL---
SEQIDNO36    PNFWAMRELA--
SEQIDNO41    PNFKKLLSLALS
SEQIDNO32    PNFKKLLELV--
SEQIDNO29    PNLKKLLALVRF
SEQIDNO40    PNFKKLLELI--
SEQIDNO34    PDFGKLKDLVLS
SEQIDNO39    PNFQKLKDLVLS
SEQIDNO38    PDFQKLKELVLS
SEQIDNO37    PDFEKLRQLVLS
SEQIDNO54    PDFMKLKELVLS
SEQIDNO55    PDFMKLKELVLS
SEQIDNO42    PDFLRLQ-----
SEQIDNO43    PLFDKLLA----
SEQIDNO31    PNFPKLLKAA--
SEQIDNO51    PNFQKLLKSAV-
SEQIDNO53    PNFATMRKLA--
SEQIDNO50    PNFAAMRKLA--
SEQIDNO52    PNFATMRKLA--
SEQIDNO47    PDFAKLKALVL-
SEQIDNO48    PDFGKLKDLAL-
SEQIDNO49    PNFAKMLELA--
SEQIDNO30    PDFAELKKLA--
SEQIDNO46    PDFAKLRGV---
SEQIDNO33    PNFQALTKVL--
SEQIDNO44    PLFDKLLNL---
SEQIDNO45    PDFKQLLTLAT-
             * :   :
```

# FIG. 17 Continued

FIG. 18

FIG. 19

*FIG. 20*

*FIG. 21*

FIG. 22

FIG. 23

*FIG. 24*

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- US 5821104 A
- US 4683202 A
- WO 9218645 A
- US 5723323 A
- US 5605793 A
- WO 0058517 A
- WO 9117243 A
- WO 9735956 A
- EP 0238023 A
- EP 0449375 A
- US 5741665 A
- US 5674707 A
- US 3817837 A
- US 3850752 A
- US 3939350 A
- US 3996345 A
- US 4277437 A
- US 4275149 A
- US 4366241 A
- US 4816567 A
- WO 2007044968 A
- US 8592194 B2
- US 7972808 B2

### Non-patent literature cited in the description

- **CHEN et al.** *J. Anim. Sci.,* 2009, vol. 77, 1277-1283
- **XUE et al.** *Food Funct.,* 25 April 2013, vol. 4 (4), 640-7
- **ZHANG et al.** *Biomacromolecules,* 2011, vol. 12, 2894-2901
- **IVERSEN ; JORGENSEN.** *Biotechnology Techniques,* 1995, vol. 9, 573-576
- **CARUTHERS MH et al.** *Nuc Acids Res Symp Ser,* 1980, 215-23
- **HORN T et al.** *Nuc Acids Res Symp Ser,* 1980, 225-232
- **BEUCAGE S.L. et al.** *Tetrahedron Letters,* 1981, vol. 22, 1859-1869
- **MATTHES et al.** *EMBO J.,* 1984, vol. 3, 801-805
- **SAIKI R K et al.** *Science,* 1988, vol. 239, 487-491
- **AUSUBEL et al.** Short Protocols in Molecular Biology. 1999
- *FEMS Microbiol Lett,* 1999, vol. 174 (2), 247-50
- *FEMS Microbiol Lett,* 1999, vol. 177 (1), 187-8
- **ALTSCHUL et al.** *J. Mol. Biol.,* 1990, 403-410
- **HIGGINS DG ; SHARP PM.** *Gene,* 1988, vol. 73 (1), 237-244
- **SIMON RJ et al.** *PNAS,* 1992, vol. 89 (20), 9367-9371
- **HORWELL DC.** *Trends Biotechnol.,* 1995, vol. 13 (4), 132-134
- **MORINAGA et al.** *Biotechnology,* 1984, vol. 2, 646-649
- **NELSON ; LONG.** *Analytical Biochemistry,* 1989, vol. 180, 147-151
- **J. SAMBROOK ; E. F. FRITSCH ; T. MANIATIS.** Molecular Cloning: A Laboratory Manual. Cold Spring Harbor Laboratory Press, 1989, 1-3
- **NAKAMURA, Y. et al.** Codon usage tabulated from the international DNA sequence databases: status for the year 2000. *Nucl. Acids Res.,* 2000, vol. 28, 292
- **SAMBROOK et al.** Molecular Cloning: A Laboratory Manual. Cold Spring Harbor Laboratory Press, 1989
- **POTRYKUS.** *Annu Rev Plant Physiol Plant Mol Biol,* 1991, vol. 42, 205-225
- **CHRISTOU.** *Agro-Food-Industry Hi-Tech,* March 1994, 17-27
- **DAVIS ; DE SERRES.** *Methods Enzymol,* 1971, vol. 17A, 79-143
- **PUNT et al.** *Trends Biotechnol,* May 2002, vol. 20 (5), 200-6
- **ARCHER ; PEBERDY.** *Crit Rev Biotechnol,* 1997, vol. 17 (4), 273-306
- Aspergillus: 50 years on. Progress in industrial microbiology. **TURNER G.** Vectors for genetic manipulation. Elsevier, 1994, vol. 29, 641-666
- *Methods Mol Biol,* 1995, vol. 49, 341-54
- *Curr Opin Biotechnol,* October 1997, vol. 8 (5), 554-60
- *FEMS Microbiol Rev,* 2000, vol. 24 (1), 45-66
- Yeast as a vehicle for the expression of heterologous genes. **E HINCHCLIFFE E KENNY.** Yeasts. Academic Press Ltd, 1993, vol. 5

- **HINNEN et al.** *Proceedings of the National Academy of Sciences of the USA,* 1978, vol. 75, 1929
- **BEGGS, J D.** *Nature,* 1978, vol. 275, 104
- **ITO, H et al.** *J Bacteriology,* 1983, vol. 153, 163-168
- *Methods Enzymol,* 1990, vol. 182, 132-43
- *Curr Opin Biotechnol,* 1995, vol. 6 (5), 501-6
- Current Protocols in Molecular Biology. John Wiley & Sons
- **B. ROE ; J. CRABTREE ; A. KAHN.** DNA Isolation and Sequencing: Essential Techniques. John Wiley & Sons, 1996
- Oligonucleotide Synthesis: A Practical Approach. Irl Press, 1984
- Methods of Enzymology: DNA Structure Part A: Synthesis and Physical Analysis of DNA. **D. M. J. LILLEY ; J. E. DAHLBERG.** Methods in Enzymology. Academic Press, 1992
- **SINGLETON et al.** DICTIONARY OF MICROBIOLOGY AND MOLECULAR BIOLOGY. John Wiley and Sons, 1994

- **HALE ; MARHAM.** THE HARPER COLLINS DICTIONARY OF BIOLOGY. Harper Perennial, 1991
- **THOMAS NORDAHL PETERSEN ; SØREN BRUNAK ; GUNNAR VON HEIJNE ; HENRIK NIELSEN.** *Nature Methods,* 2011, vol. 8, 785-786
- **PENTTILÄ et al.** *Gene,* 1987, vol. 61, 155-164
- **REICHARD et al.** *Applied and Environmental Microbiology,* 2006, vol. 72, 1739-1748
- **P.M. NIELSEN ; D. PETERSEN ; C. DAMBMANN.** Improved Method for Determining Food Protein Degree of Hydrolysis. *Journal of Food Science,* 2001, vol. 66, 642-646
- **S. YU ; A. COWIESON ; C. GILBERT ; P. PLUMSTEAD ; S. DALSGAARD.** *J. Anim. Sci.,* 2012, vol. 90, 1824-1832
- **BEDFORD, M.R. ; CLASSEN, H.L.** An in vitro assay for prediction of broiler intestinal viscosity and growth when fed rye-based diets in the presence of exogenous enzymes. *Poultry Science,* 1993, vol. 72, 137-143